# EUROPEAN PATENT APPLICATION

(11) **EP 4 631 524 A2**
(43) Date of publication of application: **15.10.2025**
(21) Application number: 25196012.6
(22) Date of filing: 09.07.2020
(51) Int. Cl.: A61K 47/54

(54) **COMBINATION COMPRISING AN ADC OR AN AOC COMPRISING A VHH, AND A SAPONIN OR A LIGAND-SAPONIN CONJUGATE**

(30) Priority: 24.06.2020 NL 2025901
(62) Divisional of application: 20736364.9
(71) Applicant: Sapreme Technologies B.V., 3584 CM Utrecht (NL)
(72) Inventor: POSTEL, Ruben, 3721 MA Bilthoven (NL); Hermans, Guy, 3721 MA Bilthoven (NL); Fuchs, Hendrik, 13353 Berlin (DE)
(74) Representative: Nederlandsch Octrooibureau

(57) **Abstract**

The invention relates to a pharmaceutical combination comprising: a first conjugate comprising at least one effector molecule and a single-domain antibody (sdAb) for binding to a first cell-surface molecule; and comprising a saponin, a derivative thereof, or a second conjugate comprising a binding molecule for binding to a second cell-surface molecule and the saponin and/or the derivative thereof, wherein the saponin or the derivative thereof is a monodesmosidic or bidesmosidic triterpene glycoside. The invention also relates to a composition comprising the first conjugate and the saponin (derivative) or the second conjugate comprising the saponin (derivative). In addition, the invention relates to a pharmaceutical combination or composition of the invention, for use as a medicament, and for use in the treatment or the prophylaxis of a cancer, an auto-immune disease such as rheumatoid arthritis, an enzyme deficiency, a gene defect, a disease relating to a gene defect, an amyloidosis, a disease related to an enzyme deficiency, an infection such as a viral infection, hypercholesterolemia, primary hyperoxaluria, haemophilia A, haemophilia B, alpha-1 antitrypsin related liver disease, acute hepatic porphyria, transthyretin-mediated amyloidosis. Furthermore, the invention relates to an *in vitro* or *ex vivo* method for transferring the first conjugate of the invention from outside a cell to inside said cell, preferably to the cytosol of said cell.

## Description

### TECHNOLOGICAL FIELD

The invention relates to a pharmaceutical combination comprising: a first conjugate comprising at least one effector molecule and a single-domain antibody (sdAb) for binding to a first cell-surface molecule; and comprising a saponin, a derivative thereof, or a second conjugate comprising a binding molecule for binding to a second cell-surface molecule and the saponin and/or the derivative thereof, wherein the saponin or the derivative thereof is a monodesmosidic or bidesmosidic triterpene glycoside. The invention also relates to a composition comprising the first conjugate and the saponin (derivative) or relates to a composition comprising the first conjugate and the second conjugate comprising the saponin (derivative). In addition, the invention relates to a pharmaceutical combination or composition of the invention, for use as a medicament, and for use in the treatment or the prophylaxis of a cancer, an auto-immune disease such as rheumatoid arthritis, an enzyme deficiency, a gene defect, a disease relating to a gene defect, an amyloidosis, a disease related to an enzyme deficiency, an infection such as a viral infection, hypercholesterolemia, primary hyperoxaluria, haemophilia A, haemophilia B, alpha-1 antitrypsin related liver disease, acute hepatic porphyria, transthyretin-mediated amyloidosis. Furthermore, the invention relates to an *in vitro* or *ex vivo* method for transferring the first conjugate of the invention from outside a cell to inside said cell, preferably to the cytosol of said cell. The invention also relates to an *in vitro* or ex *vivo* method for transferring the first conjugate of the invention from outside a cell to inside said cell, and for transferring the effector molecule comprised by the first conjugate into the cytosol of said cell once the first conjugate is transferred from outside the cell to inside said cell such as in the endosome, endolysosome or lysosome of said cell.

### BACKGROUND

Molecules with a therapeutic biological activity are in many occasions in theory suitable for application as an effective therapeutic drug for the treatment of a disease such as a cancer in human patients in need thereof. A typical example are small-molecule biologically active moieties. However, many if not all potential drug-like molecules and therapeutics currently used in the clinic suffer from at least one of a plethora of shortcomings and drawbacks. When administered to a human body, therapeutically active molecules may exert off-target effects, in addition to the biologically activity directed to an aspect underlying a to-be-treated disease or health problem. Such off-target effects are undesired and bear a risk for induction of health- or even life-threatening side effects of the administered molecule. It is the occurrence of such adverse events that cause many drug-like compounds and therapeutic moieties to fail phase III clinical trials or even phase IV clinical trials (post-market entry follow-up). Therefore, there is a strong desire to provide drug molecules such as small-molecule therapeutics, wherein the therapeutic effect of the drug molecule should, e.g., (1) be highly specific for a biological factor or biological process driving the disease, (2) be sufficiently safe, (3) be sufficiently efficacious, (4) be sufficiently directed to the diseased cell with little to no off-target activity on non-diseased cells, (5) have a sufficiently timely mode of action (e.g. the administered drug molecule should reach the targeted site in the human patient within a certain time frame and should remain at the targeted site for a certain time frame ), and/or (6) have sufficiently long lasting therapeutic activity in the patient's body, amongst others. Unfortunately, to date, 'ideal' therapeutics with many or even all of the beneficial characteristics here above outlined, are not available to the patients, despite already long-lasting and intensive research and despite the impressive progress made in several areas of the individually addressed encountered difficulties and drawbacks.

Chemotherapy is one of the most important therapeutic options for cancer treatment. However, it is often associated with a low therapeutic window because it has no specificity towards cancer cells over dividing cells in healthy tissue. The invention of monoclonal antibodies offered the possibility of exploiting their specific binding properties as a mechanism for the targeted delivery of cytotoxic agents to cancer cells, while sparing normal cells. This can be achieved by chemical conjugation of cytotoxic effectors (also known as payloads or warheads) to antibodies, to create antibody-drug conjugates (ADCs). Typically, very potent payloads such as emtansine (DM1) are used which have a limited therapeutic index (a ratio that compares toxic dose to efficacious dose) in their unconjugated forms. The conjugation of DM1 to trastuzumab (ado-trastuzumab emtansine), also known as Kadcycla, enhances the tolerable dose of DM1 at least two-fold in monkeys. In the past few decades tremendous efforts and investments have been made to develop therapeutic ADCs. However, it remains challenging to bring ADCs into the clinic, despite promising preclinical data. The first ADC approved for clinical use was gemtuzumab ozogamicin (Mylotarg, CD33 targeted, Pfizer/Wyeth) for relapsed acute myelogenous leukemia (AML) in 2000. Mylotarg was however, withdrawn from the market at the request of the Federal Drug Administration (FDA) due to a number of concerns including its safety profile. Patients treated with Mylotarg were more often found to die than patients treated with conventional chemotherapy. Mylotarg was admitted to the market again in 2017 with a lower recommended dose, a different schedule in combination with chemotherapy or on its own, and a new patient population. To date, only five ADCs have been approved for clinical use, and meanwhile clinical development of approximately fifty-five ADCs has been halted. However, interest remains high and approximately eighty ADCs are still in clinical development in nearly six-hundred clinical trials at present.

Despite the potential to use toxic payloads that are normally not tolerated by patients, a low therapeutic index (a ratio that compares toxic dose to efficacious dose) is a major problem accounting for the discontinuance of many ADCs in clinical development, which can be caused by several mechanisms such as off-target toxicity on normal cells, development of resistance against the cytotoxic agents and premature release of drugs in the circulation. A systematic review by the FDA of ADCs found that the toxicity profiles of most ADCs could be categorized according to the payload used, but not the antibody used, suggesting that toxicity is mostly determined by premature release of the payload. Of the approximately fifty-five ADCs that were discontinued, it is estimated that at least twenty-three were due to a poor therapeutic index. For example, development of a trastuzumab tesirine conjugate (ADCT-502, HER-2 targeted, ADC therapeutics) was recently discontinued due to a narrow therapeutic index, possibly due to an on-target, off-tissue effect in pulmonary tissue which expresses considerable levels of HER-2. In addition, several ADCs in phase 3 trials have been discontinued due to missing primary endpoint. For example, phase 3 trials of a depatuxizumab mafodotin conjugate (ABT-414, EGFR targeted, AbbVie) tested in patients with newly diagnosed glioblastoma, and a mirvetuximab soravtansine conjugate (IMGN853, folate receptor alpha (FRα) targeted, ImmunoGen) tested in patients with platinum-resistant ovarian cancer, were recently stopped, showing no survival benefit. It is important to note that the clinically used dose of some ADCs may not be sufficient for its full anticancer activity. For example, ado-trastuzumab emtansine has an MTD of 3.6 mg/kg in humans. In preclinical models of breast cancer, ado-trastuzumab emtansine induced tumor regression at dose levels at or above 3 mg/kg, but more potent efficacy was observed at 15 mg/kg. This suggests that at the clinically administered dose, ado-trastuzumab emtansine may not exert its maximal potential anti-tumor effect.

ADCs are mainly composed of an antibody, a cytotoxic moiety such as a payload, and a linker. Several novel strategies have been proposed and carried out in the design and development of new ADCs to overcome the existing problems, targeting each of the components of ADCs. For example, by identification and validation of adequate antigenic targets for the antibody component, by selecting antigens which have high expression levels in tumor and little or no expression in normal tissues, antigens which are present on the cell surface to be accessible to the circulating ADCs, and antigens which allows internalizing of ADCs into the cell after binding; and alternative mechanisms of activity; design and optimize linkers which enhance the solubility and the drug-to-antibody ratio (DAR) of ADCs and overcome resistance induced by proteins that can transport the chemotherapeutic agent out of the cells; enhance the DAR ratio by inclusion of more payloads, select and optimize antibodies to improve antibody homogeneity and developability. In addition to the technological development of ADCs, new clinical and translational strategies are also being deployed to maximize the therapeutic index, such as, change dosing schedules through fractionated dosing; perform biodistribution studies; include biomarkers to optimize patient selection, to capture response signals early and monitor the duration and depth of response, and to inform combination studies.

An example of ADCs with clinical potential are those ADCs such as brentuximab vedotin, inotuzumab ozogamicin, moxetumomab pasudotox, and polatuzumab vedotin, which are evaluated as a treatment option for lymphoid malignancies and multiple myeloma. Polatuzumab vedotin, binding to CD79b on (malignant) B-cells, and pinatuzumab vedotin, binding to CD22, are tested in clinical trials wherein the ADCs each were combined with co-administered rituximab, a monoclonal antibody binding to CD20 and not provided with a payload *[*B. Yu and D. Liu, Antibody-drug conjugates in clinical trials for lymphoid malignancies and multiple myeloma; Journal of Hematology & Oncology (2019) 12:94*].* Combinations of monoclonal antibodies such as these examples are yet a further approach and attempt to arrive at the 'magic bullet' which combines many or even all of the aforementioned desired characteristics of ADCs.

Meanwhile in the past few decades, nucleic acid-based therapeutics are under development. Therapeutic nucleic acids can be based on deoxyribonucleic acid (DNA) or ribonucleic acid (RNA), Anti-sense oligonucleotides (ASOs, AONs), and short interfering RNAs (siRNAs), MicroRNAs, and DNA and RNA aptamers, for approaches such as gene therapy, RNA interference (RNAi). Many of them share the same fundamental basis of action by inhibition of either DNA or RNA expression, thereby preventing expression of disease-related abnormal proteins. The largest number of clinical trials is being carried out in the field of gene therapy, with almost 2600 ongoing or completed clinical trials worldwide but with only about 4% entering phase 3. This is followed by clinical trials with ASOs. Similarly to ADCs, despite the large number of techniques being explored, therapeutic nucleic acids share two major issues during clinical development: delivery into cells and off-target effects. For instance, ASOs such as peptide nucleic acid (PNA), phosphoramidate morpholino oligomer (PMO), locked nucleic acid (LNA) and bridged nucleic acid (BNA), are being investigated as an attractive strategy to inhibit specifically target genes and especially those genes that are difficult to target with small molecules inhibitors or neutralizing antibodies. Currently, the efficacy of different ASOs is being studied in many neurodegenerative diseases such as Huntington's disease, Parkinson's disease, Alzheimer's disease, and amyotrophic lateral sclerosis and also in several cancer stages. The application of ASOs as potential therapeutic agents requires safe and effective methods for their delivery to the cytoplasm and/or nucleus of the target cells and tissues. Although the clinical relevance of ASOs has been demonstrated, inefficient cellular uptake, both in vitro and in vivo, limit the efficacy of ASOs and has been a barrier to therapeutic development. Cellular uptake can be < 2% of the dose resulting in too low ASO concentration at the active site for an effective and sustained outcome. This consequently requires an increase of the administered dose which induces off-target effects. Most common side-effects are activation of the complement cascade, the inhibition of the clotting cascade and toll-like receptor mediated stimulation of the immune system.

Chemotherapeutics are most commonly small molecules, however, their efficacy is hampered by the severe off-target side toxicity, as well as their poor solubility, rapid clearance and limited tumor exposure. Scaffold-small-molecule drug conjugates such as polymer-drug conjugates (PDCs) are macromolecular constructs with pharmacologically activity, which comprises one or more molecules of a small-molecule drug bound to a carrier scaffold (e.g. polyethylene glycol (PEG)).

Such conjugate principle has attracted much attention and has been under investigation for several decades. The majority of conjugates of small-molecule drugs under pre-clinical or clinical development are for oncological indications. However, up-to-date only one drug not related to cancer has been approved (Movantik, a PEG oligomer conjugate of opioid antagonist naloxone, AstraZeneca) for opioid-induced constipation in patients with chronic pain in 2014, which is a non-oncology indication. Translating application of drug-scaffold conjugates into treatment of human subjects provides little clinical success so far. For example, PK1 (N-(2-hydroxypropyl)methacrylamide (HPMA) copolymer doxorubicin; development by Pharmacia, Pfizer) showed great anti-cancer activity in both solid tumors and leukemia in murine models, and was under clinical investigation for oncological indications. Despite that it demonstrated significant reduction of nonspecific toxicity and improved pharmacokinetics in man, improvements in anticancer efficacy turned out to be marginal in patients, and as a consequence further development of PK1 was discontinued.

The failure of scaffold-small-molecule drug conjugates is at least partially attributed to its poor accumulation at the tumor site. For example, while in murine models PK1 showed 45-250 times higher accumulation in the tumor than in healthy tissues (liver, kidney, lung, spleen, and heart), accumulation in tumor was only observed in a small subset of patients in the clinical trial.

A potential solution to the aforementioned problems is application of nanoparticle systems for drug delivery such as liposomes. Liposomes are sphere-shaped vesicles consisting of one or more phospholipid bilayers, which are spontaneously formed when phospholipids are dispersed in water. The amphiphilicity characteristics of the phospholipids provide it with the properties of self-assembly, emulsifying and wetting characteristics, and these properties can be employed in the design of new drugs and new drug delivery systems. Drug encapsulated in a liposomal delivery system may convey several advantages over a direct administration of the drug, such as an improvement and control over pharmacokinetics and pharmacodynamics, tissue targeting property, decreased toxicity and enhanced drug activity. An example of such success is liposome-encapsulated form of a small molecule chemotherapy agent doxorubicin (Doxil: a pegylated liposome-encapsulated form of doxorubicin; Myocet: a non-pegylated liposomal doxorubicin), which have been approved for clinical use.

Therefore, a solution still needs to be found that allows for drug therapies such as anti-tumor therapies, applicable for non-systemic use when desired, wherein the drug has for example an acceptable safety profile, little off-target activity, sufficient efficacy, sufficiently low clearance rate from the patient's body, a sufficiently wide therapeutic window, etc.

In European patent EP1623715B1, a composition comprising a pharmacologically active agent coupled to a target-cell specific binding molecule, combined with a saponin, has been described, wherein the target-cell specific binding molecule is a full-length monoclonal immunoglobulin G type antibody. The pharmacologically active agent is for example a toxin.

### SUMMARY OF THE INVENTION

A first aspect of the invention relates to a pharmaceutical combination comprising:
- a first conjugate comprising at least one effector molecule and a single-domain antibody (sdAb) for binding to a first cell-surface molecule, which are covalently linked to each other, directly or via a linker;
- a saponin and/or a derivative thereof and/or a second conjugate comprising a binding molecule for binding to a second cell-surface molecule and the saponin and/or the derivative thereof, which binding molecule and the saponin or the derivative thereof are covalently linked to each other, directly or via a linker, wherein the saponin or the derivative thereof is a monodesmosidic triterpene glycoside or a bidesmosidic triterpene glycoside, wherein the second cell-surface molecule is the same as the first cell-surface molecule or different from the first cell-surface molecule; and optionally
- a pharmaceutically acceptable excipient and/or pharmaceutically acceptable diluent.

An embodiment is the pharmaceutical combination of the invention in the form of at least two, preferably two, pharmaceutical compositions comprising:
- a first pharmaceutical composition comprising the first conjugate and optionally comprising a pharmaceutically acceptable excipient and/or pharmaceutically acceptable diluent; and
- a second pharmaceutical composition comprising the saponin and/or the derivative thereof and/or the second conjugate, and optionally comprising a pharmaceutically acceptable excipient and/or pharmaceutically acceptable diluent.

An embodiment is the pharmaceutical combination of the invention in the form of a single pharmaceutical composition comprising the first conjugate and at least one, preferably one, of:
- the saponin;
- the saponin derivative; and
- the second conjugate,
and optionally comprising a pharmaceutically acceptable excipient and/or pharmaceutically acceptable diluent.

A second aspect of the invention relates to the pharmaceutical combination of the invention, for use as a medicament.

A third aspect of the invention relates to the pharmaceutical combination of the invention, for use in the treatment or the prophylaxis of a cancer, an auto-immune disease such as rheumatoid arthritis, an enzyme deficiency, a gene defect, a disease relating to a gene defect, an amyloidosis, a disease related to an enzyme deficiency, an infection such as a viral infection, hypercholesterolemia, primary hyperoxaluria, haemophilia A, haemophilia B, alpha-1 antitrypsin related liver disease, acute hepatic porphyria, transthyretin-mediated amyloidosis.

A fourth aspect of the invention relates to an *in vitro* or ex *vivo* method for transferring the first conjugate of the invention from outside a cell to inside said cell, preferably to the cytosol of said cell, comprising the steps of:
a) providing a cell which expresses the first cell-surface molecule according to the invention on its surface, and optionally expresses the second cell-surface molecule according to the invention on its surface, said cell preferably selected from a liver cell, an aberrant cell such as a virally infected cell, an auto-immune cell and a tumor cell;
b) providing the first conjugate of the invention for transferring into the cell provided in step a);
c) providing the saponin or the saponin derivative of the invention, or, when the cell provided in step a) expresses the second cell-surface molecule according to the invention on its surface, providing the second conjugate of the invention;
d) contacting the cell of step a) *in vitro* or ex *vivo* with the first conjugate of step b) and any one or more of the saponin, the saponin derivative and the conjugate of step c),
therewith establishing the transfer of the first conjugate from outside the cell into said cell, preferably into the cytosol of said cell.

### DEFINITIONS

The term "proteinaceous" has its regular scientific meaning and here refers to a molecule that is protein-like, meaning that the molecule possesses, to some degree, the physicochemical properties characteristic of a protein, is of protein, relating to protein, containing protein, pertaining to protein, consisting of protein, resembling protein, or being a protein. The term "proteinaceous" as used in for example 'proteinaceous molecule' refers to the presence of at least a part of the molecule that resembles or is a protein, wherein 'protein' is to be understood to include a chain of amino-acid residues at least two residues long, thus including a peptide, a polypeptide and a protein and an assembly of proteins or protein domains. In the proteinaceous molecule, the at least two amino-acid residues are for example linked via (an) amide bond(s), such as (a) peptide bond(s). In the proteinaceous molecule, the amino-acid residues are natural amino-acid residues and/or artificial amino-acid residues such as modified natural amino-acid residues. In a preferred embodiment, a proteinaceous molecule is a molecule comprising at least two amino-acid residues, preferably between two and about 2.000 amino-acid residues. In one embodiment, a proteinaceous molecule is a molecule comprising from 2 to 20 (typical for a peptide) amino acids. In one embodiment, a proteinaceous molecule is a molecule comprising from 21 to 1.000 amino acids (typical for a polypeptide, a protein, a protein domain, such as an antibody, a Fab, an scFv, a single domain antibody, a ligand for a receptor such as EGF). Preferably, the amino-acid residues are (typically) linked via (a) peptide bond(s). According to the invention, said amino-acid residues are or comprise (modified) (non-)natural amino acid residues.

The term "effector molecule", or "effector moiety" when referring to the effector molecule as part of e.g. a covalent conjugate, has its regular scientific meaning and here refers to a molecule that can selectively bind to for example any one or more of the target molecules: a protein, a peptide, a carbohydrate, a saccharide such as a glycan, a (phospho)lipid, a nucleic acid such as DNA, RNA, an enzyme, and regulates the biological activity of such one or more target molecule(s). The effector molecule is for example a molecule selected from any one or more of a small molecule such as a drug molecule, a toxin such as a protein toxin, an oligonucleotide such as a BNA, a xeno nucleic acid or an siRNA, an enzyme, a peptide, a protein, or any combination thereof. Thus, for example, an effector molecule or an effector moiety is a molecule or moiety selected from any one or more of a small molecule such as a drug molecule, a toxin such as a protein toxin, an oligonucleotide such as a BNA, a xeno nucleic acid or an siRNA, an enzyme, a peptide, a protein, or any combination thereof, that can selectively bind to any one or more of the target molecules: a protein, a peptide, a carbohydrate, a saccharide such as a glycan, a (phospho)lipid, a nucleic acid such as DNA, RNA, an enzyme, and that upon binding to the target molecule regulates the biological activity of such one or more target molecule(s). Typically, an effector molecule can exert a biological effect inside a cell such as a mammalian cell such as a human cell, such as in the cytosol of said cell. Typical effector molecules are thus drug molecules, plasmid DNA, toxins such as toxins comprised by antibody-drug conjugates (ADCs), oligonucleotides such as siRNA, BNA, nucleic acids comprised by an antibody-oligonucleotide conjugate (AOC). For example, an effector molecule is a molecule which can act as a ligand that can increase or decrease (intracellular) enzyme activity, gene expression, or cell signalling. In the context of the invention, an effector molecule or effector moiety when the effector molecule is part of a conjugate, is not a saponin, and is not a cell-surface molecule binding molecule such as an antibody such as an sdAb.

The term "modified saponin" has its regular scientific meaning and here refers to a saponin, i.e. a saponin derivative, which has one or more chemical modifications at positions where previously any of an aldehyde group, a carboxyl group, an acetate group and/or an acetyl group was present in the non-derivatised saponin before being subjected to chemical modification for provision of the modified saponin. For example, the modified saponin is provided by chemical modification of any one or more of an aldehyde group, a carboxyl group, an acetate group and/or an acetyl group in a saponin upon which the modified saponin is based, i.e. the saponin is provided and any of an aldehyde group, a carboxyl group, an acetate group and/or an acetyl group is chemically modified therewith providing the modified saponin. For example, the saponin that is modified for provision of the modified saponin is a naturally occurring saponin. Typically, the modified saponin is a synthetic saponin, typically the modified saponin is a modification of a natural saponin, and is thus derived from a natural saponin, although a modified saponin can also be derived from a synthetic saponin which may or may not have a natural counterpart. Typically, the modified saponin has not a natural counterpart, i.e. the modified saponin is not produced naturally by e.g. plants or trees.

The term "aglycone core structure" has its regular scientific meaning and here refers to the aglycone core, or aglycone in short, of a saponin without the one or two carbohydrate antenna or saccharide chains (glycans) bound thereto. For example, quillaic acid is the aglycone core structure for SO1861, QS-7 and QS21. Typically, the glycans of a saponin are mono-saccharides or oligosaccharides, such as linear or branched glycans.

The term "saccharide chain" has its regular scientific meaning and here refers to any of a glycan, a carbohydrate antenna, a single saccharide moiety (mono saccharide) or a chain comprising multiple saccharide moieties (oligosaccharide, polysaccharide). The saccharide chain can consist of only saccharide moieties or may also comprise further moieties such as any one of 4E-Methoxycinnamic acid, 4Z-Methoxycinnamic acid, and 5-O-[5-O-Ara/Api-3,5-dihydroxy-6-methyl-octanoyl]-3,5-dihydroxy-6-methyl-octanoic acid), such as for example present in QS-21.

The term "chemically modified" has its regular scientific meaning and here refers to the chemical modification of a first chemical group or first chemical moiety such that a second chemical group or second chemical moiety is provided. Examples are the chemical modification of a carbonyl group into a - (H)C-OH group, the chemical modification of an acetate group into a hydroxyl group, the provision of a saponin conjugated at its aldehyde group with an N-ε-maleimidocaproic acid hydrazide (EMCH) moiety via a chemical reaction, etc.

The term "chemically modified aldehyde group" has its regular scientific meaning and here refers to the chemical reaction product obtained by the chemical reaction involving the aldehyde group of a saponin resulting in replacement of the initial aldehyde group by a new chemical group. For example, the formation of a - (H)C-OH group from the initial aldehyde group of a saponin.

The term "chemically modified carboxyl group" has its regular scientific meaning and here refers to the chemical reaction product obtained by the chemical reaction involving the carboxyl group of a saponin, such as the carboxyl group of a glucuronic acid moiety, and a further molecule, resulting in replacement of the initial carboxyl group by a new chemical group. For example, the formation of the conjugate between a saponin and any one of 2-amino-2-methyl-1,3-propanediol (AMPD), N-(2-aminoethyl)maleimide (AEM) or 1-[Bis(dimethylamino)methylene]-1H-1,2,3-triazolo[4,5-b]pyridinium 3-oxid hexafluorophosphate (HATU), involving the carboxyl group of the glucuronic acid of the saponin.

The term "Api/Xyl-" or "Api- or Xyl-" in the context of the name of a saccharide chain has its regular scientific meaning and here refers to the saccharide chain either comprising an apiose (Api) moiety, or comprising a xylose (Xyl) moiety.

The term "saponin on which the modified saponin is based" has its regular scientific meaning and here refers to a saponin that has been modified in order to provide the modified saponin. Typically, the saponin on which the modified saponin is based is a naturally occurring saponin, which is subjected to chemical modification for the provision of the modified saponin.

The term "modified saponin based on a saponin" has its regular scientific meaning and here refers to a saponin that has been subjected to a chemical modification step such that the modified saponin is provided, wherein the saponin from which the modified saponin has been made is typically a naturally occurring saponin.

The term "oligonucleotide" has its regular scientific meaning and here refers to amongst others any natural or synthetic string of nucleic acids encompassing DNA, modified DNA, RNA, mRNA, modified RNA, synthetic nucleic acids, presented as a single-stranded molecule or a double-stranded molecule, such as a BNA, an antisense oligonucleotide (ASO), a short or small interfering RNA (siRNA; silencing RNA), an anti-sense DNA, anti-sense RNA, etc.

The term "antibody-drug conjugate" or "ADC" has its regular scientific meaning and here refers to any conjugate of an antibody such as an IgG, a Fab, an scFv, an immunoglobulin, an immunoglobulin fragment, one or multiple V_{H} domains, single-domain antibodies, a V_{HH}, a camelid V_{H}, etc., and any molecule that can exert a therapeutic effect when contacted with cells of a subject such as a human patient, such as an active pharmaceutical ingredient, a toxin, an oligonucleotide, an enzyme, a small molecule drug compound, etc.

The term "antibody-oligonucleotide conjugate" or "AOC" has its regular scientific meaning and here refers to any conjugate of an antibody such as an IgG, a Fab, an scFv, an immunoglobulin, an immunoglobulin fragment, one or multiple V_{H} domains, single-domain antibodies, a V_{HH}, a camelid V_{H}, etc., and any oligonucleotide molecule that can exert a therapeutic effect when contacted with cells of a subject such as a human patient, such as an oligonucleotide selected from a natural or synthetic string of nucleic acids encompassing DNA, modified DNA, RNA, mRNA, modified RNA, synthetic nucleic acids, presented as a single-stranded molecule or a double-stranded molecule, such as a BNA, an antisense oligonucleotide (ASO), a short or small interfering RNA (siRNA; silencing RNA), an anti-sense DNA, anti-sense RNA, etc.

The term "bridged nucleic acid", or "BNA" in short, or "locked nucleic acid" or "LNA" in short, has its regular scientific meaning and here refers to a modified RNA nucleotide. A BNA is also referred to as 'constrained RNA molecule' or 'inaccessible RNA molecule'. A BNA monomer can contain a five-membered, six-membered or even a seven-membered bridged structure with a "fixed" C₃'-endo sugar puckering. The bridge is synthetically incorporated at the 2', 4'-position of the ribose to afford a 2', 4'-BNA monomer. A BNA monomer can be incorporated into an oligonucleotide polymeric structure using standard phosphoramidite chemistry known in the art. A BNA is a structurally rigid oligonucleotide with increased binding affinity and stability.

The term 'S' as used such as in an antibody-saponin conjugate comprising a linker, represents 'stable linker' which remains intact in the endosome, endolysosome and in the lysosome of mammalian cells, such as human cells, such as a human tumor cell, thus under slightly acid conditions (pH < 6.6, such as pH 4.0 - 5.5).

The term 'L' as used such as in an antibody-saponin conjugate comprising a linker, represents 'labile linker' which is cleaved under slightly acid conditions (pH < 6.6, such as pH 4.0 - 5.5) in the endosome, endolysosome and in the lysosome of mammalian cells, such as human cells, such as a human tumor cell.

The terms first, second, third and the like in the description and in the claims, are used for distinguishing between for example similar elements, compositions, constituents in a composition, or separate method steps, and not necessarily for describing a sequential or chronological order. The terms are interchangeable under appropriate circumstances and the embodiments of the invention can operate in other sequences than described or illustrated herein, unless specified otherwise.

The embodiments of the invention described herein can operate in combination and cooperation, unless specified otherwise.

Furthermore, the various embodiments, although referred to as "preferred" or "e.g." or "for example" or "in particular" and the like are to be construed as exemplary manners in which the invention may be implemented rather than as limiting the scope of the invention.

The term "comprising", used in the claims, should not be interpreted as being restricted to for example the elements or the method steps or the constituents of a compositions listed thereafter; it does not exclude other elements or method steps or constituents in a certain composition. It needs to be interpreted as specifying the presence of the stated features, integers, (method) steps or components as referred to, but does not preclude the presence or addition of one or more other features, integers, steps or components, or groups thereof. Thus, the scope of the expression "a method comprising steps A and B" should not be limited to a method consisting only of steps A and B, rather with respect to the present invention, the only enumerated steps of the method are A and B, and further the claim should be interpreted as including equivalents of those method steps. Thus, the scope of the expression "a composition comprising components A and B" should not be limited to a composition consisting only of components A and B, rather with respect to the present invention, the only enumerated components of the composition are A and B, and further the claim should be interpreted as including equivalents of those components.

In addition, reference to an element or a component by the indefinite article "a" or "an" does not exclude the possibility that more than one of the element or component are present, unless the context clearly requires that there is one and only one of the elements or components. The indefinite article "a" or "an" thus usually means "at least one".

The term "Saponinum album" has its normal meaning and here refers to a mixture of saponins produced by Merck KGaA (Darmstadt, Germany) containing saponins from *Gypsophila paniculata* and *Gypsophila arostii,* containing SA1657 and mainly SA1641.

The term "Quillaja saponin" has its normal meaning and here refers to the saponin fraction of Quillaja saponaria and thus the source for all other QS saponins, mainly containing QS-18 and QS-21.

"QS-21" or "QS21" has its regular scientific meaning and here refers to a mixture of QS-21 A-apio (~63%), QS-21 A-xylo (~32%), QS-21 B-apio (~3.3%), and QS-21 B-xylo (~1.7%).

Similarly, "QS-21A" has its regular scientific meaning and here refers to a mixture of QS-21 A-apio (~65%) and QS-21 A-xylo (~35%).

Similarly, "QS-21B" has its regular scientific meaning and here refers to a mixture of QS-21 B-apio (~65%) and QS-21 B-xylo (~35%).

The term "Quil-A" refers to a commercially available semi-purified extract from Quillaja saponaria and contains variable quantities of more than 50 distinct saponins, many of which incorporate the triterpene-trisaccharide substructure Gal-(1→2)-[Xyl-(1→3)]-GlcA- at the C-3beta-OH group found in QS-7, QS-17, QS18, and QS-21. The saponins found in Quil-A are listed in van Setten (1995), Table *2 [*Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995)]. Quil-A and also Quillaja saponin are fractions of saponins from Quillaja saponaria and both contain a large variety of different saponins with largely overlapping content. The two fractions differ in their specific composition as the two fractions are gained by different purification procedures.

The term "QS1861" and the term "QS1862" refer to QS-7 and QS-7 api. QS1861 has a molecular mass of 1861 Dalton, QS1862 has a molecular mass of 1862 Dalton. QS1862 is described in Fleck et al. (2019) in Table 1, row no. 28 *[*Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171*].* The described structure is the api-variant QS1862 of QS-7. The molecular mass is 1862 Dalton as this mass is the formal mass including proton at the glucuronic acid. At neutral pH, the molecule is deprotonated. When measuring in mass spectrometry in negative ion mode, the measured mass is 1861 Dalton.

### BRIEF DESCRIPTION OF THE DRAWINGS

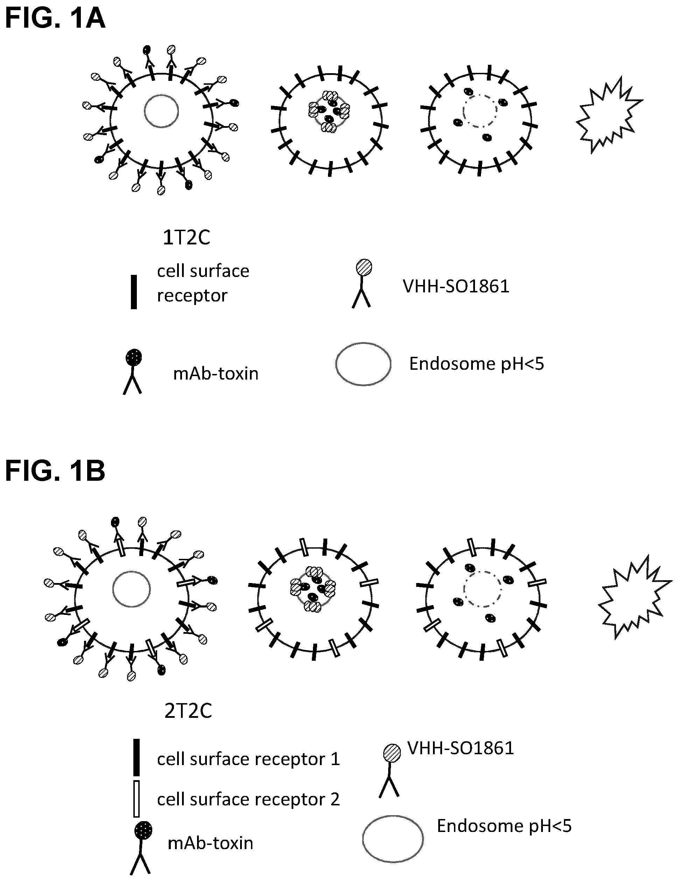

**Figure 1A (Fig. 1A****):** *The targeted 2-component approach (1 target).* SO1861 and toxin (ribosomal inactivating protein) are each, separately, conjugated to a V_{HH} or antibody (mAb) for delivery and internalization into target cells. 1) mAb-toxin and V_{HH}-SO1861 bind to the cell surface receptor, 2) receptor-mediated endocytosis of both conjugates occurs (binding of conjugates to the receptor is followed by internalization of the conjugate/receptor complex), 3) at low endolysosomal pH and appropriate concentration, SO1861 becomes active to enable endolysosomal escape, 4) release of toxin into cytoplasm occurs and induces cell death.
**Figure 1B (Fig. 1B****):** *The targeted 2-component approach (2 targets).* SO1861 and toxin (ribosomal inactivating protein) are each, separately, conjugated to a V_{HH} or antibody (mAb) for delivery and internalization into target cells. 1) mAb-toxin and V_{HH}-SO1861 bind to their corresponding cell surface receptor, 2) receptor-mediated endocytosis of both conjugates occurs (binding of conjugates to the receptor is followed by internalization of the conjugate/receptor complex), 3) at low endolysosomal pH and appropriate concentration, SO1861 becomes active to enable endolysosomal escape, 4) release of toxin into cytoplasm occurs and induces cell death.

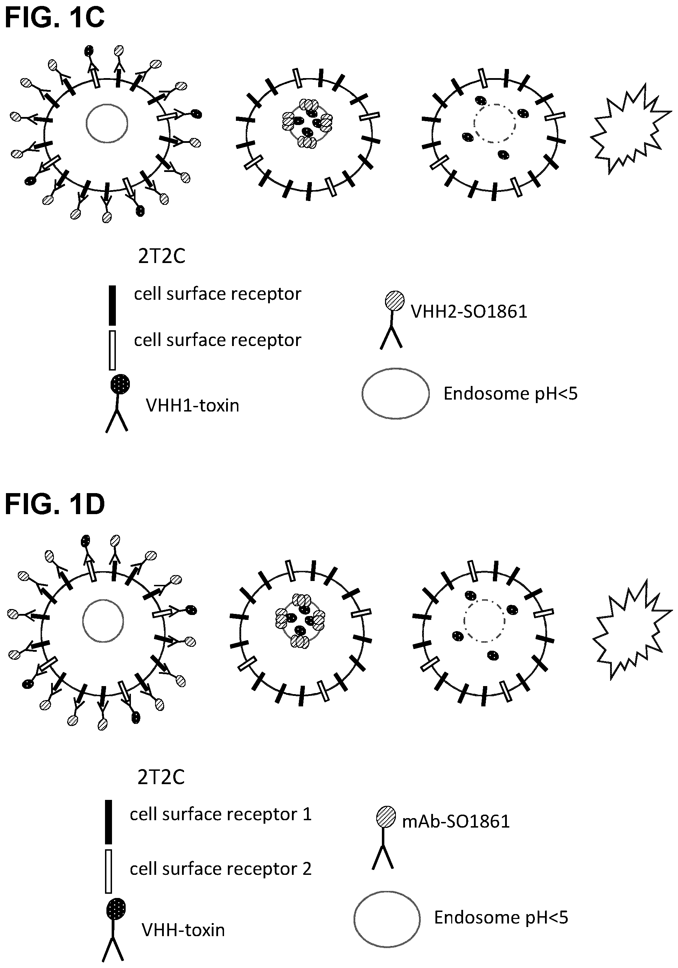

**Figure 1C (Fig. 1C****):** *The targeted 2-component approach (2 targets).* SO1861 and toxin (ribosomal inactivating protein) are each, separately, conjugated to a V_{HH} for delivery and internalization into target cells. 1) V_{HH}-toxin and V_{HH}-SO1861 bind to their corresponding cell surface receptor, 2) receptor-mediated endocytosis of both conjugates occurs (binding of conjugates to the receptor is followed by internalization of the conjugate/receptor complex), 3) at low endolysosomal pH and appropriate concentration, SO1861 becomes active to enable endolysosomal escape, 4) release of toxin into cytoplasm occurs and induces cell death.
**Figure 1D (Fig. 1D****):** *The targeted 2-component approach (2 targets).* SO1861 and toxin (ribosomal inactivating protein) are each, separately, conjugated to a V_{HH} or mAb for delivery and internalization into target cells. 1) V_{HH}-toxin and mAb-SO1861 bind to their corresponding cell surface receptor, 2) receptor-mediated endocytosis of both conjugates occurs (binding of conjugates to the receptor is followed by internalization of the conjugate/receptor complex), 3) at low endolysosomal pH and appropriate concentration, SO1861 becomes active to enable endolysosomal escape, 4) release of toxin into cytoplasm occurs and induces cell death.

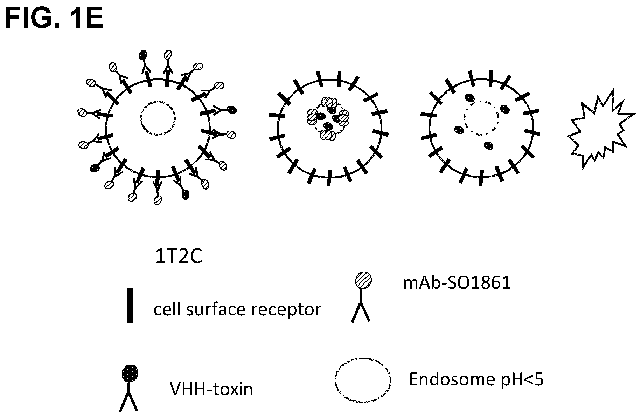

**Figure 1E (Fig. 1E****):** *The targeted 2-component approach (1 target).* SO1861 and toxin (ribosomal inactivating protein) are each, separately, conjugated to a V_{HH} or antibody (mAb) for delivery and internalization into target cells. 1) V_{HH}-toxin and mAb-SO1861 bind to the cell surface receptor, 2) receptor-mediated endocytosis of both conjugates occurs (binding of conjugates to the receptor is followed by internalization of the conjugate/receptor complex), 3) at low endolysosomal pH and appropriate concentration, SO1861 becomes active to enable endolysosomal escape, 4) release of toxin into cytoplasm occurs and induces cell death.

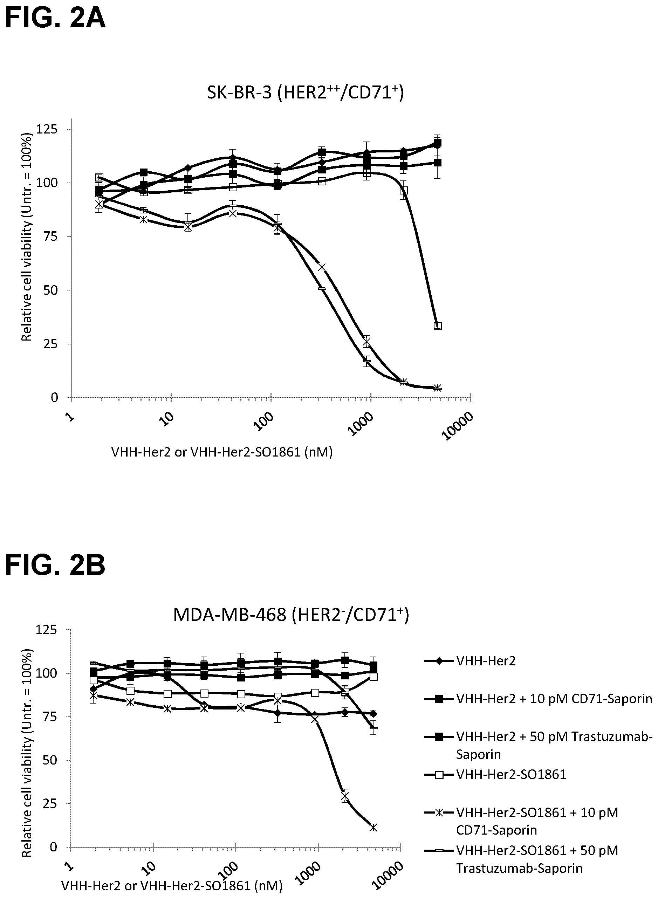

**Figure 2 (Fig. 2****):** Cell killing (MTS) assay) with the combination treatment according to the invention of V_{HH}(HER2)-SO1861 (DAR1) + 50pM trastuzumab-saporin (DAR4) or 10 pM CD71mab-saporin (DAR4) on SK-BR-3 cells (HER2⁺⁺/CD71⁺) (A) and MD-MB-468 cells (HER2⁻/CD71⁺) (B).

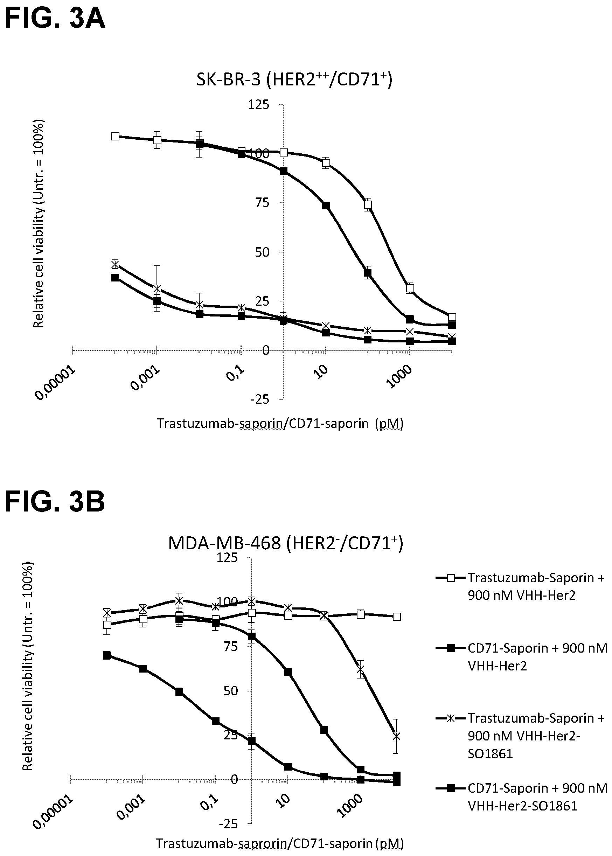

**Figure 3 (Fig. 3****):** Cell killing (MTS) assay) with the combination treatment according to the invention of Trastuzumab-saporin (DAR4) or CD71-saporin (DAR4) + 900nM HER2V_{HH}-SO1861 (DAR1) on SK-BR-3 cells (HER2⁺⁺/CD71⁺) (A) and MD-MB-468 cells (HER2⁻/CD71⁺) (B).

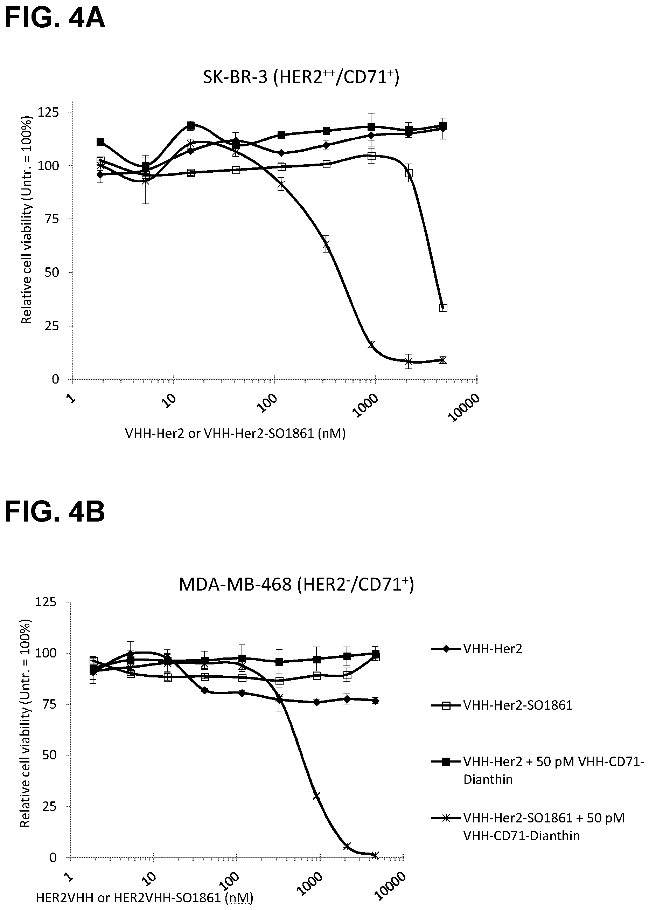

**Figure 4 (Fig. 4****):** Cell killing (MTS) assay) with the combination treatment according to the invention of V_{HH}(HER2)-SO1861 (DAR1) + 50pM CD71V_{HH}-dianthin (DAR1) on SK-BR-3 cells (HER2⁺⁺/CD71⁺) (A) and MD-MB-468 cells (HER2⁻/CD71⁺) (B).

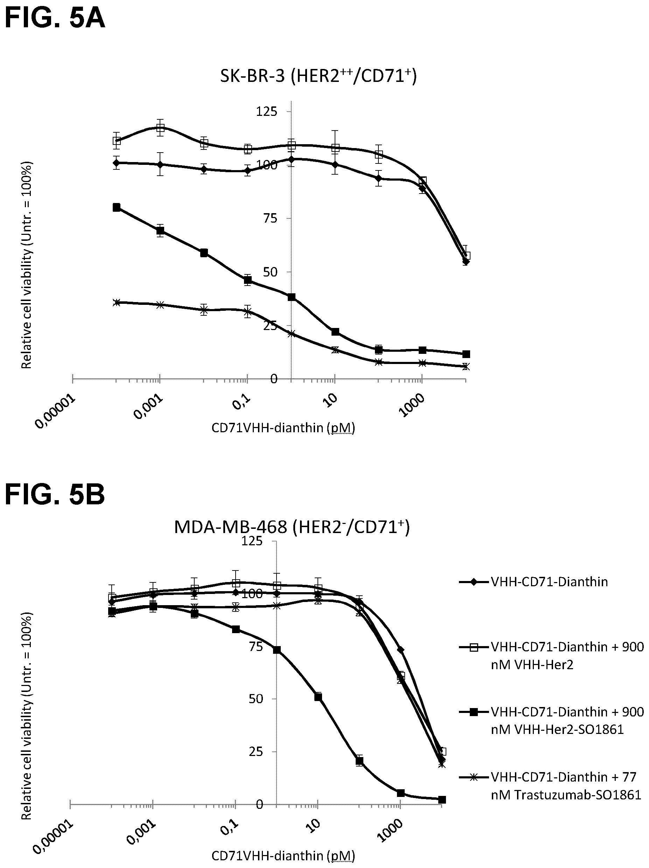

**Figure 5 (Fig. 5****):** Cell killing (MTS) assay) with the combination treatment according to the invention of CD71V_{HH}-dianthin (DAR1) + 900nM HER2V_{HH}-SO1861 (DAR1) on SK-BR-3 cells (HER2⁺⁺/CD71⁺) (A) and MD-MB-468 cells (HER2⁻/CD71⁺) (B).

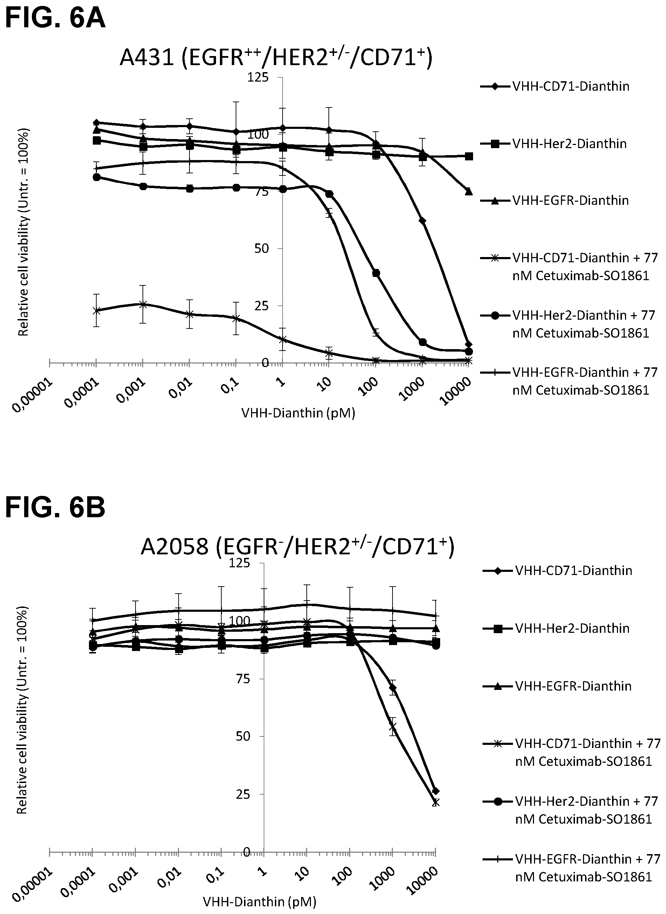

**Figure 6 (Fig. 6****):** Cell killing (MTS) assay) with the combination treatment according to the invention of CD71V_{HH}-dianthin (DAR1) + cetuximab-SO1861 (DAR4) or HER2V_{HH}-dianthin (DAR1) + cetuximab-SO1861 (DAR4) or EGFRV_{HH}-dianthin (DAR1) + cetuximab-SO1861 (DAR4) on A431 cells (EGFR⁺⁼/HER^{+/-}/CD71⁺) (A) and A2058 cells (EGFR⁻/HER^{+/-}/CD71⁺) (B).

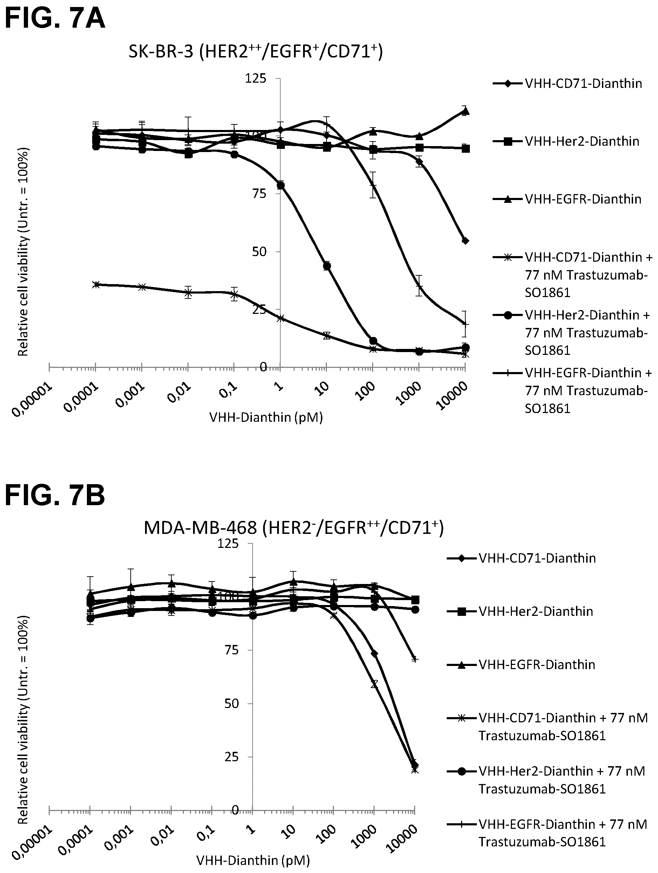

**Figure 7 (Fig. 7****):** Cell killing (MTS) assay) with the combination treatment according to the invention of CD71V_{HH}-dianthin (DAR1) + 77 nM cetuximab-SO1861 (DAR4) or HER2V_{HH}-dianthin (DAR1) + 77 nM cetuximab-SO1861 (DAR4) or EGFRV_{HH}-dianthin (DAR1) + 77 nM cetuximab-SO1861 (DAR4) on A431 cells (EGFR⁺⁼/HER^{+/-}/CD71⁺) (A) and MDA-MB-468 cells (HER2⁻/EGFR⁺⁺/CD71⁺) (B).

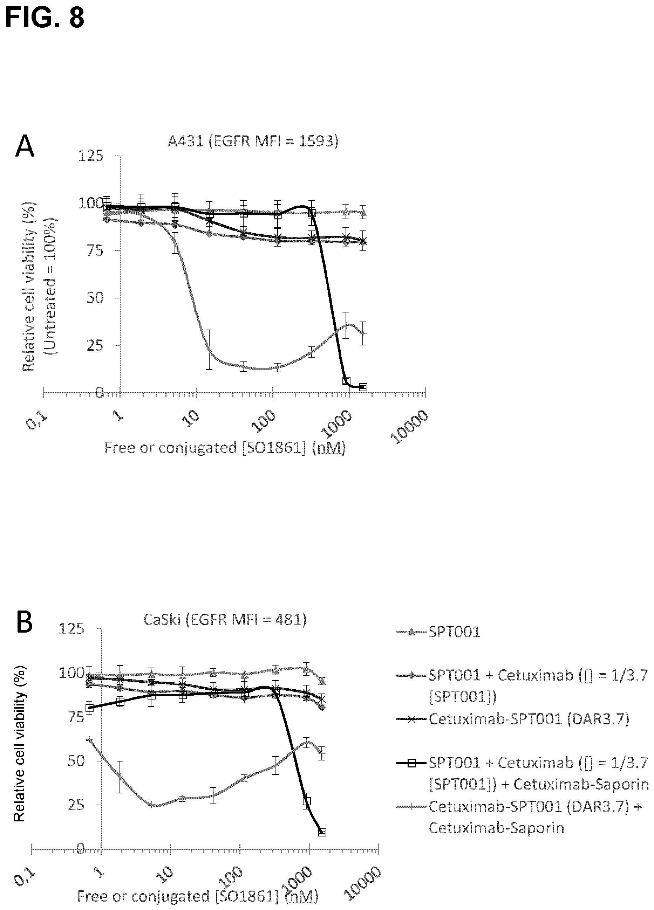

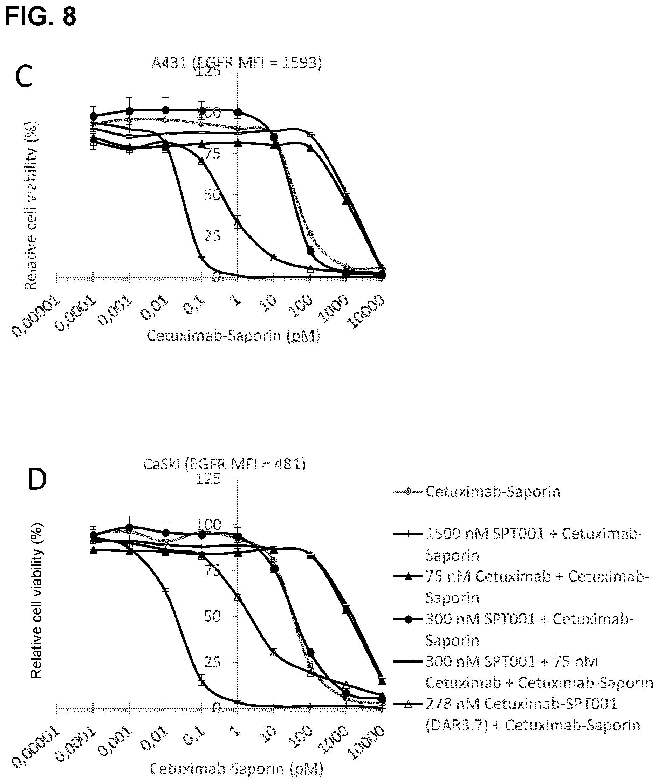

**Figure 8. (FIG. 8****):** *1-target 2-component (EGFR high expression).* EGFR targeted cell killing in A431 (EGFR⁺⁺⁺) and CaSKi (EGFR⁺⁺), by a therapeutic combination. A, B) cetuximab-SO1861 titration in combination with a fixed concentration of 10 pM cetuximab-saporin shows that a 100-400 fold reduced concentration of conjugated SO1861 is required, versus unconjugated SO1861, to induce cell killing by cetuximab-saporin. C, D), Cetuximab-saporin titration in combination with 278 nM cetuximab-SO1861 kills cells in contrast to 300 nM unconjugated SO1861 + cetuximab-saporin. 1500 nM unconjugated SO1861 + cetuximab-saporin is more efficient compared to the therapeutic combination, since both cetuximab conjugates compete for the same EGFR receptor. Only simultaneous targeted delivery of both cetuximab conjugates leads to efficient cell-killing, in contrast to monotherapy with either conjugate alone. REMARK: the legend displayed in Fig. 8B also relates to the graphs in Fig. 8A. REMARK: the legend displayed in Fig. 8D also relates to the graphs in Fig. 8C.

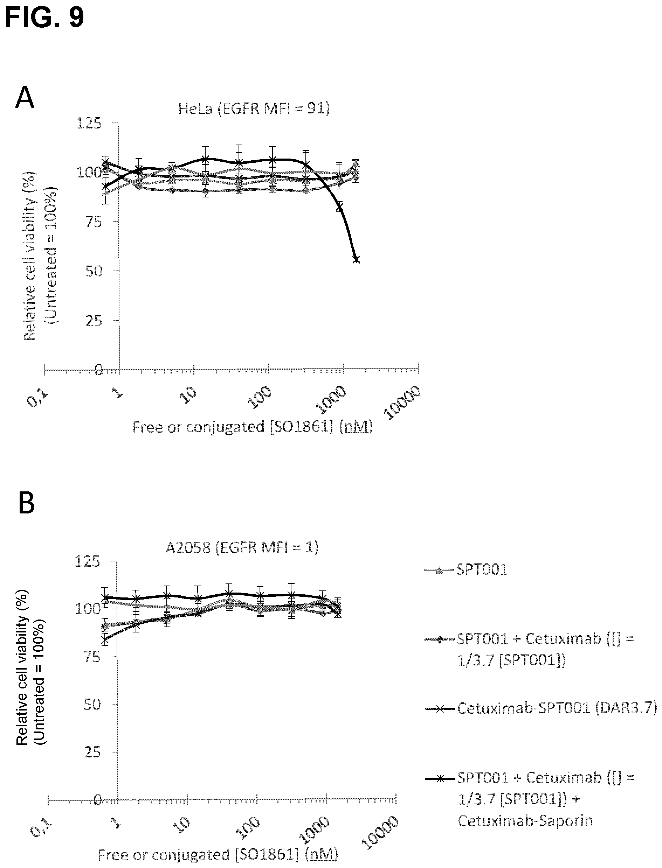

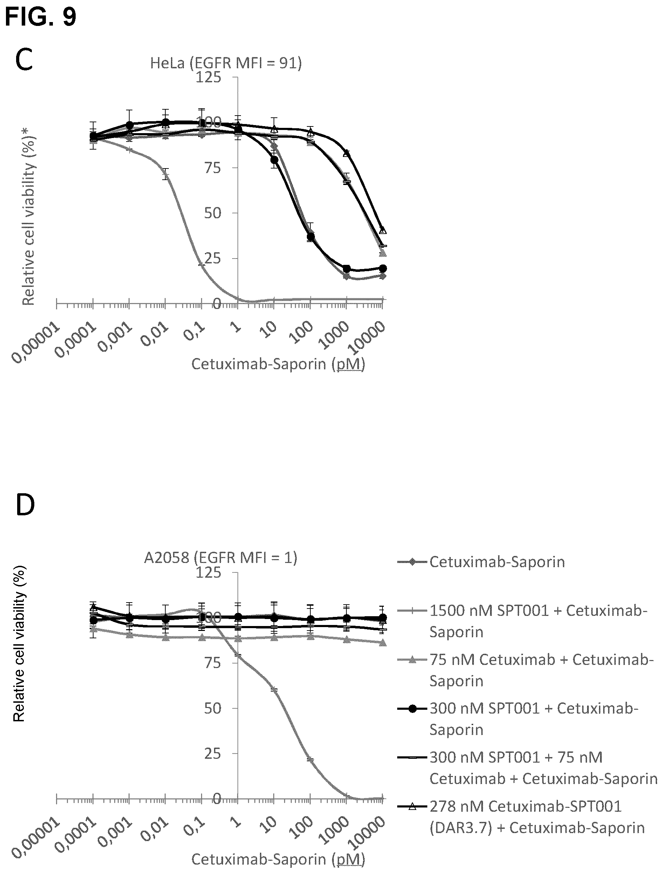

**Figure 9 (FIG. 9****):** *1-target 2-component (EGFR no*/*low expression).* EGFR targeted cell killing in HeLa (EGFR⁺) and A2058 (EGFR⁻) cells, by a therapeutic combination. A, B) cetuximab-SO1861 titration in combination with a fixed concentration of 10 pM cetuximab-saporin does not induce cell killing by cetuximab-saporin. C, D), Cetuximab-saporin titration in combination with 278 nM cetuximab-SO1861 cannot induce cell killing. Low EGFR receptor expression is prohibitive for sufficient SO1861 to be delivered via antibody-mediated delivery, while 1500 nM of unconjugated SO1861 induces cell killing. REMARK: the legend displayed in Fig. 9B also relates to the graphs in Fig. 9A. REMARK: the legend displayed in Fig. 9D also relates to the graphs in Fig. 9C.

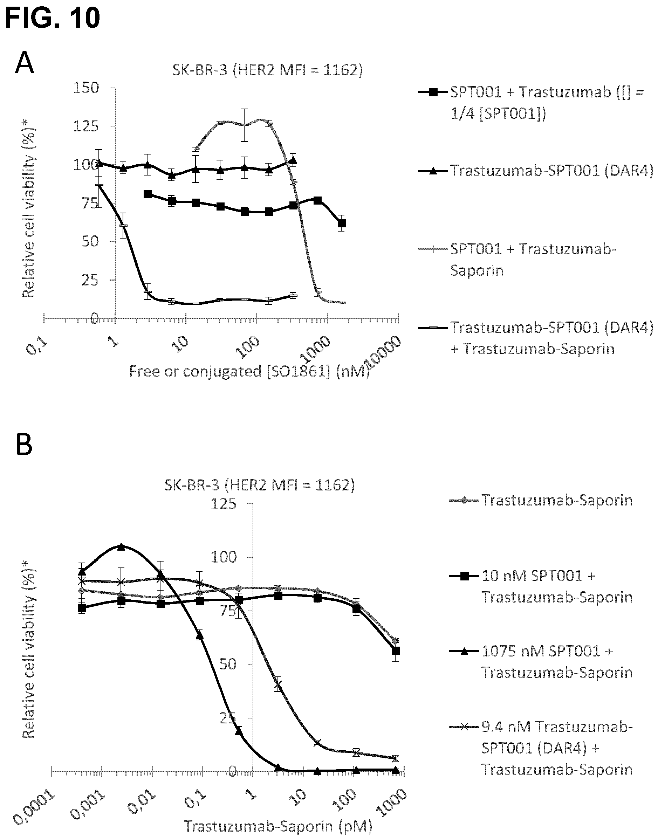

**Figure 10 (FIG. 10****):** *1-target 2-component (HER2 high expression).* HER2 targeted cell killing in SKBR3 (HER2⁺⁺⁺) cells by a therapeutic combination. A) Trastuzumab-SO1861 titration in combination with a fixed concentration of 673 pM trastuzumab-saporin shows that a 1000 fold reduced concentration of conjugated SO1861 is required, versus unconjugated SO1861, to induce cell killing by trastuzumab-saporin. C, D), Trastuzumab-saporin titration in combination with 9,4 nM Trastuzumab-SO1861 kills cells in contrast to 10 nM unconjugated SO1861 + trastuzumab-saporin. 1075 nM unconjugated SO1861 + trastuzumab-saporin is more efficient compared to the therapeutic combination, since both trastuzumab conjugates compete for the same HER2 receptor. Only simultaneous targeted delivery of both trastuzumab conjugates leads to efficient cell-killing, in contrast to monotherapy with either conjugate alone. SPT001 is SO1861.

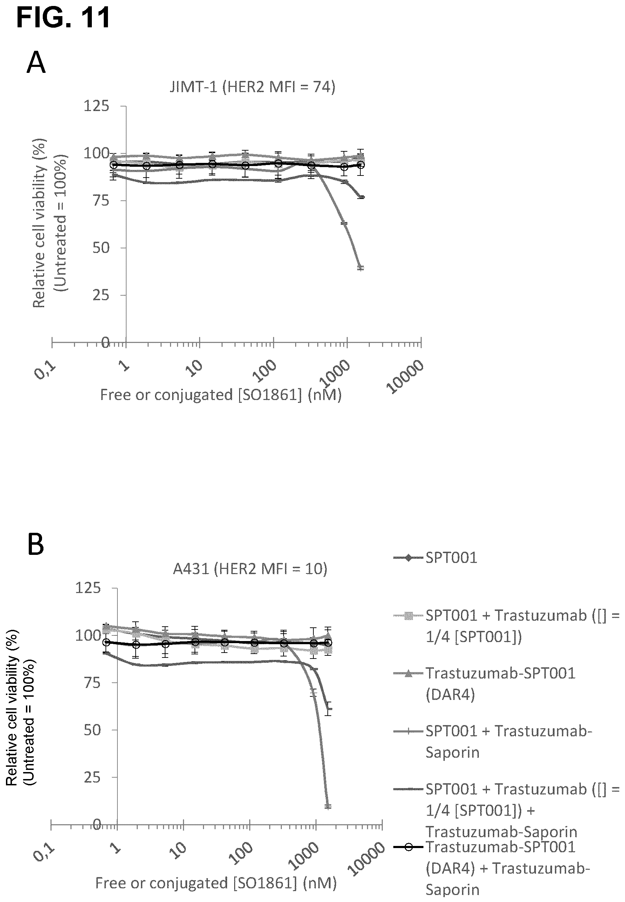

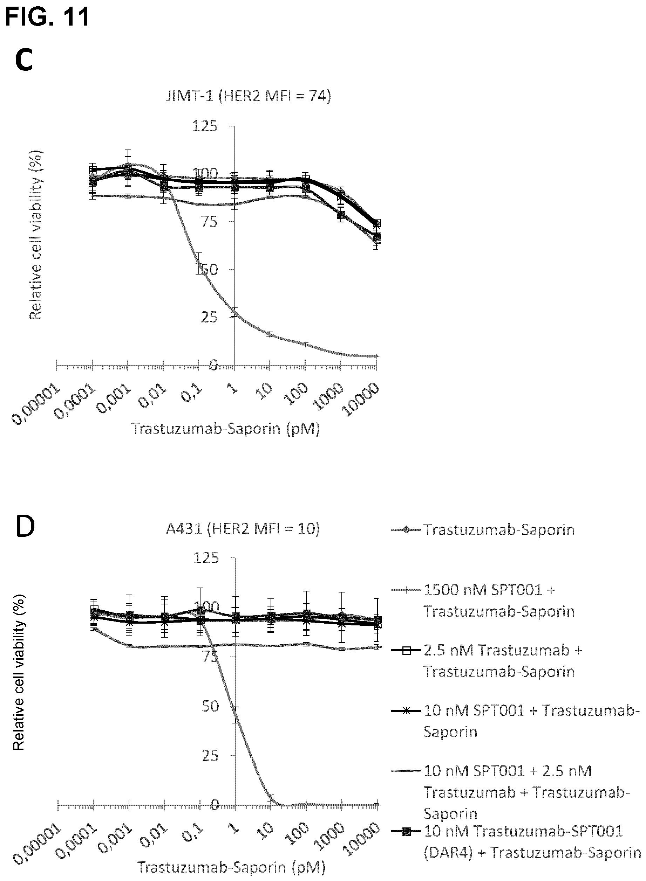

**Figure 11 (FIG. 11****):** *1-target 2-component(HER2 no*/*low expression). HER2* targeted cell killing in JIMT1 (HER2⁺) and A431 (HER2^{+/-}) cells, by a therapeutic combination. A, B) trastuzumab-SO1861 titration in combination with a fixed concentration of 50 pM trastuzumab-saporin does not induce cell killing by trastuzumab-saporin. C, D), Trastuzumab-saporin titration in combination with 10 nM trastuzumab-SO1861 can not induce cell killing. Low HER2 receptor expression is prohibitive for sufficient SO1861 to be delivered via antibody-mediated delivery, while 1500 nM of unconjugated SO1861 induces efficient cell killing. REMARK: the legend displayed in Fig. 11B also relates to the graphs in Fig. 11A. REMARK: the legend displayed in Fig. 11D also relates to the graphs in Fig. 11C. SPT001 is SO1861.

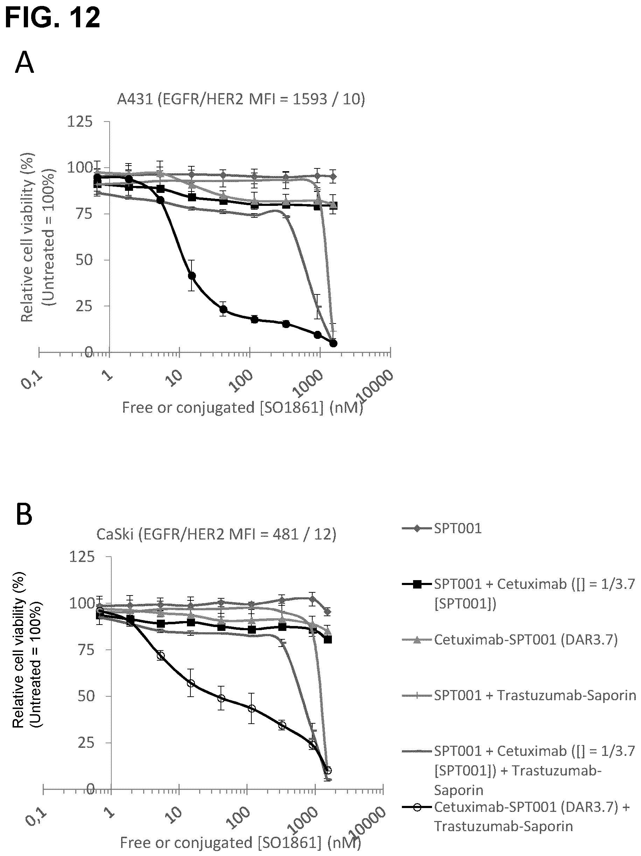

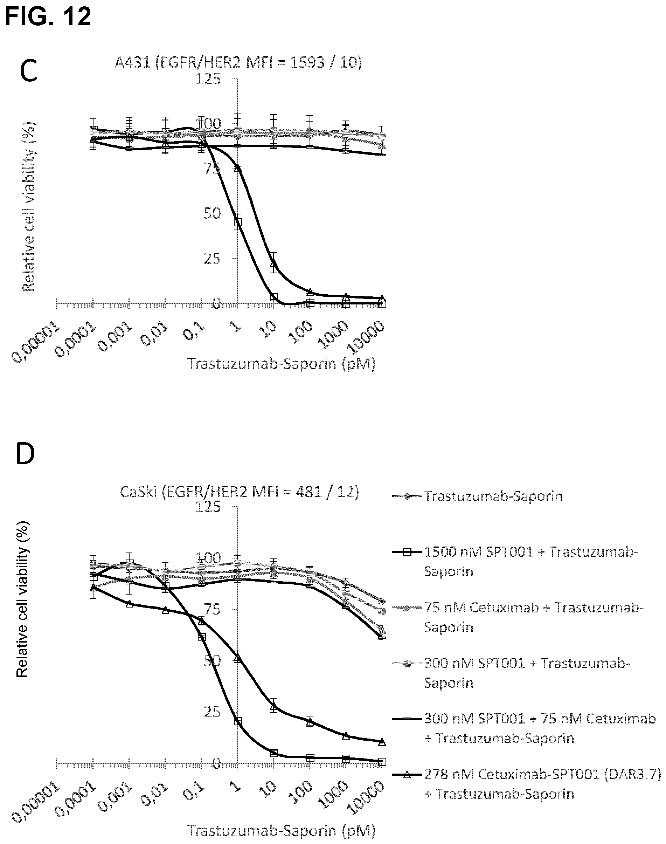

**Figure 12 (FIG. 12****):** *2-target 2-component (EGFR high expression and HER2 low expression). EGFR*/*HER2* targeted cell killing in A431 (EGFR⁺⁺⁺/HER2^{+/-}) and Caski (EGFR⁺⁺/HER2^{+/-}) cells by a therapeutic combination. A, B) Cetuximab-SO1861 titration in combination with a fixed concentration of 50 pM trastuzumab-saporin shows a 100 fold reduced concentration of conjugated SO1861 is required, versus unconjugated SO1861, to induce cell killing by trastuzumab-saporin. C, D), Trastuzumab-saporin titration in combination with 278 nM cetuximab-SO1861 kills cells in contrast to 300 nM unconjugated SO1861 + trastuzumab-saporin. 1500 nM unconjugated SO1861 + trastuzumab-saporin has comparable cell killing efficiency compared to the therapeutic combination, 278 nM cetuximab-SO1861 + trastuzumab-saporin, since both conjugates do not compete for the same receptor. Only simultaneous targeted delivery of both conjugates leads to efficient cell-killing, in contrast to monotherapy with either conjugate alone. REMARK: the legend displayed in Fig. 12B also relates to the graphs in Fig. 12A. REMARK: the legend displayed in Fig. 12D also relates to the graphs in Fig. 12C. SPT001 is SO1861.

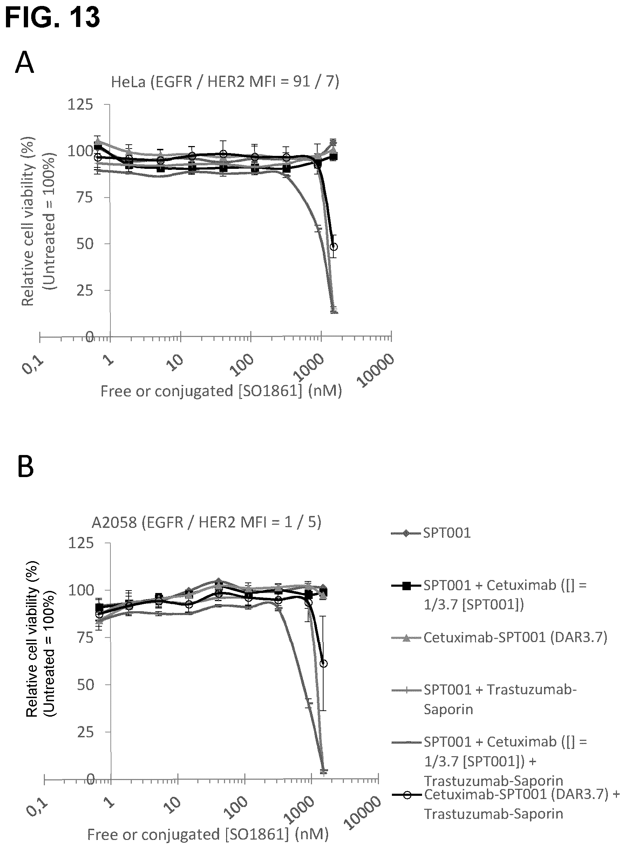

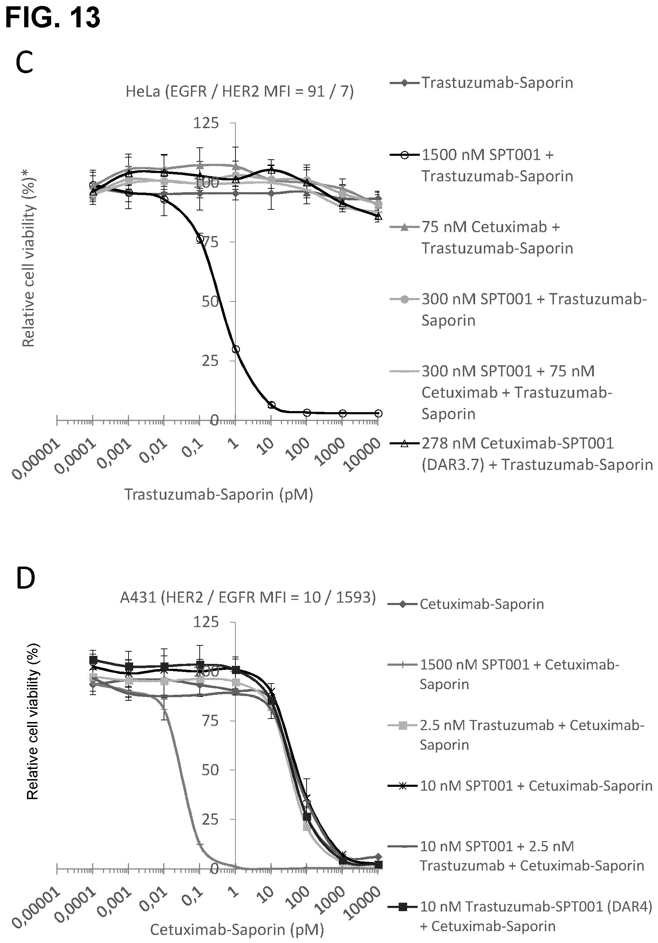

**Figure 13. (FIG. 13****):** *2-target 2-component (EGFR low expression and HER2 nollow expression).* EGFR/HER2 targeted cell killing in HeLa (EGFR⁺/HER2^{+/-}) and A2058 (EGFR⁻/HER2^{+/-}) cells by a therapeutic combination. A, B) Cetuximab-SO1861 titration in combination with a fixed concentration of 50 pM trastuzumab-saporin does not induce cell killing by trastuzumab-saporin. C, D), Trastuzumab-saporin titration in combination with 278 nM cetuximab-SO1861 does not potentiate cell killing, while 1500 nM of unconjugated SO1861 induces efficient cell killing. Low EGFR receptor expression is prohibitive for sufficient SO1861 to be delivered via antibody-mediated delivery. REMARK: the legend displayed in Fig. 13B also relates to the graphs in Fig. 13A. REMARK: the legend displayed in Fig. 13D also relates to the graphs in Fig. 13C. SPT001 is SO1861.

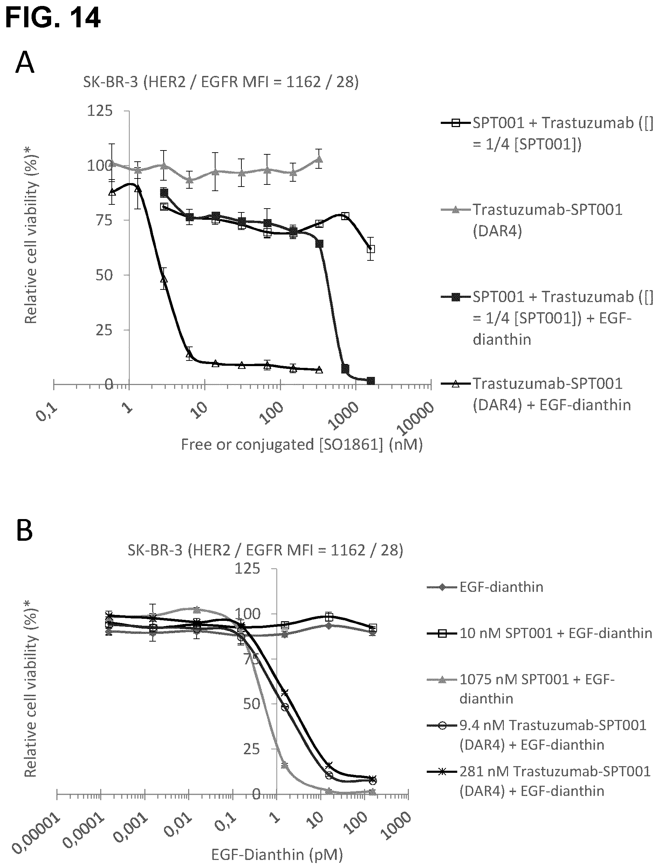

**Figure 14. (FIG. 14****):** *2-target 2-component (HER2 high expression and EGFR low expression). HER2* targeted cell killing in SKBR3 (HER2⁺⁺⁺/EGFR^{+/-}) cells by a therapeutic combination. A) Trastuzumab-SO1861 titration in combination with a fixed concentration of 1.5 pM EGFdianthin shows that a 400 fold reduced concentration of conjugated SO1861 is required, versus unconjugated SO1861, to induce cell killing by EGFdianthin. B), EGFdianthin titration in combination with 9,4 nM trastuzumab-SO1861 can kill cells in contrast to 10 nM unconjugated SO1861 + EGFdianthin. 1075 nM unconjugated SO1861 + EGFdianthin has comparable cell killing efficiency compared to the therapeutic combination, 9,4 nM trastuzumab-SO1861 + EGFdianthin, since both conjugates do not compete for the same receptor. Only simultaneous targeted delivery of both conjugates leads to efficient cell-killing, in contrast to monotherapy with either conjugate alone. SPT001 is SO1861.

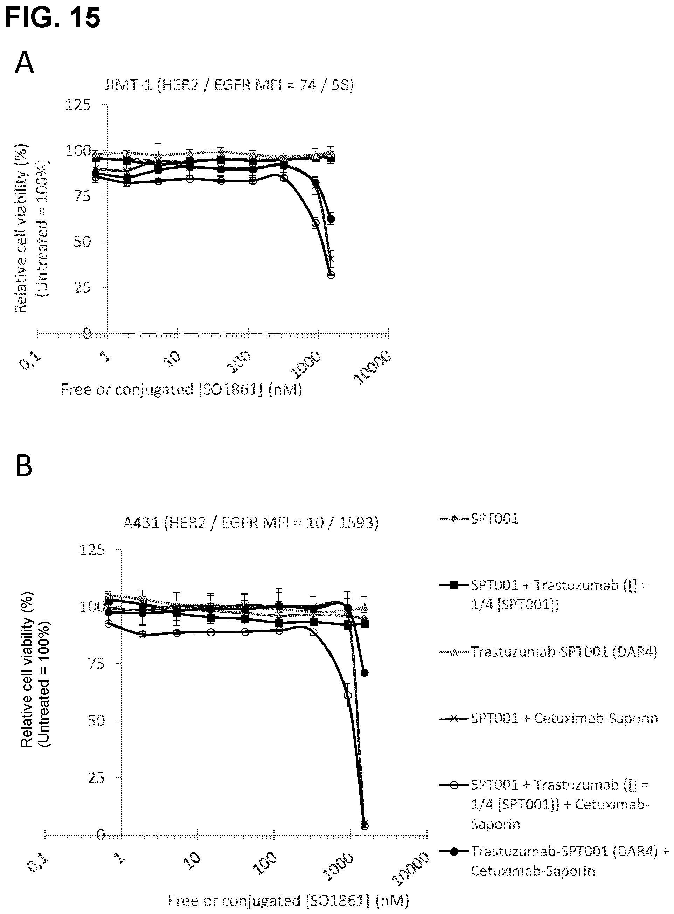

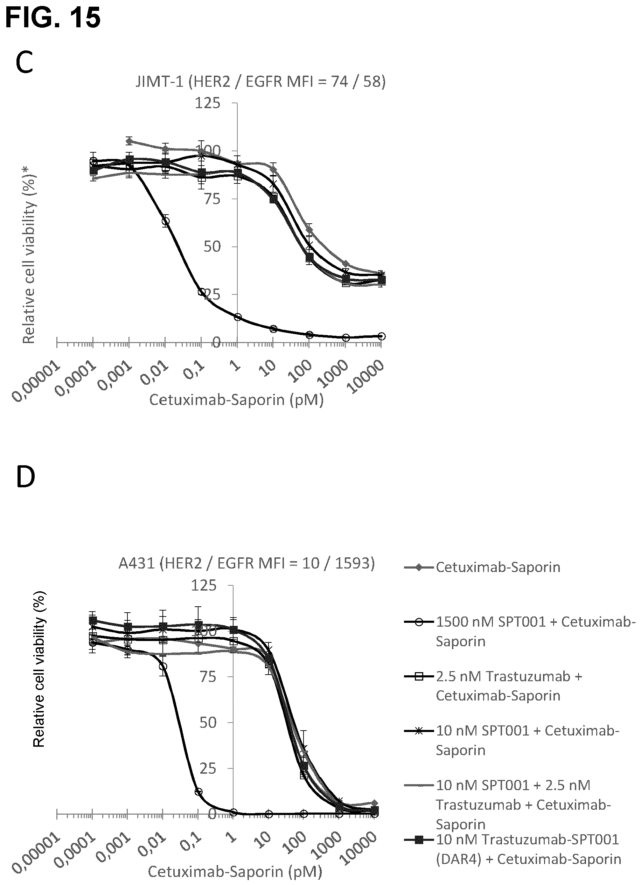

**Figure 15. (FIG. 15****):** *2-target 2-component (HER2 low expression and EGFR low or high expression). HER2* targeted cell killing in JIMT1 (HER2⁺) and A431 (HER2^{+/-}) cells, by a therapeutic combination according to the invention. A, B) trastuzumab-SO1861 titration in combination with a fixed concentration of 5 pM cetuximab-saporin does not induce cell killing by cetuximab-saporin. C, D), Cetuximab-saporin titration in combination with 10 nM trastuzumab-SO1861 can not induce cell killing. Low HER2 receptor expression is prohibitive for sufficient SO1861 to be delivered via antibody-mediated delivery, while 1500 nM of unconjugated SO1861 induces efficient cell killing. Even a high EGFR receptor expression level (D) for delivery of cetuximab-saporin does not change its potency in the presence of trastuzumab-SO1861, indicating that the bottleneck for cell-killing activity is a too low HER2 expression level, leading to insufficient SO1861 inside target cells to switch on endosomal escape. REMARK: the legend displayed in Fig. 15B also relates to the graphs in Fig. 15A. REMARK: the legend displayed in Fig. 15D also relates to the graphs in Fig. 15C. SPT001 is SO1861.

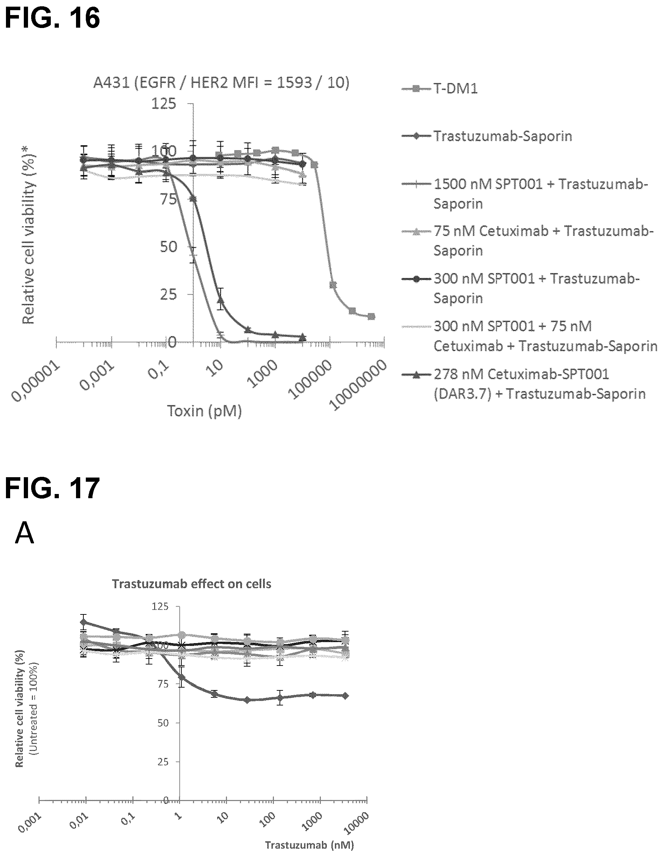

**Figure 16. (FIG. 16****):** 2-target 2-component versus T-DM1. Cells with high EGFR expression and low HER2 expression (A431) are efficiently killed with the therapeutic combination, however T-DM1 is not effective at such low toxin concentrations. T-DM1 is Trastuzumab-emtansine (Kadcyla^{®}), carrying ~3.5 DM1 toxin molecules per antibody.

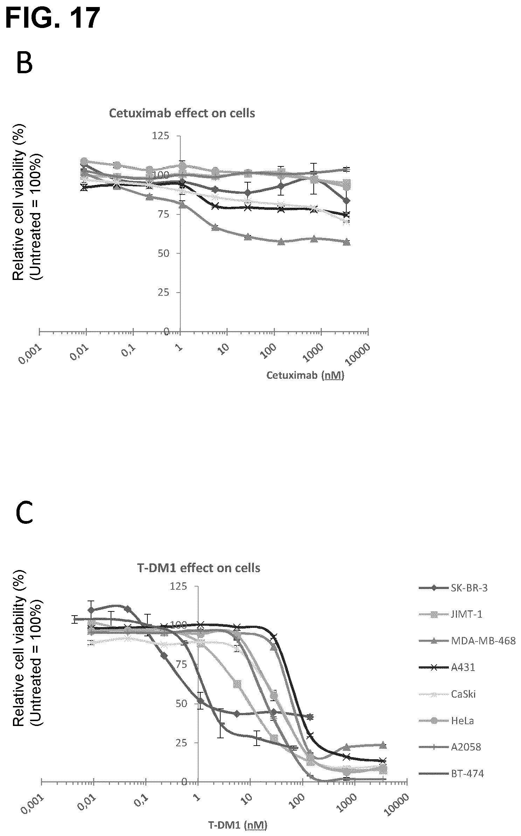

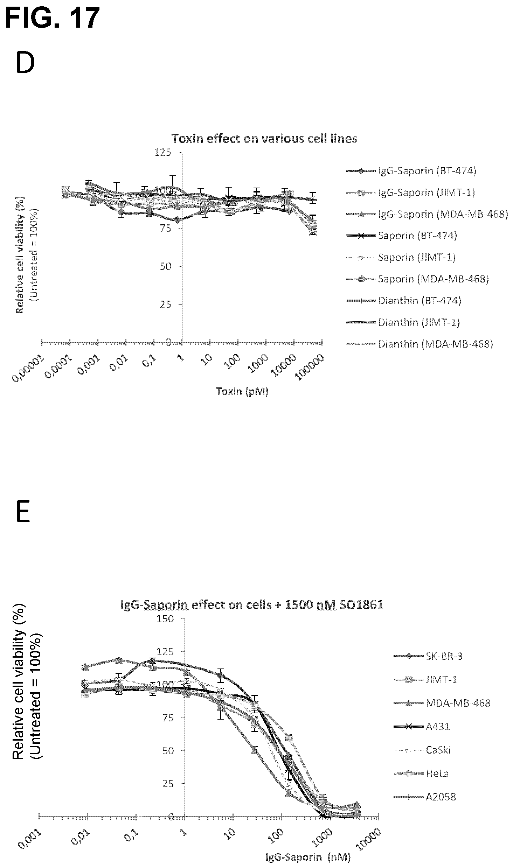

**Figure 17A-E. (Figure 17A-E****):** displays the relative cell viability when trastuzumab (Fig. 17A), cetuximab (Fig. 17B) or T-DM1 (Fig. 17C), free toxins saporin (Fig. 17D) and dianthin (Fig. 17D), saporin coupled to a non-cell binding IgG (Fig. 17D), and saporin coupled to a non-cell binding IgG combined with free saponin SO1861 (Fig. 17E) are contacted with the indicated cell lines SK-BR-3, JIMT-1, MDA-MB-468, A431, CaSki, HeLa, A2058, BT-474. REMARK: the legend displayed in Fig. 17C also relates to the curves in Fig. 17A and 17B.

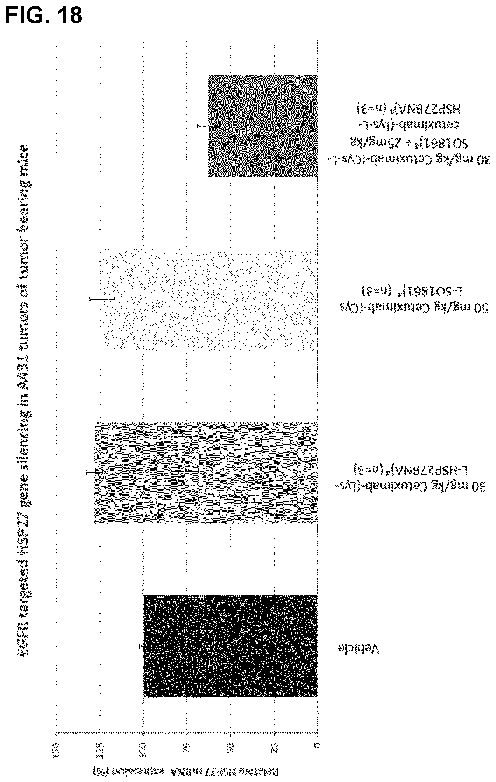

**Figure 18. (FIG. 18****):** *1T2C in vivo activity.* The 1T2C combination of 50 mg/kg cetuximab-(Cys-L-SO1861)⁴ + 25 mg/kg cetuximab-(-L-HSP27BNA)⁴ in A431 tumor bearing mice reveals strong tumor targeted gene silencing, compared to the controls.

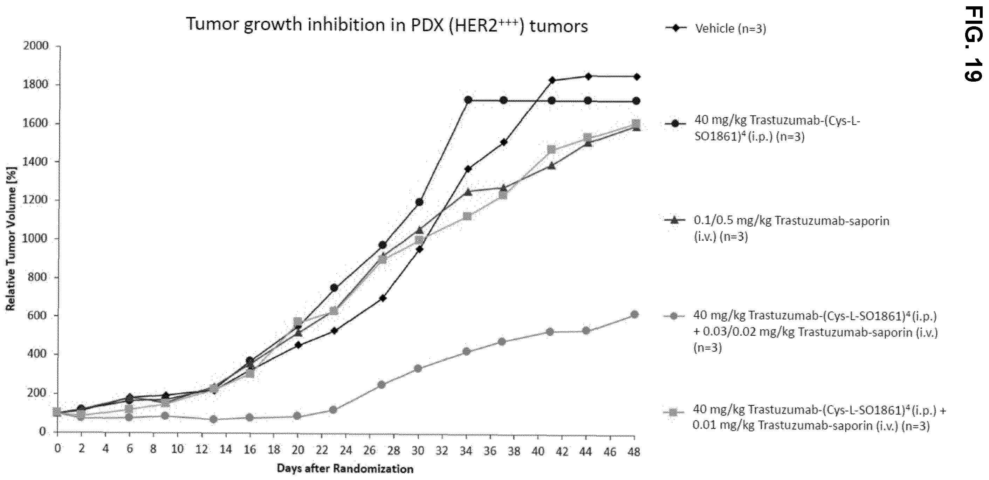

**Figure 19. (FIG. 19****):** *1T2C in vivo activity.* The 1T2C combination of 40 mg/kg trastuzumab-(Cys-L-SO1861)⁴ + 0.02/0.03 mg/kg trastuzumab-saporin in a PDX tumor mouse model (high HER2 expression) shows effective tumor growth inhibition.

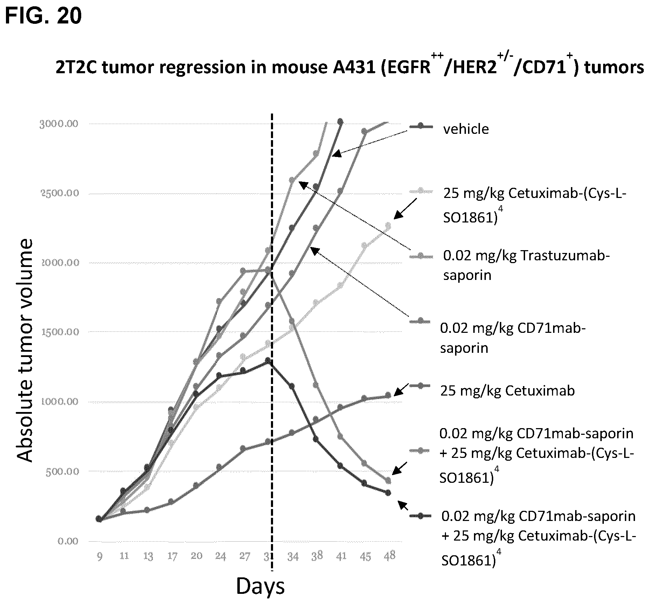

**Figure 20. (FIG. 20****):** *The 2T2 component system tested in A431 tumor bearing mice model reveals tumor regression.*

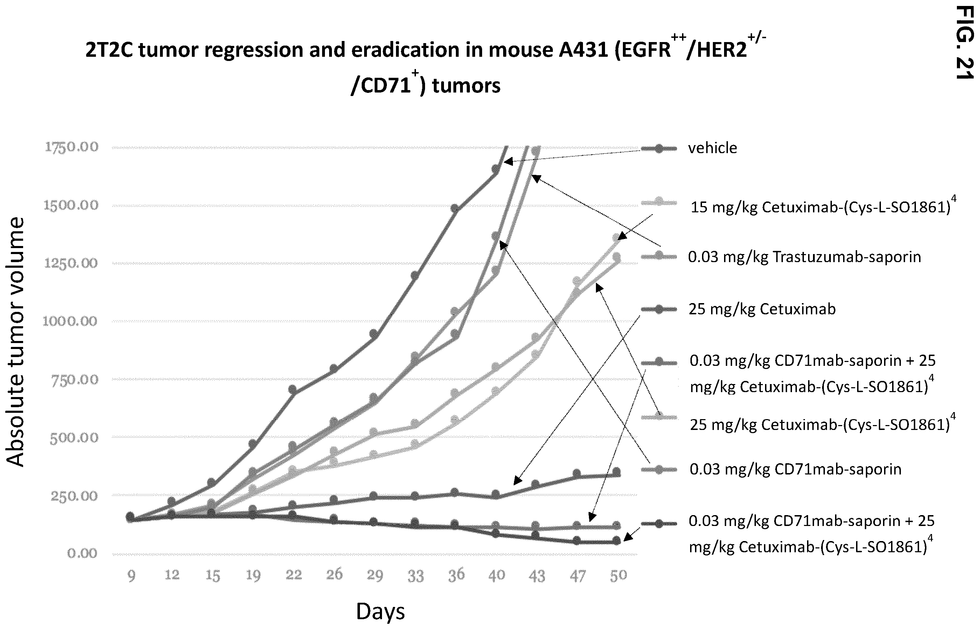

**Figure 21. (FIG. 21****):** *The 2T2 component system tested in A431 tumor bearing mice model reveals tumor regression and eradication.*

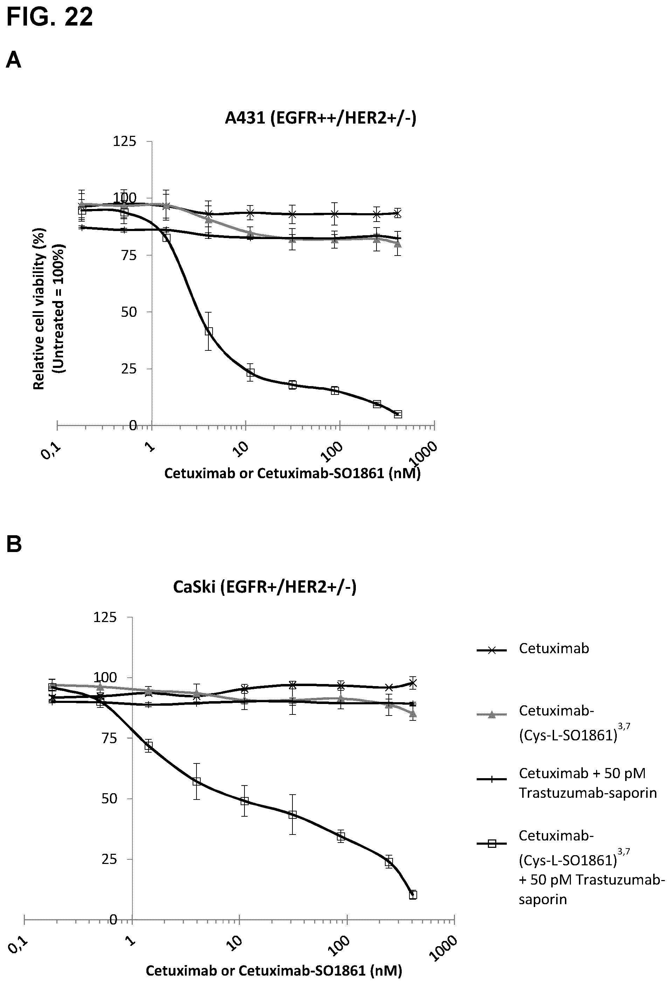

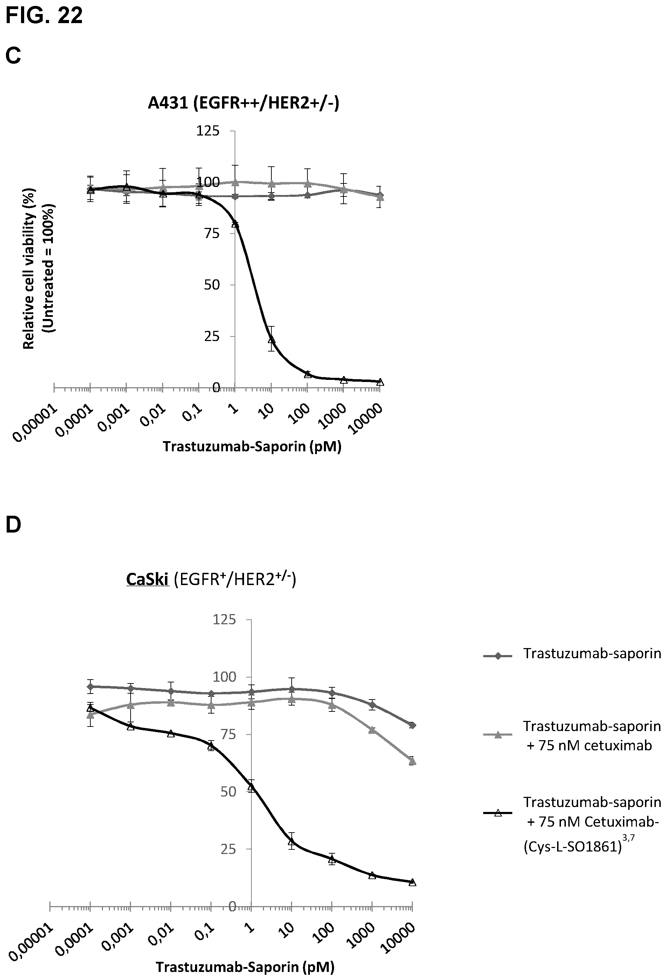

**Figure 22 (FIG. 22****):** *2-target 2-component.* EGFR/HER2 targeted cell killing in A431 cells (EGFR⁺⁺/HER2^{+/-}) (A, C) and CaSKi cells (EGFR⁺⁺/HER2^{+/-}) (B, D) by a therapeutic combination according to the invention. A, B) Cetuximab-(Cys-L-SO1861)^{3,7} titration + fixed concentration 50 pM trastuzumab-saporin and controls on A431 cells. C, D) Trastuzumab-saporin titration + fixed concentration of 75 nM cetuximab-(Cys-L-SO1861)^{3,7} and controls on Caski cells. The legends and/or axes are the same for A,B, and the legends are the same for figures C and D.

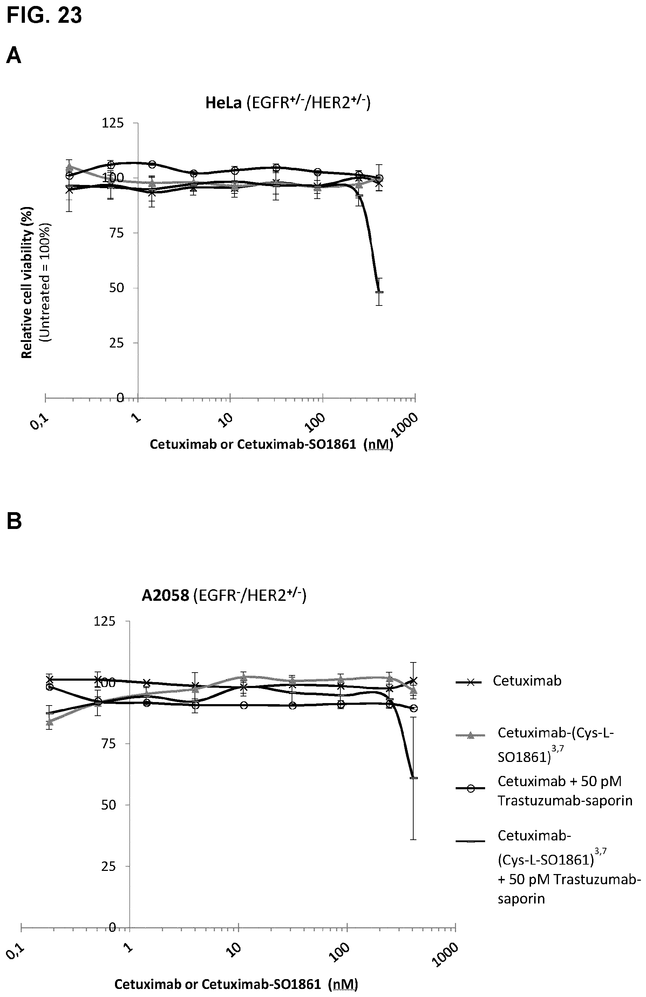

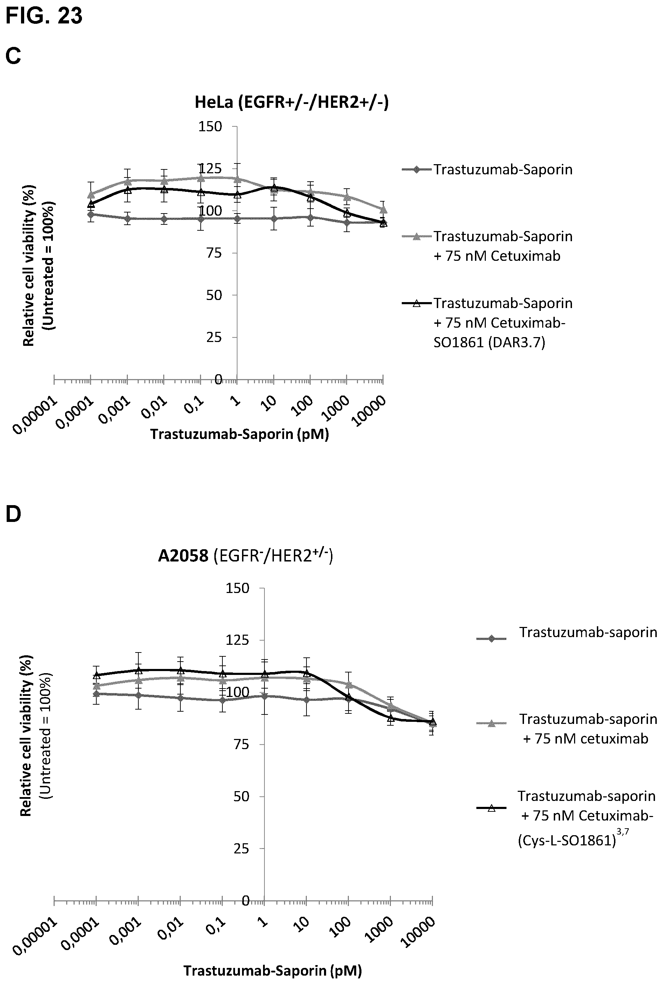

**Figure 23. (FIG. 23****):** *2-target 2-component.* EGFR/HER2 targeted cell killing in HeLa cells (EGFR^{+/-}/HER2^{+/-}) (A, C) and A2058 cells (EGFR⁻/HER2^{+/-}) (B, D) by a therapeutic combination according to the invention. A, B) Cetuximab-(Cys-L-SO1861)^{3,7} titration + fixed concentration 50 pM trastuzumab-saporin and controls on HeLa cells. C, D) Trastuzumab-saporin titration + fixed concentration of 75 nM cetuximab-(Cys-L-SO1861)^{3,7} and controls on A2058 cells. The legends and/or axes are the same for C and D.

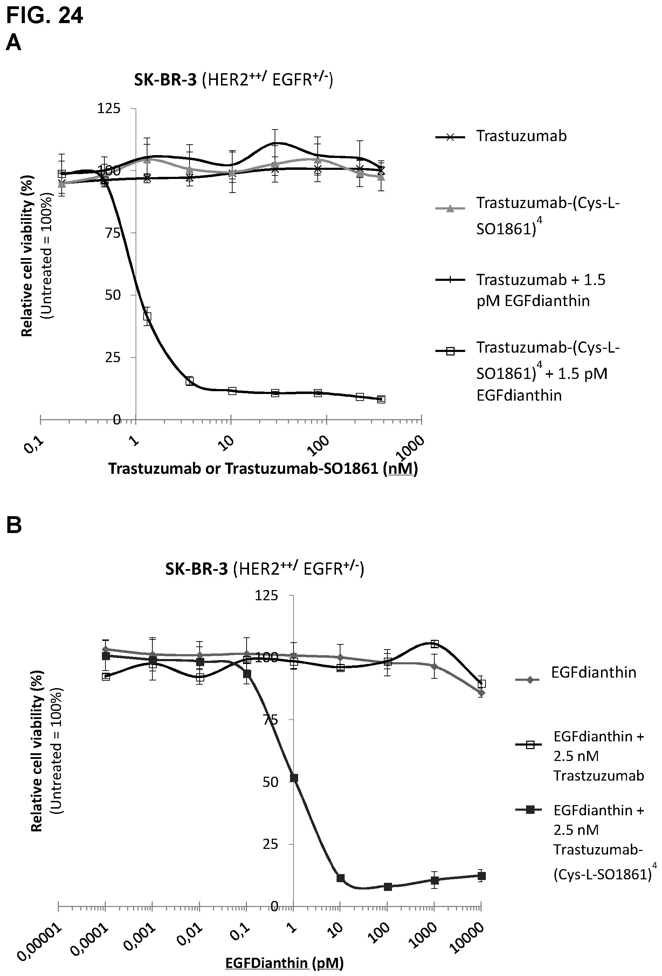

**Figure 24. (FIG. 24****):** *2-target 2-component.* HER2/EGFR targeted cell killing in SKBR3 cells (HER2⁺⁺/EGFR^{+/-}) (A, B) by a therapeutic combination according to the invention. A Trastuzumab-(Cys-L-SO1861)⁴ titration + fixed concentration 1.5 pM EGFdianthin and controls on SKBR3 cells. B) EGFdianthin titration + fixed concentration of 2.5 nM trastuzumab-(Cys-L-SO1861)⁴ and controls on SKBR3 cells.

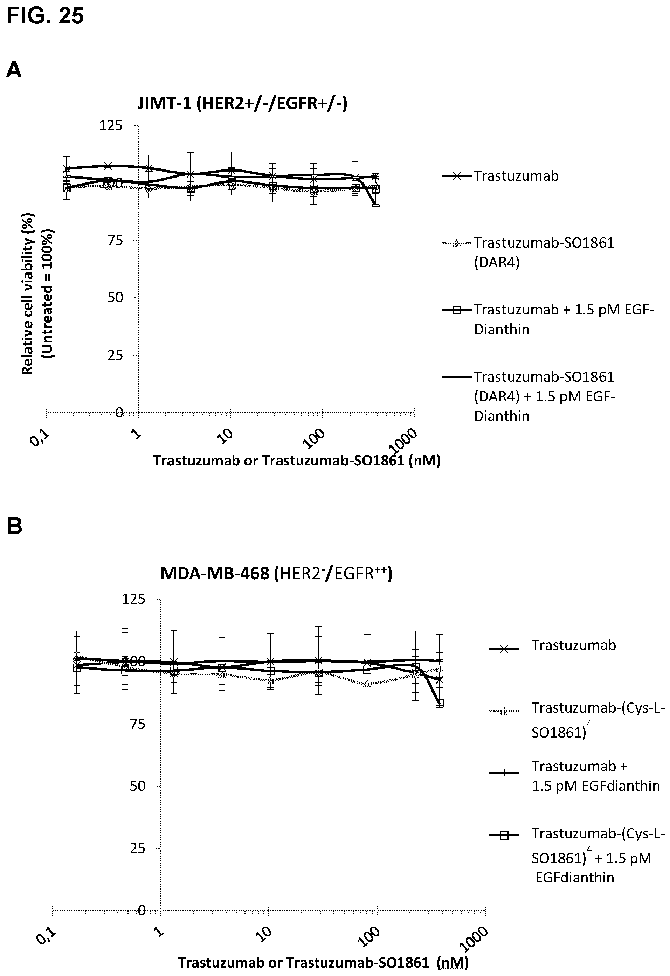

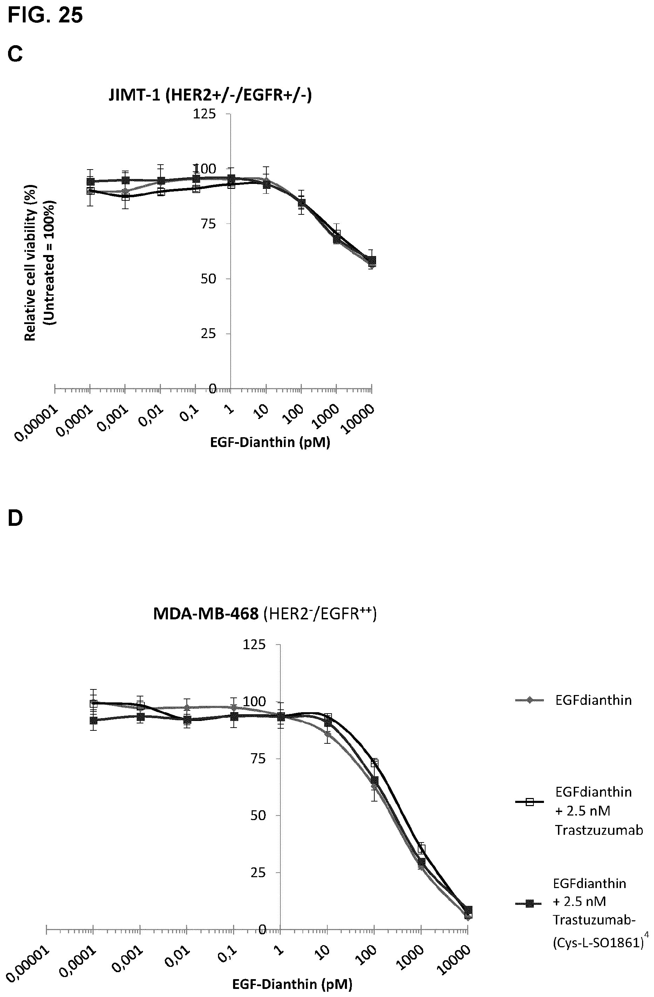

**Figure 25. (FIG. 25****):** *2-target 2-component.* HER2/EGFR targeted cell killing in JIMT-1 cells (HER2^{+/-}EGFR^{+/-}) (A, C) and MDA-MB-468 cells (HER2⁻/EGFR⁺⁺) (B, D) by a therapeutic combination according to the invention. A, B) Trastuzumab-(Cys-L-SO1861)⁴ titration + fixed concentration 1.5 pM EGFdianthin and controls on JIMT-1 cells. C, D) EGFdianthin titration + fixed concentration of 2.5 nM trastuzumab-(Cys-L-SO1861)⁴ and controls on MDA-MB-468 cells. The legends and/or axes are the same for C and D.

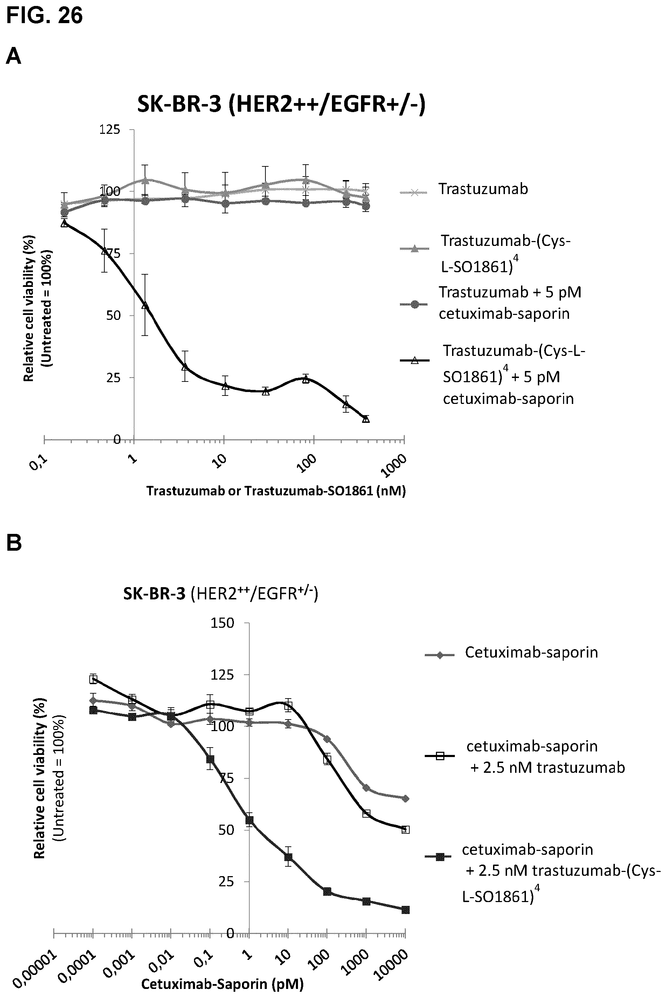

**Figure 26. (FIG. 26****):** *2-target 2-component.* HER2/EGFR targeted cell killing in SKBR3 cells (HER2⁺⁺/EGFR^{+/-}) (A, B) by a therapeutic combination according to the invention. A) Trastuzumab-(Cys-L-SO1861)⁴ titration + fixed concentration 10 pM cetuximab-saporin and controls on SKBR3 cells. B) Cetuximab-saporin titration + fixed concentration of 2.5nM trastuzumab-(Cys-L-SO1861)⁴ and controls on SKBR3 cells.

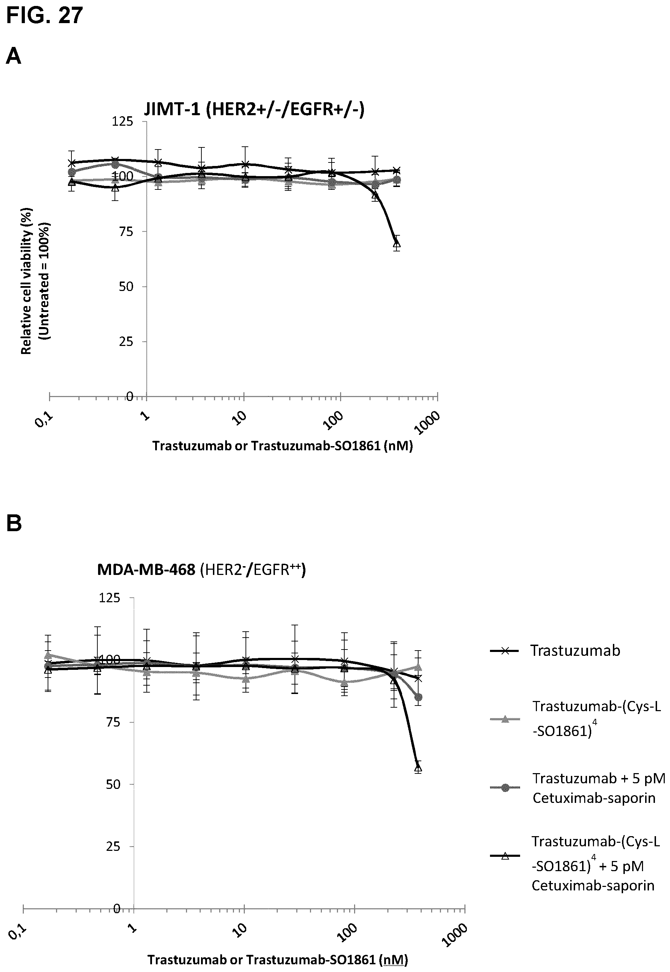

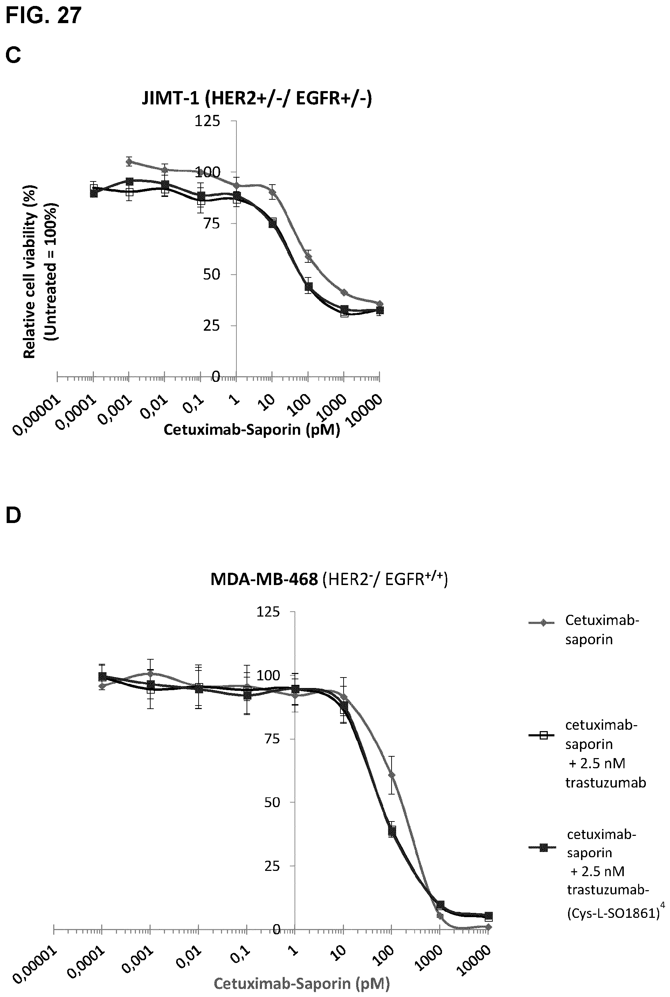

**Figure 27. (FIG. 27****):** *2-target 2-component.* HER2/EGFR targeted cell killing in JIMT-1 cells (HER2^{+/-}EGFR^{+/-}) (A, C) and MDA-MB-468 cells (HER2 /EGFR⁺⁺) (B, D) by a therapeutic combination according to the invention. A, B) Trastuzumab-(Cys-L-S01861)⁴ titration + fixed concentration 10 pM cetuximab-saporin and controls on JIMT-1 cells. C, D) Cetuximab-saporin titration + fixed concentration of 2.5 nM trastuzumab-(Cys-L-S01861)⁴ and controls on MDA-MB-468 cells. The legends and/or axes are the same for A and B, and the legends are the same for C and D.

### DETAILED DESCRIPTION

It is a first goal of the present invention to provide improved ADCs and AOCs with increased therapeutic window, and to provide an improved therapeutic combination or an improved pharmaceutical composition for delivery of an effective amount or dose of an effector molecule, when for example the delivery from outside a target cell into said cell, is considered, or more in particular when the delivery of the effector molecule in the cytosol of said target cell is considered. It is a second goal of the present invention to provide an improved method of treatment of a (human) patient suffering from a disease to be treated with (an effective amount of) a conjugate comprising an effector molecule and a ligand (cell-surface molecule binding molecule for targeting the effector molecule at and in a target aberrant cell such as a tumor cell) for e.g. a target tumor cell, i.e. to improve the therapeutic window of the ADC or the AOC comprising the effector molecule to be delivered in the cytosol of target tumor cells.

It is an objective of the current invention to provide a combination of effector-molecule activity enhancing molecules and improved ADCs or AOCs for use in therapy such as anti-cancer therapy. It is also an objective of the current invention to provide a therapeutic composition or a therapeutic combination of e.g. two therapeutic compositions, comprising such an improved ADC or AOC and comprising an effector-molecule activity enhancing molecule. Such an effector-molecule activity enhancing molecule is capable of improving a biological effect of an effector molecule once said effector molecule is delivered inside a target cell in e.g. and preferably the endosome, endolysosome and/or lysosome, and e.g. subsequently and preferably into the cytosol of said cell, which target cell comprises the molecular target for the effector molecule. Said provided effector-molecule activity enhancing molecule, when administered to a (human) patient in need thereof, bearing the target cell, results in the experience by said patient of an improved therapeutic effect or at least a sufficient effect at preferably a lower dose of the effector molecule than the currently required dose for reaching an effective dose of the effector molecule. This way, the therapeutic window of the effector molecule, such as an effector molecule which is part of a conjugate, such as an ADC or an AOC, is widened effectively.

In order for a bioactive molecule (e.g. an effector molecule) to work, the molecule must be able to engage with its target, e.g. in the blood serum, on the outside of the cell surface or inside a cell or an organelle. The active moiety of almost all protein-based targeted toxins, e.g., must enter the cytosol of the target cell to mediate its target modulatory effect. In many constellations the toxin remains ineffective since (1) the targeting moiety is poorly internalized and remains bound to the outside of the cells, (2) is recycled back to the cell surface after internalization or (3) transported to the endolysosomes where it is degraded. Although these fundamental issues are known for decades and more than 500 targeted toxins have been investigated in the past decades, the problems have not been solved yet and only a couple of antibody-targeted protein toxin have been admitted to the market, albeit with warning labels for severe toxicity. Moxetumomab pasudotox-tdfk (LUMOXITI^{®}, AstraZeneca Pharmaceuticals LP), has been approved for relapsed or refractory hairy cell leukemia by the FDA to date. Other of such approved ADCs are Elzonris, Ontak.

To overcome these problems, many strategies have been described including approaches to redirect the toxins to endogenous cellular membrane transport complexes of the biosynthetic pathway in the endoplasmic reticulum and techniques to disrupt or weaken the membrane integrity of endosomes, i.e. the compartments of the endocytic pathway in a cell, and thus facilitating the endosomal escape. This comprises the use of lysosomotropic amines, carboxylic ionophores, calcium channel antagonists, various cell-penetrating peptides of viral, bacterial, plant, animal, human and synthetic origin, other organic molecules and light-induced techniques. Although the efficacy of the targeted toxins was typically augmented in cell culture hundred- or thousand-fold, in exceptional cases more than million-fold, the requirement to co-administer endosomal escape enhancers with other substances harbors new problems including additional side effects, loss of target specificity, difficulties to determine the therapeutic window and cell type-dependent variations.

All strategies, including physicochemical techniques, require enhancer molecules that interact more or less directly with membranes and comprise essentially small chemical molecules, secondary metabolites, peptides and proteins. A common feature of all these substances is that they are *per se* not target cell-specific and distribute with other kinetics than the targeted toxins. This is one major drawback of the current approaches.

It is a further goal of the present invention to provide improved enhancers for sdAb based ADCs and for sdAb based AOCs, and to provide an improved therapeutic combination or an improved pharmaceutical composition for delivery of an effective amount of an effector molecule, when for example the delivery from outside a target cell into said cell, is considered, or more in particular when the delivery of the effector molecule in the cytosol of said target cell is considered. It is a further goal of the present invention to provide an improved method of treatment of a (human) patient suffering from a disease to be treated with a conjugate comprising an effector molecule and a ligand, i.e. an sdAb, for e.g. a target tumor cell, i.e. to improve the therapeutic window of the ADC or the AOC comprising the effector molecule and comprising the sdAb to be delivered in the cytosol of target tumor cells.

It is an objective of the current invention to provide effector-molecule enhancing molecules for use in therapy such as anti-cancer therapy, a therapeutic composition or a therapeutic combination of e.g. two therapeutic compositions, comprising such a molecule that is capable of improving a biological effect of an effector molecule once delivered inside a target cell which comprises the molecular target for the effector molecule, which provided molecule, when administered to a (human) patient in need thereof, bearing the target cell, experience an improved therapeutic effect or a sufficient effect at a lower dose of the effector molecule than the currently required dose for reaching an effective dose of the effector molecule. This way, the therapeutic window of the effector molecule, such as an effector molecule which is part of a conjugate, such as an ADC comprising an sdAb or an AOC comprising an sdAb, is widened effectively.

The present invention will be described with respect to particular embodiments but the invention is not limited thereto but only by the claims. While the invention has been described in terms of several embodiments, it is contemplated that alternatives, modifications, permutations and equivalents thereof will become apparent to one having ordinary skill in the art upon reading the specification and upon study of the drawings and graphs. The invention is not limited in any way to the illustrated embodiments. Changes can be made without departing from the scope which is defined by the appended claims.

The inventors invented and established that the therapeutic window of a first conjugate of the invention such as an antibody drug conjugate or an antibody-oligonucleotide conjugate, increases when such an ADC or AOC preferably comprises a cell-surface molecule binding sdAb for targeting the ADC or AOC to a cell-surface molecule, and when to the same subject in need thereof such as a human cancer patient or a tumor bearing mammal, is administered a second conjugate comprising at least one covalently bound saponin (see for example the examples in Tables A5-A7, and see for example Figures 4-7 for conjugates comprising V_{HH} and Figures 2, 3, 8-13, 16, 16, 18-23, 26 and 27 for conjugates comprising a monoclonal antibody (mAb), in the Examples section for a series of in vitro and in vivo tumor cell-model examples of the effect of saponin covalently bound to an antibody such as an IgG or a V_{HH}, when combined with an ADC or an AOC comprising an antibody such as a mAb or an sdAb). The saponin is conjugated with an antibody such as an IgG or a V_{HH}, and an effector molecule such as protein toxins or an oligonucleotide such as a BNA is conjugated with a second antibody such as an IgG or an sdAb. The inventors were the first who established and determined that conjugating a saponin of the invention with a ligand for binding to a cell-surface molecule, such as an antibody such as a full-length intact IgG, or such as an sdAb such as a V_{HH}, provides a conjugate for cell-specific delivery of the saponin at the cell surface of a target cell exposing the cell-surface molecule at its cell surface, and subsequent delivery of the (free) saponin inside the cell, such as the cell endosome, endolysosome, lysosome and ultimately in the cell cytosol (preferred are saponin comprising conjugates wherein the saponin is covalently linked via a bond that is cleavable in the endosome, endolysosome, lysosome of mammalian cells such as human tumor cells, such that free saponin is present once the conjugate comprising the saponin entered the cell endosome, endolysosome, lysosome and the bond is cleaved, e.g. entry via receptor-mediated endocytosis once the conjugate has bound to the cell-surface molecule, i.e. a receptor). Examples of such cell-targeting saponin conjugates are for example provided in Figures 2-27 for saponin-V_{HH} conjugates and for saponin-Mab conjugates, as outlined in the Examples section here below. Saponin is conjugated to ligands such as EGF, Her2 targeting V_{HH} or IgG, EGFR targeting IgG, etc.

At least one of the above objectives is achieved by providing the combination of improved ADCs, or improved AOCs comprising a cell-surface molecule binding sdAb and effector-molecule enhancing molecules of the invention such as a second conjugate comprising covalently linked bidesmosidic triterpenoid glycosides (triterpene saponins).

A first aspect of the invention relates to a pharmaceutical combination comprising:
∘ a first conjugate comprising at least one effector molecule and a single-domain antibody (sdAb) for binding to a first cell-surface molecule, which are covalently linked to each other, directly or via a linker;
∘ a saponin and/or a derivative thereof and/or a second conjugate comprising a binding molecule for binding to a second cell-surface molecule and the saponin and/or the derivative thereof, which binding molecule and the saponin or the derivative thereof are covalently linked to each other, directly or via a linker, wherein the saponin or the derivative thereof is a monodesmosidic triterpene glycoside or a bidesmosidic triterpene glycoside, wherein the second cell-surface molecule is the same as the first cell-surface molecule or different from the first cell-surface molecule; and optionally
∘ a pharmaceutically acceptable excipient and/or pharmaceutically acceptable diluent.

The inventors established that an effector molecule which is part of the first conjugate comprising the sdAb is delivered inside a cell with high efficiency under influence of a saponin, such as a saponin comprised by the second conjugate of the invention, when the effect of the effector molecule inside the cell is considered. More specifically, the inventors established that an effector molecule which is part of the first conjugate comprising the sdAb is delivered in the cytosol of a cell with high efficiency under influence of a saponin of the invention, such as a saponin comprised by the second conjugate of the invention, when the effect of the effector molecule inside the cell is considered. That is to say, contacting tumor cells with a sub-optimal dose of the first conjugate does not result in intracellular effector molecule activity (the target cell is not efficiently killed upon biological activity of the effector molecule), in the absence of the saponin, e.g. as part of the second conjugate. However, when the target tumor cell is contacted with both the first conjugate comprising the effector molecule and the free saponin and/or the second conjugate comprising the saponin, efficient tumor cell killing is determined.

To explain the invention in more detail, the process of cellular uptake of substances and the used terminology in this invention is described first. The uptake of extracellular substances into a cell by vesicle budding is called endocytosis. Said vesicle budding can be characterized by (1) receptor-dependent ligand uptake mediated by the cytosolic protein clathrin, (2) lipid-raft uptake mediated by the cholesterol-binding protein caveolin, (3) unspecific fluid uptake (pinocytosis), or (4) unspecific particle uptake (phagocytosis). All types of endocytosis run into the following cellular processes of vesicle transport and substance sorting called the endocytic pathways. The endocytic pathways are complex and not fully understood. Earlier, it was thought that organelles are formed *de novo* and mature into the next organelle along the endocytic pathway. Nowadays, it is hypothesized that the endocytic pathways involve stable compartments that are connected by vesicular traffic. A compartment is a complex, multifunctional membrane organelle that is specialized for a particular set of essential functions for the cell. Vesicles are considered to be transient organelles, simpler in composition, and are defined as membrane-enclosed containers that form *de novo* by budding from a preexisting compartment. In contrast to compartments, vesicles can undergo maturation, which is a physiologically irreversible series of biochemical changes. Early endosomes and late endosomes represent stable compartments in the endocytic pathway while primary endocytic vesicles, phagosomes, multivesicular bodies (also called endosome carrier vesicles), secretory granules, and even lysosomes represent vesicles. The endocytic vesicle, which arises at the plasma membrane, most prominently from clathrin-coated pits, first fuses with the early endosome, which is a major sorting compartment of approximately pH 6.5. A large part of the cargo and membranes internalized are recycled back to the plasma membrane through recycling vesicles (recycling pathway). Components that should be degraded are transported to the acidic late endosome (pH lower than 6) via multivesicular bodies. Lysosomes are vesicles that can store mature lysosomal enzymes and deliver them to a late endosomal compartment when needed. The resulting organelle is called the hybrid organelle or endolysosome. Lysosomes bud off the hybrid organelle in a process referred to as lysosome reformation. Late endosomes, lysosomes, and hybrid organelles are extremely dynamic organelles, and distinction between them is often difficult. Degradation of the endocytosed molecules occurs inside the endolysosomes. *Endosomal escape* is the active or passive release of a substance from the inner lumen of any kind of compartment or vesicle from the endocytic pathway, preferably from clathrin-mediated endocytosis, or recycling pathway into the cytosol. Endosomal escape thus includes but is not limited to release from endosomes, endolysosomes or lysosomes, including their intermediate and hybrid organelles. After entering the cytosol, said substance might move to other cell units such as the nucleus. Glycoside molecules (saponins) in the context of the invention are compounds that are able to enhance the effect of an effector molecule, in particular by facilitating the endosomal escape. The glycoside molecules interact with the membranes of compartments and vesicles of the endocytic and recycling pathway and make them leaky for said effector molecules resulting in augmented endosomal escape.

With the term "improving an effect of an effector molecule" is meant that a saponin increases the functional efficacy of the effector molecule (e.g. the therapeutic index of a toxin or a drug; the metabolic efficacy of a modifier in biotechnological processes; the transfection efficacy of genes in cell culture research experiments), preferably by enabling or improving its target engagement. Acceleration, prolongation, or enhancement of antigen-specific immune responses are preferably not included. Therapeutic efficacy includes but is not limited to a stronger therapeutic effect with lower dosing and/or less side effects. "Improving an effect of an effector molecule" can also mean that an effector molecule, which could not be used because of lack of effect (and was e.g. not known as being an effector molecule), becomes effective when used as part of the first conjugate of the invention comprising a cell-surface molecule targeting sdAb and in combination with free saponin (derivative) and/or in combination with a second conjugate of the present invention comprising covalently bound saponin (derivative). Any other effect, which is beneficial or desired and can be attributed to the combination of an effector moiety, e.g. as part of an ADC comprising the cell-targeting sdAb or an AOC comprising the cell-targeting sdAb according to the first conjugate of the invention, and a saponin comprised by a second conjugate of the invention, as provided by the invention, is considered to be "an improved effect". In the context of the invention, a saponin such as a saponin comprised by the second conjugate of the invention is an "enhancer" of the functional efficacy of an effector molecule, which is comprised by the first, separate conjugate such as an ADC comprising a cell-surface molecule binding sdAb or an AOC comprising a cell-surface molecule binding sdAb, according to the invention.

One major drawback of targeted toxin enhancement by free glycosides not part of a cell-surface molecule targeting conjugate, such as for instance saponins of the invention, up to the present invention is that the targeted toxins are internalized by receptor-mediated endocytosis (binding of targeted toxins to the receptor is followed by internalization of the targeted toxin conjugate/receptor complex) while glycosides passively diffuse through the plasma membrane and reach the endosomal membranes presumably via interaction with cholesterol. In principal, glycosides such as the saponins in the second conjugates of the invention, when in free non-conjugated form, can enter any cell, also non-target cells (off-target cells), resulting in inefficient enhancer availability in the target cells for effective release of the targeted toxin and possible side effects in non-target cells. One major problem is that entry of the targeted toxin and the glycosides proceed with different kinetics and that these kinetics are different from cell (line) to cell (line) and from tissue to tissue, so that the correct time difference for the application of the two substances (e.g. ADC or AOC comprising an sdAb for binding to a cell-surface molecule, with a free saponin) can widely vary from tumor (cell (line)) to tumor (cell (line)). Moreover, in living organisms, liberation, absorption, distribution, metabolism and excretion of these substances is also different. Furthermore, the a-specific uptake of glycosides by non-targeted cells may induce unwanted effects in these cells. This can, e.g., be cytosolic delivery of compounds that should have been delivered to the lysosomes, disturbed antigen presentation, etc. Non-targeted administration of the glycoside and the targeted drug may also be problematic in drug development and may hinder or at least postpone marketing authorization by the relevant authorities (e.g. FDA or EMA). With targeted toxin or targeted drug in the context of the present invention is meant that a toxin or drug is specifically targeted to a membrane bound molecule on a target cell (cell-surface molecule), e.g. a toxin or drug bound to an antibody such as an sdAb that specifically recognizes a structure (binding site, epitope) on the cell membrane of a target cell.

It is thus very useful to direct the glycoside (a saponin of the invention) via the same route as the effector molecule, e.g., via a targeting ligand to the target cell in order for the enhancer to be available at effective concentration inside the acidic compartments of the endocytic pathway of the target cell and in order to exhibit a synergistic effect with the toxin. The present invention, therefore, provides novel approaches to redirect both the effector molecule and the endosomal escape enhancer (i.e. a saponin of the invention) via a targeting ligand (binding molecule) to the acidic compartments of the endocytic pathway of the target cell. The targeting ligand comprising the saponin in the second conjugate of the invention can be the same as, or can be different from the targeting ligand, i.e. the sdAb, comprised by the first conjugate comprising the effector molecule of the invention. When the targeting ligands are different, it is appreciated that both targeting ligands bind to a first and second cell-surface molecule that are different but that are present on the same target cell such as a tumor cell. For example, the second conjugate of the invention, comprising a saponin moiety, can bind to EGFR or CD71 or HER2, and for example the first conjugate comprising an effector moiety and a cell-surface molecule binding sdAb can bind to EGFR or CD71 or HER2, wherein the first conjugate of the invention and the second conjugate bind to the same cell receptor or to a different cell receptor.

The inventors established that an effector molecule which is part of the first conjugate comprising a targeting ligand such as an sdAb is delivered inside a cell with high efficiency under influence of a saponin which is comprised by the second conjugate of the invention, when the effect of the effector molecule inside the cell is considered. Surprisingly, despite the relative small size of an sdAb such as a V_{HH}, binding of the first conjugate of the invention comprising the effector molecule and comprising such sdAb, to the target cell surface receptor is still occurring when an effector molecule and an sdAb are comprised by the first conjugate, the sdAb preferably being a V_{HH}. Surprisingly, despite the relative small size of an sdAb such as a V_{HH}, binding of the second conjugate of the invention comprising the saponin and comprising an sdAb, to the target cell surface receptor is still occurring when a saponin and an sdAb are comprised by the second conjugate, the sdAb preferably being a V_{HH}. The binding of an effector molecule to the sdAb such as a V_{HH}, forming the first conjugate of the invention, does not result in e.g. steric hindrance when the capacity of the V_{HH} to bind to the cell surface molecule is considered. The binding of a saponin to the sdAb such as a V_{HH}, forming the second conjugate of the invention, does not result in e.g. steric hindrance when the capacity of the V_{HH} to bind to the cell surface molecule is considered. That is to say, contacting e.g. tumor cells with a sub-optimal dose of e.g. an ADC comprising the sdAb or an AOC comprising the sdAb (i.e. the first conjugate of the invention) does not result in intracellular effector molecule activity (the target cell is not efficiently killed upon biological activity of the effector molecule), in the absence of the second conjugate of the invention comprising the saponin covalently coupled to a cell-surface molecule binding ligand such as an antibody such as the sdAb according to the second conjugate of the invention. However, when the target tumor cell is contacted with the first conjugate of the invention comprising the effector molecule and an sdAb, e.g. an ADC or an AOC, together with free saponin and/or with the second conjugate of the invention, comprising saponin, efficient tumor cell killing is achieved.

By targeting a single cell-surface molecule with the second conjugate of the invention and an ADC or an AOC, i.e. the first conjugate of the invention, or by targeting a second cell-surface molecule with the second conjugate of the invention and targeting a first cell-surface molecule with an ADC or an AOC, i.e. the first conjugate of the invention, wherein the first and second cell-surface molecules are different and are present at the same target cell, the delivery of the saponin bound to the cell-surface molecule targeting antibody such as an sdAb in the second conjugate of the invention, and the delivery of the ADC or AOC comprising the effector molecule and the sdAb for binding to the first cell-surface molecule, at and inside the cytosol of the targeted cell, exposing the cell-surface molecule(s) on the cell surface, is improved and more specific, compared to for example contacting the cell with only an ADC without contacting the cell simultaneously with the second conjugate comprising the saponin of the invention, thus without the presence of the cell-targeted saponin (second conjugate of the invention). An aberrant cell selected for targeting by the cell-surface molecule targeting sdAb of the first conjugate and for targeting by the cell-surface molecule binding molecule comprised by the second conjugate such as an antibody such as an sdAb, ideally bears the epitope on the cell-surface molecule to which the cell-surface molecule targeting molecule can bind, to a high extent (i.e. relatively higher expression of the targeted cell-surface molecule on the targeted cell such as for example a tumor cell or an auto-immune cell, than the expression on a non-targeted cell such as for example a healthy cell) and/or expose the epitope in the targeted cell-surface molecule for binding of the cell-surface molecule targeting sdAb of the first conjugate and for binding of the cell-surface molecule targeting antibody such as an sdAb of the second conjugate, specifically, when (neighboring) healthy cells in a patient are considered. Preferably, the cell-surface molecule targeted by the cell-surface molecule targeting sdAb of the first conjugate of the invention and the cell-surface molecule targeted by the cell-surface molecule targeting molecule such as an antibody such as an sdAb of the second conjugate of the invention, is relatively highly and/or specifically expressed on the targeted (diseased, tumor) cell compared to healthy cells. An embodiment is the first conjugate of the invention, wherein the target cell-surface molecule for the cell-surface molecule targeting sdAb of the first conjugate such as a tumor-cell receptor, is expressed specifically or to a relatively higher extent when compared to expression of the cell-surface molecule on the surface of a healthy (neighboring) cell. An embodiment is the second conjugate of the invention, wherein the target cell-surface molecule for the cell-surface molecule targeting molecule such as an antibody such as an sdAb of the second conjugate such as a tumor-cell receptor, is expressed specifically or to a relatively higher extent when compared to expression of the cell-surface molecule on the surface of a healthy (neighboring) cell. Thus, the epitope on the targeted cell-surface molecule is ideally unique to the targeted diseased cells, and is at least specifically present and exposed at the surface of the targeted cells. Binding of the first conjugate of the invention to the epitope on the cell-surface molecule on a targeted cell is followed by endocytosis of the complex of the conjugate and the target cell-surface molecule (binding of conjugates to the receptor (the target cell-surface molecule) is followed by internalization of the conjugate/receptor complex). Binding of the second conjugate of the invention to the epitope on the cell-surface molecule on a targeted cell is followed by endocytosis of the complex of the second conjugate and the target cell-surface molecule (binding of conjugates to the target cell-surface molecule (cell-surface receptor) is followed by internalization of the conjugate/receptor complex). Since the first and second conjugate only can enter the target cell through binding interaction with a cell-surface molecules specifically expressed to a sufficient extent or uniquely expressed on the targeted cell when compared to healthy cells that should not be targeted, accumulation of a therapeutically active amount of effector moiety comprised by an ADC or an AOC (first conjugate of the invention) which targets the same cell-surface molecule as the second conjugate of the invention or which targets a different cell-surface molecule though present at the same target cell as the cell-surface molecule targeted by the second conjugate of the invention, and saponin comprised by the second conjugate of the invention, inside the target cells is only possible and occurring if expression levels of the targeted cell-surface molecule(s) is above a certain minimal expression threshold. At the same time, the fact that the effector moiety bound to a cell-surface molecule targeting sdAb, of the first conjugate is only capable of exerting its intracellular (e.g. cytotoxic or gene silencing) activity in the presence of the second conjugate bearing the covalently bound saponin of the invention, also provides a safeguard against negative and undesired side effects of the effector moiety towards e.g. healthy cells and healthy tissue not meant to be targeted and affected by the effector moiety, when compared to exposure of cells to an ADC without the presence of the second conjugate of the invention comprising the covalently bound saponin(s). That is to say, sufficiently low expression or even absence of exposed cell-surface molecule(s), to which a first conjugate of the invention such as an ADC or an AOC comprising the sdAb, and a second conjugate comprising saponin could bind, does ideally not allow entrance into (non-targeted) healthy cells of the first conjugate of the invention comprising the effector molecule and the second conjugate comprising the saponin, to amounts that would result in endosomal escape of the effector moiety of the ADC or the AOC under influence of the saponin comprised by the second conjugate of the invention. Since the ADC or the AOC can be used at lower dose in the presence of the saponin comprising second conjugate of the invention, compared to when the ADC or AOC was applied in the therapeutic regimen in the absence of the second conjugate of the invention thus in the absence of saponin, entrance of ADC or entrance of AOC in healthy cells to low extent already bears a lower risk for occurrence of unwanted side effects when for example the targeting and killing of target diseased cells such as tumor cells and auto-immune cells is considered.

Inclusion of an sdAb in the first conjugate has many advantages compared to inclusion of an antibody such as an IgG (full-length immunoglobulin), or of a binding fragment or binding domain thereof. Importantly, since sdAbs do not comprise the Fc tail present in IgGs, risk for off-target side effects due to binding of the conjugate to Fc receptors on cells such as endothelial cells of a host to whom the conjugate is administered, is absent when the first conjugate of the invention is administered to a mammal such as a human subject. Thus, the risk profile of the first conjugate of the invention is improved compared to IgG-based ADCs and AOCs, or compared to ADCs or AOCs comprising an Fc tail. In addition, since the first conjugate of the invention cannot be bound by Fc receptors, the first conjugate is already effective at a dose which is lower than the dose required for reaching the same effector molecule activity with full-length antibody-based ADCs and AOCs, due to less or no undesired capturing of the first conjugate by cell-surface receptors, different from the aimed target first cell-surface molecule. Furthermore, due to the relatively small size of sdAbs compared to e.g. Fab, scFv, IgG, tissue penetration of the first conjugate of the invention is improved, which is beneficial for reaching the target cells once the first conjugate is administered to a patient in need of therapy based on the biological activity of the effector molecule inside (tumor) cells of such a patient. All these advantages of the application of an sdAb in the first conjugate of the invention, when compared to the application of larger antibodies or fragments thereof, such as IgGs comprising an Fc tail, in similar ADCs or AOCs, result in an improved therapeutic window for the effector molecule, when comprised by the first conjugate of the invention. Improved target cell killing in case of a targeted tumor cell when the effector molecule is for example a toxin, at the same dose at which an ADC based on an IgG and comprising the same effector molecule, is not or only sub-optimally effective, for example. Due to the aspects of the invention, it is now possible to treat patients with a lower dose of effector molecule as part of a conjugate comprising the sdAb, i.e. the first conjugate of the invention, therewith reaching the same or improved effector molecule mediated effect in the target cells, compared to a higher dose required when an antibody-based ADC or AOC would be used, which comprises the same effector molecule. Administering such first conjugate of the invention at lower dose lowers the risk for the patient for occurrence of side effects, e.g. by non-specific entrance of non-targeted, healthy cells. This is for example important when the first cell-surface molecule that is targeted by the sdAb comprised by the first conjugate is expressed to a higher extent on target (tumor) cells, but is not uniquely expressed on such target cells. A lower dose of the first conjugate lowers the risk for binding of the first conjugate to such low expressors, such as non-tumor healthy cells.

The inventors also found that the therapeutic window of the first conjugate of the invention is widened when the first conjugate is contacted with target cells in the presence of a saponin or more in particular the second conjugate of the invention which comprises a saponin. When target cells that bear the first cell-surface molecule, i.e. the target for the sdAb comprised by the first conjugate, and that bear the second cell-surface molecule (which is intrinsically true when both the first and the second cell-surface molecule are the same cell-surface molecule, according to embodiments of the invention), i.e. the target for the binding molecule comprised by the second conjugate, are contacted with both the first conjugate and the second conjugate, an effective dose of the effector molecule is lower than when the target cells are contacted with the first conjugate in the absence of the second conjugate (or in the absence of free saponin). The presence of the second conjugate comprising saponin or the presence of free saponin potentiates the activity of the effector molecule in the target cells, such that the therapeutic window of the first conjugate, and therewith the therapeutic window of the effector molecule is widened. Sufficient effector molecule efficiency is achieved at lower dose when target cells are contacted with both the first and second conjugate of the invention. The similar effect is found by the inventors when the first conjugate is contacted with the target cells in the presence of saponin or a functional derivative thereof, when effector-molecule potentiating activity of the saponin is considered, however, at a 100-fold to 1000-fold higher concentration of the free saponin (derivative) compared to the effective dose established when the second conjugate comprising covalently bound saponin is combined with the first conjugate. Thus, providing the saponin or the derivative thereof with a binding molecule (i.e. the second conjugate of the invention) results in an improved effector-molecule activity potentiating effect, when the first and second conjugate are contacted together with the target cell that expresses both cell-surface molecules on its surface. Targeted saponin is already effective at lower dose than free saponin, in delivery of the effector molecule inside the target cell, and in delivery from the endosome, endolysosome or lysosome of said cell into the cytosol, where the effector molecule **should bind** its target binding partner and should exerts its biological activity (e.g. cell killing in case of the target cell being a tumor cell and the effector molecule being e.g. a toxin).

In combination, the inventors provide a combination of a first pharmaceutical composition comprising the first conjugate and a second pharmaceutical composition comprising the free saponin (derivative) or the second conjugate comprising the saponin (derivative), and provide a pharmaceutical composition comprising the first conjugate and the free saponin (derivative) or the second conjugate, which have improved therapeutic window, less risk for inducing side effects when an effective dose of the effector molecule comprised by the first conjugate is administered to a patient in need of effector molecule based therapy, and improved effector molecule activity due to improved delivery of the first conjugate inside target cells, more specifically improved effector molecule activity due to improved delivery of the effector molecule inside the cytosol of such target cells, when compared to current ADCs or AOCs based on full-length antibodies or Fc comprising constructs thereof. It is part of the invention that such first conjugates of the invention are administered to patients in need of effector molecule based therapy together with the free saponin (derivative) or the second conjugate, although the application of the second conjugate in combination with the first conjugate is preferred.

An embodiment is the pharmaceutical combination of the invention in the form of at least two, preferably two, pharmaceutical compositions comprising:
- a first pharmaceutical composition comprising the first conjugate and optionally comprising a pharmaceutically acceptable excipient and/or pharmaceutically acceptable diluent; and
- a second pharmaceutical composition comprising the saponin and/or the derivative thereof and/or the second conjugate, and optionally comprising a pharmaceutically acceptable excipient and/or pharmaceutically acceptable diluent.

An embodiment is the pharmaceutical combination of the invention in the form of a single pharmaceutical composition comprising the first conjugate and at least one, preferably one, of:
- the saponin;
- the saponin derivative; and
- the second conjugate,
and optionally comprising a pharmaceutically acceptable excipient and/or pharmaceutically acceptable diluent.

An aspect of the invention relates to a pharmaceutical composition comprising:
∘ the first conjugate comprising at least one effector molecule and a single-domain antibody (sdAb) for binding to a first cell-surface molecule, which are covalently linked to each other, directly or via a linker;
∘ the saponin and/or the derivative thereof and/or the second conjugate comprising the binding molecule for binding to the second cell-surface molecule and the saponin and/or the derivative thereof, which binding molecule and the saponin or the derivative thereof are covalently linked to each other, directly or via a linker, wherein the saponin or the derivative thereof is a monodesmosidic triterpene glycoside or a bidesmosidic triterpene glycoside, wherein the second cell-surface molecule is the same as or different from the first cell-surface molecule; and optionally
o a pharmaceutically acceptable excipient and/or pharmaceutically acceptable diluent.

An embodiment is the pharmaceutical combination or the pharmaceutical composition of the invention, wherein the first conjugate comprises an sdAb which is any one or more of: a V_{H} domain derived from a heavy chain of an antibody, preferably of immunoglobulin G origin, preferably of human origin; a V_{L} domain derived from a light chain of an antibody, preferably of immunoglobulin G origin, preferably of human origin; a V_{HH} domain such as derived from a heavy-chain only antibody (HCAb) such as from *Camelidae* origin or *Ig-NAR* origin such as a variable heavy chain new antigen receptor (V_{NAR}) domain, preferably the HCAb is from *Camelidae* origin; and preferably the sdAb is a V_{HH} domain derived from an HCAb from *Camelidae* origin (camelid V_{H}) such as derived from an HCAb from camel, lama, alpaca, dromedary, vicuna, guanaco and Bactrian camel.

In particular, V_{HH} domains are suitable for application in the first conjugate of the invention, and in fact also for application in the second conjugate of the invention. Such V_{HH} domains are commonly renown for their high stability, i.e. resistance to unfolding, for their capability to bind to a binding partner without the requirement of the presence of a second V domain, such as present in e.g. IgG and required for the IgG to bind to its binding partner via the two V domains, for their ease of generation by techniques known in the art (camelid immunization, phage display techniques, etc.), for their capability of penetrating tissue to a higher extent than seen for full-length IgGs, which is beneficial when target (tumor) cells are located inside or as part of such (organ) tissue. Additionally, such V_{HH} domains lack an Fc tail common to immunoglobulin G type of antibodies, such that Fc-receptor mediated side effects of administering a conjugate comprising an sdAb such as a V_{HH} domain cannot occur, which side effects may occur in subjects to whom a therapeutic full-length immunoglobulin comprising the Fc tail is administered.

An embodiment is the pharmaceutical combination or the pharmaceutical composition of the invention, wherein the first conjugate comprises at least two sdAbs with a single first sdAb covalently bound to the at least one effector molecule, or with two or more sdAbs bound to the at least one effector molecule, or with all of the at least two sdAbs bound to the at least one effector molecule, for example wherein each of the at least two sdAbs is bound to one or more of the at least one effector molecule.

An embodiment is the pharmaceutical combination or the pharmaceutical composition of the invention, wherein the sdAb comprises at least two sdAbs, which are the same sdAbs, preferably two - eight sdAbs, more preferably two - four sdAbs.

An embodiment is the pharmaceutical combination or the pharmaceutical composition of the invention, comprising one - eight sdAbs, capable of binding to a same binding site on the first cell-surface molecule, wherein the at least one effector molecule is bound to a single first sdAb of the one - eight sdAbs or wherein the at least one effector molecule is bound to two or more of the sdAbs, if present, and/or wherein each of the at least one effector molecule is bound to a single sdAb separately such that one or more of the sdAbs is/are each bound to a single effector molecule.

Providing a first conjugate of the invention (and/or a second conjugate of the invention) which comprises a (linear) string of multiple sdAbs covalently linked to each other, can provide the benefit of the capacity of the conjugate to bind with higher avidity to the target cell, which can result in improved uptake (endocytosis) of the conjugate by the target cell.

Synchronization is the missing link between a successful delivery strategy for mice and its application in humans, when the application of the endosomal escape enhancing effect of saponin towards effector molecules is considered. Indeed, the inventors established in a series of in vivo mouse tumor models that separately administering to the mice a dose of free saponin and a dose of e.g. ADC without the presence of a second conjugate comprising saponin according to the invention, did not result in any desired anti-tumor activity such as delayed tumor growth, tumor regression, diminished and slower tumor growth, compared to control animals not treated with the ADC in the presence of free saponin. See also the Figures 18-21 for in vivo tumor models and the Examples described in the Examples section, here below. The free saponin was administered using various routes of administration and using various time points of administering the free saponin compared to the moment of administering the ADC (administering free saponin before, during and after administering the ADC). The ADC tested in in vivo tumor models was cetuximab-dianthin (with free SO1861), or trastuzumab-saporin (with free SO1861). Varying the dose of free saponin did not provide for an efficacious anti-tumor activity. The ADCs referred to were administered at a dose that in itself did not inflict any beneficial anti-tumor effect on the tumor-bearing animals. Surprisingly, the inventors established that beneficial anti-tumor activity in various in vitro mammalian cell-based bioassays using human tumor cells and/or in various in vivo animal tumor models can be achieved by treating the cells or animals with the second conjugates according to the invention in combination with an ADC or an AOC, i.e. the first conjugate of the invention.

The second conjugates of the invention optionally comprising a scaffold according to the invention (see below; a scaffold such as a covalent saponin conjugate comprising an oligomeric or polymeric structure with one or multiple saponin moieties covalently bound thereto). The scaffold for example being a tri-functional linker with a covalently bound saponin (e.g. SO1861, QS-21) via a cleavable or non-cleavable linkage, the scaffold linked with a covalent bond to the cell-surface molecule targeting molecule of the second conjugate such as a monoclonal antibody such as cetuximab, trastuzumab, OKT-9, but preferably an sdAb such as a V_{HH}, or the scaffold being a dendron, such as a dendron, for example G4-dendron or G5-dendron, to which for example four moieties can bind such as four saponin molecules, or a dendron for binding for example eight saponins, the dendron comprising a chemical group for (covalent) coupling to the cell-surface molecule targeting antibody such as an sdAb, of the second conjugate. Reference is made to the further embodiments and the Examples section, exemplifying several of these scaffolds according to the invention, showing in vivo and/or in vitro anti-tumor cell activity when cell toxicity exerted by e.g. a proteinaceous toxin comprised by the first conjugate of the invention is considered or when gene silencing in the tumor cell is considered.

Without wishing to be bound by any theory, in view of the failures observed when treatment of tumor-bearing animals with an ADC together with free saponin is considered, it is preferred to synchronize the presence of both, the at least one saponin, and the effector moiety, preferably a toxin or an oligonucleotide, in compartments or vesicles of the endocytic pathway of the target cell, e.g. a tumor cell or an auto-immune cell. With ADC and free saponin, synchronizing the presence of the molecules in the late endosomes, in order to obtain the synergistic effects *in vivo* was not beneficially obtainable according to attempts of the inventors. In one aspect, the invention preferably solves at least the following problem with respect to combining a first conjugate comprising the effector moiety and a (tumor) cell surface molecule binding sdAb (e.g. an ADC or an AOC) with a second conjugate of the invention comprising the saponin(s): without wishing to be bound by any theory the only reasonable chemical group within, e.g., the saponins that can be used for (covalent), in particular single and cleavable, retainable coupling is required for the endosomal escape activity. Known restrictions are most likely the reason why saponins have not been used in combination with pharmaceutically active substances in clinical investigations other than the application of saponins in vaccination regimes wherein the use of an immune-potentiating adjuvant substance was implied, although the striking endosomal escape enhancer effect of, e.g., saponins of the invention and exemplified herein, is known for more than 10 years. For example providing a second conjugate of the invention with a covalently bound saponin, for example in the context of a scaffold carrying several saponins, solves these difficulties, at least in part. For example providing a second conjugate of the invention with a covalently bound saponin via a cleavable bond that is cleaved under influence of the slightly acidic pH (pH < 6,5) as apparent in the endosome, endolysosome and lysosome of target cells, such that free saponin cleaved off from the second conjugate is present inside said endosome, endolysosome or lysosome once the second conjugate is endocytosed and delivered inside the cell, solves these difficulties, at least in part. Surprisingly, the saponins previously applied for their immune-potentiating activity in the vaccination context involving saponins as adjuvant component, are now also suitably for (covalent) coupling to the cell-surface molecule targeting antibody, such as an sdAb, comprised by the second conjugate of the invention, for anti-tumor activity in vitro and in vivo when used in combination with an ADC or an AOC comprising an sdAb, i.e. the first conjugate of the invention.

An embodiment is the pharmaceutical combination or the pharmaceutical composition of the invention, wherein the sdAb is a single sdAb or are at least two, preferably two sdAbs, wherein the sdAb(s) can bind to the first cell-surface molecule such as HIVgp41 or which first cell-surface molecule is a tumor-cell surface receptor such as a tumor-cell specific receptor, more preferably a receptor selected from: CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, prostate specific membrane antigen (PSMA), CanAg, integrin-alphaV, CA6, CD33, mesothelin, Cripto, CD3, CD30, CD239, CD70, CD123, CD352, DLL3, CD25, ephrinA4, MUC-1, Trop2, CEACAM5, CEACAM6, HER3, CD74, PTK7, Notch3, FGF2, C4.4A, FLT3, CD38, FGFR3, CD7, PD-L1, CTLA-4, CD52, PDGFRA, VEGFR1, VEGFR2, c-Met (HGFR), EGFR1, RANKL, ADAMTS5, CD16, CXCR7 (ACKR3), glucocorticoid-induced TNFR-related protein (GITR), most preferably selected from: HER2, c-Met, VEGFR2, CXCR7, CD71 and EGFR1.

It is part of the invention that the sdAb comprised by the first conjugate of the invention has binding specificity for a cell-surface molecule that is specifically expressed on the target cell. 'Specifically expressed' should here be understood as the unique expression of the cell-surface molecule on the target cell only, wherein e.g. healthy cells that should not bind the first conjugate, are not targeted, or should here be understood as the upregulated or relatively high expression of the target cell-surface molecule on the target cells, compared to lower expression of the cell-surface molecule on e.g. healthy cells that should not or at least to a much lower extent, bind the conjugate. These listed cell receptors are such cell-surface molecules that are sufficiently specific for the cells that are the target of the first conjugate, and are therewith preferred candidates for binding by the first conjugate. It will be appreciated that the higher the specificity of a certain cell-surface molecule, when expression of the cell-surface molecule on the target cell is compared to the expression on other cells not meant to be targeted by the first conjugate of the invention, the better the therapeutic window is, when the activity of the effector molecule inside the cells is considered. For example, suitable targets for targeting by the first conjugate are amongst other tumor cell specific receptors, HER2, EGFR, such as EGFR1, and CD71.

An embodiment is the pharmaceutical combination or the pharmaceutical composition of the invention, wherein the sdAb comprised by the first conjugate is a single sdAb or are at least two, preferably two sdAbs, wherein the sdAb(s) is/are selected from: an anti-CD71 sdAb, an anti-HER2 sdAb, an anti-CD20 sdAb, an anti-CA125 sdAb, an anti-EpCAM (17-1A) sdAb, an anti-EGFR sdAb, an anti-CD30 sdAb, an anti-CD33 sdAb, an anti-vascular integrin alpha-v beta-3 sdAb, an anti-CD52 sdAb, an anti-CD22 sdAb, an anti-CEA sdAb, an anti-CD44v6 sdAb, an anti-FAP sdAb, an anti-CD19 sdAb, an anti-CanAg sdAb, an anti-CD56 sdAb, an anti-CD38 sdAb, an anti-CA6 sdAb, an anti-IGF-1R sdAb, an anti-integrin sdAb, an anti-syndecan-1 sdAb, an anti-CD79b, an anti-c-Met sdAb, an anti-EGFR1 sdAb, an anti-VEGFR2 sdAb, an anti-CXCR7 sdAb and an anti-HIVgp41, wherein the sdAb(s) is/are preferably V_{HH}(s), more preferably camelid V_{H}.

An embodiment is the pharmaceutical combination or the pharmaceutical composition of the invention, wherein the first conjugate comprises an sdAb that can bind to HER2, CD71, HIVgp41 and/or EGFR, wherein the sdAb is preferably a V_{HH}, more preferably a camelid V_{H}.

An embodiment is the pharmaceutical combination or the pharmaceutical composition of the invention, wherein the first conjugate comprises an sdAb for binding to HER2 selected from: sdAb produced by clone 11A4, clone 18C3, clone 22G12, clone Q17 or clone Q17-C-tag; or an sdAb for binding to EGFR and produced by clone anti-EGFR Q86-C-tag; or an sdAb for binding to CD71 and produced by clone anti-CD71 Q52-C-tag; or an sdAb for binding to HIVgp41 and produced by clone anti-HIVgp41 Q8-C-tag; or an sdAb encoded by a cDNA of any one of the SEQ ID NO: 1, 3, 5, 7, 9, 11, 13, 15, 17, 19, 21, 23, 25, 27, 29 and 31; or any one of the sdAbs with an amino-acid sequence of SEQ ID NO: 2, 4, 6, 8, 10, 12, 14, 16, 18, 20, 22, 24, 26, 28, 30, 32, 36-72, wherein optionally the first conjugate further comprises a covalently bound sdAb for binding to albumin, such as any one or more of the sdAbs with an amino-acid sequence of SEQ ID NO: 33, 34 and 35, preferably the sdAb is a V_{HH}, more preferably a camelid V_{H}. V_{HH}s suitable for incorporation in the first conjugate of the invention are for example found in the single domain antibody database (Wilton, E.E. et al. (2018)), in patent applications US20160251440 (anti-CD123, anti-CEACAM), US9683045 (anti-c-Met), US20090252681 (anti-EGFR, anti-IGF-1R), US9969805 (anti-HER2), US20190023796A1 (anti-HER3), and in Kijanka et al. (2013), for anti-HER2 and in Mercier et al. (2019) for anti-HER2. The amino-acid sequences and/or the cDNA sequences of a series of suitable V_{HH}s is also provided here below for anti-HER2, anti-HER3, anti-CD123, anti-CEACAM, anti-c-Met, anti-EGFR, anti-IGF-1R, anti-PD-L1, anti-CTLA-4, anti-CD19, anti-HER1 and anti-VGFR2, as SEQ ID NOs 1-32 and 36-72, in view of their ability to bind to tumor-cell specific receptors. In particular, a V_{HH} capable of binding to a binding site on any of the tumor-cell specific receptors HER2, VEGFR and CD71 is suitable for incorporation in the first conjugate of the invention. The inventors revealed that an ADC comprising a V_{HH} that targets any one of such receptors is effective in delivery of the effector molecule bound to the sdAb. See for example the Examples section, and Figures 4-7.

An embodiment is the pharmaceutical combination or the pharmaceutical composition of the invention, wherein the effector molecule comprises or consists of at least one of a small molecule such as a drug molecule, a toxin such as a protein toxin, an oligonucleotide such as a BNA, a xeno nucleic acid or an siRNA, an enzyme, a peptide, a protein, or any combination thereof.

An embodiment is the pharmaceutical combination or the pharmaceutical composition of the invention, wherein the effector molecule is selected from any one or more of a vector, a gene, a cell suicide inducing transgene, deoxyribonucleic acid (DNA), ribonucleic acid (RNA), anti-sense oligonucleotide (ASO, AON), short interfering RNA (siRNA), anti-microRNA (anti-miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), www.biosyn.com/literaturevault/superior-silencing-by-2-4-bnanc-based-short-antisense-oligonucleotides.aspx, 3'-fluoro hexitol nucleic acid (FHNA), a plasmid, glycol nucleic acid (GNA) and threose nucleic acid (TNA), or a derivative thereof, more preferably a BNA, for example a BNA for silencing HSP27 protein expression or a BNA for silencing apolipoprotein B expression.

An embodiment is the pharmaceutical combination or the pharmaceutical composition of the invention, wherein the effector molecule is an oligonucleotide selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), mRNA, DNA, antisense DNA, locked nucleic acid (LNA), bridged nucleic acid (BNA), 2'-O,4'-aminoethylene bridged nucleic Acid (BNA^{NC}), BNA-based siRNA, and BNA-based antisense oligonucleotide (BNA-AON).

An embodiment is the pharmaceutical combination or the pharmaceutical composition of the invention, wherein the effector molecule is an oligonucleotide selected from any one of an anti-miRNA, a BNA-AON or an siRNA, such as BNA-based siRNA, preferably selected from chemically modified siRNA, metabolically stable siRNA and chemically modified, metabolically stable siRNA.

An embodiment is the pharmaceutical combination or the pharmaceutical composition of the invention, wherein the effector molecule is an oligonucleotide that capable of silencing a gene, when present in a cell comprising such gene, wherein the gene is any one of genes: apolipoprotein B (apoB), transthyretin (TTR), proprotein convertase subtilisin/kexin type 9 (PCSK9), delta-aminolevulinate synthase 1 (ALAS1), antithrombin 3 (AT3), glycolate oxidase (GO), complement component C5 (CC5), X gene of hepatitis B virus (HBV), S gene of HBV, alpha-1 antitrypsin (AAT) and lactate dehydrogenase (LDH), and/or is capable of targeting an aberrant miRNA when present in a cell comprising such miRNA.

An embodiment is the pharmaceutical combination or the pharmaceutical composition of the invention, wherein the effector molecule is an oligonucleotide that is capable of targeting an mRNA, when present in a cell comprising such mRNA, wherein the mRNA is involved in expression of any one of proteins: apoB, TTR, PCSK9, ALAS1, AT3, GO, CC5, expression product of X gene of HBV, expression product of S gene of HBV, AAT and LDH, or is capable of antagonizing or restoring an miRNA function such as inhibiting an oncogenic miRNA (onco-miR) or suppression of expression of an onco-miR, when present in a cell comprising such an miRNA.

An embodiment is the pharmaceutical combination or the pharmaceutical composition of the invention, wherein the effector molecule comprises or consists of at least one proteinaceous molecule, preferably selected from any one or more of a peptide, a protein, an enzyme and a protein toxin. The inventors found that very effective tumor cell killing is achieved when sdAbs are selected that bind any of HER2, VEGFR, CD71, which sdAb is combined in the first conjugate with a toxin such as a protein toxin, such as dianthin or saporin. Examples demonstrating the high efficacy of such first conjugates of the invention are provided in the Examples section and are displayed in Figures 4-7.

An embodiment is the pharmaceutical combination or the pharmaceutical composition of the invention, wherein the effector molecule comprises or consists of at least one of: urease and Cre-recombinase, a proteinaceous toxin, a ribosome-inactivating protein, a protein toxin, a bacterial toxin, a plant toxin, more preferably selected from any one or more of a viral toxin such as apoptin; a bacterial toxin such as Shiga toxin, Shiga-like toxin, Pseudomonas aeruginosa exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or human angiogenin, or any toxic fragment or toxic derivative thereof; preferably the protein toxin is dianthin and/or saporin.

An embodiment is the pharmaceutical combination or the pharmaceutical composition of the invention, wherein the effector molecule comprises or consists of at least one payload.

An embodiment is the pharmaceutical combination or the pharmaceutical composition of the invention, wherein the effector molecule comprises or consists of at least one of: a toxin targeting ribosomes, a toxin targeting elongation factors, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-γ-calicheamicin, a pyrrolobenzodiazepine (PBD) dimer, a benzodiazepine, a CC-1065 analogue, a duocarmycin, Doxorubicin, paclitaxel, docetaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, α-amanitin, a spliceostatin, a thailanstatin, ozogamicin, tesirine, Amberstatin269 and soravtansine, or a derivative thereof.

An embodiment is the pharmaceutical combination or the pharmaceutical composition of the invention, wherein the first conjugate comprises or consists of an antibody-drug conjugate (ADC), such as an ADCs comprising at least one sdAb and a toxin derived from any one of ADCs: gemtuzumab ozogamicin, brentuximab vedotin, trastuzumab emtansine, inotuzumab ozogamicin, moxetumomab pasudotox and polatuzumab vedotin. It will be appreciated when a sdAb is derived from such a human antibody, the V_{H} domain of such a human antibody may require some improvements with regard to domain stability ('camelization' of the human V_{H} domain), known in the art.

An effector moiety useful as part of an ADC or an AOC in the present invention (i.e. the first conjugate of the invention) preferably relies on late endosomal escape for exerting its effect. Some effector molecules, such as, e.g., a pseudomonas exotoxin, are rerouted to other organelles prior to the "late endosomal stage" and, thus, would normally not benefit from incorporation in an ADC for combining with the second conjugate according to the present invention. However, such toxin may be adapted for use with the present invention, e.g., by deleting the signal peptide responsible for rerouting. In particular toxins that are highly toxic and would require only one molecule to escape the endosomes to kill a cell maybe modified to be less potent. It is preferred to use a toxin that kills a cell if at least 2, more preferably at least 5, more preferably at least 10, more preferably at least 20, more preferably at least 50, most preferably at least 100 toxin molecules escape the endosome (and enter the cytosol). It is further preferred that a first conjugate of the invention comprised by the pharmaceutical composition or comprised by the pharmaceutical combination of the invention, comprises a covalently conjugated functionalized scaffold, i.e. a scaffold such as an oligomeric or polymeric molecule or a tri-functional linker, comprising covalently bound effector moiety or moieties for targeting the scaffold comprising the bound effector moiety/moieties at a target cell such as a tumor cell or an auto-immune cell. Further, in order to reduce off-target toxicity, cell membrane non-permeable small molecule toxins are preferred effector molecules over cell membrane permeable toxins.

Preferably, the effector moiety comprised by the ADC or the AOC (i.e. the first conjugate of the invention) for combination with the second conjugate of the invention, which effector moiety effect is enhanced by the saponins comprised by the second conjugate of the invention, detaches from the first conjugate (i.e. an ADC, an AOC), e.g. detaches from the antibody, such as an sdAb, present in the first conjugate as the cell-surface molecule targeting moiety of the first conjugate, when endocytosed. This can be achieved by a cleavable bond that breaks, e.g. under acidic, reductive, enzymatic or light-induced conditions.

The inventors show that a tumor-cell targeting monoclonal antibody or an sdAb provided with covalently coupled antisense BNA such as BNA(HSP27) (first conjugate of the invention) and combined with a tumor-cell targeting monoclonal antibody or an sdAb, which is the same or different, and which is provided with covalently coupled saponin (i.e. the second conjugate of the invention), that is contacted with tumor cells, both the BNA coupled to the cell-targeting ligand (i.e. the first conjugate) (via a cleavable bond) and the saponin coupled to the antibody (e.g. sdAb in the second conjugate of the invention) (via a cleavable bond), is capable of silencing HSP27 in vivo in tumors, compared to control and compared to the AOC bearing the BNA in the absence of the second conjugate of the invention comprising the saponin (SO1861). Administering an ADC (first conjugate of the invention) together with a second conjugate comprising saponin of the invention, or administering an antibody-oligonucleotide conjugate such as an antibody-BNA conjugate of the invention wherein the antibody is a V_{HH}, together with a second conjugate comprising saponin of the invention, thus endows the ADC or AOC with anti-tumor cell activity not seen with only the ADC or only the AOC, in the absence of the second conjugate of the invention, at the same dose. The antisense BNA (HSP27) was a BNA with oligonucleic acid sequence according to Zhang et al. (2011) *[*Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333]. Noteworthy, to the best of the knowledge of the inventors, BNA is designed for application as a free nucleic acid. The inventors are now the first to demonstrate that the antisense BNA can be covalently coupled through a (non-)cleavable linker with a ligand or an antibody such as a mAb or an sdAb such as a V_{HH}, in a way that gene-silencing activity is retained in vitro and more importantly in vivo in the tumor cells of a tumor-bearing animal. This approach of providing BNA based AOCs, i.e. a first conjugate of the invention, opens new ways to administer targeted BNA to human (cancer) patients in need thereof.

An embodiment is the pharmaceutical combination or the pharmaceutical composition of the invention, wherein the saponin comprises an aglycone core structure selected from, or the saponin derivative comprises an aglycone core structure derived from:
2alpha-hydroxy oleanolic acid;
16alpha-hydroxy oleanolic acid;
hederagenin (23-hydroxy oleanolic acid);
16alpha,23-dihydroxy oleanolic acid;
gypsogenin;
quillaic acid;
protoaescigenin-21(2-methylbut-2-enoate)-22-acetate;
23-oxo-barringtogenol C-21,22-bis(2-methylbut-2-enoate);
23-oxo-barringtogenol C-21(2-methylbut-2-enoate)-16,22-diacetate;
digitogenin;
3,16,28-trihydroxy oleanan-12-en; and
gypsogenic acid,
preferably, the saponin comprises or the saponin derivative is derived from an aglycone core structure selected from quillaic acid and gypsogenin, more preferably the saponin comprises or the saponin derivative is derived from aglycone core structure quillaic acid.

Without wishing to be bound by any theory, presence of an aldehyde group (or derivative thereof) in the aglycone core structure of the saponin (here, also referred to as 'aglycone') is beneficial for the capacity of the saponin to stimulate and/or potentiate the endosomal escape of the effector molecule comprised by the first conjugate such as an ADC or AOC, according to the invention, when such a saponin co-localizes in a cell, in the endosome of said cell, with these effector molecules, as part of the second conjugate of the invention or when in free form inside the endosome (e.g. split off from the second conjugate once the second conjugate is delivered inside the target cell endosome or lysosome), and when exposed to the cells in the presence of the first conjugate bearing the effector molecule and the cell-targeting sdAb. Therefore, the second conjugates of the invention comprising saponin which has an aglycone with an aldehyde group is preferred. In quillaic acid and in gypsogenin the aldehyde group is at the C₂₃ atom of the aglycone.

An embodiment is the pharmaceutical combination or the pharmaceutical composition of the invention, wherein the saponin or the saponin derivative optionally comprises a first saccharide chain bound to the C₃ atom or the C₂₈ atom of the aglycone core structure of the saponin or saponin derivative, preferably bound to the C₃ atom, and/or wherein the saponin or the saponin derivative optionally comprises a second saccharide chain bound to the C₂₈ atom of the aglycone core structure of the saponin or the saponin derivative, preferably, the at least one saponin comprises the first and second saccharide chain. Thus, when the saponin comprised by the second conjugate of the invention bears two glycans (saccharide chains), the first saccharide chain is bound at position C₃ of the aglycone core structure and the second saccharide chain is bound at position C₂₈ of the aglycone core structure.

An embodiment is the pharmaceutical combination or the pharmaceutical composition of the invention, wherein
- the saponin or the saponin derivative comprises the first saccharide chain selected from:
   GlcA-,
   Glc-,
   Gal-,
   Rha-(1→2)-Ara-,
   Gal-(1→2)-[Xyl-(1→3)]-GlcA-,
   Glc-(1→2)-[Glc-(1→4)]-GlcA-,
   Glc-(1→2)-Ara-(1→3)-[Gal-(1→2)]-GlcA-,
   Xyl-(1→2)-Ara-(1→3)-[Gal-(1→2)]-GlcA-,
   Glc-(1→3)-Gal-(1→2)-[Xyl-(1→3)]-Glc-(1→4)-Gal-,
   Rha-(1→2)-Gal-(1→3)-[Glc-(1→2)]-GlcA-,
   Ara-(1→4)-Rha-(1→2)-Glc-(1→2)-Rha-(1→2)-GlcA-,
   Ara-(1→4)-Fuc-(1→2)-Glc-(1→2)-Rha-(1→2)-GlcA-,
   Ara-(1→4)-Rha-(1→2)-Gal-(1→2)-Rha-(1→2)-GlcA-,
   Ara-(1→4)-Fuc-(1→2)-Gal-(1→2)-Rha-(1→2)-GlcA-,
   Ara-(1→4)-Rha-(1→2)-Glc-(1→2)-Fuc-(1→2)-GlcA-,
   Ara-(1→4)-Fuc-(1→2)-Glc-(1→2)-Fuc-(1→2)-GlcA-,
   Ara-(1→4)-Rha-(1→2)-Gal-(1→2)-Fuc-(1→2)-GlcA-,
   Ara-(1→4)-Fuc-(1→2)-Gal-(1→2)-Fuc-(1→2)-GlcA-,
   Xyl-(1→4)-Rha-(1→2)-Glc-(1→2)-Rha-(1→2)-GlcA-,
   Xyl-(1→4)-Fuc-(1→2)-Glc-(1→2)-Rha-(1→2)-GlcA-,
   Xyl-(1→4)-Rha-(1→2)-Gal-(1→2)-Rha-(1→2)-GlcA-,
   Xyl-(1→4)-Fuc-(1→2)-Gal-(1→2)-Rha-(1→2)-GlcA-,
   Xyl-(1→4)-Rha-(1→2)-Glc-(1→2)-Fuc-(1→2)-GlcA-,
   Xyl-(1→4)-Fuc-(1→2)-Glc-(1→2)-Fuc-(1→2)-GlcA-,
   Xyl-(1→4)-Rha-(1→2)-Gal-(1→2)-Fuc-(1→2)-GlcA-,
   Xyl-(1→4)-Fuc-(1→2)-Gal-(1→2)-Fuc-(1→2)-GlcA-, and
   any derivative thereof,
      and/or
- the saponin or the saponin derivative comprises the second saccharide chain selected from:
   Glc-,
   Gal-,
   Rha-(1→2)-[Xyl-(1→4)]-Rha-,
   Rha-(1→2)-[Ara-(1→3)-Xyl-(1→4)]-Rha-,
   Ara-,
   Xyl-,
   Xyl-(1→4)-Rha-(1→2)-[R1-(→4)]-Fuc- wherein R1 is 4E-Methoxycinnamic acid,
   Xyl-(1→4)-Rha-(1→2)-[R2-(→4)]-Fuc- wherein R2 is 4Z-Methoxycinnamic acid,
   Xyl-(1→4)-[Gal-(1→3)]-Rha-(1→2)-4-OAc-Fuc-,
   Xyl-(1→4)-[Glc-(1→3)]-Rha-(1→2)-3,4-di-OAc-Fuc-,
   Xyl-(1→4)-[Glc-(1→3)]-Rha-(1→2)-[R3-(→4)]-3-OAc-Fuc- wherein R3 is 4E-Methoxycinnamic acid,
   Glc-(1→3)-Xyl-(1→4)-[Glc-(1→3)]-Rha-(1→2)-4-OAc-Fuc-,
   Glc-(1→3)-Xyl-(1→4)-Rha-(1→2)-4-OAc-Fuc-,
   (Ara- or Xyl-)(1→3)-(Ara- or Xyl-)(1→4)-(Rha- or Fuc-)(1→2)-[4-OAc-(Rha- or Fuc-)(1→4)]-(Rha- or Fuc-),
   Xyl-(1→3)-Xyl-(1→4)-Rha-(1→2)-[Qui-(1→4)]-Fuc-,
   Api-(1→3)-Xyl-(1→4)-[Glc-(1→3)]-Rha-(1→2)-Fuc-,
   Xyl-(1→4)-[Gal-(1→3)]-Rha-(1→2)-Fuc-,
   Xyl-(1→4)-[Glc-(1→3)]-Rha-(1→2)-Fuc-,
   Ara/Xyl-(1→4)-Rha/Fuc-(1→4)-[Glc/Gal-(1→2)]-Fuc-,
   Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[Rha-(1→3)]-4-OAc-Fuc-,
   Api-(1→3)-Xyl-(1→4)-[Glc-(1→3)]-Rha-(1→2)-[Rha-(1→3)]-4-OAc-Fuc-,
   6-OAc-Glc-(1→3)-Xyl-(1→4)-Rha-(1→2)-[3-OAc-Rha-(1→3)]-Fuc-,
   Glc-(1→3)-Xyl-(1→4)-Rha-(1→2)-[3-OAc--Rha-(1→3)]-Fuc-,
   Xyl-(1→3)-Xyl-(1→4)-Rha-(1→2)-[Qui-(1→4)]-Fuc-,
   Glc-(1→3)-[Xyl-(1→4)]-Rha-(1→2)-[Qui-(1→4)]-Fuc-,
   Glc-(1→3)-Xyl-(1→4)-Rha-(1→2)-[Xyl-(1→3)-4-OAc-Qui-(1→4)]-Fuc-,
   Xyl-(1→3)-Xyl-(1→4)-Rha-(1→2)-[3,4-di-OAc-Qui-(1→4)]-Fuc-,
   Glc-(1→3)-[Xyl-(1→4)]-Rha-(1→2)-Fuc-,
   6-OAc-Glc-(1→3)-[Xyl-(1→4)]-Rha-(1→2)-Fuc-,
   Glc-(1→3)-[Xyl-(1→3)-Xyl-(1→4)]-Rha-(1→2)-Fuc-,
   Xyl-(1→3)-Xyl-(1→4)-Rha-(1→2)-[Xyl-(1→3)-4-OAc-Qui-(1→4)]-Fuc-,
   Api/Xyl-(1→3)-Xyl-(1→4)-[Glc-(1→3)]-Rha-(1→2)-[Rha-(1→3)]-40Ac-Fuc-,
   Api-(1→3)-Xyl-(1→4)-[Glc-(1→3)]-Rha-(1→2)-[Rha-(1→3)]-40Ac-Fuc-,
   Api/Xyl-(1→3)-Xyl-(1→4)-[Glc-(1→3)]-Rha-(1→2)-[R6-(→4)]-Fuc- wherein R6 is 5-O-[5-O-Rha-(1→2)-Ara/Api-3,5-dihydroxy-6-methyl-octanoyl]-3,5-dihydroxy-6-methyl-octanoic acid),
   Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
   Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
   Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
   Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
   Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid)
   Glc-(1→3)-[Glc-(1→6)]-Gal-, and
   any derivative thereof.

Thus, when the saponin comprised by the second conjugate of the invention bears two glycans (saccharide chains), the first saccharide chain is bound at position C₃ of the aglycone core structure and the second saccharide chain is bound at position C₂₈ of the aglycone core structure.

An embodiment is the pharmaceutical combination or the pharmaceutical composition of the invention, wherein the saponin or the saponin derivative in the second conjugate comprises the first saccharide chain and comprises the second saccharide chain of claim 24 or 25, wherein the first saccharide chain comprises more than one saccharide moiety and the second saccharide chain comprises more than one saccharide moiety, and wherein the aglycone core structure is, or is a derivative of, quillaic acid or gypsogenin, wherein one, two or three, preferably one or two, of:
i. an aldehyde group in the aglycone core structure has been derivatised,
ii. a carboxyl group of a glucuronic acid moiety in the first saccharide chain has been derivatised, and
iii. at least one acetoxy (Me(CO)O-) group in the second saccharide chain has been derivatised.

An embodiment is the pharmaceutical combination or the pharmaceutical composition of the invention, wherein the saponin or the saponin derivative in the second conjugate comprises:
i. an aglycone core structure comprising an aldehyde group which has been derivatised by:
   - reduction to an alcohol;
   - transformation into a hydrazone bond through reaction with N-ε-maleimidocaproic acid hydrazide (EMCH) wherein the maleimide group of the EMCH is optionally derivatised by formation of a thioether bond with mercaptoethanol;
   - transformation into a hydrazone bond through reaction with N-[β-maleimidopropionic acid] hydrazide (BMPH) wherein the maleimide group of the BMPH is optionally derivatised by formation of a thioether bond with mercaptoethanol; or
   - transformation into a hydrazone bond through reaction with N-[κ-maleimidoundecanoic acid] hydrazide (KMUH) wherein the maleimide group of the KMUH is optionally derivatised by formation of a thioether bond with mercaptoethanol;
ii. a first saccharide chain comprising a carboxyl group, preferably a carboxyl group of a glucuronic acid moiety, which has been derivatised by transformation into an amide bond through reaction with 2-amino-2-methyl-1,3-propanediol (AMPD) or N-(2-aminoethyl)maleimide (AEM);
iii. a second saccharide chain comprising an acetoxy group (Me(CO)O-) which has been derivatised by transformation into a hydroxyl group (HO-) by deacetylation; or
iv. any combination of two or three derivatisations i., ii. and/or iii., preferably any combination of two derivatisations i., ii. and/or iii.

An embodiment is the pharmaceutical combination or the pharmaceutical composition of the invention, wherein the saponin or the saponin derivative is selected from: *Quillaja* bark saponin, dipsacoside B, saikosaponin A, saikosaponin D, macranthoidin A, esculentoside A, phytolaccagenin, aescinate, AS6.2, NP-005236, AMA-1, AMR, alpha-Hederin, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1904, SO1862, QS-7, QS1861, QS-7 api, QS1862, QS-17, QS-18, QS-21 A-apio, QS-21 A-xylo, QS-21 B-apio, QS-21 B-xylo, beta-Aescin, Aescin la, Teaseed saponin I, Teaseedsaponin J, Assamsaponin F, Digitonin, Primula acid 1 and AS64R, or a derivative thereof, or a stereoisomer thereof, and/or a combination thereof, preferably the saponin or the saponin derivative is any one or more of QS-21 or a QS-21 derivative, SO1861 or a SO1861 derivative, SA1641 or a SA1641 derivative and GE1741 or a GE1741 derivative, more preferably a QS-21 derivative or a SO1861 derivative, most preferably a SO1861 derivative, wherein preferably such saponin derivative is a saponin derivative of according to the invention.

An embodiment is the pharmaceutical combination or the pharmaceutical composition of the invention, wherein the saponin or the saponin derivative is selected from: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillaja saponin, Saponinum album, QS-18, Quil-A, Gyp1, gypsoside A, AG1, AG2, SO1542, SO1584, SO1658, SO1674, SO1832, SO1862, SO1904, or a derivative thereof, or a stereoisomer thereof and/or any combinations thereof, preferably the saponin derivative is a SO1861 derivative and/or a GE1741 derivative and/or a SA1641 derivative and/or a QS-21 derivative, more preferably the saponin derivative is a SO1861 derivative or a QS21 derivative, most preferably, the saponin derivative is a SO1861 derivative according to the invention.

Such saponins of the triterpene glycoside type are capable of enhancing the endosomal escape of the effector molecules comprised by the first conjugate, and that are present in the endosome (or lysosome) of a cell, when the saponin as part of the second conjugate or in free form co-localizes with such effector molecule inside the cell. The inventors established that the endosomal escape enhancing activity of these saponins is about 100 to 1000 times more potent when the saponin is contacted with a cell as part of the second conjugate of the invention. The free saponin is capable of stimulating the delivery of effector molecules in the cytosol of cells, when such cells are contacted with the effector molecules as part of the first conjugate, and the saponin, at 100-1000 times higher saponin concentration, compared to the concentration of the same saponin which is comprised by the second conjugate of the invention, required to achieve the same extent of delivery of the effector molecule from outside the target cell to inside the endosome and finally in the cytosol of said cell. Saponins which display such endosomal escape enhancing activity are listed in Table A1, as well as saponins with high structural similarity with saponins for which the ability to potentiate the cytosolic delivery of the effector molecules comprised by the first conjugate has been established. When the saponin is part of the second conjugate of the invention, the targeted delivery of the saponin upon binding of the binding molecule of the second conjugate, such as an antibody or an sdAb, to the second cell-surface binding site on the target cell, on said cell, and after endocytosis, into the endosome of said cell, is thus about 100 to 1000 times more effective compared to contacting the same cell with free, untargeted saponin (derivative) which is not provided with a binding molecule such as an antibody or an sdAb for binding to a second cell-surface molecule of a target cell. For certain embodiments, the binding molecule comprised by the second conjugate is an sdAb. The small size of an sdAb of the second conjugates of the invention, compared to e.g. IgG type of antibodies, or fragments thereof such as Fab, scFv, contributes to efficient uptake by the target cell that exposes the second binding site for binding of the sdAb comprised by the second conjugate, e.g. uptake by endocytosis. Typically, the sdAbs in the second conjugates of the invention are capable of binding to a cell-surface receptor of a target cell, such as a tumor cell specific cell-surface receptor, which is the same as or a different cell-surface receptor from the cell-surface receptor targeted by the sdAb comprised by the first conjugate of the invention. This way, the first and second conjugates of the invention are particularly suitable for endocytosis into e.g. tumor cells expressing the first and second cell-surface receptor, wherein these first and second cell-surface receptor can be the same or can be different.

An embodiment is the pharmaceutical combination or the pharmaceutical composition of the invention, wherein the second conjugate comprises a binding molecule for binding to the second cell-surface molecule which binding molecule is any one or more of: a ligand, such as a proteinaceous ligand, for binding to the second cell-surface molecule such as EGF or a cytokine, or an immunoglobulin or at least one binding fragment or binding domain of said immunoglobulin capable of binding to the second cell-surface molecule, wherein the immunoglobulin is preferably any one or more of an antibody such as a monoclonal antibody, preferably a human antibody, an IgG, a molecule comprising or consisting of a sdAb, at least one V_{HH} domain or at least one V_{H} domain, a variable heavy chain new antigen receptor (V_{NAR}) domain, a Fab, an scFv, an Fv, a dAb, an F(ab)2, a Fcab fragment.

An embodiment is the pharmaceutical combination or the pharmaceutical composition of the invention, wherein the second conjugate comprises a binding molecule for binding to the second cell-surface molecule, wherein the second cell-surface molecule is a cell-surface receptor present on an aberrant cell such as a tumor cell, an auto-immune cell, an infected cell such as a virally infected cell.

An embodiment is the pharmaceutical combination or the pharmaceutical composition of the invention, wherein the second conjugate comprises a binding molecule for binding to the second cell-surface molecule such as an antibody or a binding fragment or binding domain thereof, such as a sdAb, or a receptor ligand, wherein preferably the second cell-surface receptor is HIVgp41 or a tumor-cell surface receptor such as a tumor-cell specific receptor, more preferably a cell-surface receptor selected from: CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, prostate specific membrane antigen (PSMA), CanAg, integrin-alphaV, CA6, CD33, mesothelin, Cripto, CD3, CD30, CD239, CD70, CD123, CD352, DLL3, CD25, ephrinA4, MUC-1, Trop2, CEACAM5, CEACAM6, HER3, CD74, PTK7, Notch3, FGF2, C4.4A, FLT3, CD38, FGFR3, CD7, PD-L1, CTLA-4, CD52, PDGFRA, VEGFR1, VEGFR2, c-Met (HGFR), EGFR1, RANKL, ADAMTS5, CD16, CXCR7 (ACKR3), glucocorticoid-induced TNFR-related protein (GITR), most preferably selected from: HER2, c-Met, VEGFR2, CXCR7, CD71, EGFR and EGFR1.

An embodiment is the pharmaceutical combination or the pharmaceutical composition of the invention, wherein the second conjugate comprises a binding molecule for binding to the second cell-surface molecule which is an immunoglobulin selected from, preferably a sdAb derived from or based on: an anti-CD71 antibody such as IgG type OKT-9, an anti-HER2 antibody such as trastuzumab (Herceptin), pertuzumab, an anti-CD20 antibody such as rituximab, ofatumumab, tositumomab, obinutuzumab ibritumomab, an anti-CA125 antibody such as oregovomab, an anti-EpCAM (17-1A) antibody such as edrecolomab, an anti-EGFR antibody such as cetuximab, matuzumab, panitumumab, nimotuzumab, an anti-CD30 antibody such as brentuximab, an anti-CD33 antibody such as gemtuzumab, huMy9-6, an anti-vascular integrin alpha-v beta-3 antibody such as etaracizumab, an anti-CD52 antibody such as alemtuzumab, an anti-CD22 antibody such as epratuzumab, pinatuzumab, binding fragment (Fv) of anti-CD22 antibody moxetumomab, humanized monoclonal antibody inotuzumab, an anti-CEA antibody such as labetuzumab, an anti-CD44v6 antibody such as bivatuzumab, an anti-FAP antibody such as sibrotuzumab, an anti-CD19 antibody such as huB4, an anti-CanAg antibody such as huC242, an anti-CD56 antibody such as huN901, an anti-CD38 antibody such as daratumumab, OKT-10 anti-CD38 monoclonal antibody, an anti-CA6 antibody such as DS6, an anti-IGF-1R antibody such as cixutumumab, 3B7, an anti-integrin antibody such as CNTO 95, an anti-syndecan-1 antibody such as B-B4, an anti-CD79b such as polatuzumab, an anti-HIVgp41 antibody, preferably any one of an anti-HIVgp41 antibody, an anti-CD71 antibody, an anti-HER2 antibody and an anti-EGFR antibody, more preferably any one of: trastuzumab, pertuzumab, cetuximab, matuzumab, an anti-CD71 antibody, OKT-9, most preferably trastuzumab, cetuximab, the anti-CD71 antibody OKT-9.

An embodiment is the pharmaceutical combination or the pharmaceutical composition of the invention, wherein the second conjugate comprises an sdAb that can bind to HER2, CD71, HIVgp41 and/or EGFR, wherein the sdAb is preferably a V_{HH}, more preferably a camelid V_{H}.

An embodiment is the pharmaceutical combination or the pharmaceutical composition of the invention, wherein the second conjugate comprises an sdAb for binding to HER2 selected from: sdAb produced by clone 11A4, clone 18C3, clone 22G12, clone Q17 or clone Q17-C-tag; or an sdAb for binding to EGFR and produced by clone anti-EGFR Q86-C-tag; or an sdAb for binding to CD71 and produced by clone anti-CD71 Q52-C-tag; or an sdAb for binding to HIVgp41 and produced by clone anti-HIVgp41 Q8-C-tag; or an sdAb encoded by a cDNA of any one of the SEQ ID NO: 1, 3, 5, 7, 9, 11, 13, 15, 17, 19, 21, 23, 25, 27, 29 and 31; or any one of the sdAbs with an amino-acid sequence of SEQ ID NO: 2, 4, 6, 8, 10, 12, 14, 16, 18, 20, 22, 24, 26, 28, 30, 32, 36-72, wherein optionally the first conjugate further comprises a covalently bound sdAb for binding to albumin, such as any one or more of the sdAbs with an amino-acid sequence of SEQ ID NO: 33, 34 and 35, preferably the sdAb is a V_{HH}, more preferably a camelid V_{H}. V_{HH}s suitable for incorporation in the second conjugate of the invention are for example found in the single domain antibody database (Wilton, E.E. et al. (2018)), in patent applications US20160251440 (anti-CD123, anti-CEACAM), US9683045 (anti-c-Met), US20090252681 (anti-EGFR, anti-IGF-1R), US9969805 (anti-HER2), US20190023796A1 (anti-HER3), and in Kijanka et al. (2013), for anti-HER2 and in Mercier et al. (2019) for anti-HER2. The amino-acid sequences and/or the cDNA sequences of a series of suitable V_{HH}s is also provided here below for anti-HER2, anti-HER3, anti-CD123, anti-CEACAM, anti-c-Met, anti-EGFR, anti-IGF-1R, anti-PD-L1, anti-CTLA-4, anti-CD19, anti-HER1 and anti-VGFR2, as SEQ ID NOs 1-32 and 36-72, in view of their ability to bind to tumor-cell specific receptors. In particular, a V_{HH} capable of binding to a binding site on any of the tumor-cell specific receptors HER2, VEGFR and CD71 is suitable for incorporation in the second conjugate of the invention. The inventors revealed that a conjugate of a saponin and a binding molecule comprising a V_{HH} that targets any one of such receptors is effective in delivery of the saponin bound to the sdAb. See for example the Examples section, and Figures 2-4.

These cell-surface molecules are typically present on tumor cells with tumor cell specificity, at least to a certain extent. Tumor cell specificity makes these receptors suitable targets for the first and second conjugates of the invention, and therefore the sdAb in the first conjugate is capable of binding to such a cell-surface receptor and therefore the binding molecule comprised by the second conjugate of the invention such as an antibody or an sdAb, is capable of binding to such a cell-surface receptor. Tumor cells are commonly targeted by the second conjugates comprising a tumor-cell receptor targeting binding molecule such as an antibody or a binding fragment or domain thereof, and comprising a saponin to be delivered inside the target tumor cell, more specifically in the cytosol of said tumor cell. Since the saponins comprised by the second conjugate of the invention are capable of stimulating the release and delivery of the effector molecules comprised by the first conjugate of the invention, in the cytosol of cells, such as the (tumor) cells targeted by the binding molecule of the second conjugate such as an sdAb comprised by the second conjugate of the invention, it is particularly suitable to select as the target (tumor) cell surface molecule for the binding molecule such as an sdAb, a cell-surface receptor known for its suitability to serve as the target for e.g. ADCs and AOCs. The first and second conjugates of the invention are therewith suitable for co-delivery of the effector molecule that is part of the first conjugate, which is an ADC or an AOC comprising an sdAb, together with the saponin comprised by the second conjugate of the invention. Targeting a tumor cell specific receptor with the second conjugate of the invention promotes endocytosis and delivery of the saponin as part of the second conjugate into the target cell endosome and/or lysosome. When the tumor cell is also contacted with an ADC or an AOC, such as the first conjugate of the invention, the effector molecule comprised by such ADC or AOC or first conjugate of the invention is co-delivered into the endosome or lysosome, and under influence of the saponin, the effector molecule is subsequently delivered into the cytosol of the target cell. As said before, the application of targeted saponin when part of the second conjugate of the invention results in an about 100 times to 1000 times potentiation of the stimulatory effect of the saponin, when biological activity of the effector molecule comprised by the first conjugate of the invention is considered, compared to the application of free saponin lacking a cell-targeting binding molecule such as a receptor ligand, an antibody or an sdAb. Application of the small sdAb such as a camelid V_{H} in the second conjugate of the invention prevents or slows down clearance of the second conjugate of the invention from the circulation and from the body of a human subject to whom the second conjugate was administered, when compared to clearance rates commonly observed for antibody based ADCs, which as said is also apparent for the first conjugate comprising the sdAb. In addition, due to the relatively small size of the sdAb, the risk for limiting or hampering the saponin activity inside a target cell due to the presence of the linked protein domain is limited, compared to the larger size of e.g. an antibody bound to the saponin. In general, the smaller the size of the molecule linked to the saponin, the smaller the risk for interference with the saponin activity inside cells due to the presence of the bound molecule, e.g. an antibody domain such as a V_{HH}. Moreover, the relative small size of the sdAbs leads to their rapid distribution in tissue, such as tumor tissue, allowing for improved reaching of target cells by the second conjugate of the invention, and therewith to improved (extent of) binding to the target cells, compared to the relatively large-sized IgGs commonly applied in e.g. ADCs, OACs. One of the many benefits of applying sdAbs in the second conjugates of the invention (which also applies for applying sdAbs in e.g. ADCs and AOCs, i.e. the first conjugates of the invention), is the absence of an Fc tail common to regular antibodies of e.g. the IgG type. Absence of an Fc tail in the sdAb in the first and second conjugate of the invention prevents occurrence of Fcy-Receptor mediated off-target effects such as undesired side effects relating to Fcy-Receptor activation, when the first and second conjugate is administered to a patient in need thereof. Absence of an Fc tail eliminates the risk of side effects generated by the binding of an Fc to cells of a patient to whom e.g. an antibody-based ADC is administered. The sdAb comprising conjugates of the invention do not bear this risk for Fc-mediated undesired side effects, whereas the inventors revealed that such first and second conjugates are highly efficient and effective in potentiation of the effector molecule comprised by the first conjugate, when tumor cells are contacted with both the ADC (at relatively low dose; see also the Examples section) and the second conjugate of the invention (at relatively low dose; see also the Examples section).

An embodiment is the pharmaceutical combination or the pharmaceutical composition of the invention, wherein the second conjugate comprises the saponin or the saponin derivative which is covalently bound via a thio-ether bond to a sulfhydryl group in the binding molecule, the covalent bonding via linker N-ε-maleimidocaproic acid hydrazide (EMCH) that is covalently bound to an aldehyde group in position C₂₃ of the aglycone core structure of the saponin or the saponin derivative and that is covalently bound to the sulfhydryl group in the binding molecule, such as a sulfhydryl group of a cysteine.

An embodiment is the pharmaceutical combination or the pharmaceutical composition of the invention, wherein the second conjugate comprises the saponin or the saponin derivative which is a bidesmosidic triterpene saponin or derivative thereof belonging to the type of a 12,13-dehydrooleanane with optionally an aldehyde function in position C₂₃ of the aglycone core structure of the saponin or the derivative thereof, and comprising a glucuronic acid unit in a first saccharide chain at the C₃beta-OH group of the aglycone core structure of the saponin, wherein the saponin or the derivative thereof is covalently bound to an amino-acid residue of the binding molecule via the carboxyl group of the glucuronic acid unit in the first saccharide chain, preferably via at least one linker, wherein the amino-acid residue preferably is selected from cysteine and lysine.

An embodiment is the pharmaceutical combination or the pharmaceutical composition of the invention, wherein the second conjugate comprises the saponin or the derivative thereof which comprises a glucuronic acid unit in the first saccharide chain at the C₃beta-OH group of the aglycone core structure of the at least one saponin or the derivative thereof, which glucuronic acid unit is covalently bound to a linker, which linker is preferably covalently bound via an amide bond to an amine group in the binding molecule, such as an amine group of a lysine or an N-terminus of the binding molecule, preferably the glucuronic acid unit is covalently bound linker 1-[Bis(dimethylamino)methylene]-1H-1,2,3-triazolo[4,5-b]pyridinium 3-oxid hexafluorophosphate (HATU).

An embodiment is the pharmaceutical combination or the pharmaceutical composition of the invention, wherein the second conjugate comprises more than one covalently bound saponin or a derivative thereof, preferably 2, **3, 4,** 5, 6, 8, 10, 16, 32, 64, 128 or 1-100, or any number therein between, such as 7, 9, 12 saponins or a derivative thereof.

An embodiment is the pharmaceutical combination or the pharmaceutical composition of the invention, wherein the more than one covalently bound saponin or saponin derivative are covalently bound directly to an amino-acid residue of the binding molecule, preferably to a cysteine and/or to a lysine, and/or are covalently bound via at least one linker and/or via at least one cleavable linker. Coupling of the saponin to the binding molecule such as an antibody, an sdAb, or a ligand for the second cell-surface molecule, via a linker provides flexibility when the binding site on the binding molecule for coupling of the saponin is considered. Furthermore, such a linker may act as a spacer between the binding molecule such as an sdAb and the saponin, such that both the binding molecule maintains its capability to bind to a binding site on a cell surface molecule and the saponin maintains its capability to enhance endosomal escape of the effector molecule comprised by the first conjugate, when the second conjugate co-localizes in the endosome or lysosome of the cell targeted by the first conjugate upon binding of the binding molecule such as an sdAb to the cell-surface molecule, together with the effector molecule.

An embodiment is the pharmaceutical combination or the pharmaceutical composition of the invention, wherein the more than one covalently bound saponin or saponin derivative are part of a covalent saponin conjugate comprising at least one oligomeric molecule or polymeric molecule and with the more than one saponin or saponin derivative covalently bound thereto, wherein the covalent saponin conjugate is covalently bound to the binding molecule. Such a covalent saponin conjugate serves as a carrier for multiple saponin moieties, which can be bound to the binding molecule comprised by the second conjugate, such as an antibody or an sdAb, via a single bond, preferably via a (cleavable) linker. Since the covalent saponin conjugate can bear any selected number of covalently bound saponin moieties, such as 1-200 saponin moieties, relating to the type of selected oligomeric or polymeric structure comprising binding sites for covalent linking these saponins, application of such covalent saponin conjugate provides freedom when the number of saponin moieties in the second conjugate of the invention is considered. For example, for cytosolic delivery of a selected effector molecule comprised by the first conjugate of the invention, the number of saponins present in the second conjugate of the invention can be adapted by providing the covalent saponin conjugate with a number of saponin moieties sufficient and enough for stimulating the cytosolic delivery of the effector molecule, when the covalent saponin conjugate is part of the second conjugate of the invention, and when the effector molecule co-localizes with the second conjugate in the endosome or lysosome of a target cell in which the effector molecule should exert its biological activity.

An embodiment is the pharmaceutical combination or the pharmaceutical composition of the invention, wherein 1-8 of such covalent saponin conjugates are bound to the binding molecule, more preferably 2-4 of such of such covalent saponin conjugates, wherein the at least one covalent saponin conjugate is optionally based on a dendron, wherein optionally 1-32 saponin(s) or saponin derivative(s), preferably 2, 3, 4, 5, 6, 8, 10, 16, 32, or any number therein between, such as 7, 9, 12 saponins or saponin derivatives, are covalently bound to the oligomeric molecule or to the polymeric molecule of the at least one covalent saponin conjugate, either directly or via a linker. Preferably, one or two of the covalent saponin conjugates is/are bound to a single binding molecule such as an sdAb in the second conjugate of the invention. For many purposes, coupling of a single saponin or coupling of a single covalent saponin conjugate to a single binding molecule such as an antibody or an sdAb comprised by the second conjugate, suffices for efficient stimulation of the effector molecule delivery into a target cell and into the cytosol of said cell, wherein the effector molecule is comprised by the first conjugate of the invention. Typically, 4, 8 or 16 saponins are comprised by the second conjugate of the invention, such as 4 or 8 saponins comprised by a single covalent saponin conjugate coupled to a binding molecule such as an sdAb in the second conjugate of the invention. Typically, such second conjugates of the invention comprise a single antibody or a single sdAb, to which the saponin or saponins or the covalent saponin conjugate(s) is/are bound, preferably a single saponin or a single covalent saponin conjugate is part of the second conjugate.

An embodiment is the pharmaceutical combination or the pharmaceutical composition of the invention, wherein the saponin or the saponin derivative is covalently bound to the binding molecule via a cleavable linker.

An embodiment is the pharmaceutical combination or the pharmaceutical composition of the invention, wherein the cleavable linker is subject to cleavage under acidic conditions, reductive conditions, enzymatic conditions and/or light-induced conditions, and preferably the cleavable linker comprises a cleavable bond selected from a hydrazone bond and a hydrazide bond subject to cleavage under acidic conditions, and/or a bond susceptible to proteolysis, for example proteolysis by Cathepsin B, and/or a bond susceptible for cleavage under reductive conditions such as a disulfide bond.

An embodiment is the pharmaceutical combination or the pharmaceutical composition of the invention, wherein the cleavable linker is subject to cleavage *in vivo* under acidic conditions as for example present in endosomes and/or lysosomes of mammalian cells, preferably human cells, preferably at pH 4.0 - 6.5, and more preferably at pH ≤ 5.5. Such cleavable linkers that are cleavable under the conditions as apparent in endosomes and lysosomes facilitates the delivery of free saponin inside the endosome or lysosome, upon cleavage of the saponin from the remainder of the second conjugate of the invention. This way, the second conjugate of the invention combines the benefits of cell-targeted delivery of the saponin upon specific binding of the binding molecule such as an antibody or an sdAb, to the second cell-surface molecule on the target cell, and of the presence of the free saponin inside the cell, i.e. inside the endosome (or lysosome), which contributes to the ability of the free saponin to stimulate and/or facilitate the delivery of the effector molecule comprised by the first conjugate of the invention, out of the endosome (or lysosome) and into the cytosol of the target cell.

An embodiment is the pharmaceutical combination or the pharmaceutical composition of the invention, wherein the oligomeric molecule or the polymeric molecule of the covalent saponin conjugate is covalently bound to the binding molecule, preferably to an amino-acid residue of the binding molecule.

An embodiment is the pharmaceutical combination or the pharmaceutical composition of the invention, wherein the saponin or the saponin derivative is covalently bound to the oligomeric molecule or to the polymeric molecule of the covalent saponin conjugate via a cleavable linker according to the invention.

An embodiment is the pharmaceutical combination or the pharmaceutical composition of the invention, wherein the saponin or the saponin derivative is covalently bound to the oligomeric molecule or to the polymeric molecule of the covalent saponin conjugate via any one or more of an imine bond, a hydrazone bond, a hydrazide bond, an oxime bond, a 1,3-dioxolane bond, a disulfide bond, a thio-ether bond, an amide bond, a peptide bond or an ester bond, preferably via a linker.

An embodiment is the pharmaceutical combination or the pharmaceutical composition of the invention, wherein the saponin or the saponin derivative comprises an aglycone core structure comprising an aldehyde function in position C₂₃ and the saponin comprising optionally a glucuronic acid function in a first saccharide chain at the C₃beta-OH group of aglycone core structure of the saponin, which aldehyde function is involved in the covalent bonding to the oligomeric molecule or to the polymeric molecule of the covalent saponin conjugate, and/or, if present, the glucuronic acid function is involved in the covalent bonding to the oligomeric molecule or to the polymeric molecule of the covalent saponin conjugate, the bonding of the saponin or the saponin derivative either via a direct covalent bond, or via a linker, wherein the linker is a cleavable linker or a stable linker. Here, stable refers to a bond between the saponin and the binding molecule such as an sdAb or to a bond between the saponin and the oligomeric or polymeric structure, which bond remains intact (is not cleaved) under the acidic conditions inside a cell, in particular the acidic conditions in the endosome or lysosome of such a cell. In addition, such a stable bond remains intact (i.e. is not cleaved) in e.g. the circulation and in the organs of a human subject to whom the second conjugate of the invention comprising the covalent saponin conjugate, is administered. In contrast, a cleavable linker in the context of the binding of a saponin to a binding molecule comprised by the second conjugate such as an antibody or an sdAb, or to an oligomeric structure or a polymeric structure refers to a bond that is cleaved under the acidic conditions as apparent inside endosomes and lysosomes of mammalian cells such as a human cell, e.g. a tumor cell, whereas such cleavable linker remains intact (is not cleaved) when a second conjugate comprising such cleavable bonds is present in the circulation or in organs, i.e. outside cells, of e.g. a human subject to whom the second conjugate of the invention is administered.

An embodiment is the pharmaceutical combination or the pharmaceutical composition of the invention, wherein the aldehyde function in position C₂₃ of aglycone core structure of the saponin or the saponin derivative is covalently bound to linker EMCH, which EMCH is covalently bound via a thio-ether bond to a sulfhydryl group in the oligomeric molecule or in the polymeric molecule of the covalent saponin conjugate, such as a sulfhydryl group of a cysteine. Binding of the EMCH linker to the aldehyde group of the aglycone of the saponin results in formation of a hydrazone bond. Such a hydrazone bond is a typical example of a cleavable bond under the acidic conditions inside endosomes and lysosomes. A saponin that is coupled to a binding molecule comprised by the second conjugate, such as an antibody or an sdAb in the second conjugate of the invention, or to an oligomeric structure or polymeric structure of a covalent saponin conjugate, wherein such a covalent saponin conjugate is coupled to the binding molecule of the second conjugate, e.g. to an sdAb in the second conjugate of the invention, is releasable from the second conjugate of the invention once delivered in the endosome or lysosome of a target cell that exposes the second cell-surface molecule to which the binding molecule such as an antibody or an sdAb of the second conjugate can bind. This way, saponin coupled to the binding molecule such as an sdAb in the second conjugate of the invention is transferred from outside the cell into the endosome (or lysosome), and in the endosome (or the lysosome), the saponin is released from the second conjugate upon pH driven cleavage of the hydrazone bond. In the endosome (or the lysosome) the free saponin can exert its stimulatory activity when the delivery of the effector molecule comprised by the first conjugate of the invention, which is co-localized in the endosome (or lysosome), into the cytosol is considered. Surprisingly, the inventors established that for the saponin it is not a prerequisite for endosomal escape enhancing activity of the saponin, that the saponin is present in endosomes or lysosomes in free form. Also saponins comprised by e.g. second conjugates of the invention, are potentiating the delivery of the effector molecule comprised by the first conjugate, out of the endosome / lysosome into the cytosol of targeted cells, once the effector molecule and the saponin as part of a first and second conjugate, respectively, are both contacted with the same target cell that expresses the first cell-surface molecule and the second cell-surface molecule, which can be the same or which can be different.

An embodiment is the pharmaceutical combination or the pharmaceutical composition of the invention, wherein the glucuronic acid function in the first saccharide chain at the C₃beta-OH group of the aglycone core structure of the saponin or of the saponin derivative is covalently bound to linker HATU, which HATU is covalently bound via an amide bond to an amine group in the oligomeric molecule or in the polymeric molecule of the covalent saponin conjugate, such as an amine group of a lysine or an N-terminus of a protein. When the HATU linker is coupled to the saponin and to the binding molecule of the second conjugate, e.g. to an sdAb, the saponin is for example bound to the N-terminus of the sdAb or to the amine group of a lysine present in the sdAb.

An embodiment is the pharmaceutical combination or the pharmaceutical composition of the invention, wherein the polymeric molecule or the oligomeric molecule of the covalent saponin conjugate is bound to the binding molecule, preferably to an amino-acid residue of the binding molecule, involving a click chemistry group on the polymeric molecule or the oligomeric molecule of the covalent saponin conjugate, the click chemistry group preferably selected from a tetrazine, an azide, an alkene or an alkyne, or a cyclic derivative of these groups, more preferably the click chemistry group is an azide.

An embodiment is the pharmaceutical combination or the pharmaceutical composition of the invention, wherein the polymeric molecule or the oligomeric molecule of the covalent saponin conjugate comprises a polymeric structure and/or an oligomeric structure selected from: a linear polymer, a branched polymer and/or a cyclic polymer, an oligomer, a dendrimer, a dendron, a dendronized polymer, a dendronized oligomer, a DNA, a polypeptide, a poly-lysine, a poly-ethylene glycol, an oligo-ethylene glycol (OEG), such as OEG₃, OEG₄ and OEG₅, or an assembly of these polymeric structures and/or oligomeric structures which assembly is preferably built up by covalent cross-linking, preferably the polymeric molecule or the oligomeric molecule of the covalent saponin conjugate is a dendron such as a poly-amidoamine (PAMAM) dendrimer. Driven by the number of selected saponins to be incorporated in the second conjugate of the invention, the type and size or length of the oligomeric structure or polymeric structure is selected. That is to say, the number of saponins to be coupled to the binding molecule comprised by the second conjugate such as an sdAb, for formation of the second conjugate of the invention, can determine the selection of a suitable oligomeric or polymeric structure, bearing the sufficient amount of binding sites for coupling the desired number of saponins, therewith providing a covalent saponin conjugate bearing the selected number of saponin moieties to be coupled to the binding molecule such as an antibody, a receptor ligand or an sdAb for provision of the second conjugate of the invention. For example, length of an OEG or size of a Dendron or poly-lysine molecule determines the maximum number of saponins which can be covalently linked to such oligomeric or polymeric structure.

A second conjugate according to the invention thus comprises at least one saponin. With "at least one" in this context is meant that the second conjugate comprises one saponin molecule but may also comprise a couple (e.g. two, three or four) of saponins or a multitude (e.g. 10, 20 or 100) of saponins. Depending on the application, the second conjugate may comprise a covalently bound scaffold (covalent saponin conjugate) with covalently bound saponins, wherein the scaffold may be designed such that it comprises a defined number of saponins. Preferably, a second conjugate according to the invention comprises a defined number or range of saponins, rather than a random number. This is especially advantageous for drug development in relation to marketing authorization. A defined number in this respect means that a second conjugate preferably comprises a previously defined number of saponins. This is, e.g., achieved by designing a scaffold comprising a polymeric structure with a certain number of possible moieties for the saponin(s) to (covalently) attach. Under ideal circumstances, all of these moieties are coupled to a saponin and the scaffold than comprises the prior defined number of saponins. It is envisaged to offer a standard set of scaffolds, comprising, e.g., two, four, eight, sixteen, thirty-two, sixty-four, etc., saponins so that the optimal number can be easily tested by the user according to his needs. An embodiment is the second conjugate of the invention comprising the scaffold of the invention (covalent saponin conjugate of the invention), wherein the saponin is present in a defined range as, e.g., under non-ideal circumstances, not all moieties present in a polymeric structure bind a saponin. Such ranges may for instance be 2 - 4 saponin molecules per scaffold, 3 - 6 saponin molecules per scaffold, 4 - 8 saponin molecules per scaffold, 6 - 8 saponin molecules per scaffold, 6 - 12 saponin molecules per scaffold and so on. In such case, a second conjugate comprising a scaffold according to the invention (covalent saponin conjugate of the invention), thus comprises 2, 3 or 4 saponins if the range is defined as 2 - 4.

The scaffold is fundamentally independent of the type of saponin covalently bound to the scaffold, the scaffold subsequently (in sequential order) covalently coupled to the cell-surface molecule binding molecule such as a receptor ligand, an antibody such as an sdAb, in the second conjugate of the invention. Thus, the second conjugate of the invention comprising the scaffold (covalent saponin conjugate of the invention) is a basis product for a platform technology. Since the at least one covalently bound saponin in the second conjugate of the invention mediates intracellular delivery of the effector moiety bound to the cell-surface molecule targeting sdAb, comprised by the first conjugate of the invention such as an ADC or an AOC comprising an sdAb, according to the invention, the scaffold technology according to the invention is a system that mediates controlled intracellular effector moiety delivery by saponins, by combining an ADC or an AOC (i.e. the first conjugate of the invention) with the second conjugate of the invention which comprises the saponin and for example and preferably an antibody such as an sdAb for binding to a target cell cell-surface molecule such as and preferably a receptor involved in endocytosis of the receptor and the conjugate bound thereto. The scaffold provides an optimized and functionally active unit that can be linked to the saponin(s) and to the cell-surface molecule targeting ligand such as an antibody such as an sdAb comprised by the second conjugate, at a single and defined position in the ligand such as an sdAb.

An embodiment is the second conjugate of the invention comprising a scaffold according to the invention (covalent saponin conjugate of the invention), wherein the number of monomers of the polymeric or oligomeric structure is an exactly defined number or range. Preferably, the polymeric or oligomeric structure comprises structures such as poly(amines), e.g., polyethylenimine and poly(amidoamine), or structures such as polyethylene glycol, poly(esters), such as poly(lactides), poly(lactams), polylactide-co-glycolide copolymers, poly(dextrin), or a peptide or a protein, or structures such as natural and/or artificial polyamino acids, e.g. poly-lysine, DNA polymers, stabilized RNA polymers or PNA (peptide nucleic acid) polymers, either appearing as linear, branched or cyclic polymer, oligomer, dendrimer, dendron, dendronized polymer, dendronized oligomer or assemblies of these structures, either sheer or mixed. Preferably, the polymeric or oligomeric structures are biocompatible, wherein biocompatible means that the polymeric or oligomeric structure does not show substantial acute or chronic toxicity in organisms and can be either excreted as it is or fully degraded to excretable and/or physiological compounds by the body's metabolism. Assemblies can be built up by covalent cross-linking or non-covalent bonds and/or attraction. They can therefore also form nanogels, microgels, or hydrogels, or they can be attached to carriers such as inorganic nanoparticles, colloids, liposomes, micelles or particle-like structures comprising cholesterol and/or phospholipids. Said polymeric or oligomeric structures preferably bear an exactly defined number or range of coupling moieties (chemical groups) for the coupling of glycoside molecules (and/or carrier molecules such as a ligand, monoclonal antibody or a fragment thereof such as an sdAb, wherein the sdAb is preferred according to the invention). Preferably at least 50%, more preferably at least 75%, more preferably at least 85%, more preferably at least 90%, more preferably at least 95%, more preferably at least 98%, more preferably at least 99%, most preferably (about) 100% of the exactly defined number or range of coupling moieties (chemical groups) in the polymeric or oligomeric structure is occupied by a glycoside molecule (saponin of the invention) in a scaffold according to the invention (covalent saponin conjugate of the invention).

Preferably, a dendron is a branched, clearly defined tree-like polymer with a single chemically addressable group at the origin of the tree, called the focal point. A dendrimer is a connection of two or more dendrons at their focal point. A dendronized polymer is a connection of the focal point of one or more dendrons to a polymer. In a preferred embodiment, a scaffold according to the invention is provided, wherein the polymeric or oligomeric structure comprises a linear, branched or cyclic polymer, oligomer, dendrimer, dendron, dendronized polymer, dendronized oligomer or assemblies of these structures, either sheer or mixed, wherein assemblies can be built up by covalent cross-linking or non-covalent attraction and can form nanogels, microgels, or hydrogels, and wherein, preferably, the polymer is a derivative of a poly(amine), e.g., polyethylenimine and poly(amidoamine), and structures such as polyethylene glycol, poly(esters), such as poly(lactids), poly(lactams), polylactide-co-glycolide copolymers, and poly(dextrin), and structures such as natural and/or artificial polyamino acids such as poly-lysine, or a peptide or a protein or DNA polymers, stabilized RNA polymers or PNA (peptide nucleic acid) polymers. Preferably, the polymeric or oligomeric structures are biocompatible.

It will be appreciated that when the binding molecule such as an sdAb in the second conjugate of the invention and the sdAb in the first conjugate of the invention, i.e. an ADC or an AOC, target different cell-surface molecules such as different tumor-cell specific receptors, the two different targets are both present at the surface of the target cell in which the effector molecule comprised by the first conjugate, i.e. the ADC or the AOC, and the saponin comprised by the second conjugate of the invention, should be co-delivered. When the ADC or the AOC (the first conjugate of the invention) and the second conjugate of the invention target different (tumor) cell receptors, the advantage is that e.g. the risk for off-target effects on e.g. cells that have one of the two cell-surface molecules on their surface, is reduced, when doses of the first conjugate of the invention (the ADC or the AOC) and of the second conjugate of the invention are contacted with a mixture of different cell types including the target cells, such as occurs when the combination of the first conjugate of the invention (the ADC or the AOC) and the second conjugate of the invention are (co-)administered to a patient in need thereof. By administering a dose of the first conjugate of the invention (the ADC or the AOC) which is too low to deliver a therapeutically effective amount of the effector molecule inside target cells, when the ADC or the AOC binds to such target cells, in the absence of the targeted saponin in the form of the second conjugate of the invention, entrance of cells that may express the cell-surface molecule to which the ADC or the AOC (the first conjugate of the invention) can bind, but that do not express the target for the binding molecule such as an sdAb in the second conjugate of the invention, will accordingly not induce e.g. a cytotoxic effect when the ADC is considered. Only in the target cell bearing both receptors for binding of the ADC or the AOC (the first conjugate of the invention) and for binding of the second conjugate of the invention, the dose of the effector molecule will be sufficient and enough for exerting its biological effect in the cytosol of said cells, once endosomal escape occurred under influence of the saponin that co-localizes inside said target cells expressing both receptors.

In some embodiments, the binding molecule such as an sdAb comprised by the second conjugate of the invention and the sdAb comprised by the first conjugate of the invention (i.e. the ADC or the AOC) target the same cell-surface molecule such as the same tumor-cell specific receptor. This way, presence of only a single tumor-cell specific receptor allows for co-delivery of both the ADC or the AOC (the first conjugate of the invention) and the second conjugate of the invention to inside the target cell bearing such single type of target receptor. An advantage of such co-delivery inside target cells using a single cell-specific surface molecule such as a tumor-cell specific receptor, e.g. HER2, EGFR, CD71, is the possibility to target tumor cells which only express a single receptor which is specific enough for specific targeting of such a target tumor cell by the second conjugate of the invention bearing the saponin and by an ADC or an AOC (i.e. the first conjugate of the invention). Selecting and targeting such a single specific receptor makes it possible to co-deliver the targeted saponin and the targeted effector molecule inside the tumor cell, such that such difficult-to-treat tumor cells can still be effectively provided with an effective dose of the effector molecule.

An aspect of the invention relates to a pharmaceutical combination of the invention or to a pharmaceutical composition according to the invention, for use as a medicament.

An aspect of the invention relates to a pharmaceutical combination of the invention or to a pharmaceutical composition according to the invention, for use in the treatment or the prophylaxis of a cancer, an auto-immune disease such as rheumatoid arthritis, an enzyme deficiency, a gene defect, a disease relating to a gene defect, an amyloidosis, a disease related to an enzyme deficiency, an infection such as a viral infection, hypercholesterolemia, primary hyperoxaluria, haemophilia A, haemophilia B, alpha-1 antitrypsin related liver disease, acute hepatic porphyria, transthyretin-mediated amyloidosis.

Surprisingly, the inventors found that a dose of the first conjugate of the invention, i.e. an ADC, which does not result in any tumor-cell killing is sufficient and enough for efficient tumor-cell killing when the first conjugate of the invention comprising the effector molecule is contacted with the tumor cells in the presence of the second conjugate of the invention. Examples are provided in Figures 4-7. For example, a dose of first conjugate of the invention such as ADC anti-CD71 V_{HH}-toxin, anti-HER2 V_{HH}-toxin or anti-EGFR V_{HH}-toxin does not exert a toxic effect on tumor cells when contacted with the tumor cells. Typical toxins are protein toxins, e.g. dianthin and saporin. However, when the first conjugate of the invention is co-administered together with a second conjugate of the invention, efficient tumor cell killing is achieved. The second conjugate of the invention is for example anti-HER2 V_{HH} - SO1861, anti-CD71 V_{HH} - SO1861, anti-EGFR V_{HH} - SO1861, cetuximab-SO1861, trastuzumab-SO1861. The first conjugate of the invention is for example an ADC such as for example an ADC comprising a V_{HH} capable of binding to HER2, CD71 or EGFR, and for example, the ADC comprises a protein toxin such as dianthin or saporin. As said before, by applying the second conjugate of the invention, the saponin dose required to achieve an efficient biological activity of the effector molecule comprised by the first conjugate of the invention, i.e. an ADC or an AOC, inside a target cell, is about 100 to 1000 fold lower when the second conjugate comprising the saponin is co-administered with the first conjugate of the invention, i.e. the ADC or the AOC to the target cells, compared to the dose of free saponin that is required when first conjugate of the invention is co-administered to the target cell together with the free saponin. Herewith, the second conjugate of the invention potentiates the first conjugate of the invention, i.e. an sdAb comprising ADC or AOC, at a dose of the ADC or the AOC that would otherwise not be effective in tumor cells when it would be administered to a patient in need thereof in the absence of the targeted saponin (second conjugate of the invention), and in addition, herewith the second conjugate of the invention is already sufficiently effective when potentiation of the effector molecule of the first conjugate of the invention, i.e. an ADC or an AOC, is considered, already at a relatively low dose i.e. at a dose at which a free saponin that is not provided with a tumor-cell targeting binding molecule such as an sdAb, is not sufficiently effective in effector molecule potentiation. In combination, the inventors provide for an improved method for treating a human patient in need of treatment with a therapeutically effective amount of the first conjugate of the invention, i.e. an ADC or an AOC comprising a tumor-cell targeting sdAb, since the ADC or the AOC is already effective at lower dose when co-administered to patients in the presence of the second conjugate of the invention. One of the many benefits of the first and second conjugates of the invention resides in the absence of an Fc tail in the sdAb part of the first conjugate and preferably also in the second conjugates. Absence of the Fc tail prevents unwanted binding of conjugates to off-target patient cells bearing Fc receptors, which binding could otherwise result in side-effects if such an Fc tail would be present, such as seen for many conventional ADCs, AOCs. Antibody-based ADCs and AOCs comprising an Fc tail suffer from a decreased efficacy due to the undesired binding of such IgG based ADCs, AOCs to Fc receptors. As a result of Fc receptor binding, the effective dose of such IgG-based ADCs and AOCs is lowered. Therewith, absence of the Fc tail provides for several benefits in this regard. Off-target and undesired binding of the first conjugate and preferably also the second conjugate to Fc receptors cannot occur. Herewith, the first and second conjugate of the invention have a lower effective dose when target receptor mediated endocytosis and delivery of the first and second conjugate of the invention inside endosomes is considered, since no conjugate is 'lost' due to Fc receptor binding, a drawback seen with IgG-based conjugates. As a result, the therapeutic window of the first conjugates of the invention is wider than the therapeutic window that would have been achieved when the sdAb is replaced by a conventional IgG comprising the Fc tail. Similarly, as a result, the therapeutic window of the second conjugates of the invention is wider than the therapeutic window that would have been achieved when the sdAb in the second conjugate is replaced by a binding molecule for binding the second cell-surface molecule on the target cell, such as a conventional IgG comprising the Fc tail.

An embodiment is the pharmaceutical combination for use of the invention or the pharmaceutical composition for use according to the invention, wherein the saponin is SO1861 or QS-21, or wherein the saponin derivative is a SO1861 derivative or a QS-21 derivative, preferably a saponin derivative of the invention.

An embodiment is the pharmaceutical combination for use of the invention or the pharmaceutical composition for use according to the invention, wherein:
- said use is in the treatment or prevention of cancer in a human subject; and/or
- said use is in the treatment or prophylaxis of cancer in a patient in need thereof, wherein the first and the second cell-surface molecules are the same or a different tumor-cell surface molecules, preferably (a) tumor cell-specific surface molecule(s), and wherein the sdAb and the binding molecule of the second conjugate bind to the tumor-cell surface molecules, preferably tumor cell-specific surface molecules; and/or
- the pharmaceutical combination, preferably a therapeutically effective amount of the pharmaceutical combination, is administered to a patient in need thereof, preferably a human patient.

A further aspect of the invention relates to an *in vitro* or ex *vivo* method for transferring the first conjugate of the invention from outside a cell to inside said cell, preferably to the cytosol of said cell, comprising the steps of:
a) providing a cell which expresses the first cell-surface molecule according to the invention on its surface, and optionally expresses the second cell-surface molecule according to the invention on its surface, said cell preferably selected from a liver cell, an aberrant cell such as a virally infected cell, an auto-immune cell and a tumor cell;
b) providing the first conjugate of the invention for transferring into the cell provided in step a);
c) providing the saponin or the saponin derivative of the invention, and/or, when the cell provided in step a) expresses the second cell-surface molecule according to the invention on its surface, providing the second conjugate of the invention;
d) contacting the cell of step a) *in vitro* or ex *vivo* with the first conjugate of step b) and any one or more of the saponin, the saponin derivative and the conjugate of step c),
therewith establishing the transfer of the first conjugate from outside the cell into said cell, preferably into the cytosol of said cell.

An embodiment is the *in vitro* or *ex vivo* method for transferring the first conjugate of the invention from outside a cell to inside said cell according to the invention, the method, once the first conjugate is transferred to inside said cell, further for transferring the effector molecule comprised by the first conjugate from the cell endosome, endolysosome or lysosome into the cytosol of the cell, comprising the steps of:
a) providing a cell which expresses the first cell-surface molecule according to the invention on its surface, and optionally expresses the second cell-surface molecule according to the invention on its surface, said cell preferably selected from a liver cell, an aberrant cell such as a virally infected cell, an auto-immune cell and a tumor cell;
b) providing the first conjugate of the invention for transferring into the cell provided in step a);
c) providing the saponin or the saponin derivative of any one of the invention, or, preferably, when the cell provided in step a) expresses the second cell-surface molecule according to the invention on its surface, providing the second conjugate of the invention;
d) contacting the cell of step a) *in vitro* or ex *vivo* with the first conjugate of step b) and any one or more of the saponin, the saponin derivative and the conjugate of step c),
therewith establishing the transfer of the first conjugate from outside the cell into said cell, and therewith establishing the delivery of the effector molecule comprised by the first conjugate into the cytosol of said cell, optionally either as part of the first conjugate, or as free effector molecule cleaved off from the first conjugate in the endosome, endolysosome or lysosome.

In formal terms, a glycoside is any molecule in which a sugar group is bound through its anomeric carbon to another group via a glycosidic bond. Glycoside molecules, such as saponins, in the context of the invention are such molecules that are further able to enhance the effect of an effector moiety, without wishing to be bound by any theory, in particular by facilitating the endosomal escape of the effector moiety. Without wishing to be bound by any theory, the glycoside molecules (saponins of the invention, such as those exemplified herein and in the claims) interact with the membranes of compartments and vesicles of the endocytic and recycling pathway and make them leaky for said effector moieties resulting in augmented endosomal escape. With the term "the scaffold is able to augment endosomal escape of the effector moiety" is meant that the at least one saponin (glycoside molecule), which is coupled via a linker or directly to the cell-surface molecule targeting antibody such as an sdAb or via the polymeric or oligomeric structure of the scaffold (covalent saponin conjugate of the invention) in the second conjugate of the invention, is able to enhance endosomal escape of an effector moiety comprised by the first conjugate of the invention, when both molecules are within an endosome, e.g. a late endosome, optionally and preferably after the at least one saponin is released from the second conjugate of the invention such as from a linker or polymeric or oligomeric structure comprised by said second conjugate, e.g., by cleavage of a cleavable bond between the at least one glycoside (saponin) and the second conjugate (for example via a polymeric or oligomeric structure of a scaffold and/or via a linker). Even though a bond between the at least one saponin according to the invention and the cell-surface molecule targeting ligand such as an antibody such as an sdAb of the second conjugate of the invention, optionally via a linker or a scaffold, may be a "stable bond", that does not mean that such bond cannot be cleaved in the endosomes by, e.g., enzymes. For instance, the saponin, optionally together with a linker or a part of the oligomeric or polymeric structure of a scaffold, may be cleaved off from the remaining linker fragment or oligomeric or polymeric structure. It could, for instance be that a protease cuts a (proteinaceous) linker or proteinaceous polymeric structure, e.g., albumin, thereby releasing the at least one saponin. It is, however, preferred that the glycoside molecule (preferably saponin) is released in an active form, preferably in the original form that it had before it was (prepared to be) coupled to the cell-surface molecule targeting molecule such as an sdAb of the second conjugate of the invention optionally via a linker and/or an oligomeric or polymeric scaffold (covalent saponin conjugate of the invention); thus the glycoside (saponin) has its natural structure after such cleavage or the glycoside (saponin) has (part of) a chemical group or linker bound thereto, after such cleavage, while glycoside biological activity (saponin biological activity), e.g. endosomal/lysosomal escape enhancing activity towards an effector moiety present in the same endosome or lysosome, is maintained or restored upon said cleavage of the bond between the glycoside (saponin) and the cell-surface molecule targeting antibody such as an sdAb, optionally comprising a linker and/or a scaffold of the invention. With regard to the present invention the term "stable" with respect to bonds between e.g. saponins and amino-acid residues of the cell-surface molecule targeting sdAb in the second conjugate, a linker, a polymeric or oligomeric structures (of the scaffold, a.k.a. the covalent saponin conjugate of the invention), ligands, (monoclonal) immunoglobulins or binding domains or -fragments thereof, and/or effectors (effector moieties, effector molecules), is meant that the bond is not readily broken or at least not designed to be readily broken by, e.g., pH differences, salt concentrations, or UV-light, reductive conditions. With regard to the present invention the term "cleavable" with respect to bonds between e.g. saponins and the cell-surface molecule targeting sdAb, linkers, amino-acid residues, polymeric or oligomeric structures of the covalent saponin conjugate, ligands, antibodies and/or effectors, is meant that the bond is designed to be readily broken by, e.g., pH differences, salt concentrations, under reductive conditions, and the like. The skilled person is well aware of such cleavable bonds and how to prepare them.

Before the present invention one of the major hurdles of introducing ADCs and AOCs on the market was the small therapeutic window: a therapeutically effective dose of an ADC or an AOC is accompanied with (unacceptable) side effects, hampering development and implication in treatment of patients with the ADCs. By the application of the first conjugate of the invention comprising the effector molecule and the cell-surface molecule targeting sdAb and the second conjugate of the invention, e.g. an sdAb-saponin conjugate, it has now become possible to guide one or multiple glycoside molecules (saponin(s)) to a (target) cell, together with the ADC carrying a payload or together with an sdAb conjugated with an oligonucleotide such as a BNA according to the invention. In particular, it was previously not possible to specifically guide an effector moiety of an ADC or AOC or any other conjugate of a payload and a (proteinaceous) cell-surface molecule targeting molecule, and a (predefined, controllable) particular number or range of glycoside molecules (saponins) per effector moiety at the same time to the cytosol of cells, such as via the endocytic pathway of a cell. As said before, the ADC or the AOC (i.e. the first conjugate of the invention) and the second conjugate of the invention can target the same cell-surface molecule or can target different cell surface molecules, wherein the two different cell-surface molecules are expressed at the surface of the same target cell such as a tumor cell or an auto-immune cell.

A solution provided for by the invention comprises the covalent binding of at least one saponin to the cell-surface molecule targeting molecule of the second conjugate of the invention, e.g. an antibody, preferably an sdAb. A further solution provided for by the invention comprises (first) polymerizing the glycoside molecules (saponins) using an oligomeric or polymeric scaffold, and providing the cell-surface molecule targeting molecule comprised by the second conjugate of the invention with a cluster of covalently bound saponins, enabling re-monomerization of the one or more saponins at the intracellular site where the mode of action of the saponin is desired, e.g. after endocytosis. "Polymerizes" in this context means the reversible and/or irreversible multiple conjugation of saponin molecules to the cell-surface molecule binding molecule of the second conjugate, e.g. an antibody such as an sdAb, either via linker, or directly or via a polymeric or oligomeric structure to form a scaffold (covalent saponin conjugate of the invention) or the reversible and/or irreversible multiple conjugation of (modified) saponins thereby forming a polymeric or oligomeric structure to form a scaffold (covalent saponin conjugate of the invention). "Re-monomerization" in this context means the cleavage of the saponins from the second conjugate, from the linker linking the saponin(s) to the cell-surface molecule targeting ligand such as an sdAb of the second conjugate or from the scaffold, for example after endocytosis, and regaining the (native) chemical state of the unbound saponins, which unbound saponins may or may not comprise additional chemical groups such as a chemical group for linking the saponin to a linker, an amino-acid residue of the second conjugate or to the scaffold, and/or a (chemical) linker bound to a chemical group of the saponin such as an aldehyde group or carboxylic acid group. Due to the complex chemistry of the saponins for example the "polymerization" of saponins at a scaffold or other linking linker and their 're-monomerization' at a desired location such as intracellularly e.g. after endocytosis, was a challenging task. In particular, the chemical reactions used for providing the linkers and the scaffold comprising covalently linked glycosides for covalent binding to the cell-surface molecule binding molecule for providing the second conjugate, e.g. triterpenoid saponins (polymerization of the glycosides), normally occur in water-free organic solvents, but saponins and for example biocompatible polymers applied as a scaffold for bearing bound saponins, are water-soluble molecules. The chemical properties of the unmodified saponin further prohibited polymerization by itself and, one other possible solution, to bind multiple saponins (directly) to the cell-surface molecule binding molecule such as an antibody, e.g. an sdAb, was estimated not to be very promising, as an antibody such as an sdAb does typically not provide sufficient binding sites and because the coupling product would become quite heterogeneous and/or coupling biologically active molecules such as a saponin and a cell-surface molecule targeting antibody such as an sdAb together bears the risk for influencing and hampering the activity of the saponin and/or the risk for influencing and hampering the binding of e.g. the sdAb to the cell-surface molecule, bound together in such saponin-comprising second conjugate of the invention. Embodiments of the present invention solves at least one of these drawbacks.

Whether or not a second conjugate of the invention comprising saponins, either or not further comprising one or more (cleavable) linkers and/or optionally a scaffold (covalent saponin conjugate of the invention), is able to disturb the acidic environment and inhibit the endosomal escape function of the at least one glycoside (saponin) can be easily determined with an assay as described in the examples section, and as known in the art. The inhibition is described as "fold amount increases of glycoside (saponin of the invention) necessary to induced 50% cell killing". It is preferred that the scaffold or the second conjugate of the invention does not lead to an increase that is at least the increase in glycoside molecules (saponins) necessary to obtain 50% cell killing observed when using Chloroquine as a positive control. Alternatively, and preferably, the second conjugate comprising saponins, either or not further comprising one or more (cleavable) linkers and/or optionally a scaffold does not lead to an at least 4-fold increase of glycoside molecules to induce 50% cell killing, more preferably does not lead to an at least 2-fold increase. The fold increase is to be measured in assay, wherein Chloroquine, as a positive control, induces a 2-fold increase in glycoside amount, preferably saponin amount wherein the saponin is any one or more of the saponins of the invention (previous embodiments) to observe 50% cell killing.

As said before, the at least one saponin that is comprised by the second conjugate according to the invention increases the efficacy of at least current and new effector moieties as defined in this invention. Potential side-effects will be decreased due to lowering of dosing of the effector moiety comprised by the first conjugate of the invention which further comprises a cell-surface molecule binding sdAb, such as an ADC or an AOC, without lowering the efficacy. Therefore, the invention provides a combination of a second conjugate according to the invention and a first conjugate according to the invention and the invention provides a first conjugate according to the invention, for use in medicine or for use as a medicament. An aspect of the invention relates to a pharmaceutical composition of the invention, for use as a medicament, wherein the pharmaceutical composition comprises the first conjugate of the invention. An aspect of the invention relates to a pharmaceutical composition of the invention, for use as a medicament, wherein the pharmaceutical composition comprises the first conjugate of the invention and comprises the second conjugate of the invention.

A number of preferred features can be formulated for endosomal escape enhancers comprised by the second conjugate of the invention, i.e. a saponin of the invention: (1) they are preferably not toxic and do not invoke an immune response, (2) they preferably do not mediate the cytosolic uptake of the effector moiety into off-target cells, (3) their presence at the site of action is preferably synchronized with the presence of the effector moiety, (4) they are preferably biodegradable or excretable, and (5) they preferably do not substantially interfere with biological processes of the organism unrelated to the biological activity of the effector molecule with which the endosomal escape enhancer is combined with, e.g. interact with hormones. Examples of saponins of the invention that fulfill the before mentioned criteria, at least to some extent, are bidesmosidic triterpenes, preferably bidesmosidic triterpene saponins, such as SO1861, SA1641, QS-21, GE1741, and the further saponins according to the invention and listed throughout the specification.

Also provided is the use of a first conjugate according to the invention for manufacturing a medicament. Also provided is the use of a first conjugate according to the invention and a second conjugate of the invention for manufacturing a medicament. Especially cancer medicines, and in particular the classical chemotherapy medicaments, are notorious for their side effects. Because of targeting and synchronization in time and place of both the pharmaceutically active substance comprised by the first conjugate such as an ADC or an AOC, and the saponin comprised by the second conjugate molecule of the invention, a first conjugate according to the invention is especially valuable for use as a medicament or the combination of a first conjugate according to the invention and a second conjugate of the invention is especially valuable for use as a medicament, in particular for use in a method of treating cancer, wherein the medicament is a single composition comprising an ADC or an AOC of the invention (first conjugate of the invention) and the second conjugate of the invention, or is a therapeutic combination of a first pharmaceutical composition comprising a first conjugate of the invention, e.g. an ADC or an AOC, and a second pharmaceutical composition comprising the second conjugate of the invention. The invention thus provides a first conjugate according to the invention for use in a method of treating cancer. The invention also provides a first conjugate according to the invention for use in a method of treating acquired or hereditary disorders, in particular monogenic deficiency disorders. The second conjugate thus comprises the at least one saponin and an sdAb for targeting the second conjugate at an aberrant target cell such as a tumor cell or an auto-immune cell, and the second conjugate of the invention is combined with at least one first conjugate of the invention comprising an effector moiety and an sdAb such as an ADC or an AOC of the invention. Thus, an aspect of the invention relates to a second conjugate according to the invention, wherein the second conjugate comprises a covalently bound saponin and a cell-surface molecule binding antibody such as an sdAb, for use in a method for the treatment of a cancer or an auto-immune disease, the method further comprising the administration of a first conjugate according to the invention to a subject such as a cancer patient in need thereof.

A further application of the first and second conjugates of the invention in medicine is the use in a method for the substitution of intracellular enzymes in target cells that produce these enzymes in insufficient amount or insufficient functionality. The second conjugate of the invention is for example administered to a patient in need thereof in combination with the first conjugate of the invention comprising the enzyme to be substituted or an oligonucleotide for gene therapy, for substitution of a target intracellular enzyme. The resulting disease might be hereditary or acquired. In most cases, only symptomatic treatment is possible and for a number of rare diseases, insufficient treatment options lead to a shortened life span of concerned patients. An example for such a disease is phenylketonuria, which is an inborn error of metabolism that results in decreased metabolism of the amino acid phenylalanine. The disease is characterized by mutations in the gene for the hepatic enzyme phenylalanine hydroxylase. Phenylketonuria is not curable to date. The incidence is approximately 1:10,000 with the highest known incidence in Turkey with 1:2,600. A cell-surface molecule targeting antibody comprised by the second conjugate of the invention, preferably an sdAb such as a V_{HH}, with bound saponin and a first conjugate comprising the same or a different cell-surface molecule binding sdAb and phenylalanine hydroxylase or a first conjugate comprising the same or a different cell-surface molecule binding sdAb with a bound polynucleotide that encodes phenylalanine hydroxylase, can be used to target liver cells by use of a suitable specific antibody or sdAb in the second conjugate of the invention and by use of a suitable specific sdAb in the first conjugate according to the invention, and to substitute the defect enzyme in hepatocytes. This is one example of use of the second conjugate of the invention comprising a saponin bound thereto and, in this example, a first conjugate of the invention comprising an effector molecule, such as the enzyme or the oligonucleotide bound thereto according to the invention, for substitution or gene therapy. In a preferred embodiment, a second conjugate according to the invention for use in a method of gene therapy or substitution therapy is provided in combination with a first conjugate of the invention comprising a cell-surface molecule binding sdAb and an enzyme or a nucleic acid encoding for the enzyme.

With the first and second conjugates of the invention it has now become possible to design and manufacture a two-component, non-viral clinically applicable gene delivery technology. For example, the second conjugate of the invention allows for development of non-viral based combinations of first and second conjugates for gene delivery technology, which enhances therapeutic efficacy with lower therapeutic dose thereby improving the health of patients. The second conjugate of the invention, in particular when comprising a covalently bound cell-surface molecule targeting antibody such as a monoclonal antibody or sdAb for binding to a (tumor, auto-immune) cell-surface specific molecule, and combined with a first conjugate comprising an effector moiety such as an oligonucleotide for example a BNA, and comprising a cell-surface molecule targeting sdAb such as a V_{HH}, allows for overcoming a longstanding and major bottleneck in the field of gene delivery, namely efficient, safe and cost-effective transfer of gene therapeutic products across the endosomal membrane into the cytosol/nucleosol. Indeed, gene therapy is one of the most promising treatment options for future advanced therapies in a broad range of diseases. Successful gene delivery requires the recognition of target cells as well as cytosolic and nucleosolic uptake of the gene. One of the major problems in the field of non-viral gene therapy is the inefficient and insufficiently safe delivery of genetic material for therapeutic use in patients.

Thus, when applying the second conjugate of the invention, comprising a cell-targeting cell-surface molecule targeting molecule such as a ligand or preferably an antibody (fragment, domain thereof, preferably sdAb) and combined with a first conjugate of the invention such as an AOC or an sdAb comprising first conjugate comprising an oligonucleotide such as an antisense BNA and comprising a cell-surface molecule targeting sdAb, the inventors now made it possible to overcome a longstanding and major bottleneck in the field of gene delivery: safe transfer of gene therapeutic products across the endosomal membrane into the cytosol/nucleosol. The combination of the first and second conjugates of the invention represents technology designed for allowing targeting of any addressable cell type with all known genetic agents and subsequent efficient cytosolic delivery of a gene, thereby ensuring better patient therapy not limited to inherited disorders, but also for cancer therapy and therefore of importance for large patient groups. The technology based on the first and second conjugates of the invention may comprise a second conjugate of the invention which comprises a polymeric or oligomeric scaffold (covalent saponin conjugate of the invention) that serves as a carrier for endosomal escape enhancers (EEEs), such as the saponins as exemplified herein, and the saponins of the embodiments according to the invention, for the cell-surface molecule targeting molecule such as a targeting ligand or (monoclonal) (tumor-cell specific) antibody, or a fragment thereof, or preferably an sdAb such as a V_{HH}. A first conjugate of the invention then comprises a first cell-surface molecule binding molecule such as an sdAb, and an effector moiety, here an effector gene such as an LNA or BNA. Use of the second conjugate of the invention, e.g. comprising a cell-targeting antibody (fragment) or sdAb in combination with a first conjugate of the invention comprising the same or a different cell-surface targeting sdAb and an oligonucleotide such as a BNA, has potential to bring any kind of biological macromolecules into the cytosol and the nucleus. Development of new targeting ligands, sdAbs and monoclonal (human, humanized) antibodies is under continuous investigation by numerous research groups and companies worldwide. The same for the oligonucleotides that are aimed for delivery in the cytosol of diseases cells such as cancer cells. The first and second conjugates of the invention thus also present as a molecular tool in which present and future targeting sdAbs and antibodies can be used by click chemistry, allowing for customized drug applications and for future developments in the field of tissue and cell targeting techniques, and the first conjugates according to the invention thus also present as a molecular tool in which present and future therapeutic oligonucleotides (as well as payloads such as protein toxins) are linked or can be linked to for example an sdAb by click chemistry, allowing for customized drug applications and for future developments in the field of tissue and cell targeting techniques. The first and second conjugates of the invention can comprise antibodies and ligands as the cell-surface molecule targeting molecule, but an sdAb is preferred. The worldwide market of gene therapeutics is rapidly growing and is covering potential treatments for a wide range of disease areas such as, cancer, cardiovascular diseases, Parkinson's, Alzheimer, HIV and many rare (monogenetic) diseases. The current viral vector-based gene therapeutic technologies have significant challenges, such as safety, manufacturing logistics, and associated high costs. The second conjugate of the invention allows for use in a technology platform which represents an alternative for a current viral gene delivery technology, when combined with a first conjugate of the invention comprising the gene to be delivered and a cell-surface targeting molecule i.e. an sdAb. Therefore, the first and second conjugates of the invention are suitable for implementing in approaches for developing non-viral gene treatments for diseases such as cancers, cardiovascular diseases, Parkinson's disease, Alzheimer's disease, HIV infection and many rare (monogenetic) diseases. The first and second conjugates of the invention are suitable for developing novel treatments for transforming the field of antibody-drug conjugates (ADCs) and oligonucleotide-based therapeutics by making non-viral vector based gene therapeutics such as based on targeted antisense BNA. The application of the second conjugate of the invention, in particular in a covalent conjugate with an antibody such as an sdAb and a saponin, and combined with a first conjugate comprising a cell-surface molecule binding molecule, i.e. an sdAb, and an oligonucleotide such as a BNA, is one of the many beneficial approaches made possible due to the present invention. For example, use of the first and second conjugates of the invention now allows for exploitation of the endocytic pathway of mammalian cells. Endocytosis is exploited for the delivery of therapeutics, wherein the second conjugate of the invention contributes to improved uptake and endosomal escape of e.g. siRNAs which are comprised by the first conjugate. The first and second conjugates of the invention are suitably used together with small molecules that act as delivery enhancers for e.g. payloads, oligonucleotides. Herewith, the first conjugate of the invention bearing the covalently coupled oligonucleotide such as a BNA and bearing the covalently coupled cell targeting moiety i.e. an sdAb, preferably a V_{HH}, in combination with the second conjugate of the invention, bearing the saponins of the invention and bearing the covalently coupled cell targeting moiety such as a ligand and preferably an antibody (domain or fragment, preferably a V_{HH}), provides a solution for the current problem seen with current endosomal escape enhancers and gene therapeutic product, relating to their application as two components comprising free non-targeted saponin, thus complicating therapeutic approval and clinical applicability, since such a second conjugate of the invention encompassing the saponin, combined with a first conjugate of the invention comprising a gene product such as a BNA and a (tumor) cell targeting moiety i.e. an sdAb. Thus the invention provides a non-viral gene delivery technology where endosomal escape enhancers (e.g. the glycosides of the embodiments of the invention and of the examples provided) and a second targeting ligand or antibody (according to e.g. the embodiments of the invention and the sdAbs exemplified here below in the Examples section) are comprised by the second conjugate of the invention, and are combined with a first conjugate of the invention comprising a gene therapeutic product (oligonucleotides according to the invention such as a BNA) and a first cell-surface molecule targeting sdAb such as the same or a different cell-surface targeting sdAb as encompassed by the second conjugate of the invention, wherein the first cell-surface molecule targeting sdAb binds to the same or to a different cell surface molecule as the cell surface molecule targeting molecule of the second conjugate of the invention. Such a first and second conjugate of the invention thus provide therapeutic opportunities for current and future macromolecule drugs for a broad range of diseases and large patient groups. With the application of such a second conjugate of the invention comprising at least one saponin and at least one specific cell-targeting moiety such as an immunoglobulin or sdAb, in combination with a first conjugate of the invention comprising at least one oligonucleotide and at least one specific cell-targeting moiety i.e. an sdAb, the problem is addressed which is apparent for current methods of applying non-cell-surface molecule targeted free endosomal escape enhancers (e.g. saponin not conjugated with a cell-surface molecule binding antibody) and gene therapeutic product separately, which current methods do not ensure that both compounds are at the same time at the site of interaction. This problem is now overcome by using the second conjugate of the invention in combination with a first conjugate of the invention comprising the nucleic acid, wherein the second conjugate of the invention and the first conjugate both comprise a cell-surface molecule targeting molecule, e.g. such as an antibody such as an sdAb when the second conjugate is considered and an sdAb when the first conjugate is considered, according to the invention. That is to say, such a combination of a second conjugate of the invention and a first conjugate comprising an oligonucleotide provides a non-viral gene delivery technology with increased synchronization (in time and place) of both compounds, i.e. the saponin and the gene product such as a BNA.

Gene therapies could help with hereditary, previously incurable diseases such as cystic fibrosis, chorea, Huntington's disease or hemophilia. However, currently some problems have not been overcome: for example, the therapeutic genes must precisely reach specific target cells in the body. On the other hand, the therapeutic genes should be absorbed by the targeted cells, but the therapeutic genes should not be destroyed. The current gene therapy approaches use viruses as a ferry for genes. However, these procedures involve considerable risks and cannot be transferred to the introduction of other biomolecules. An embodiment is the second conjugate of the invention comprising (plant-derived) glycosides (e.g. any one of the saponins of the invention) for use a platform technology that allows not only delivery of genes when such genes are comprised by the first conjugate of the invention as the carrier molecule, but also allows for the delivery of different therapeutic biomolecules to be introduced into target cells. Therefore, the second conjugate of the invention is used for developing treatments based on nucleic acids for cystic fibrosis, chorea, Huntington's disease or hemophilia. Herewith, with the second conjugate of the invention, a new gene therapy strategy is available for improving the health of patients with genetic diseases, including those patients with cystic fibrosis, Huntington's disease, and hemophilia. As part of the invention, a non-viral gene delivery technology is developed that combines plant-derived endosomal escape enhancers (glycosides; i.e. the saponins of the invention) and a targeting ligand (e.g. an antibody such as an sdAb) that are comprised in a single second conjugate of the invention, for combination with a first conjugate of the invention comprising a cell-surface molecule binding sdAb such as a V_{HH} and a gene therapeutic products. The resulting non-viral gene therapy based on the first and second conjugates of the invention displays about 40 times increased delivery efficiency at a lower dosage of the first conjugate comprising the gene over currently available strategies. Herewith, the second conjugate of the invention is for use in clinical applications such as for the repair or replacement of defective genes, like in cystic fibrosis patients, and for the targeted delivery of specific genes, for instance, to destroy cancer cells. In fact, the first and second conjugates of the invention are suitable for application in treatment regimens for any disease caused by a genetic defect - such as cystic fibrosis, Huntington's disease and hemophilia and which are currently incurable. Gene therapy which makes use of the first and second conjugates of the invention helps in overcoming two current problems: Firstly, it is possible with the second conjugate of the invention to deliver the first conjugate which comprises therapeutic genes to specific target cells in the body; secondly, the therapeutic genes enter the interior of these cells, but are not destroyed, due to the presence of saponin(s) and a targeting moiety such as an antibody or an sdAb for binding a target cell (second conjugate of the invention), and the presence of the oligonucleotide product and a targeting sdAb (first conjugate of the invention), for binding a target cell, for example by using an oligomeric or polymeric scaffold of the invention (covalent effector molecule conjugate of the invention) as part of the first and second conjugates of the invention.

The present invention also provides a method of treating cancer, the method comprising administering a medicament comprising a second conjugate according to the invention to a patient in need thereof in combination with a first conjugate of the invention, i.e. an ADC or an AOC comprising an effector molecule and a cell-surface molecule binding sdAb, preferably administering an effective dose of said medicament to a patient in need thereof, preferably a human cancer patient.

Considerations concerning forms suitable for administration are known in the art and include toxic effects, solubility, route of administration, and maintaining activity. For example, pharmacological compositions injected into the bloodstream should be soluble.

Suitable dosage forms, in part depend upon the use or the route of entry, for example transdermal or by injection. Such dosage forms should allow the compound to reach a target cell whether the target cell is present in a multicellular host. Other factors are known in the art, and include considerations such as toxicity and dosage form which retard the compound or composition from exerting its effect.

| TABLE A1. Saponins displaying (late) endosomal/lysosomal escape enhancing activity, and saponins comprising a structure reminiscent to such saponins displaying (late) endosomal/lysosomal escape enhancing activity | | | |
|---|---|---|---|
| **Saponin Name** | **Aglycone core** | **Carbohydrate substituent at the C-3beta-OH group** | **Carbohydrate substituent at the C-28-OH group** |
| NP-005236 | 2alpha-Hydroxyoleanolic acid | GlcA- | Glc/Gal- |
| AMA-1 | 16alpha-Hydroxyoleanolic acid | Glc- | Rha-(1→2)-[Xyl-(1→4)]-Rha- |
| AMR | 16alpha-Hydroxyoleanolic acid | Glc- | Rha-(1→2)-[Ara-(1→3)-Xyl-(1→4)]-Rha- |
| alpha-Hederin | Hederagenin (23-Hydroxyoleanolic acid) | Rha-(1→2)-Ara- | - |
| NP-012672 | 16alpha,23-Dihydroxyoleanolic acid | Ara/Xyl-(1→4)-Rha/Fuc-(1→2)-Glc/Gal-(1→2)-Rha/Fuc-(1→2)-GlcA- | Ara/Xyl- |
| NP-017777 | Gypsogenin | Gal-(1→2)-[Xyl-(1→3)]-GlcA- | Xyl-(1→4)-Rha-(1→2)-[R-(→4)]-Fuc- (R = 4E-Methoxycinnamic acid) |
| NP-017778 | Gypsogenin | Gal-(1→2)-[Xyl-(1→3)]-GlcA- | Xyl-(1→4)-Rha-(1→2)-[R-(→4)]-Fuc- (R = 4Z-Methoxycinnamic acid) |
| NP-017774 | Gypsogenin | Gal-(1→2)-[Xyl-(1→3)]-GlcA- | Xyl-(1→4)-[Gal-(1→3)]-Rha-(1→2)-4-OAc-Fuc- |
| NP-018110^{c}, NP-017772^{d} | Gypsogenin | Gal-(1→2)-[Xyl-(1→3)]-GlcA- | Xyl-(1→4)-[Glc-(1→3)]-Rha-(1→2)-3,4-di-OAc-Fuc- |
| NP-018109 | Gypsogenin | Gal-(1→2)-[Xyl-(1→3)]-GlcA- | Xyl-(1→4)-[Glc-(1→3)]-Rha-(1→2)-[R-(->4)]-3-OAc-Fuc- (R = 4E-Methoxycinnamic acid) |
| NP-017888 | Gypsogenin | Gal-(1→2)-[Xyl-(1→3)]-GlcA- | Glc-(1→3)-Xyl-(1→4)-[Glc-(1→3)]-Rha-(1→2)-4-OAc-Fuc- |
| NP-017889 | Gypsogenin | Gal-(1→2)-[Xyl-(1→3)]-GlcA- | Glc-(1→3)-Xyl-(1→4)-Rha-(1→2)-4-OAc-Fuc- |
| NP-018108 | Gypsogenin | Gal-(1→2)-[Xyl-(1→3)]-GlcA- | Ara/Xyl-(1→3)-Ara/Xyl-(1→4)-Rha/Fuc-(1→2)-[4-OAc-Rha/Fuc-(1->4)]-Rha/Fuc- |
| SA1641^{a}, AE X55^{b} | Gypsogenin | Gal-(1→2)-[Xyl-(1→3)]-GlcA- | Xyl-(1→3)-Xyl-(1→4)-Rha-(1→2)-[Qui-(1->4)]-Fuc- |
| NP-017674 | Quillaic acid | Gal-(1→2)-[Xyl-(1→3)]-GlcA- | Api-(1→3)-Xyl-(1→4)-[Glc-(1→3)]-Rha-(1→2)-Fuc- |
| NP-017810 | Quillaic acid | Gal-(1→2)-[Xyl-(1→3)]-GlcA- | Xyl-(1→4)-[Gal-(1→3)]-Rha-(1→2)-Fuc- |
| AG1 | Quillaic acid | Gal-(1→2)-[Xyl-(1→3)]-GlcA- | Xyl-(1→4)-[Glc-(1→3)]-Rha-(1→2)-Fuc- |
| NP-003881 | Quillaic acid | Gal-(1→2)-[Xyl-(1→3)]-GlcA- | Ara/Xyl-(1→4)-Rha/Fuc-(1→4)-[Glc/Gal-(1→2)]-Fuc- |
| NP-017676 | Quillaic acid | Gal-(1→2)-[Xyl-(1→3)]-GlcA- | Api-(1→3)-Xyl-(1→4)-[Glc-(1→3)]-Rha-(1->2)-[R-(->4)]-Fuc-(R = 5-O-[5-O-Ara/Api-3,5-dihydroxy-6-methyl-octanoyl]-3,5-dihydroxy-6-methyl-octanoic acid) |
| NP-017677 | Quillaic acid | Gal-(1→2)-[Xyl-(1→3)]-GlcA- | Api-(1→3)-Xyl-(1→4)-Rha-(1→2)-[R-(->4)]-Fuc-(R = 5-O-[5-O-Ara/Api-3,5-dihydroxy-6-methyl-octanoyl]-3,5-dihydroxy-6-methyl-octanoic acid) |
| NP-017706 | Quillaic acid | Gal-(1→2)-[Xyl-(1→3)]-GlcA- | Api-(1→3)-Xyl-(1→4)-Rha-(1→2)-[Rha-(1→3)]-4-OAc-Fuc- |
| NP-017705 | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4-OAc-Fuc- |
| NP-017773 | Quillaic acid | Gal-(1→2)-[Xyl-(1→3)]-GlcA- | 6-OAc-Glc-(1→3)-Xyl-(1→4)-Rha-(1→2)-[3-OAc-Rha-(1→3)]-Fuc- |
| NP-017775 | Quillaic acid | Gal-(1→2)-[Xyl-(1→3)]-GlcA- | Glc-(1→3)-Xyl-(1→4)-Rha-(1→2)-[3-OAc--Rha-(1->3)]-Fuc- |
| SA1657 | Quillaic acid | Gal-(1→2)-[Xyl-(1→3)]-GlcA- | Xyl-(1→3)-Xyl-(1→4)-Rha-(1→2)-[Qui-(1->4)]-Fuc- |
| AG2 | Quillaic acid | Gal-(1→2)-[Xyl-(1→3)]-GlcA- | Glc-(1→3)-[Xyl-(1→4)]-Rha-(1→2)-[Qui-(1->4)]-Fuc- |
| SO1861 | Quillaic acid | Gal-(1→2)-[Xyl-(1→3)]-GIcA- | Glc-(1→3)-Xyl-(1→4)-Rha-(1→2)-[Xyl-(1→3)-4-OAc-Qui-(1→4)]-Fuc- |
| GE1741 | Quillaic acid | Gal-(1→2)-[Xyl-(1→3)]-GlcA- | Xyl-(1→3)-Xyl-(1→4)-Rha-(1→2)-[3,4-di-OAc-Qui-(1→4)]-Fuc- |
| SO1542 | Quillaic acid | Gal-(1→2)-[Xyl-(1→3)]-GlcA- | GIc-(1→3)-[Xyl-(1→4)]-Rha-(1→2)-Fuc- |
| SO1584 | Quillaic acid | Gal-(1→2)-[Xyl-(1→3)]-GlcA- | 6-OAc-Glc-(1→3)-[Xyl-(1→4)]-Rha-(1→2)-Fuc- |
| SO1658 | Gypsogenin | Gal-(1→2)-[Xyl-(1→3)]-GlcA- | Glc-(1→3)-[Xyl-(1→3)-Xyl-(1→4)]-Rha-(1→2)-Fuc- |
| SO1674 | Quillaic acid | Gal-(1→2)-[Xyl-(1→3)]-GlcA- | Glc-(1→3)-[Xyl-(1→3)-Xyl-(1→4)]-Rha-(1→2)-Fuc- |
| SO1832 | Quillaic acid | Gal-(1→2)-[Xyl-(1→3)]-GlcA- | Xyl-(1→3)-Xyl-(1→4)-Rha-(1→2)-[Xyl-(1→3)-4-OAc-Qui-(1→4)]-Fuc- |
| QS-7 (also referred to as QS1861) | Quillaic acid | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-apio | Quillaic acid | Gal-(1→2)-[Xyl-(1→3)]-GlcA- | Api-(1→3)-Xyl-(1→4)-Rha-(1→2)-[R-(->4)]-Fuc-(R = 5-O-[5-O-Ara/Api-3,5-dihydroxy-6-methyl-octanoyl]-3,5-dihydroxy-6-methyl-octanoic acid) |
| QS-21 A-xylo | Quillaic acid | Gal-(1→2)-[Xyl-(1→3)]-GlcA- | Xyl-(1→3)-Xyl-(1→4)-Rha-(1→2)-[R-(->4)]-Fuc-(R = 5-O-[5-O-Ara/Api-3,5-dihydroxy-6-methyl-octanoyl]-3,5-dihydroxy-6-methyl-octanoic acid) |
| QS-21 B-apio | Quillaic acid | Gal-(1→2)-[Xyl-(1→3)]-GlcA- | Api-(1→3)-Xyl-(1→4)-Rha-(1→2)-[R-(->3)]-Fuc-(R = 5-O-[5-O-Ara/Api-3,5-dihydroxy-6-methyl-octanoyl]-3,5-dihydroxy-6-methyl-octanoic acid) |
| QS-21 B-xylo | Quillaic acid | Gal-(1→2)-[Xyl-(1→3)]-GlcA- | Xyl-(1→3)-Xyl-(1→4)-Rha-(1→2)-[R-(->3)]-Fuc-(R = 5-O-[5-O-Ara/Api-3,5-dihydroxy-6-methyl-octanoyl]-3,5-dihydroxy-6-methyl-octanoic acid) |
| beta-Aescin (described: Aescin la) | Protoaescigenin-21 (2-methylbut-2-enoate)-22-acetat | Glc-(1→2)-[Glc-(1→4)]-GlcA- | - |
| Teaseed saponin I | 23-Oxo-barringtogenol C-21,22-bis(2-methylbut-2-enoate) | Glc-(1→2)-Ara-(1→3)-[Gal-(1->2)]-GlcA- | - |
| Teaseedsaponin J | 23-Oxo-barringtogenol C - 21,22-bis(2-methylbut-2-enoate) | Xyl-(1→2)-Ara-(1→3)-[Gal-(1→2)]-GlcA- | - |
| Assamsaponin F | 23-Oxo-barringtogenol C-21 (2-methylbut-2-enoate)-16,22-diacetat | Glc-(1→2)-Ara-(1→3)-[Gal-(1→2)]-GlcA- | - |
| Digitonin | Digitogenin | Glc-(1→3)-Gal-(1→2)-[Xyl-(1→3)]-Glc-(1→4)-Gal- | - |
| Primula acid 1 | 3,16,28-Trihydroxyoleanan-12-en | Rha-(1→2)-Gal-(1→3)-[Glc-(1→2)]-GlcA- | - |
| AS64R | Gypsogenic acid | - | Glc-(1→3)-[Glc-(1→6)]-Gal- |
| | | **Carbohydrate substituent at the C-23-OH group** | |
| AS6.2 | Gypsogenic acid | Gal- | Glc-(1→3)-[Glc-(1→6)]-Gal- |
| a, b: Different names refer to different isolates of the same structure | | | |
| c, d: Different names refer to different isolates of the same structure | | | |

### EXAMPLES AND EXEMPLARY EMBODIMENTS

### Example 1. V_{HH}-SO1861 + mAb-saporin (1T2C and 2T2C)

The 1 target 2-components system (1T2C) is the combination treatment of V_{HH}-SO1861 and mAb-protein toxin, where V_{HH} and mAb recognize and bind the same cell surface receptor (Figure 1A). The 2 target 2-components system (2T2C) is the combination treatment of V_{HH}-SO1861 and mAb-protein toxin, where V_{HH} recognizes and binds a different cell surface receptor as the mAb (Figure 1B). SO1861-EMCH was conjugated (labile) via the terminal cysteine residue (Cys) to antiHER2V_{HH}, with a DAR 1, (HER2V_{HH}-SO1861). HER2V_{HH}-SO1861 was titrated on a fixed concentration of 10 pM CD71mab-saporin (CD71 monoclonal antibody conjugated to the protein toxin, saporin, with a DAR4) or 50 pM trastuzumab-saporin (trastuzumab conjugated to the protein toxin, saporin, with a DAR4). Targeted protein toxin mediated cell killing on SK-BR-3 (HER2⁺⁺ /CD71⁺) and MDA-MB-468 (HER2⁻/CD71⁺) was determined. This revealed enhanced cell killing at low concentrations of HER2V_{HH}-SO1861 in SK-BR-3 for both combinations with 10 pM CD71mab-saporin or 50 pM trastuzumab-saporin (IC50= 300 nM; Figure 2A). Equivalent concentrations of HER2V_{HH}-SO1861 alone induced cell killing at high concentrations (IC50= 4.000 nM), whereas equivalent concentrations of HER2V_{HH}, HER2V_{HH} + CD71mab-saporin or HER2V_{HH} + trastuzumab-saporin could not induce cell killing activity (IC50> 5000 nM; Figure 2A). In MDA-MB-468 (HER2⁻/CD71⁺) the combination of HER2V_{HH}-SO1861 + 10 pM CD71mab-saporin revealed cell killing activity at high concentrations (IC50= 2.000nM; Figure 2B), whereas the combination of HER2V_{HH}-SO1861 + 50 pM trastuzumab-saporin showed cell killing activity at much higher concentrations (IC50>5.000 nM; Figure 2B). Equivalent concentrations of HER2V_{HH}, HER2V_{HH} + CD71mab-saporin or HER2V_{HH} + trastuzumab-saporin could not induce cell killing activity in MDA-MB-468 cells (IC50>5.000 nM; Figure 2B).

All this shows that conjugation of SO1861-EMCH to a HER2 targeting V_{HH} enhances the endosomal escape and cytoplasmic delivery of a targeted protein toxin (targeting the same or different cell surface receptor) resulting in cell killing of HER2 expressing cells.

Next, trastuzumab-saporin or CD71mab-saporin was titrated on a fixed concentration of 900 nM HER2V_{HH}-SO1861 and targeted protein toxin mediated cell killing on SK-BR-3 (HER2⁺⁺ /CD71⁺) and MDA-MB-468 (HER2⁻/CD71⁺) was determined. This revealed that 900 nM HER2V_{HH}-SO1861 in combination with low concentrations trastuzumab-saporin or CD71mab-saporin induced already efficient cell killing of SK-BR-3 (IC50 = 0,0001 pM; Figure 3A), whereas CD71mab-saporin + 900 nM HER2V_{HH} or trastuzumab-saporin + 900 nM HER2V_{HH} could only induce cell killing at high concentrations (IC50 = 50 pM; IC50 = 400 pM resp; Figure 3). In MDA-MB-468 cells (HER2⁻/CD71⁺) CD71mab-saporin + 900 nM HER2V_{HH}-SO1861 showed cell killing at IC50 = 0,01 pM, whereas trastuzumab-saporin + 900 nM HER2V_{HH}-SO1861 showed activity at IC50 = 2.000 pM. Trastuzumab-saporin + 900 nM HER2V_{HH} or CD71mab-saporin + 900 nM HER2V_{HH} showed cell killing only at (IC50> 10.000pM and IC50 = 20 pM resp. Figure 3B). All this shows that relatively low concentrations of trastuzumab-saporin or CD71mab-saporin can be effective and induce cell killing in combination with relatively low HER2V_{HH}-SO1861 (DAR1) concentrations in HER2⁺⁺ /CD71⁺ expressing cells.

Note: throughout the description, including the examples, and in the claims, 'V_{HH}' and 'VHH' are to be understood as referring to the same type of single domain antibody (sdAb).

### Example 2. V_{HH}-SO1861 + V_{HH}-dianthin (2T2C)

The 2 target 2-components system (2T2C) is the combination treatment of V_{HH}1-SO1861 and V_{HH}2-protein toxin, where each V_{HH} recognizes another cell surface receptor (Figure 1C). SO1861-EMCH was conjugated to the terminal cysteine residues of the V_{HH} targeting HER2, producing HER2V_{HH}-SO1861 (DAR1). HER2V_{HH}-SO1861 was titrated on a fixed concentration of 50 pM CD71V_{HH}-dianthin and targeted protein toxin mediated cell killing on SK-BR-3 (HER2⁺⁺ /CD71⁺) and MDA-MB-468 (HER2⁻/CD71⁺) was determined. This revealed enhanced cell killing at relatively low concentrations of V_{HH}HER2-L-SO1861 (SK-BR-3: IC50 = 300 nM; Figure 4A). Equivalent concentrations of HER2V_{HH}-SO1861 alone induced cell killing at high concentrations (IC50 = 4.000 nM), whereas equivalent concentrations of HER2V_{HH}, HER2V_{HH} + 50 pM CD71V_{HH}-dianthin could not induce cell killing (IC50 > 5.000 nM; Figure 4A). In MDA-MB-468 (HER2⁻/CD71⁺) the combination of HER2V_{HH}-SO1861 + 50 pM CD71V_{HH}-dianthin revealed cell killing activity at higher concentrations (IC50 = 600 nM; Figure 4B), whereas equivalent concentrations of HER2V_{HH}, HER2V_{HH}-SO1861 or HER2V_{HH} + 50 pM CD71V_{HH}-dianthin could not induce cell killing activity (IC50 > 5.000 nM; Figure 4B).

Next, CD71V_{HH}-dianthin was titrated on a fixed concentration of 900 nM HER2V_{HH}-SO1861 and targeted protein toxin mediated cell killing on SK-BR-3 (HER2⁺⁺ /CD71⁺) and MDA-MB-468 (HER2⁻/CD71⁺) was determined. This revealed that 900nM HER2V_{HH}-SO1861 in combination with low concentrations CD71V_{HH}-dianthin induced efficient cell killing of SK-BR-3 cells (IC50 = 0,05 pM; Figure 5A), whereas CD71V_{HH}dianthin or CD71V_{HH}-dianthin + 900 nM HER2V_{HH} could only induce cell killing at high concentrations (IC50 > 10.000 pM); Figure 5A). Besides, CD71V_{HH}-dianthin was also titrated on a fixed concentration of 77 nM trastuzumab-SO1861 (DAR4) and this revealed also a strong enhancement in cell killing activity in SK-BR-3 (HER2⁺⁺ /CD71⁺) cells ((IC50 < 0,0001 pM). In MDA-MB-468 cells (HER2⁻/CD71⁺) CD71V_{HH}-dianthin + 900 nM HER2V_{H1}-SO1861 showed cell killing only at much higher concentrations (IC50 = 10 pM, Figure 5B), whereas CD71V_{HH}-dianthin, CD71V_{HH}-dianthin + 900 nM HER2V_{HH} or CD71V_{HH}-dianthin + trastuzumab-SO1861 (DAR4) showed cell killing only at IC50 = 2.000 pM; Figure 5B).

All this shows that relatively low concentrations of V_{HH}CD71-dianthin can be effective and induce cell killing in combination with low V_{HH}HER2-SO1861 conjugate concentrations in high HER2/CD71 expressing cells.

The combination according to the invention in MDA-MB-468 cells (HER2⁻/CD71⁺) did not reveal any cell killing activity. This shows that in the absence of sufficient receptor expression, effective intracellular delivered SO1861 concentrations are not reached (threshold) to induce endosomal escape and cytoplasmic delivery of the protein toxin.

### Example 3. V_{HH}-dianthin + mAb-SO1861 (1T2C and 2T2C)

The 1 target 2-components system (1T2C) is the combination treatment of mAb-SO1861 and V_{HH}-protein toxin, where mAb and V_{HH} recognize and bind the same cell surface receptor (Figure 1E). The 2 target 2-components system (2T2C) is also the combination treatment of mAb-SO1861 and V_{HH}-protein toxin, where the mAb and V_{HH} recognize another cell surface receptor (Figure 1D).

Dianthin-C (dianthin with a terminal cysteine) was conjugated to the terminal cysteine residues of the V_{HH} targeting HER2, V_{HH} targeting CD71 or V_{HH} targeting EGFR producing HER2V_{HH}-dianthin (DAR1), CD71V_{HH}-dianthin (DAR1) and EGFRV_{HH}-dianthin (DAR1).

CD71V_{HH}-dianthin, HER2V_{HH}-dianthin or EGFRV_{HH}-dianthin was titrated on a fixed concentration of cetuximab-SO1861 (DAR4) and targeted protein toxin mediated cell killing on A431 (EGFR⁺⁺/HER2^{+/-}/CD71⁺) and A2058 (EGFR⁻/HER2^{+/-}/CD71⁺) was determined. This revealed that very low concentrations CD71V_{HH}-dianthin in combination with 77 nM cetuximab-SO1861 induced efficient cell killing of A431 cells (IC50 < 0,0001 pM; Figure 6A), whereas CD71V_{HH}-dianthin alone showed activity at IC50 = 2000 pM. The other two combinations EGFRV_{HH}-dianthin + 77 nM cetuximab-SO1861 and HER2V_{HH}-dianthin + 77 nM cetuximab-SO1861 showed efficient cell killing at respectively IC50 = 20 pM and IC50 = 50 pM, whereas EGFRV_{HH}-dianthin or HER2V_{HH}-dianthin alone could not induce efficient cell killing in A431 cells (IC50 > 10.000 pM; Figure 6A). In A2058 cells (EGFR⁻/HER2^{+/-}/CD71⁺), CD71V_{HH}-dianthin and CD71V_{HH}-dianthin + 77 nM cetuximab-SO1861 showed cell killing activity at respectively, IC50 = 3.000 and IC50 = 1.000 pM, whereas all other treatments or combinations showed no cell killing up to IC50 = 10.000 pM V_{HH}-toxin in A2058 cells (Figure 6B).

This shows that cetuximab-SO1861 (DAR4) can efficiently induce endosomal escape of three different V_{HH}-dianthin conjugates, thereby inducing enhanced cell killing in A431 cells.

Next, CD71V_{HH}-dianthin, HER2V_{HH}-dianthin or EGFRV_{HH}-dianthin was titrated on a fixed concentration of trastuzumab-SO1861 (DAR4) and targeted protein toxin mediated cell killing on SK-BR-3 (HER2⁺⁺/EGFR⁻/CD71⁺) and MDA-MB-468 cells (HER2⁻/EGFR⁺⁺/CD71⁺) was determined. This revealed that very low concentrations CD71V_{HH}-dianthin in combination with 77 nM trastuzumab-SO1861 induced efficient cell killing of SK-BR-3 cells (IC50 < 0,0001 pM; Figure 7A), whereas CD71V_{HH}-dianthin alone showed activity at IC50 = 10.000 pM. The other two combinations EGFRV_{HH}-dianthin + 77 nM trastuzumab-SO1861 and HER2V_{HH}-dianthin + 77 nM trastuzumab-SO1861 showed efficient cell killing at respectively IC50 = 400 pM and IC50 = 6 pM, whereas EGFRV_{HH}-dianthin or HER2V_{HH}-dianthin alone could not induce efficient cell killing in SK-BR-3 cells (IC50 > 10.000 pM; Figure 7A). In MDA-MB-468 cells (HER2⁻/EGFR⁺⁺/CD71⁺), CD71V_{HH}-dianthin and CD71V_{HH}-dianthin + 77 nM cetuximab-SO1861 showed cell killing activity at respectively, IC50 = 3000 pM and IC50 = 2000 pM, whereas all other treatments or combinations showed no cell killing up to IC50 = 10.000 pM V_{HH}-dianthin in MDA-MB-468 cells (Figure 7B). This shows that trastuzumab-SO1861 (DAR4) can efficiently induce endosomal escape of three different V_{HH}-dianthin conjugates, thereby inducing enhanced cell killing in SK-BR-3 cells.

### Materials and methods

### materials

SO1861 was isolated and purified by Analyticon Discovery GmbH from raw plant extract obtained from *Saponaria officinalis.* V_{HH} were purchased from QVQ, Utrecht, The Netherlands (HER2V_{HH}: clone name: Q17c; CD71V_{HH}: clone name: Q52c EGFRV_{HH}: clone name: Q86c). Trastuzumab (Tras, Herceptin^{®}, Roche), Cetuximab (Cet, Erbitux^{®}, Merck KGaA) were purchased from the pharmacy (Charite, Berlin). CD71 monoclonal antibody was purchased from BioCell (Okt9, #BE0023). Custom trastuzumab-saporin and antiCD71mab-saporin conjugate was produced and purchased from Advanced Targeting Systems (San Diego, CA). Dianthin-Cys (Dia-Cys, Dianthin mutant with a single C-terminal cysteine was produced by Proteogenix, France.

Tris(2-carboxyethyl)phosphine hydrochloride (TCEP, 98 %, Sigma-Aldrich), 5,5-Dithiobis(2-nitrobenzoic acid) (DTNB, Ellman's reagent, 99%, Sigma-Aldrich), Zeba^{™} Spin Desalting Columns (2 mL, Thermo-Fisher), NuPAGE^{™} 4-12% Bis-Tris Protein Gels (Thermo-Fisher), NuPAGE^{™} MES SDS Running Buffer (Thermo-Fisher), Novex^{™} Sharp Pre-stained Protein Standard (Thermo-Fisher), PageBlue^{™} Protein Staining Solution (Thermo-Fischer), Pierce^{™} BCA Protein Assay Kit (Thermo-Fisher), N-Ethylmaleimide (NEM, 98 %, Sigma-Aldrich), 1,4-Dithiothreitol (DTT, 98 %, Sigma-Aldrich), Sephadex G25 (GE Healthcare), Sephadex G50 M (GE Healthcare), Superdex 200P (GE Healthcare), Isopropyl alcohol (IPA, 99.6 %, VWR), Tris(hydroxymethyl)aminomethane (Tris, 99%, Sigma-Aldrich), Tris(hydroxymethyl)aminomethane hydrochloride (Tris.HCL, Sigma-Aldrich), L-Histidine (99%, Sigma-Aldrich), D-(+)-Trehalose dehydrate (99%, Sigma-Aldrich), Polyethylene glycol sorbitan monolaurate (TWEEN 20, Sigma-Aldrich), Dulbecco's Phosphate-Buffered Saline (DPBS, Thermo-Fisher), Guanidine hydrochloride (99%, Sigma-Aldrich), Ethylenediaminetetraacetic acid disodium salt dihydrate (EDTA-Na₂, 99 %, Sigma-Aldrich), sterile filters 0.2 µm and 0.45 µm (Sartorius), Succinimidyl 4-(N-maleimidomethyl)cyclohexane-1-carboxylate (SMCC, Thermo-Fisher), , Vivaspin T4 and T15 concentrator (Sartorius), Superdex 200PG (GE Healthcare), Tetra(ethylene glycol) succinimidyl 3-(2-pyridyldithio)propionate (PEG₄-SPDP, Thermo-Fisher), HSP27 BNA disulfide oligonucleotide (Biosynthesis), [O-(7-Azabenzotriazol-1-yl)-N,N,N,N-tetramethyluronium-hexafluorphosphat] (HATU, 97%, Sigma-Aldrich), Dimethyl sulfoxide (DMSO, 99%, Sigma-Aldrich), N-(2-Aminoethyl)maleimide trifluoroacetate salt (AEM, 98 %, Sigma-Aldrich), L-Cysteine (98.5 %, Sigma-Aldrich), deionized water (DI) was freshly taken from Ultrapure Lab Water Systems (MilliQ, Merck), Nickel-nitrilotriacetic acid agarose (Ni-NTA agarose, Protino), Glycine (99.5 %, VWR), 5,5-Dithiobis(2-nitrobenzoic acid (Ellman's reagent, DTNB, 98 %, Sigma-Aldrich), S-Acetylmercaptosuccinic anhydride Fluorescein (SAMSA reagent, Invitrogen) Sodium bicarbonate (99.7 %, Sigma-Aldrich), Sodium carbonate (99.9 %, Sigma-Aldrich), PD MiniTrap desalting columns with Sephadex G-25 resin (GE Healthcare), PD10 G25 desalting column (GE Healthcare), Zeba Spin Desalting Columns in 0.5, 2, 5, and 10 mL (Thermo-Fisher), Vivaspin Centrifugal Filters T4 10 kDa MWCO, T4 100 kDa MWCO, and T15 (Sartorius), Biosep s3000 aSEC column (Phenomenex), Vivacell Ultrafiltration Units 10 and 30 kDa MWCO (Sartorius), Nalgene Rapid-Flow filter (Thermo-Fisher),

### methods

### SO1861-EMCH synthesis

To SO1861 (121 mg, 0.065 mmol) and EMCH.TFA (110 mg, 0.325 mmol) was added methanol (extra dry, 3.00 mL) and TFA (0.020 mL, 0.260 mmol). The reaction mixture stirred at room temperature. After 1.5 hours the reaction mixture was subjected to preparative MP-LC.¹ Fractions corresponding to the product were immediately pooled together, frozen and lyophilized overnight to give the title compound (120 mg, 90%) as a white fluffy solid. Purity based on LC-MS 96%.
LRMS (m/z): 2069 [M-1]¹⁻
LC-MS r.t. (min): 1.08⁴

### Cell viability assay

After treatment the cells were incubated for 72 hr at 37 °C before the cell viability was determined by a MTS-assay, performed according to the manufacturer's instruction (CellTiter 96^{®} AQueous One Solution Cell Proliferation Assay, Promega). Briefly, the MTS solution was diluted 20× in DMEM without phenol red (PAN-Biotech GmbH) supplemented with 10% FBS. The cells were washed once with 200 µL/PBS well, after which 100 µL diluted MTS solution was added/well. The plate was incubated for approximately 20-30 minutes at 37 °C. Subsequently, the OD at 492 nm was measured on a Thermo Scientific Multiskan FC plate reader (Thermo Scientific). For quantification the background signal of 'medium only' wells was subtracted from all other wells, before the cell viability percentage of treated/untreated cells was calculated, by dividing the background corrected signal of treated wells over the background corrected signal of the untreated wells (x 100).

### FACS analysis

Cells were seeded in DMEM (PAN-Biotech GmbH) supplemented with 10% fetal calf serum (PAN-Biotech GmbH) and 1% penicillin/streptomycin (PAN-Biotech GmbH), at 500,000 c/plate in 10 cm dishes and incubated for 48 hrs (5% CO₂, 37 °C), until a confluency of 90% was reached. Next, the cells were trypsinized (TryplE Express, Gibco Thermo Scientific) to single cells. 0.75 x 10⁶ Cells were transferred to a 15 mL falcon tube and centrifuged (1,400 rpm, 3 min). The supernatant was discarded while leaving the cell pellet submerged. The pellet was dissociated by gentle tapping the falcon tube on a vortex shaker and the cells were washed with 4 mL cold PBS (Mg²⁺ and Ca²⁺ free, 2% FBS). After washing the cells were resuspended in 3 mL cold PBS (Mg²⁺ and Ca²⁺ free, 2% FBS) and divided equally over 3 round bottom FACS tubes (1 mL/tube). The cells were centrifuged again and resuspended in 200 µL cold PBS (Mg²⁺ and Ca²⁺ free, 2% FBS) or 200 µL antibody solution; containing 5 µL antibody in 195 µL cold PBS (Mg²⁺ and Ca²⁺ free, 2% FBS). APC Mouse IgG1, κ APC anti-human EGFR (#352906, Biolegend) was used to stain the EGFR receptor. PE anti-human HER2 APC anti-human CD340 (erbB2/HER-2) (#324408 Biolegend ) was used to stain the HER2 receptor, PE Mouse IgG2a, κ Isotype Ctrl FC (#400212, Biolegend) was used as its matched isotype control. PE anti-human CD71 (#334106, Biolegend ) was used to stain the CD71 receptor, PE Mouse IgG2a, κ Isotype Ctrl FC (#400212, Biolegend) was used as its matched isotype control. Samples were incubated for 30 min at 4 °C on a tube roller mixer. Afterwards, the cells were washed 3x with cold PBS (Mg²⁺ and Ca²⁺ free, 2% FBS) and fixated for 20 min at room temperature using a 2% PFA solution in PBS. Cells were washed 2x with cold PBS, and resuspended in 250-350 µL cold PBS for FACS analysis. Samples were analyzed with a BD FACSCanto II flow cytometry system (BD Biosciences) and FlowJo software. Results of the analyses of the cell-surface expression of EGFR, HER2 and CD71 on the various cells is summarized in Table A2.

**Table A2. Cell surface expression levels of EGFR, HER2 and CD71 of various cells**

| **Cell line** | | **EGFR expression level (MFI)** | | **HER2 expression level (MFI)** | | **CD71 expression level (MFI)** | |
|---|---|---|---|---|---|---|---|
| | **MDA-MB-468** | | 1656 | | 1 | | 186 |
| | **A431** | | 1593 | | 10 | | 322 |
| | **SK-BR-3** | | 28 | | 1162 | | 331 |
| | **A2058** | | 1 | | 5 | | 59 |

### Procedure for the conjugation of V_{HH}-SO1861

To an aliquot of V_{HH} was added an aliquot of freshly prepared TCEP solution (10.0 mg/ml), the mixture vortexed briefly then incubated for 30 minutes at 20°C with roller-mixing. After incubation, the resulting V_{HH}-SH was purified by gel filtration using zeba spin desalting column into TBS pH 7.5. To the resulting V_{HH}-SH was added freshly prepared SPT-EMCH solution the mixture vortexed briefly then incubated overnight at 20°C.

After incubation, an aliquot of V_{HH}-SO1861 mixture was removed and characterised by Ellman's assay to ascertain SO1861 incorporation. The conjugate was purified by 1.6 × 35 cm Superdex 200PG column eluting with DPBS pH 7.5 to give purified V_{HH}-SO1861. The aliquot was filtered to 0.2 µm, concentrated and normalised to 1.0 mg/ml to afford V_{HH}-SO1861.

### Procedure for the conjugation of V_{HH}-Dianthin

Dianthin-Cys was concentrated by ultrafiltration using a vivaspin T15 10KDa MWCO centrifugal filter and buffer exchanged into TBS pH 7.5. To the concentrated Dianthin-Cys was added an aliquot of freshly prepared TCEP solution (10.0 mg/ml), the mixture vortexed briefly then incubated for 60 minutes at 20 °C with roller-mixing. After incubation, the resulting Dianthin-SH was purified by gel filtration using a zeba spin desalting column then repeated centrifugal-wash cycles using a vivaspin T15 10KDa MWCO centrifugal filter into TBS pH 7.5. The resulting Dianthin-SH was reacted with freshly prepared DTME solution (10 mg/ml) in DMSO, the mixture vortexed briefly then incubated for 60 minutes at 20°C. After, the Dianthin-DTME was obtained following purification by gel filtration using a zeba spin desalting column into TBS pH 7.5. The Dianthin-DTME was stored at 20°C until conjugated. At the same time, an aliquot of V_{HH} was concentrated by ultrafiltration using a vivaspin T15 10KDa MWCO centrifugal filter and buffer exchanged into TBS pH 7.5. To the concentrated V_{HH} was added an aliquot of freshly prepared TCEP solution (10.0 mg/ml), the mixture vortexed briefly then incubated for 60 minutes at 37°C with roller-mixing. After incubation, the resulting V_{HH} was purified by gel filtration using a zeba spin desalting column then repeated centrifugal-wash cycles using a vivaspin T4 5KDa MWCO centrifugal filter into TBS pH 7.5. An aliquot of the resulting V_{HH}-SH was reacted with Dianthin-DTME, the mixture vortexed briefly then incubated overnight at 20 °C. After, the reaction mixture was concentrated using a vivaspin T4 10KDa MWCO centrifuge tube and purified by gel filtration using a 1.6 × 35 cm Superdex 200PG column eluting into DPBS pH 7.5.

### Antibody-(L-SO1861)⁴

Trastuzumab, Cetuximab, are referred hereafter as "Ab". Ab was conjugated to the saponin SO1861-EMCH via Michael-type thiol-ene conjugation reaction at DARs of 1, 2, 3, 4, 5, and 6. The SO1861-EMCH molecule obtains a labile (L) hydrazone bond between its structure and its maleimide function generating a labile bond between the saponin and Ab. The procedure is exemplary described for Trastuzumab-(L-SO1861)₄:

To a solution of Cetuximab (40 mg, 8.0 ml) was added 10 µl/ml each of Tris concentrate (127 mg/ml, 1.05M), Tris.HCl concentrate (623 mg/ml, 3.95 M) and EDTA-Na₂ concentrate (95 mg/ml, 0.26 M) to give a 50 mM TBS, 2.5 mM EDTA buffer pH 7.5.

To Cetuximab divided into four portions (each of 9.73 mg, 4.864 mg/ml, 65 nmol) was added an aliquot of freshly prepared TCEP solution (0.5 - 2.0 mg/ml, 1.15 - 7.02 mole equivalents, 75 - 455 nmol), the mixtures vortexed briefly then incubated for 300 minutes at 20 °C with roller-mixing. After incubation (prior to addition of SO1861-EMCH), a *ca.* 1 mg (0.210 ml) aliquot of Ab-SH was removed from each mixture and purified by gel filtration using a zeba spin desalting column into TBS pH 7.5. These aliquots were characterized by UV-vis analysis and Ellman's assay (thiol to Ab ratio = 2.0, 4.2, 5.9 and 6.8 respectively). To each of the bulk Ab-SH was added an aliquot of freshly prepared SO1861-EMCH solution (2 mg/ml, 1.3 mole equivalents per 'thiol', 0.15 - 0.61 µmol, 0.16 - 0.63 ml), the mixtures vortexed briefly then incubated for 120 minutes at 20 °C. Besides each conjugation reaction, two aliquots of desalted Ab-SH (0.25 mg, 1.67 nmol) were reacted with NEM (1.3 mole equivalents per 'thiol', 4.3 - 17.4 nmol, 2.2 - 8.7 µl of a 0.25 mg/ml solution) or TBS pH 7.5 buffer (2.2 - 8.7 µl) for 120 minutes at 20 °C, as positive and negative controls, respectively. After incubation (prior to addition of NEM), a 0.200 ml aliquot of Ab - SO1861-EMCH mixture was removed and purified by gel filtration using zeba spin desalting column into TBS pH 7.5. This aliquot was characterized by UV-vis and alongside positive and negative controls were characterized by Ellman's assay to obtain SO1861-EMCH incorporations. To the bulk Ab - SO1861-EMCH mixture was added an aliquot of freshly prepared NEM solution (2.5 mg/ml, 2.5 - 10 mole equivalents, 0.15 - 0.58 µmol) and the mixtures purified by zeba spin desalting columns eluting with DPBS pH 7.5 to give purified Cetuximab - (L-SO1861) conjugates. The products were normalized to 2.5 mg/ml and filtered to 0.2µm prior to dispensing for biological evaluation. The reaction conditions and results for Trastuzumab-L-SO1861 conjugates and the reaction conditions and results for Cetuximab-L-SO1861 conjugates are summarized in Table A3 and Table A4.

**Table A3. Summarized reaction conditions and results for Trastuzumab-L-SO1861 conjugates**

| **Batch** | **Ab feed** | **TCEP feed mole equivalents** | **SO1861-EMCH feed** | **Obtained DAR** | **Purity by analytical SEC (%)** | **Yield (%)** |
|---|---|---|---|---|---|---|
| **Tras-(L-SO1861)₄** | 9.91 mg | 3.83 | 0.46 µmol | 4.0 | 98.4 | 81 |
| | 66 nmol | | | | | |

**Table A4. Summarized reaction conditions and results for Cetuximab-L-SO1861 conjugates**

| **Batch** | **Ab feed** | **TCEP feed mole equivalents** | **SO1861-EMCH feed** | **Obtained DAR** | **Purity by analytical SEC (%)** | **Yield (%)** |
|---|---|---|---|---|---|---|
| **Cet-(L-SO1861)₄** | 9.73 mg | 4.19 | 0.46 µmol | 4.1 | 99.0 | 77 |
| | 65 nmol | | | | | |

### Summary of a number of embodiments relating to antibodies and receptor ligands covalently bound to a protein toxin or to a saponin

**mAb:** trastuzumab (HER2) or cetuximab (EGFR)
**Ligand:** EGF
**Protein toxin:** Ribosome inactivating protein, saporin or dianthin
**endosomal escape enhancing conjugates of saponin with a ligand:**
**mAb-SO1861 endosomal escape enhancing conjugates**

| | |
|---|---|
| • contain a cleavable hydrazone linker | |
| • Trastuzumab-SO1861 | DAR 4.0 |
| • Cetuximab -SO1861 | DAR 3.7; |

**the endosomal escape enhancing conjugates of saponin with a ligand combined in *in vitro* or *in vivo* test models, with:**
**mAb/ligand-protein toxin conjugates**

| | |
|---|---|
| • contain a non-cleavable chemical linker or are recombinant fusion proteins | |
| • Trastuzumab-Saporin | DAR 3.0 |
| • Cetuximab-Saporin | DAR 2.6 |
| • Trastuzumab-Dianthin | DAR 1.0 |
| • EGF-Dianthin (fusion protein) | DAR 1.0 |
| • IgG-Saporin | DAR 2.2 |

### For Examples 4-9:

### Materials:

Trastuzumab and cetuximab were purchased from the pharmacy (Charite, Berlin). SO1861 was isolated and purified by Analyticon Discovery GmbH from raw plant extract obtained from *Saponaria officinalis L.*

### Methods

### SO1861-EMCH synthesis

SO1861 was from Saponaria officinalis L (Analyticon Discovery GmbH), and was coupled to EMCH according to conventional steps known in the art.

### Conjugation of SO1861 to antibodies

Custom production of trastuzumab-SO1861 and cetuximab-SO1861 was performed by FleetBioprocessing (UK). SO1861-EMCH was conjugated to cysteines of the antibody.

### Conjugation of Saporin to Trastuzumab and cetuximab

Custom trastuzumab-saporin and cetuximab-saporin conjugates were produced and purchased from Advanced Targeting Systems (San Diego, CA). IgG-saporin and saporin was purchased from Advanced Targeting Systems

### FACS analyses

FACS analysis was performed on a BD FACSCanto II, data analysis with FlowJo V10 software, FACS antibodies were: 1) Isotype: APC Mouse IgG1, κ Isotype Ctrl (FC) (400122, Biolegend). EGFR: APC anti-human EGFR (352906, Biolegend) HER2: APC anti-human CD340 (erbB2/HER-2) (324408, Biolegend).

### Dianthin production

Dianthin was expressed in a bacterium culture and the protein was purified following conventional cell culturing and protein purification steps known in the art.

### Conjugation of antibody to dianthin

Conjugation of antibody and dianthin was according to common procedures known in the art.

### Cell culture

Cells were cultured in DMEM (PAN-Biotech GmbH) supplemented with 10% fetal bovine serum (FBS) (PAN-Biotech GmbH) at 37 °C and 5% CO₂.

### Cell viability assay

Cells were seeded in a 96 well plate at 5.000-10.000 c/w in 100 µL/well and incubated overnight at 37°C. The next day 10x concentrated treatment-mix samples were prepared in PBS, which contain antibody-conjugated SO1861 (i.e. a 'binding molecule' or an 'endosomal escape enhancing conjugate' of the invention) and targeted-toxin (i.e. a 'binding molecule') both at 10x final concentration. The media was removed from the cell culture plate and replaced by 180 µL culture media, followed by the addition of 20 µL treatment-mix/well. For control, 10x treatment-mix samples were prepared that contained the corresponding concentrations of only antibody-conjugated SO1861, only antibody, only SO1861, only targeted-toxin, or PBS without compound as vehicle control. In the case that endosomal acidification inhibitors (chloroquine (Sigma Aldrich) or bafilomycin A1 (Enzo Life Sciences)) were used, the cell culture media in step 1 of treatment was replaced by 180 µL media containing 1 µM chloroquine or 0.2 µM bafilomycin A1. The plate was incubated for 1 hour at 37°C, before the 10x treatment-mix samples were added. The remaining incubation and treatment steps were performed according to standard procedures known in the field.

After treatment the cells were incubated for 72 hr at 37°C before the cell viability was determined by a MTS-assay, performed according to the manufacturer's instruction (CellTiter 96^{®} AQueous One Solution Cell Proliferation Assay, Promega). Briefly, the MTS solution was diluted 20x in DMEM without phenol red (PAN-Biotech GmbH) supplemented with 10% FBS (PAN-Biotech GmbH). The cells were washed once with 200 µL PBS per well, after which 100 µL diluted MTS solution was added per well. The plate was incubated for approximately 20-30 minutes at 37°C. Subsequently, the optical density at 492 nm was measured on a Thermo Scientific Multiskan FC plate reader (Thermo Scientific). For quantification the background signal of 'medium only' wells was subtracted from all other wells, before the ratio of untreated/treated cells was calculated, by dividing the background corrected signal of untreated wells over the background corrected signal of the treated wells.

### Results

### Example 4. 1 target 2-component system

1 target 2-components system is the combination treatment of mAb1-protein toxin and mAb1-SO1861 (see Figure 1A, E), whereas the 2 target 2-component system is the combination of mAb1-protein toxin and mAb2-SO1861 or mAb2-protein toxin + mAb1-SO1861 (Figure 1B-D).

Cetuximab-SO1861 (monoclonal antibody recognizing and binding EGFR, conjugated to the saponin molecule, SO1861; an endosomal escape enhancing conjugate) was titrated (calculated on concentration SO1861) on a fixed concentration of 10 pM cetuximab-saporin (monoclonal antibody recognizing and binding EGFR, conjugated to the protein toxin, saporin) and cell killing on high EGFR expressing cells was determined. High EGFR expressing cells (A431or CaSKi) showed efficient cell killing when 10 pM cetuximab-saporin was combined with high concentrations of non-targeted unconjugated SO1861 (A431: [SO1861] IC50 = 600 nM and Caski: [SO1861] IC50 = 700 nM; Figure 8A, 8B; Table A6). However, when cetuximab-saporin was combined with cetuximab-SO1861 strong cell killing was induced already at low concentrations of SO1861 (A431: [SO1861] IC50 = 5 nM and Caski [SO1861] IC50 = 8 nM; Figure 8A, 8B ; Table A6). This shows that targeted conjugated SO1861 is more effective in inducing endosomal escape compared to non-targeted unconjugated SO1861. Next, cetuximab-saporin was titrated on a fixed concentration of 300 nM cetuximab-SO1861 and targeted protein toxin mediated cell killing on EGFR expressing cells was determined. High EGFR expressing cells (A431 or CaSKi) show cell killing only at high cetuximab-saporin concentrations in combination with non-targeted unconjugated 300nM SO1861 (A431: [toxin] IC50 = 40 pM; CaSki: [toxin] IC50= 40 pM; Figure 8C, 8D; Table A7) whereas 300 nM cetuximab-SO1861 in combination with low cetuximab-saporin concentrations already induced efficient cell killing (A431: [toxin] IC50 = 0.4 pM; CaSKi: [toxin] IC50= 2 pM; Figure 8C and 8D; Table A7). Highest cell killing efficiency is achieved when high concentrations of non-targeted unconjugated SO1861 (1500 nM) is combined with low concentrations of cetuximab-saporin (A431: [toxin] IC50 = 0,03 pM; CaSki: [toxin] IC50 = 0,02 pM; Figure 8C, 8D; Table A7). All this shows that when conjugated to cetuximab, relatively low concentrations of SO1861 efficiently can kill high EGFR expressing cells in combination with relatively low concentrations of cetuximab-saporin. High concentrations (1500 nM) of non-targeted unconjugated SO1861 in combination with low concentrations of cetuximab-saporin is still most effective since receptor competition does not play a role for SO1861 to enter the cell, since in the 1-target 2-component system both conjugates compete for the same EGFR receptor. The receptor competition principle is also clearly shown in the cetuximab-toxin titration treatments without (A431: [toxin] IC50 = 40 pM; Caski: [toxin] IC50 = 40 pM) or with 75 nM cetuximab (A431: [toxin] IC50 = 1000 pM; Caski: from IC50 = 1000 pM; Figure 8C, 8D).

Next, cetuximab-SO1861 was titrated (calculated on concentration SO1861) on a fixed concentration of 10 pM cetuximab-saporin and cell killing on low/no EGFR expressing cells was determined. Low EGFR expressing cells (HeLa) showed only cell killing when 10 pM cetuximab-saporin was combined with high concentrations of non-targeted unconjugated SO1861, whereas A2058 cells that do not express EGFR (A2058) were not sensitive at all (HeLa: [SO1861] IC50 = 1000 nM; A2058: [SO1861] IC50 > 1000 nM; Figure 9A, 9B; Table A6). The combination of 10 pM cetuximab-saporin with increasing concentrations of cetuximab-SO1861 did not induce any significant cell killing in both cell lines (HeLa: [SO1861] IC50 = 1000 nM; A2058: [SO1861] IC50 > 1000 nM; Figure 9A, 9B; Table A6). This shows that in the absence of sufficient receptor expression, effective intracellular SO1861 concentrations are not reached (threshold) to induce endosomal protein toxin escape and toxin-mediated cell killing. Next, cetuximab-saporin was titrated on a fixed concentration of 300nM cetuximab-SO1861 and targeted protein toxin mediated cell killing on low/no EGFR expressing cells was determined. Low EGFR expressing cells (HeLa) show cell killing only at very high cetuximab-saporin concentrations in combination with 278 nM cetuximab-SO1861 or 300 nM unconjugated SO1861, whereas A2058 cells (EGFR) are not sensitive at any of the tested concentrations (HeLa: [toxin] IC50 = 60 pM; A2058: [toxin] IC50> 10.000 pM; Figure 9C, 9D; Table A7). High concentrations of unconjugated SO1861 (1500 nM) in combination with low concentrations of cetuximab-saporin in low EGFR expressing cells (HeLa) show efficient cell killing, whereas in A2058 cells only at very high cetuximab-saporin concentrations in combination with 1500 nM SO1861 non-targeted, a-specific cell killing is induced (Hela: [toxin] IC50 = 0,03 pM; A2058: [toxin] IC50 = 20 pM; Figure 9C, 9D; Table A6). All this shows that cells with low or no EGFR receptor expression are not susceptible for the combination of cetuximab-SO1861 + cetuximab-saporin, due to a lack of sufficient EGFR receptor that facilitates the entry of sufficient SO1861 and toxin within the cell.

Trastuzumab-SO1861 (monoclonal antibody recognizing and binding HER2, conjugated to the saponin molecule, SO1861; an endosomal escape enhancing conjugate according to the invention) was titrated (calculated on concentration SO1861) on a fixed concentration of 50 pM trastuzumab-saporin (monoclonal antibody recognizing and binding HER2, conjugated to the protein toxin, saporin) and cell killing on high HER2 expressing cells was determined. High HER2 expressing cells (SKBR3) showed efficient cell killing when 50 pM trastuzumab-saporin was combined with high concentrations of non-targeted unconjugated SO1861 (SKBR3; Figure 10A, 10B; Table A6). However, when trastuzumab-saporin was combined with trastuzumab-SO1861 strong cell killing was induced already at low concentrations of SO1861 (SKBR3; Figure 10A, 10B; Table A6). This shows that targeted conjugated SO1861 is more effective in inducing endosomal escape compared to non-targeted unconjugated SO1861. Next, trastuzumab-saporin was titrated on a fixed concentration of 50 nM trastuzumab-SO1861 and targeted protein toxin mediated cell killing on HER2 expressing cells was determined. High HER2 expressing cells (SKBR3 or BT474) show cell killing only at high trastuzumab-saporin concentrations in combination with non-targeted unconjugated 10 nM SO1861 (Table A7) whereas 10 nM trastuzumab-SO1861 in combination with low trastuzumab-saporin concentrations already induced efficient cell killing (Table A7). Highest cell killing efficiency is achieved when high concentrations of non-targeted unconjugated SO1861 (1500 nM) is combined with low concentrations of trastuzumab-saporin (Table A7). All this shows that when conjugated to trastuzumab, low concentrations of SO1861 efficiently can kill high HER2 expressing cells in combination with relatively low concentrations of trastuzumab-saporin. High concentrations (1500 nM) of non-targeted unconjugated SO1861 in combination with low concentrations of trastuzumab-saporin is still most effective, since receptor competition does not play a role for SO1861 to enter the cell, since in the 1-target 2-component system both conjugates compete for the same EGFR receptor. The receptor competition principle is also clearly shown in the trastuzumab-toxin titration treatments without or with 2,5 nM trastuzumab (SKBR3: [toxin] IC50 = 1000 nM).

Next, trastuzumab-SO1861 was titrated (calculated on concentration SO1861) on a fixed concentration of 50 pM trastuzumab-saporin and cell killing on low/no EGFR expressing cells was determined. Low EGFR expressing cells (JIMT1; A431) showed only cell killing when 50 pM trastuzumab-saporin was combined with high concentrations of non-targeted unconjugated SO1861 (JIMT1: [SO1861] IC50 > 1000 nM; A431: [SO1861] IC50 > 1000 nM; Figure 11A, 11B; Table A6). The combination of 50 pM trastuzumab-saporin with increasing concentrations of trastuzumab-SO1861 did not induce any significant cell killing in both cell lines (JIMT1: [SO1861] IC50 > 1000 nM; A431: [SO1861] IC50 > 1000 nM; Figure 11A, 11B; Table A6). This shows that in the absence of sufficient receptor expression, effective intracellular SO1861 concentrations are not reached (threshold) to induce endosomal protein toxin escape and toxin-mediated cell killing. Next, trastuzumab-saporin was titrated on a fixed concentration of 10 nM trastuzumab-SO1861 and targeted protein toxin mediated cell killing on low/no HER2 expressing cells was determined. Low HER2 expressing cells (JIMT1; A431) show no significant cell killing at high trastuzumab-saporin concentrations in combination with 10 nM trastuzumab-SO1861 (JIMT-1: [toxin] IC50 > 10.000 pM; A431: [toxin] IC50 > 10.000 pM; Figure 11C, 11D; Table A7). High concentrations of unconjugated SO1861 (1500 nM) in combination with low concentrations of trastuzumab-saporin in low HER2 expressing cells (show efficient cell killing (JIMT1: [toxin] IC50 = 0,1 pM; A431: [toxin] IC50 = 0,8 pM; Figure 11C, 11D; Table A5). All this shows that cells with low HER2 receptor expression are not susceptible for the combination of trastuzumab-SO1861 + trastuzumab-saporin, due to a lack of sufficient HER2 receptor that facilitates the entry of sufficient SO1861 and toxin within the cell.

### Example 5. 2 target 2-component system

Cetuximab-SO1861 was titrated (calculated on concentration SO1861) on a fixed concentration of 50 pM trastuzumab-saporin and cell killing on high EGFR/low HER2 expressing cells was determined. A431 and CaSki cells showed efficient cell killing when 50 pM trastuzumab-saporin was combined with high concentrations of non-targeted unconjugated SO1861 (A431 and Caski: [SO1861] IC50 = 1000 nM; Figure 12A, 12B; Table A6). However, when trastuzumab-saporin was combined with cetuximab-SO1861 strong cell killing was induced already at low concentrations of SO1861 (A431: [SO1861] IC50 = 12 nM and Caski [SO1861] IC50 = 40 nM; Figure 12A, 12B; Table A6). This shows that targeted conjugated SO1861 is more effective in inducing endosomal escape compared to non-targeted unconjugated SO1861. Next, trastuzumab-saporin was titrated on a fixed concentration of 300 nM cetuximab-SO1861 and targeted protein toxin mediated cell killing was determined on high EGFR/low HER2 expressing cells (A431 and CaSki) No effective cell killing was observed with high trastuzumab-saporin concentrations in combination with non-targeted unconjugated 300 nM SO1861 (A431 and Caski: [toxin] IC50 > 10.000 pM; Figure 12C, 12D; Table A7) whereas 300 nM cetuximab-SO1861 in combination with low trastuzumab-saporin concentrations already induced efficient cell killing (A431: [toxin] IC50 = 3 pM; Caski: [toxin] IC50 = 1 pM; Figure 12C and 12D; Table A7). In A431 cells, comparable cell killing efficiency is achieved when high concentrations (1500 nM) of non-targeted unconjugated SO1861 is combined with low concentrations of trastuzumab-saporin (A431: [toxin] IC50 = 1 pM; see Figure 12C; Table A7). In Caski cells the response was slightly stronger compared to the combination of cetuximab-SO1861 and Trastuzumab-saporin, due to the fact that the EGFR expression in these cells is significantly lower compared to A431 and thus targeted delivery of SO1861 to Caski cells is less sufficient (Caski: [toxin] IC50 = 0,2 pM see Figure 12D; Table A7). All this shows that when conjugated to cetuximab, low concentrations of SO1861 efficiently can kill high EGFR expressing cells in combination with relatively low concentrations of trastuzumab-saporin. High concentrations (1500nM) of non-targeted unconjugated SO1861 in combination with low concentrations of trastuzumab-saporin has comparable activity, since receptor competition does not play a role for SO1861 to enter the cell, since in the 2-target 2-component system both conjugates are delivered via different receptors, SO1861 via EGFR and toxin via HER2 receptor.

Next, cetuximab-SO1861 was titrated (calculated on concentration SO1861) on a fixed concentration of 50 pM trastuzumab-saporin and cell killing on low/no EGFR/HER2 expressing cells was determined. Low EGFR/HER2 expressing cells showed only cell killing when 50 pM trastuzumab-saporin was combined with high concentrations of non-targeted unconjugated SO1861 (HeLa: [SO1861] IC50 > 1000 nM; A2058: [SO1861] IC50 > 1000 nM; Figure 13A, 13B; Table A6). The combination of 50 pM trastuzumab-saporin with increasing concentrations of cetuximab-SO1861 only showed significant cell killing at high concentrations of cetuximab-SO1861 in both cell lines (HeLa: [SO1861] IC50 > 1000 nM; A2058: [SO1861] IC50 > 1000 nM; Figure 13A, 13B; Table A6). This shows that in the absence of sufficient receptor expression, effective intracellular SO1861 concentrations are not reached (threshold) to induce endosomal protein toxin escape and toxin-mediated cell killing. Next, trastuzumab-saporin was titrated on a fixed concentration of cetuximab-SO1861 and targeted protein toxin mediated cell killing on low/no EGFR/HER2 expressing cells was determined. Low/no EGFR/HER2 expressing cells (HeLa and A2058) show no significant cell killing at high trastuzumab-saporin concentrations in combination with 278 nM cetuximab-SO1861 (HeLa: [toxin] IC50 > 10.000 pM; A2058: [toxin] IC50 > 10.000 pM; Figure 13C, 13D; Table A7). High concentrations of unconjugated SO1861 (1500 nM) in combination with low concentrations of trastuzumab-saporin show efficient cell killing (HeLa: [toxin] IC50 = 0,4 pM; A2058: [toxin] IC50 = 0,5 pM; Figure 13C, 13D; Table A5). All this shows that cells with low/no EGFR/low HER2 expression are not susceptible for the combination of cetuximab-SO1861 + trastuzumab-saporin, due to a lack of sufficient EGFR receptor that facilitates the entry of sufficient SO1861 to ensure efficient cytoplasmic delivery of the toxin within the cell.

Next, Trastuzumab-SO1861 was titrated (calculated on concentration SO1861) on a fixed concentration of 1.5 pM EGF-dianthin and cell killing on high HER2/low EGFR expressing cells was determined. SKBR3 showed efficient cell killing when 1.5 pM EGF-dianthin was combined with high concentrations of non-targeted unconjugated SO1861 (SKBR3: [SO1861] IC50 = 800 nM; Figure 14A; Table A6). However, when EGF-dianthin was combined with trastuzumab-SO1861 strong cell killing was induced already at low concentrations of conjugated SO1861 (SKBR3: [SO1861] IC50 = 2 nM; Figure 14A; Table A5). This shows that targeted conjugated SO1861 is more effective in inducing endosomal escape compared to non-targeted unconjugated SO1861. Next, EGF-dianthin was titrated on a fixed concentration of trastuzumab-SO1861 and targeted protein toxin mediated cell killing was determined on SKBR3. No effective cell killing was observed with high EGF-dianthin concentrations in combination with non-targeted unconjugated 10 nM SO1861 (SKBR3 (shown) and BT474 (not shown): [toxin] IC50 > 10.000 pM; Figure 14B; Table A7) whereas 9.4 nM trastuzumab-SO1861 in combination with low EGF-dianthin concentrations already induced efficient cell killing (SKBR3: [toxin] IC50 = 3 pM (shown); BT474: [toxin] IC50 = 1 pM (not shown); Figure 14B; Table A7). Comparable cell killing efficiency is achieved when high concentrations (1075 nM) of non-targeted unconjugated SO1861 is combined with low concentrations of EGF-dianthin; Figure 14B; Table A7). All this shows that when conjugated to trastuzumab, low concentrations of SO1861 efficiently can kill high HER2 expressing cells in combination with relatively low concentrations of EGF-dianthin. High concentrations (1500 nM) of non-targeted unconjugated SO1861 in combination with low concentrations of EGF-dianthin has comparable activity, since receptor competition does not play a role for SO1861 to enter the cell, since in the 2-target 2-component system both conjugates are delivered via different receptors, SO1861 via HER2 and toxin via EGFR receptor.

Next, Trastuzumab-SO1861 was titrated (calculated on concentration SO1861) on a fixed concentration of 5 pM cetuximab-saporin and cell killing on low HER2, low/high EGFR expressing cells was determined showed cell killing when 5 pM cetuximab-saporin was combined with high concentrations of non-targeted unconjugated SO1861 (JIMT-1: [SO1861] IC50> 1000 nM; A431: [SO1861] IC50 > 1000 nM; Figure 15A, 22B; Table A6). The combination of 5 pM cetuximab-saporin with increasing concentrations of trastuzumab-SO1861 showed cell killing only at high concentrations of cetuximab-SO1861 in both cell lines (JIMT-1: [SO1861] IC50 > 1000 nM; A431: [SO1861] IC50 > 1000 nM; Figure 15A, 15B; Table A6). This shows that in the absence of sufficient receptor expression, effective intracellular SO1861 concentrations are not reached (threshold) to induce endosomal protein toxin escape and toxin-mediated cell killing. Next, cetuximab-saporin was titrated on a fixed concentration of trastuzumab-SO1861 and targeted protein toxin mediated cell killing on JIMT-1 and A431 was determined. Cell killing was observed only at high cetuximab-saporin concentrations in combination with 10 nM trastuzumab-SO1861 (JIMT-1: [toxin] IC50 > 90 pM; A431: [toxin] IC50 > 20 pM; Figure 15C, 15D; Table A7). High concentrations of unconjugated SO1861 (1500 nM) in combination with low concentrations of cetuximab-saporin show efficient cell killing (JIMT-1: [toxin] IC50 = 0,02 pM; A431: [toxin] IC50 = 0,03 pM; Figure 15C, 15D; Table A5). All this shows that cells with low HER2, low/high EGFR expression are not susceptible for the combination of trastuzumab-SO1861 + cetuximab-saporin, due to a lack of sufficient HER2 receptor that facilitates the entry of sufficient SO1861 to ensure efficient cytoplasmic delivery of the toxin within the cell. Even a very high EGFR expression in A431 cells did not result in efficient cell killing by cetuximab-saporin, since the threshold of SO1861 was not reached due to a lack of HER2 receptors that could facilitate the uptake of SO1861 via trastuzumab-SO1861.

### Example 6.

The 2 targeted 2 component system results in cell killing of very low target expressing cells. In A431 cells T-DM1 kills cells at nanomolar concentrations, whereas the targeted 2 component system can efficiently kill cells at picomolar concentrations (~7000 fold decrease in toxin concentration) (Figure 16)

### Example 7. Mechanism of action

When endosomal acidification is blocked the targeted 2 component system is not active, due to the fact that SPT001 (a plant-derived saponin, SO1861) is only active at low endosomal pH.

### Example 8

Endosomal acidification inhibitors block the targeted 2-component system activity showing that SO1861 function is reduced when acidification of endosomes is blocked.

### Example 9

Figure 17A-E displays the relative cell viability when trastuzumab (Fig. 17A), cetuximab (Fig. 17B) or T-DM1 (Fig. 17C), free toxins saporin (Fig. 17D) and dianthin (Fig. 17D), saporin coupled to a non-cell binding IgG (Fig. 17D), and saporin coupled to a non-cell binding IgG combined with free saponin SO1861 (Fig. 17E) are contacted with the indicated cell lines SK-BR-3, JIMT-1, MDA-MB-468, A431, CaSki, HeLa, A2058, BT-474.

Trastuzumab and cetuximab do not or hardly influence cell viability when exposed to most of the cell lines, with some effect on cell viability when trastuzumab is exposed to SK-BR-3 cells at relatively high dose, and with some effect on cell viability when cetuximab is exposed to MDA-MB-468 cells at relatively high dose.

TDM-1, or ado-trastuzumab emtansine, is a targeted therapy approved by the U.S. Food and Drug Administration to treat: HER2-positive metastatic breast cancer that has previously been treated with Herceptin (chemical name: trastuzumab) and taxane chemotherapy; early-stage HER2-positive breast cancer after surgery if residual disease was found after neoadjuvant (before surgery) treatment with Herceptin and taxane chemotherapy. The TDM-1 is a combination of Herceptin and the chemotherapy medicine emtansine. Fig. 17C shows that the TDM-1 results in decreased cell viability for all cell lines tested.

The free toxins saporin and dianthin and the toxin saporin coupled to a control IgG with no affinity for any of the cell surface molecules on the cell lines tested, do not or hardly have any influence on cell viability over a wide range of concentrations toxin tested, up to 10000 pM.

When the toxin saporin is coupled to a non-cell binding IgG, combining the conjugate with the free saponin SO1861 results in an IgG-saporin dose dependent decrease of the relative cell viability (Fig. 17E).

**Table A5. summary of IC50 values for mAb, toxin, ligand toxin or mAb-toxin monotherapy +/- SO1861.**

| **Cell line** | **EGFR expressi on level (MFI)** | **HER2 expressi on level (MFI)** | **Trastuzu mab (nM)** | **Cetuxim ab (nM)** | **T-DM1 (nM)** | **Saporin (pM)** | | **IgG-saporin (pM)** | | **Trastuzumab-Saporin (pM)** | | **Trastuzumab-Dianthin (pM)** | | **Cetuximab-Saporin (pM)** | | **EGF-Dianthin (pM)** | |
|---|---|---|---|---|---|---|---|---|---|---|---|---|---|---|---|---|---|
| | | | | | | No SO1861 | 1500 nM SO1861 | No SO1861 | 1500 nM SO1861 | No SO1861 | 1500 nM SO1861 | No SO1861 | 1500 nM SO1861 | No SO1861 | 1500 nM SO1861 | No AO1861 | 1500 nM SO1861 |
| MDA-MB-468 | 1656 | 1 | > 3.000 | > 3.000 | 60 | > 10.000 | 20 | > 10.000 | 30 | > 10.000 | 25 | > 10.000 | 200 | 180 | 0,06 | 240 | 0,09 |
| A431 | 1593 | 10 | > 3.000 | > 3.000 | 80 | > 10.000 | n.d. | > 10.000 | 90 | > 10.000 | 0,8 | > 10.000 | 60 | 40 | 0,03 | 700 | 0,03 |
| CaSki | 481 | 12 | > 3.000 | > 3.000 | 40 | > 10.000 | 15 | > 10.000 | 60 | > 10.000 | 0,2 | > 10.000 | 5 | 40 | 0,02 | 2000 | 0,03 |
| SK-BR-3 | 28 | 1162 | > 3.000 ** | > 3.000 | 1 | > 10.000 | n.d. | > 10.000 | 100 | 4500 | 0,05 | > 10.000 | 1 | n.d. | n.d. | > 10.000 | 0.5 |
| JIMT-1 | 58 | 74 | > 3.000 | > 3.000 | 10 | > 10.000 | 30 | > 10.000 | 90 | > 10.000 | 0,1 | > 10.000 | 40 | 300 | 0,02 | > 10.000 | 0,07 |
| HeLa | 91 | 7 | > 3.000 | > 3.000 | 40 | > 10.000 | 30 | > 10.000 | 200 | > 10.000 | 0,4 | > 10.000 | 18 | 60 | 0,03 | 3800 | 0,04 |
| A2058 | 1 | 5 | > 3.000 | > 3.0000 | 20 | > 10.000 | n.d. | > 10.000 | 90 | > 10.000 | 0,5 | > 10.000 | 5 | > 10.000 | 20 | > 10.000 | 30 |

| | | | | | | | | | | | | | | | | | |
|---|---|---|---|---|---|---|---|---|---|---|---|---|---|---|---|---|---|
| *Legend to Table A5:* ** MDA-MB-468 cells show a 20-25% reduction in cell viability at all Cetuximab [] above 5 nM* ** *SK-BR-3 cells show a 20% reduction in cell viability at 1 nM Trastuzumab and 30-35% reduction for all Trastuzumab [] above 1 nM* | | | | | | | | | | | | | | | | | |

**Table A6. Data summary of IC50 value of untargeted SO1861targeted 2-component system mAb-SO1861 titration with fixed [mAb-toxin].**

| **UntargetedSO1861** | | | | | | | **1-target** | | | | **2-target 2-component** | | |
|---|---|---|---|---|---|---|---|---|---|---|---|---|---|
| | | | | | | | Both SO1861 and toxin to EGFR | | Both SO1861 and toxin to HER2 | | SO1861 to EGFR; toxin to HER2 | SO1861 to HER2; toxin to EGFR | |
| Cell line | EGFR expre ssion level (MFI) | HER2 expre ssion level (MFI) | SO1861 + 50 pM Trastuz umab-saporin (IC50, nM) | SO1861 + 50 pM Trastuz umab-dianthin (IC50, nM) | SO186 1 + 10 pM Cetuxi mab-sapori n (IC50, nM) | SO1 861 + 10 pM EGF diant hin ( IC50 , nM) | 10 pM Cetuxi mab-Sapori n + Cetuxi mab-SO186 1 (IC50, nM) | 10 pM Dianthin :EGF + Cetuxim ab-SO1861 (IC50, nM) | 50 pM Trastuz umab-Saporin + Trastuz umab-SO1861 (IC50, nM) | 50 pM Trastuz umab-Dianthin + Trastuz umab-SO1861 (IC50, nM) | 50 pM Trastuz umab-Saporin + Cetuxim ab-SO1861 (IC50, nM) | 5 pM Cetuxim ab-Saporin + Trastuz umab-SO1861 (IC50, nM) | 1.5 pM Dianthin :EGF + Trastuz umab-SO1861 (IC50, nM) |
| MD A-MB-468 | 1656 | 1 | > 1.000 | > 1.000 | > 1.000 | > 1.00 0 | 3 | 6 | > 1.000 | > 1.000 | 18 | > 1.000 | > 1.000 |
| A43 1 | 1593 | 10 | > 1.000 | > 1.000 | 600 | > 1.00 0 | 5 | 8 | > 1.000 | > 1.000 | 12 | > 1.000 | > 1.000 |
| Ca Ski | 481 | 12 | > 1.000 | > 1.000 | 700 | > 1.00 0 | 5-10 | 10 | > 1.000 | > 1000 | 40 | > 1.000 | > 1.000 |
| SK-BR-3 | 28 | 1162 | 700 | n.d. | 800 | 650 | > 1.000 | > 1.000 | 2* | 3* | > 1.000 | n.d. | 3 |
| JIM T-1 | 58 | 74 | > 1.000 | > 1.000 | > 1.000 | > 1.00 0 | > 1.000 | > 1.000 | > 1.000 | > 1.000 | > 1.000 | > 1.000 | > 1.000 |
| HeL a | 91 | 7 | > 1.000 | > 1.000 | > 1.000 | > 1.00 0 | > 1.000 | > 1.000 | > 1.000 | > 1.000 | > 1.000 | > 1.000 | > 1.000 |
| A20 58 | 1 | 5 | > 1.000 | > 1.000 | > 1.000 | > 1.00 0 | > 1.000 | > 1.000 | > 1.000 | > 1.000 | > 1.000 | > 1.000 | > 1.000 |

**Table A7. Data summary of IC50 values for the targeted 2-component system, mAb-toxin titration with fixed [mAb-SO1861]. IC50 for each value is calculated as a percentage relative to the component(s) with constant concentration for that treatment (100%)**

| **1-target 2-component** | | | | | | | **2-component 2-target** | | | |
|---|---|---|---|---|---|---|---|---|---|---|
| | | | Both SO1861 and toxin to EGFR | | Both SO1861 and toxin to HER2 | | SO1861 to EGFR; toxin to HER2 | | SO1861 to HER2; toxin to EGFR | |
| Cell line | EGFR expressi on level (MFI) | HER2 expressi on level (MFI) | 278 nM Cetuxim ab-SO1861 + . Cetuxim ab-Saporin (IC50, pM) | 278 nM Cetuxim ab-SO1861 + EGF-Dianthin (IC50, pM) | 300 nM Trastuzum ab-SO1861 + Trastuzum ab-Saporin (IC50, pM) | 300 nM Trastuzum ab-SO1861 + Trastuzum ab-Dianthin (IC50, pM) | 278 nM Cetuximab -SO1861 + Trastuzum ab-Saporin (IC50, pM) | 1388 nM Cetuximab -SO1861 + Trastuzum ab-Dianthin (IC50, pM) | 300 nM Trastuzum ab-SO1861 + Cetuximab -Saporin (IC50, pM) | 300 nM Trastuzum ab-SO1861 + EGF-Dianthin (IC50, pM) |
| MDA -MB-468 | 1656 | 1 | 0.5 | 1 | > 10.000 | > 10.000 | 55 | 500 | 100 | 750 |
| A43 1 | 1593 | 10 | 0.4 | 0.4 | > 10.000 | > 10.000 | 3 | 30 | 20 | 2.300 |
| CaS ki | 481 | 12 | 2 | 1 | > 10.000 | > 10.000 | 1 | 25 | 14 | 1.600 |
| SK-BR-3 | 28 | 1162 | n.d. | n.d. | 20 | n.d. | n.d. | n.d. | n.d. | 2 |
| JIMT -1 | 58 | 74 | > 10.000 | > 10.000 | > 10.000 | > 10.000 | 3.000 | > 10.000 | 90 | 4.000 |
| HeL a | 91 | 7 | 6.000 | > 10.000 | > 10.000 | > 10.000 | > 10.000 | 10.000 | 50 | 4.000 |
| A20 58 | 1 | 5 | > 10.000 | > 10.000 | > 10000 | > 10.000 | > 10.000 | > 10.000 | > 10000 | > 10.000 |

### EXAMPLE 10 - TREATING A MAMMALIAN TUMOR-BEARING ANIMAL WITH A CONJUGATE OF THE INVENTION IN COMBINATION WITH AN ADC RESULTS IN SURVIVAL AND TUMOR REGRESSION

Female Balb/c nude mice were injected subcutaneously with a suspension of human A431 tumor cells. Under the skin of the mice, a human epidermal carcinoma developed in the xenograft animal tumor model. After injection of the tumor cells, the xenograft tumor was allowed to develop to a size of approximately 170-180 mm³. The A431 tumor cells have the following characteristics: high EGFR expressors, medium CD71 expressors, low HER2 expressors.

In Table 6A, the results of the treatment of control mice and tumor-bearing mice are presented. Tumor-bearing mice were treated with the indicated antibodies directed to either human Her2/neu, human EGFR, or human CD71, which are cell-surface receptors on the xenograft tumor. Cetuximab was covalently conjugated with saponin SO1861. The SO1861 was first provided with the linker EMCH (N-ε-maleimidocaproic acid hydrazide), which EMCH is a maleimide-and-hydrazide crosslinker for covalently conjugating sulfhydryls (reduced cysteines of the antibody)) to carbonyls (aldehyde or ketones; here the carbonyl of the aldehyde at position C-23 of the saponin). The saponin-EMCH was covalently coupled to reduced cysteines of the Cetuximab, forming a covalent thio-ether bond between the EMCH and the cysteine side chain. The ADCs trastuzumab-saporin (covalent conjugate) and anti-CD71 mAb (OKT-9, IgG) - saporin (covalent conjugate) were tested for their tumor-attacking efficacy in the mice, measured as tumor volume in time after start of the treatment with the ADCs. The dose of the ADCs was sub-optimal in the tumor model. That is to say, from previous experiments, it was established at which sub-optimal dose of the ADCs no tumor-regression or arrest of tumor growth would be observable.

These results demonstrate that the combination therapy of an ADC at a dose which is ineffective when treatment of tumor-bearing mice with the ADC alone is considered (tumor growths, death of the mice is not prevented (euthanasia)), with a conjugate of the invention consisting of a tumor-cell specific receptor targeting antibody covalently bound to a saponin, i.e. SO1861, the covalent conjugate administered to the mice suffering from cancer, at a non-effective dose when administered alone (tumor growths, death of the mice is not prevented (euthanasia)), provides an efficient and efficacious treatment regimen, expressed as tumors in regression and prolonged survival of the treated animals (beyond the duration of the experiment). The sub-optimal dose of ADC combined with a covalently bound saponin-comprising conjugate of the invention which has no anti-tumor activity when administered alone, thus provide for an effective treatment option for cancer patients, wherein a relative low dose of the ADC is efficacious. A lower dose of ADC bears the promise of less risk for adverse events, or even no side effects at all. In addition, the stimulatory effect of the saponin-bearing conjugate when the efficacy of the ADC is considered, shows that ADCs which previously have proven to lack efficacy when tumor patient treatment is concerned, may gain renewed attention and value, since ADC efficacy is improved in combination therapy setting, as the current example demonstrated. Reference is made to ADCs known in the art which were previously investigated in the human clinical setting, but then were for some ADCs retracted from further clinical investigation. Especially the ADCs for which clinical development was terminated due to observed lack of efficacy and/or due to occurrence of unacceptable adverse event are ADCs which may gain renewed value for cancer patients when combined with a covalently bound saponin-comprising conjugate, such as the cetuximab-saponin tested.

| **TABLE 6A: RESULTS OF TREATING A MAMMALIAN TUMOR-BEARING ANIMAL WITH A CONJUGATE OF THE INVENTION IN COMBINATION WITH AN ADC RESULTS IN SURVIVAL AND TUMOR REGRESSION** | | | |
|---|---|---|---|
| **Treatment group** | **Patient / healthy animal** | **treatment** | **tumor size (volume in mm³ or '+' for growth, '-' for regression, and 'stable' for growth nor regression)** |
| 1 | xenograft | vehicle | 2000 mm³ (death/euthanasia) |
| 2 | xenograft | Trastuzumab-saporin | 2000 mm³ (death/euthanasia) |
| 3 | xenograft | Anti-CD71 mAb OKT-9 - saporin (covalent conjugate) | 2000 mm³ (death/euthanasia) |
| 4 | xenograft | Cetuximab-SO1861 (covalent conjugate) | 2000 mm³ (death/euthanasia) |
| 5 | xenograft | Cetuximab | > 170 mm³, but < 2000 mm³ (death/euthanasia) |
| **6** | **xenograft** | **Trastuzumab-saporin** (covalent conjugate) **+ Cetuximab-SO1861** (covalent conjugate) | **Tumor regression from 180 mm³ at the start of treatment back to 80 mm³ (survival)** |
| **7** | **xenograft** | **Anti-CD71 mAb OKT-9-saporin** (covalent conjugate) **+ Cetuximab-SO1861** (covalent conjugate) | **Tumor regression from 180 mm³ at the start of treatment back to 40 mm³ (survival)** |

### EXAMPLE 11 - saponins mixture of Quillaja saponaria comprising QS-21, with endosomal/lysosomal escape enhancing activity

Scheme Q displays the common molecular structure of a series of QS-21 saponins (in part adapted from: Conrado Pedebos, Laércio Pol-Fachin, Ramon Pons, Cilaine V. Teixeira Hugo Verli, Atomic Model and Micelle Dynamics of QS-21 Saponin, Molecules 2014, 19, 3744-3760; four isoforms, wherein each of the depicted glycans can be bound as the R group). A mixture of water-soluble saponins obtained from Quillaja saponaria (Sigma-Aldrich, product No. S4521; Roth, Item No. 6857; InvivoGen, product 'Quil-A') may be applied in an endosomal/lysosomal escape enhancing conjugate, composition and combination of the invention, based on endosomal/lysosomal escape enhancing properties of at least one individual saponin present in the mixture, e.g. QS-21, or based on a combination of two or more of the saponins comprised by the mixture, such as QS-21 and QS-7.

The inventors demonstrated that the mixture of saponins from Quillaja saponaria at 2,5 microgram/ml dose was capable of enhancing endosomal escape of dianthin, as tested with mammalian tumor cells in a cell-based bioassay. The effector molecule exposed to the cells was dianthin covalently coupled to the ligand EGF: EGF-dianthin. Cells tested were tumor cell lines HeLa for free saponins, and A431, MDA-MB-468, CaSki and A2058 for testing the saponins when covalently coupled to cetuximab.

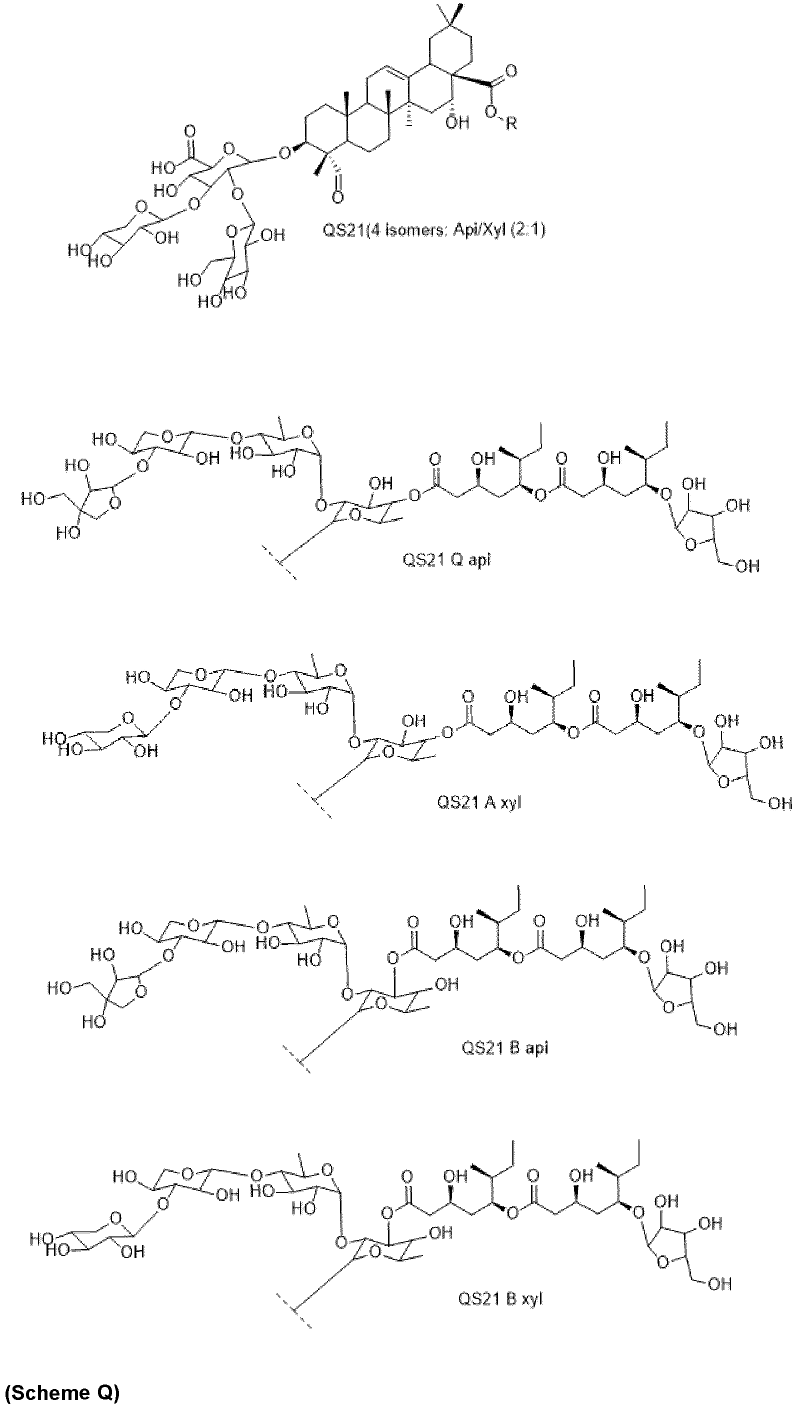

### Example 12

The 1 target 2-components system (1T2C) is the combination treatment of mAb1-protein toxin and mAb1-SO1861, as illustrated in Figure 1A, E. SO1861-EMCH was conjugated via cysteine residues (Cys) and HSP27BNA oligo was conjugated via lysine residues to cetuximab (monoclonal antibody recognizing and binding human EGFR), both with a DAR 4 resulting in the production of 2 conjugates: cetuximab-(Cys-L-SO1861)⁴ and cetuximab-(Lys-L-HSP27BNA)⁴. The combination of cetuximab-(Cys-L-SO1861)⁴ (intraperitoneal administration, (i.p.)) and cetuximab-(Lys-L-HSP27BNA)⁴ (intravenous administration, (i.v.)) was tested in a A431 xenograph mouse tumor model for EGFR tumor targeted gene silencing activity. Dosings started at day 12 when tumors reached ~150 mm³ in size and tumor samples were collected at 72 h after the first dosing and analysed for HSP27 gene expression compared to control gene mRNA expression levels (reference genes). This revealed that 1 dosing of 50 mg/kg cetuximab-(Cys-L-SO1861)⁴ + 25 mg/kg cetuximab-(Lys-L-HSP27BNA)⁴ resulted in a 50% reduction in HSP27 gene expression in the A431 tumors compared to single dosing of cetuximab-(Cys-L-SO1861)⁴ or cetuximab-(Lys-L-HSP27BNA)⁴ mono therapies (Figure 18). Compared to the vehicle control tumors, a reduction of 40% HSP27 gene silencing was observed. This shows and enables that the combination of cetuximab-conjugated SO1861 + cetuximab-conjugated HSP27BNA oligo, according to the 1T2C invention, induces efficient targeted delivery of a therapeutic antisense oligo nucleotide in the cytoplasm of solid tumor cells, thereby inducing tumor targeted gene silencing, *in vivo.*

Next, SO1861-EMCH was conjugated via cysteine residues (Cys) to trastuzumab (monoclonal antibody recognizing and binding human HER2), with a DAR 4 resulting in the production of trastuzumab-(Cys-L-SO1861)⁴. The combination of trastuzumab-(Cys-L-SO1861)⁴ and trastuzumab-saporin (trastuzumab protein toxin conjugate) was tested in a mouse tumor model (patient derived xenograph tumor model, PDX) with high HER2 expression levels and resistant for trastuzumab mono therapy. The combination, according to the 1T2C invention of 40 mg/kg trastuzumab-(Cys-L-SO1861)⁴ (intraperitoneal administration, (i.p.)) + 0.03 (Day1, 8)/ 0.02 (Day 15, 22, 30, 36, 43) mg/kg trastuzumab-saporin (intravenous administration, (i.v.)) revealed strong tumor growth inhibition compared to the vehicle control and the 40 mg/kg trastuzumab-(Cys-L-SO1861)⁴ or 0.03/0.02 mg/kg trastuzumab-saporin mono therapies (Figure 19). Besides, in tumor bearing mice that were treated with a lower dosing combination (40 mg/kg trastuzumab-(Cys-L-SO1861)⁴ + 0.01 mg/kg trastuzumab-saporin) no tumor growth inhibiting activity was observed (Figure 26). This shows and enables that the 1T2C combination of trastuzumab conjugated SO1861 + trastuzumab conjugated protein toxin induces efficient targeted delivery of a therapeutic protein toxin in the cytoplasm of solid tumor cells, thereby inducing tumor cell death and tumor growth inhibition, *in vivo.*

### Example 13. 2 target 2-component system (in vivo)

The 2 target 2-components system (2T2C) is the combination treatment of mAb1-SO1861 and mAb2-protein toxin, (Figure 1B-D). SO1861-EMCH was conjugated via cysteine residues (Cys) to cetuximab (monoclonal antibody recognizing and binding human EGFR), with a DAR 4 resulting in the production of: cetuximab-(Cys-L-SO1861)⁴. The combination of cetuximab-(Cys-L-SO1861)⁴ and trastuzumab-saporin or CD71mab-saporin was tested in a A431 (EGFR⁺⁺/HER2⁺ /CD71⁺) xenograph 'nude' mouse tumor model for EGFR tumor targeted cell killing as illustrated in Figure 1B-D. Dose escalation was performed to determine the therapeutic efficacy *(Day 9: 0.3 mg*/*kg trastuzumab-saporin or 0.1 mg*/*kg CD71mab-saporin* + *5 mg*/*kg cetuximab-(Cys-L-SO1861)⁴; Day 14, 18: 0.1 mg*/*kg trastuzumab-saporin or 0.05 mg*/*kg CD71mab-saporin* + *5 mg*/*kg cetuximab-(Cys-L-SO1861)⁴; Day 21: 0.05 mg*/*kg trastuzumab-saporin or 0.05 mg*/*kg CD71mab-saporin* + *15 mg*/*kg cetuximab-(Cys-L-SO1861)⁴; Day 28: 0.02 mg*/*kg trastuzumab-saporin or 0.02 mg*/*kg CD71mab-saporin + 15 mg*/*kg cetuximab-(Cys-L-SO1861)⁴ trastuzumab-saporin*/*cetuximab-SO1861. Controls were on the same dosing scheme respectively, only cetuximab (i.v.) was given 25 mg*/*kg every treatment day).* At day 32 (dashed vertical line), day 35 and day 39 the inventors started the combination, according to the 2T2C invention of 25 mg/kg cetuximab-(Cys-L-SO1861)⁴ (intraperitoneal injection (i.p.) + 0.02 mg/kg trastuzumab-saporin or 0.02 CD71mab-saporin (intravenous administration, (i.v.)) and this revealed strong tumor regression for both 2T2C combination groups compared to the vehicle control, 25 mg/kg cetuximab-(Cys-L-SO1861)⁴ or 0.02 mg/kg trastuzumab-saporin/CD71mab-saporin mono therapies (Figure 20). The 2T2C system even outcompetes cetuximab, the clinically used monoclonal antibody against EGFR. Next the inventors performed the same experiment but then the test was started with 25 mg/kg cetuximab-(Cys-L-SO1861)⁴ (intraperitoneal injection (i.p.) + 0.03 mg/kg trastuzumab-saporin or 0.03 CD71mab-saporin (intravenous administration, (i.v.)) treatment with a dosing at day 9 and 14 and thereafter 1 dosing per week. The 2T2C system according to the invention showed tumor regression in all mice and even in 1 mice in both 2T2C groups, complete tumor eradication (tumor volume= 0 mm³) (Figure 21). Also here the controls showed a strong increased in tumor volume whereas the positive control for this A431 mice model, cetuximab showed only tumor growth inhibition, but no regression (Figure 21). This shows and enables the 2T2C system approach, of cetuximab conjugated SO1861 + trastuzumab conjugated protein toxin or CD71mab conjugated protein toxin inducing highly efficient targeted delivery of a therapeutic protein toxin in the cytoplasm of solid tumors of tumor bearing mice, *in vivo,* thereby inducing even full tumor eradication in some mice and strong tumor regression in others even in large size tumors (2000 mm³).

### Example 14. 2 target 2-component system (in vitro)

### Results

The 2 target 2-components system (2T2C) is the combination treatment of mAb1-SO1861 and mAb2-protein toxin, (Figure 1B-D). SO1861-EMCH was conjugated via cysteine residues (Cys) to cetuximab (monoclonal antibody recognizing and binding human EGFR), with a DAR 3,7 (cetuximab-(Cys-L-SO1861)^{3,7}). Cetuximab-(Cys-L-S01861)^{3,7} was titrated on a fixed concentration of 50 pM trastuzumab-saporin (trastuzumab, conjugated to the protein toxin, saporin) and targeted protein toxin mediated cell killing on EGFR/HER2 expressing cells (A431, EGFR⁺⁺/HER2^{+/-}; CaSKi, EGFR⁺/HER2^{+/-}) was determined as illustrated in Figure 1B-D. This revealed strong cell killing at low concentrations of cetuximab-(Cys-L-SO1861)^{3,7} (A431: IC50 = 3 nM and CaSKi IC50 = 10 nM; Figure 22A, 22B) whereas equivalent concentrations cetuximab, cetuximab-(Cys-L-SO1861)^{3,7} or cetuximab + 50 pM trastuzumab -saporin could not induce any cell killing activity in EGFR/HER2 expressing cells. This shows that relatively low concentrations of cetuximab-SO1861 conjugate efficiently enhances endosomal escape of the trastuzumab conjugated protein toxin (at non-effective concentrations), thereby inducing efficient cell killing of high EGFR/low HER2 expressing cells.

Next, trastuzumab-saporin was titrated on a fixed concentration of 75 nM cetuximab-(Cys-L-SO1861)^{3,7} and targeted protein toxin mediated cell killing on EGFR/HER2 expressing cells was determined. This revealed that 75 nM cetuximab-(Cys-L-SO1861)^{3,7} in combination with low concentrations trastuzumab-saporin induced already efficient cell killing in EGFR/HER2 expressing cells (A431: IC50 = 5 pM; and CaSKi: IC50 = 1 pM; Figure 22C and 22D), whereas trastuzumab-saporin alone or trastuzumab-saporin + 75 nM cetuximab did not show significant cell killing activity (IC50 > 10.000 pM) in both cell lines (Figure 22C, 22D). All this shows that relatively low concentrations of trastuzumab-saporin can be effective and induce cell killing in combination with low cetuximab-SO1861 conjugate concentrations in high EGFR/low HER2 expressing cells.

Next, cetuximab-(Cys-L-SO1861)^{3,7} was titrated on a fixed concentration of 50 pM trastuzumab-saporin and targeted protein toxin-mediated cell killing on HeLa (EGFR^{+/-}/HER2^{+/-}) or A2058 (EGFR⁻/HER2^{+/-}) was determined as illustrated in Figure 1B-D. Both HeLa (EGFR^{+/-}/HER2^{+/-}) and A2058 (EGFR⁻/HER2^{+/-}) cells do not show cell killing at low concentrations of cetuximab-(Cys-L-SO1861)^{3,7} + 50 pM trastuzumab-saporin (HeLa: IC50 = 400 nM; A2058: IC50 > 400 nM; Figure 230A, 23B). This shows that in the absence of sufficient receptor expression, effective intracellular delivered SO1861 concentrations are not reached (threshold) to induce endosomal escape and cytoplasmic delivery of the protein toxin. Next, trastuzumab-saporin was titrated on a fixed concentration of 75 nM cetuximab-(Cys-L-SO1861)^{3,7} and targeted protein toxin mediated cell killing on HeLa (EGFR^{+/-}/HER2^{+/-}) or A2058 (EGFR⁻/HER2^{+/-}) was determined. Both HeLa (EGFR^{+/-}/HER2^{+/-}) and A2058 (EGFR⁻/HER2^{+/-}) cells showed no cell killing activity (HeLa: IC50 > 10.000 pM; A2058: IC50 > 10.000 pM; Figure 23C, 23D). All this shows that cells with low or no EGFR receptor expression are not susceptible for the combination of cetuximab-(Cys-L-SO1861)^{3,7} + trastuzumab-saporin, due to a lack of sufficient EGFR receptor that facilitates the antibody-mediated delivery of sufficient SO1861 (threshold) to ensure endosomal escape of the toxin within the cytoplasm of the cell.

Next, SO1861-EMCH was conjugated via cysteine residues (Cys) to trastuzumab (monoclonal antibody recognizing and binding human HER2), with a DAR 4 (trastuzumab-(Cys-L-SO1861)⁴). Trastuzumab-(Cys-L-SO1861)⁴ was titrated on a fixed concentration of 1.5 pM EGFdianthin (EGFR targeted ligand toxin fusion protein) and targeted protein toxin mediated cell killing on HER2/EGFR expressing cells (SK-BR-3: HER2⁺⁺/EGFR^{+/-}) was determined. This revealed strong cell killing at low concentrations of trastuzumab-(Cys-L-SO1861)⁴ + 1.5 pM EGFdianthin (SK-BR-3: IC50 = 1 nM; Figure 24A) whereas equivalent concentrations trastuzumab, trastuzumab-(Cys-L-SO1861)⁴ or trastuzumab + 1.5 pM EGFdianthin could not induce any cell killing activity in HER2⁺⁺/EGFR^{+/-} expressing cells. This shows that trastuzumab conjugated SO1861 efficiently enhances endosomal escape of the EGF fusion protein toxin (at non-effective concentrations), thereby inducing cell killing of high HER2/low EGFR expressing cells.

Next, EGFdianthin was titrated on a fixed concentration of 2.5 nM trastuzumab-(Cys-L-SO1861)⁴ and targeted protein toxin mediated cell killing on SK-BR-3 (HER2⁺⁺/EGFR^{+/-}) expressing cells was determined. This revealed that 2.5 nM trastuzumab-(Cys-L-SO1861)⁴ in combination with low concentrations EGFdianthin induced already efficient cell killing in HER2/EGFR expressing cells (SK-BR-3: IC50 = 1 pM) (Figure 24B), whereas EGFdianthin alone or EGFdianthin + 2.5 nM trastuzumab showed no cell killing activity (IC50 > 10.000 pM) (Figure 24B). All this shows that relatively low concentrations of EGFdianthin can be effective and induce cell killing only in combination with low trastuzumab-(Cys-L-SO1861)⁴ concentrations in high HER2/low EGFR expressing cells.

Next, trastuzumab-(Cys-L-SO1861)⁴ was titrated on a fixed concentration of 1.5 pM EGFdianthin and targeted protein toxin mediated cell killing on JIMT-1 (HER2^{+/-}/EGFR^{+/-}) or MDA-MB-468: HER2⁻/EGFR⁺⁺) was determined. Both cell lines were not sensitive for any combination of trastuzumab-(Cys-L-SO1861)⁴ + 1.5 pM EGFdianthin (JIMT-1: IC50 > 1000 nM; MDA-MB-468: IC50 > 1000 nM; Figure 25A, 25B). This shows that in the absence of sufficient HER2 receptor expression, effective intracellular delivered SO1861 concentrations are not reached (threshold) to induce endosomal escape and cytoplasmic delivery of the protein toxin.

Next, EGFdianthin was titrated on a fixed concentration of 2.5 nM trastuzumab-(Cys-L-SO1861)⁴ and targeted protein toxin mediated cell killing on JIMT-1 (HER2^{+/-}/EGFR^{+/-}) or MDA-MB-468 (HER2⁻/EGFR⁺⁺) was determined. Both cell lines showed cell killing at high EGFdianthin concentrations with or without 2.5 nM trastuzumab-(Cys-L-SO1861)⁴ (JIMT-1: IC50 = 10.000 pM; MDA-MB-468: IC50 = 200 pM Figure 25C, 25B).

All this shows that cells with low or no HER2 receptor expression are not susceptible for the combination of trastuzumab-(Cys-L-SO1861)^{3,7} + 1.5 pM EGFdianthin, due to a lack of sufficient HER2 receptor that facilitates the antibody-mediated delivery of sufficient SO1861 (threshold) to ensure endosomal escape of the toxin within the cytoplasm of the cell.

Next, SO1861-EMCH was conjugated via cysteine residues (Cys) to trastuzumab (monoclonal antibody recognizing and binding human HER2), with a DAR 4, (trastuzumab-(Cys-L-SO1861)⁴). Trastuzumab-(Cys-L-SO1861)⁴ was titrated on a fixed concentration of 5 pM cetuximab-saporin (EGFR targeting antibody-protein toxin conjugate) and targeted protein toxin mediated cell killing on HER2/EGFR expressing cells (SK-BR-3: HER2⁺⁺/EGFR^{+/-}) was determined as illustrated in Figure 1B-D. This revealed strong cell killing at low concentrations of trastuzumab-(Cys-L-SO1861)⁴ + 5 pM cetuximab-saporin (SK-BR-3: IC50 = 1 nM; Figure 26A) whereas equivalent concentrations trastuzumab, trastuzumab-(Cys-L-SO1861)⁴ or trastuzumab + 5 pM cetuximab-saporin could not induce any cell killing activity in HER2⁺⁺/EGFR^{+/-} expressing cells. This shows that trastuzumab conjugated SO1861 efficiently enhances endosomal escape of the cetuximab conjugated protein toxin (at non-effective concentrations), thereby inducing cell killing of HER2⁺⁺/EGFR^{+/-} expressing cells.

Next, cetuximab-saporin was titrated on a fixed concentration of 2.5 nM trastuzumab-(Cys-L-SO1861)⁴ and 75 nM trastuzumab-(Cys-L-SO1861)⁴ and targeted protein toxin mediated cell killing on HER2/EGFR expressing cells (SK-BR-3: HER2⁺⁺/EGFR^{+/-}) was determined. This revealed that 2.5 nM trastuzumab-(Cys-L-SO1861)⁴ in combination with low concentrations cetuximab-saporin induced already efficient cell killing in SK-BR-3 cells (SK-BR-3: IC50 = 1 pM; Figure 26B), whereas cetuximab-saporin alone or cetuximab-saporin + 2.5 nM trastuzumab showed cell killing only at high concentrations trastuzumab-saporin (SK-BR-3: IC50 > 4000 pM; Figure 26B). All this shows that relatively low concentrations of cetuximab-saporin can be effective and induce cell killing only in combination with low trastuzumab-(Cys-L-SO1861)⁴ concentrations in HER2⁺⁺/EGFR⁺ expressing cells.

Next, trastuzumab-(Cys-L-SO1861)⁴ was titrated on a fixed concentration of 5 pM cetuximab-saporin and targeted protein toxin mediated cell killing on JIMT-1 (HER2^{+/-}/EGFR^{+/-}) and MDA-MB-468 (HER2⁻/EGFR⁺⁺) cells was determined. Both cell lines were not sensitive for the combination of trastuzumab-(Cys-L-SO1861)⁴ + 5 pM cetuximab-saporin (JIMT-1: IC50 > 1000 nM; MDA-MB-468: IC50 > 1000 nM; Figure 27A, 27B). This shows that in the absence of sufficient HER2 receptor expression, effective intracellular delivered SO1861 concentrations are not reached (threshold) to induce endosomal escape and cytoplasmic delivery of the protein toxin.

Next, cetuximab-saporin was titrated on a fixed concentration of 2.5 nM trastuzumab-(Cys-L-SO1861)⁴ and targeted protein toxin mediated cell killing on JIMT-1 (HER2^{+/-}/EGFR^{+/-}) and MDA-MB-468 (HER2⁻/EGFR⁺⁺) cells was determined. Both cell lines showed cell killing at similar cetuximab-saporin concentrations with or without 2.5 nM trastuzumab-(Cys-L-SO1861)⁴ (JIMT-1: IC50 = 80 pM; MDA-MB-468: IC50 = 100 pM; Figure 27C, 27D).

All this shows that cells with low or no HER2 receptor expression are not susceptible for the combination of trastuzumab-(Cys-L-SO1861)⁴ + cetuximab-saporin, due to a lack of sufficient HER2 receptor that facilitates the antibody-mediated delivery of sufficient SO1861 (threshold) to ensure endosomal escape of the toxin within the cytoplasm of the cell.

### LITERATURE REFERENCES

- Wilton, E.E. et al. (2018) "sdAb-DB: The Single Domain Antibody Database", ACS Synthetic Biology 7(11): 2480-2484. DOI: 10.1021/acssynbio.8b00407
- Marta Kijanka & Frank-Jan Warnders & Mohamed El Khattabi & Marjolijn Lub-de Hooge & Gooitzen M. van Dam & Vasilis Ntziachristos & Liesbeth de Vries & Sabrina Oliveira & Paul M. P. van Bergen en Henegouwen, "Rapid optical imaging of human breast tumour xenografts using anti-HER2 VHHs site-directly conjugated to IRDye 800CW for image-guided surgery", Eur J Nucl Med Mol Imaging (2013) 40:1718-1729 DOI 10.1007/s00259-013-2471-2
- Karen Mercier, Raimond Heukers and Chiraz Frydman, "Surface Plasmon Resonance imaging (SPRi) - Production of a single domain antibody Q17c directed against recombinant HER2 protein and its binding study by Surface Plasmon Resonance imaging technology", Horiba Application Note Pharmaceuticals SPRi 42, 2019

### SEQ ID NOs

SEQ ID NO: 1: Amino-acid coding DNA sequence of Anti-HER2 sdAb 2Rb17c from camelid 
gaagttcagctgcaggaatctggtggtggtctggttcagccgggtggttctctgcgtctgtcttgcgcggcgtctggtttcatcttctctaacgacgcg
atgacctgggttcgtcaggcgccgggtaaaggtctggaatgggtttcttctatcaactggtctggtacccacaccaactacgcggactctgttaaa
 
ggtcgtttcaccatctctcgtgacaacgcgaaacgtaccctgtacctgcagatgaactctctgaaagacgaagacaccgcgctgtactactgcg
ttaccggttacggtgttaccaaaaccccgaccggtcagggtacccaggttaccgtttcttctcaccaccaccaccaccactctccgtctaccccgc
cgaccccgtctccgtctaccccgccgtgc
SEQ ID NO: 2: Amino-acid sequence of Anti-HER2 sdAb 2Rb17c from camelid 
EVQLQESGGGLVQPGGSLRLSCAASGFIFSNDAMTWVRQAPGKGLEWVSSINWSGTHTNYADSVK
GRFTISRDNAKRTLYLQMNSLKDEDTALYYCVTGYGVTKTPTGQGTQVTVSSHHHHHHSPSTPPTPS
PSTPPC
SEQ ID NO: 3: Amino-acid coding DNA sequence of Anti-HER2 sdAb NB2 from *Camelus dromedarius* 
atggaagttcagctggttgaatctggtggtggtctggttcaggcgggtggttctctgcgtctgtcttgcgcggcgtctggtatcaccttctctatcaaca
ccatgggttggtaccgtcaggcgccgggtaaacagcgtgaactggttgcgctgatctcttctatcggtgacacctactacgcggactctgttaaag
gtcgtttcaccatctctcgtgacaacgcgaaaaacaccgtttacctgcagatgaactctctgaaaccggaagacaccgcggtttactactgcaa
acgtttccgtaccgcggcgcagggtaccgactactggggtcagggtacccaggttaccgtttcttctcaccaccaccaccaccac
SEQ ID NO: 4: Amino-acid sequence of Anti-HER2 sdAb NB2 from *Camelus dromedarius* 
MEVQLVESGGGLVQAGGSLRLSCAASGITFSINTMGWYRQAPGKQRELVALISSIGDTYYADSVKGR
FTISRDNAKNTVYLQMNSLKPEDTAVYYCKRFRTAAQGTDYWGQGTQVTVSSHHHHHH
SEQ ID NO: 5: Amino-acid coding DNA sequence of Anti-HER2 sdAb pcNB2, a synthetic construct 
atggaagttcagctggttgaaaaaggtggtggtcgtgttcaggcgggtggttctctgcgtctgcgttgcgcggcgtctggtatcaccttctctatcaa
caccatgggttggtaccgtcaggcgccgggtaaacagcgtgaactggttgcgctgatctcttctatcggtgacacctactacgcggactctgttaa
aggtcgtttccgtatccgtcgtgacaacgcgaaaaacaccgtttacctgcgtatgcgtcgtctgaaaccggaagacaccgcggtttactactgca
aacgtttccgtaccgcggcgcagggtaccgactactggggtcagggtacccgtgttaccgtttctaaacaccaccaccaccaccac
SEQ ID NO: 6: Amino-acid sequence of Anti-HER2 sdAb pcNB2, a synthetic construct 
MEVQLVEKGGGRVQAGGSLRLRCAASGITFSINTMGWYRQAPGKQRELVALISSIGDTYYADSVKGR
FRIRRDNAKNTVYLRMRRLKPEDTAVYYCKRFRTAAQGTDYWGQGTRVTVSKHHHHHH
SEQ ID NO: 7: amino-acid coding DNA sequence of Anti-HER1 sdAb 7D12 from camelid 
gcggcgcaggttaaactggaagaatctggtggtggttctgttcagaccggtggttctctgcgtctgacctgcgcggcgtctggtcgtacctctcgttc
ttacggtatgggttggttccgtcaggcgccgggtaaagaacgtgaattcgtttctggtatctcttggcgtggtgactctaccggttacgcggactctgt
taaaggtcgtttcaccatctctcgtgacaacgcgaaaaacaccgttgacctgcagatgaactctctgaaaccggaagacaccgcgatctacta
ctgcgcggcggcggcgggttctgcgtggtacggtaccctgtacgaatacgactactggggtcagggtacccaggttaccgtttcttct
SEQ ID NO: 8: amino-acid sequence of Anti-HER1 sdAb 7D12 from camelid 
AAQVKLEESGGGSVQTGGSLRLTCAASGRTSRSYGMGWFRQAPGKEREFVSGISWRGDSTGYADS
VKGRFTISRDNAKNTVDLQMNSLKPEDTAIYYCAAAAGSAWYGTLYEYDYWGQGTQVTVSS
SEQ ID NO: 9: amino-acid coding DNA sequence of Anti-HER1 sdAb 9G8 from camelid 
gaagttcagctggttgaatctggtggtggtctggttcaggcgggtggttctctgcgtctgtcttgcgcggcgtctggtcgtaccttctcttcttacgcgat
gggttggttccgtcaggcgccgggtaaagaacgtgaattcgttgttgcgatcaactggtcttctggttctacctactacgcggactctgttaaaggtc
gtttcaccatctctcgtgacaacgcgaaaaacaccatgtacctgcagatgaactctctgaaaccggaagacaccgcggtttactactgcgcgg
cgggttaccagatcaactctggtaactacaacttcaaagactacgaatacgactactggggtcagggtacccaggttaccgtttcttct
SEQ ID NO: 10: amino-acid sequence of Anti-HER1 sdAb 9G8 from camelid 
EVQLVESGGGLVQAGGSLRLSCAASGRTFSSYAMGWFRQAPGKEREFVVAINWSSGSTYYADSVK
GRFTISRDNAKNTMYLQMNSLKPEDTAVYYCAAGYQINSGNYNFKDYEYDYWGQGTQVTVSS
SEQ ID NO: 11: Amino-acid coding DNA sequence of Anti-VGFR2 sdAb NTV1, a synthetic construct 
atggcgcaggttcagctgctggaatctggtggtggtctggttcagccgggtggttctctgcgtctgtcttgcgcggcgtctggttactctgttatcaac
gacttcatgacctgggttcgtcaggcgccgggtaaaggtctggaatgggtttcttatctctgttgcggacggttctacctactacgcggactctgtt
aaaggtcgtttcaccatctctcgtgacaactctaaaaacaccctgtacctgcagatgaactctctgcgtgcggaagacaccgcggtttactactg
cgcggcgcgtgttggtggtcgtgacctgggttggccgtacgaactggactactggggtcagggtaccctggttaccgtttcttct
SEQ ID NO: 12: Amino-acid sequence of Anti-VGFR2 sdAb NTV1, a synthetic construct 
MAQVQLLESGGGLVQPGGSLRLSCAASGYSVINDFMTWVRQAPGKGLEWVSSISVADGSTYYADSV
KGRFTISRDNSKNTLYLQMNSLRAEDTAVYYCAARVGGRDLGWPYELDYWGQGTLVTVSS
SEQ ID NO: 13: Amino-acid coding DNA sequence of Anti-VGFR2 sdAb NTV2, a synthetic construct 
atggcgcaggttcagctgctggaatctggtggtggtctggttcagccgggtggttctctgcgtctgtcttgcgcggcgtctggtttcaaaatcaccaa
caaaaccatggcgtgggttcgtcaggcgccgggtaaaggtctggaatgggtttcttctatcggttcttcttctggttctacctactacgcggactctgt
taaaggtcgtttcaccatctctcgtgacaactctaaaaacaccctgtacctgcagatgaactctctgcgtgcggaagacaccgcggtttactactg
cgcgcgtcgtaaaggtaaccgtctgggtccggcggcgctgcgttcttggggtcagggtaccctggttaccgtttcttct
SEQ ID NO: 14: Amino-acid sequence of Anti-VGFR2 sdAb NTV2, a synthetic construct 
MAQVQLLESGGGLVQPGGSLRLSCAASGFKITNKTMAWVRQAPGKGLEWVSSIGSSSGSTYYADSV
KGRFTISRDNSKNTLYLQMNSLRAEDTAVYYCARRKGNRLGPAALRSWGQGTLVTVSS
SEQ ID NO: 15: Amino-acid coding DNA sequence of Anti-VGFR2 sdAb NTV3, a synthetic construct 
atggcgcaggttcagctgctggaatctggtggtggtctggttcagccgggtggttctctgcgtctgtcttgcgcggcgtctggtgttcgtgttaactac
aaatctatgtcttgggttcgtcaggcgccgggtaaaggtctggaatgggtttctaccatcacctctcgtaacggttctacctactacgcggactctgtt
aaaggtcgtttcaccatctctcgtgacaactctaaaaacaccctgtacctgcagatgaactctctgcgtgcggaagacaccgcggtttactactg
cgcgaccggtcgtgcgcaccacgcgccggttcgttactggggtcagggtaccctggttaccgtttcttct
SEQ ID NO: 16: Amino-acid sequence of Anti-VGFR2 sdAb NTV3, a synthetic construct 
MAQVQLLESGGGLVQPGGSLRLSCAASGVRVNYKSMSWVRQAPGKGLEWVSTITSRNGSTYYADS
VKGRFTISRDNSKNTLYLQMNSLRAEDTAVYYCATGRAHHAPVRYWGQGTLVTVSS
SEQ ID NO: 17: Amino-acid coding DNA sequence of Anti-VGFR2 sdAb NTV4, a synthetic construct 
atggcgcaggttcagctgctggaatctggtggtggtctggttcagccgggtggttctctgcgtctgtcttgcgcggcgtctggtgttaccatcaccga
cgaagacatgacccgtgttcgtcaggcgccgggtaaaggtctggaatgggtttcttctatcctgaacaccggtggttctacctactacgcggactc
tgttaaaggtcgtttcaccatctctcgtgacaactctaaaaacaccctgtacctgcagatgaactctctgcgtgcggaagacaccgcggtttacta
ctgcgcggcggttcacgaaaaagcggcggacatgaacttctggggtcagggtaccctggttaccgtttcttct
SEQ ID NO: 18: Amino-acid sequence of Anti-VGFR2 sdAb NTV4, a synthetic construct 
AQVQLLESGGGLVQPGGSLRLSCAASGVTITDEDMTRVRQAPGKGLEWVSSILNTGGSTYYADSVK
GRFTISRDNSKNTLYLQMNSLRAEDTAVYYCAAVHEKAADMNFWGQGTLVTVSS
SEQ ID NO: 19: amino-acid coding DNA sequence of Anti-human CD19 sdAb SRB-85 from Bactrian camel 
gaagttcagctgctggaatctggtggtggtctggttcagccgggtggttctctgcgttcttgcgaagcgtctggtttcaacgcgatgacctctatcga
ctcttggaccgacgcggttaaaggttgggttcgtcagccgccgggtaaaggtctggaatgggtttctcgtttcgcgatctctcaggacaacgcga
aaaacaccgtttacctgcagatgaactctctgaaaccggaagacaccgcgatgtactactgcgcgctgtctaaatgctacacccgtgtttacga
ctactggggtcagggtacccaggttaccgtttcttctggt
SEQ ID NO: 20: amino-acid sequence of Anti-human CD19 sdAb SRB-85 from Bactrian camel 
EVQLLESGGGLVQPGGSLRSCEASGFNAMTSIDSWTDAVKGWVRQPPGKGLEWVSRFAISQDNAK
NTVYLQMNSLKPEDTAMYYCALSKCYTRVYDYWGQGTQVTVSSG
SEQ ID NO: 21: amino-acid coding DNA sequence of Anti-human CD19 sdAb SRB-37 from Bactrian camel 
gaagttcagctgcaggaatctggtggtggtctggttcagccgggtggttctctgcgtctgtcttgcgcggcgtctggtttcatctacatggttggtatca
aaaccgaacgtgacggtgttaaaggttgggttcgtcaggcgccgggtaaaggtctggaatggctgtctcgtttcaccatcccgcgtgacaacgc
gaaaaacaccctgtacctgcagatgaacaacctgaaatctgaagacaccgcgctgtactactgcgcgaccgaagaaaacgactggggtca
gggtacccaggttaccgtttcttctggt
SEQ ID NO: 22: amino-acid sequence of Anti-human CD19 sdAb SRB-37 from Bactrian camel 
EVQLQESGGGLVQPGGSLRLSCAASGFIYMVGIKTERDGVKGWVRQAPGKGLEWLSRFTIPRDNAK
NTLYLQMNNLKSEDTALYYCATEENDWGQGTQVTVSSG
SEQ ID NO: 23: Amino-acid coding DNA sequence of Anti-CTLA-4 sdAb NB16 from *Camelus dromedarius* 
caggttcagctgcaggaatctggtggtggttctgttcaggcgggtggttctctgcgtctgtcttgcaccgcgtctggtttcggtgttgacggtaccgac
atgggttggtaccgtcaggcgccgggtaacgaatgcgaactggtttcttctatctcttctatcggtatcggttactactctgaatctgttaaaggtcgttt
caccatctctcgtgacaacgcgaaaaacaccgtttacctgcagatgaactctctgcgtccggacgacaccgcggtttactactgcggtcgtcgtt
ggatcggttaccgttgcggtaactggggtcgtggtacccaggttaccgtttcttct
SEQ ID NO: 24: Amino-acid sequence of Anti-CTLA-4 sdAb NB16 from *Camelus dromedarius* 
QVQLQESGGGSVQAGGSLRLSCTASGFGVDGTDMGWYRQAPGNECELVSSISSIGIGYYSESVKGR
FTISRDNAKNTVYLQMNSLRPDDTAVYYCGRRWIGYRCGNWGRGTQVTVSS
SEQ ID NO: 25: Amino-acid coding DNA sequence of Anti-CTLA-4 sdAb NB36 from *Camelus dromedarius* 
caggttcagctgcaggaatctggtggtggttctgttcaggcgggtggttctctgcgtctgtcttgcaccggttctcgttacacctacaccatgggttgg
ttccgtcaggcgccgggtaaagaacgtgaaggtgttgcgatcaccgcgttcggttctccgttctacgcggactctgttaaaggtcgtttcaccat
ctctcgtgacaacgcgaacaacaccatcttcctgcagatgaactctctgaaaccggaagactctgcgatgtactactgcgcggcgcgtggttctt
ctggtacctcttacaaatggaacgaatacggttcttacaactactggggtcagggtacccaggttaccgtttcttct
SEQ ID NO: 26: Amino-acid sequence of Anti-CTLA-4 sdAb NB36 from *Camelus dromedarius* 
QVQLQESGGGSVQAGGSLRLSCTGSRYTYTMGWFRQAPGKEREGVVAITAFGSPFYADSVKGRFTI
SRDNANNTIFLQMNSLKPEDSAMYYCAARGSSGTSYKWNEYGSYNYWGQGTQVTVSS
SEQ ID NO: 27: Amino-acid coding DNA sequence of Anti-CTLA-4 sdAb NB91 from *Camelus dromedarius* 
caggttcagctgcaggaatctggtggtggttctgttcaggcgggtggttctctgcgtctgtcttgcgcggcgtctaaatacacctcttgcatgggttgg
ttccgtcaggcgccgggtaaagaacgtgaagttgttgcgcacatcgactctggtccgcgtaccctgtacgcggactctgttaaaggtcgtttcacc
atctctaaagacaacgcgaaaaacaccctgtacctggaaatgtctaccctgaaaccggacgacaccgcgatgtactactgcgcggcgggtc
cgatgtactctggttcttgcaactacaactactggggtcagggtacccaggttaccgtttcttct
SEQ ID NO: 28: Amino-acid sequence of Anti-CTLA-4 sdAb NB91 from *Camelus dromedarius* 
QVQLQESGGGSVQAGGSLRLSCAASKYTSCMGWFRQAPGKEREVVAHIDSGPRTLYADSVKGRFTI
SKDNAKNTLYLEMSTLKPDDTAMYYCAAGPMYSGSCNYNYWGQGTQVTVSS
SEQ ID NO: 29: Amino-acid coding DNA sequence of Anti-human PD-L1 sdAb A1 from *Camelus dromedarius* 
Caggttcagctgcaggaatctggtggtggtctggttcagccgggtggttctctgcgtctgtcttgcgcggcgtctggtttcaccctggactactacgc
gatcggttggttccgtcaggcgccgggtaaagaacgtgaaggtgtttcttgcatctcttcttctgacggttctacctactacgcggactctgttaaag
gtcgtttcaccatctctcgtgacaacgcgaaaaacaccgtttacctgcagatgtcttctctgaaaccggaagacaccgcggtttactactgcggta
tctctggttcttgcctgctggaagactacggtatggactactggggtaaaggtacccaggttaccgtttcttct
SEQ ID NO: 30: Amino-acid sequence of Anti-human PD-L1 sdAb A1 from *Camelus dromedarius* 
QVQLQESGGGLVQPGGSLRLSCAASGFTLDYYAIGWFRQAPGKEREGVSCISSSDGSTYYADSVKG
RFTISRDNAKNTVYLQMSSLKPEDTAVYYCGISGSCLLEDYGMDYWGKGTQVTVSS
SEQ ID NO: 31: Amino-acid coding DNA sequence of Anti-human PD-L1 sdAb B1 from *Camelus dromedarius* 
caggttcagctgcaggaatctggtggtggtctggttcacccgggtggttctctgcgtctgtcttgcgcggcgtctggtttctctctggacaactacgcg
atcggttggttccgtcaggcgccgggtaaagaacgtgaaggtgtttcttgcatctcttctggttctgaaggtcgtcgttactacgcggacttcgttaaa
 
ggtcgtttcaccatctctcgtgacaacgcgaaaaacaccgcgttcctgcagatgaactctctgaaaccggaagacaccgcggactactactgc
gcgaccgttggtttctgctcttctcagtacggtatggaattcgttggtgactactggggtcagggtacccaggttaccgtttcttct
SEQ ID NO: 32: Amino-acid sequence of Anti-human PD-L1 sdAb B1 from *Camelus dromedaries* 
QVQLQESGGGLVHPGGSLRLSCAASGFSLDNYAIGWFRQAPGKEREGVSCISSGSEGRRYYADFVK
GRFTISRDNAKNTAFLQMNSLKPEDTADYYCATVGFCSSQYGMEFVGDYWGQGTQVTVSS
SEQ ID NO: 33: Amino-acid sequence of Anti-mouse serum albumin sdAb MSA21 
QVQLQESGGGLVQPGGSLRLSCEASGFTFSRFGMTWVRQAPGKGVEWVSGISSLGDSTLYADSVK
GRFTISRDNAKNTLYLQMNSLKPEDTAVYYCTIGGSLNPGGQGTQVTVSS
SEQ ID NO: 34: Amino-acid sequence of Anti-human serum albumin sdAb Alb-1
SEQ ID NO: 35: Amino-acid sequence of Anti-human serum albumin sdAb Alb23 (Humanized, optimized Alb1) 
EVQLLESGGGLVQPGGSLRLSCAASGFTFRSFGMSWVRQAPGKGPEWVSSISGSGSDTLYADSVK
GRFTISRDNSKTLYLQMNSLRPEDTAVYYCTIGGSLSRSSQGTLVTVSS
SEQ ID NO: 36: Amino-acid sequence of Anti-EGFR V_{HH} 7A5 
EVQLVESGGGLVQAGGSLRLSCAASDRTFSSNNMGWFRQAPGKEREFVAAIGWGGLETHYSDSVK
GRFTISRDNAKNTVYLQMNSLKPEDTARYYCAVSSTRTVIYTLPRMYNYWGQGTQVTVSS
SEQ ID NO: 37: Amino-acid sequence of Anti-EGFR V_{HH} 7D12 
QVKLEESGGGSVQTGGSLRLTCAASGRTSRSYGMGWFRQAPGKEREFVSGISWRGDSTGYADSVK
GRFTISRDNAKNTVDLQMNSLKPEDTAIYYCAAAAGSAWYGTLYEYDYWGQGTQVTVSS
SEQ ID NO: 38: Amino-acid sequence of Anti-EGFR V_{HH} 7C12 
AVQLVESGGGSVQAGGSLRLTCAASGRTSRSYGMGWFRQAPGKEREFVSGISWRGDSTGYADSVK
GRFTISRDNAKNTVDLQMNSLKPEDTAIYYCAAAAGSTWYGTLYEYDYWGQGTQVTVSS
SEQ ID NO: 39: Amino-acid sequence of Anti-insulin-like growth factor 1 receptor V_{HH} 4B11 
EVQLVESGGGLVQPGGSLRLSCAASGSIFTFNAMGWYRQAPGKQRELVAVIISGGSTHYVDSVKGRF
TISRDNAKKMVYLQMNSLKPEDTAVYYCNVKKFGDYWGQGTQVTVSS
SEQ ID NO: 40: Amino-acid sequence of Anti-insulin-like growth factor 1 receptor V_{HH} 3G7 
DVQLVESGGGLVQAGGSLRLSCAASESISTINVMAWYRQAPGKQRELVAEITRSGRTNYVDSVKGRF
TISRDNAKNTMYLQMNSLNLEDTAVYYCRTIDGSWREYWGQGTQVTVSS
SEQ ID NO: 41: Amino-acid sequence of Anti-insulin-like growth factor 1 receptor V_{HH} 2C7 
QVKLEESGGGLVQPGGSLRLSCVASGRTFSNYAIVIGWFRQAPGQEREFVAAINWNSRSTYYADSVK
GRFTISRLNARNTVYLQMNRLKPEDTAVYDCAASHDSDYGGTNANLYDYWGQGTQVTVSS
SEQ ID NO: 42: Amino-acid sequence of Anti-insulin-like growth factor 1 receptor V_{HH} 1 C7 
QVKLEESGGGLVQAGGSLRLSCVASGRTFSRTANAWFRQAPGKEREFVATITWNSGTTRYADSVKG
RFFISKDSAKNTIYLEMNSLEPEDTAVYYCAATAAAVITPTRGYYNYWGQGTQVTVSS
SEQ ID NO: 43: Amino-acid sequence of Anti-CEACAM V_{HH} NbCEA5 
EVQLVESGGGSVQAGGSLRLSCAASGDTYGSYWMGWFRQAPGKEREGVAAINRGGGYTVYADSV
KGRFTISRDTAKNTVYLQMNSLRPDDTADYYCAASGVLGGLHEDWFNYWGQGTLVTVSS
SEQ ID NO: 44: Amino-acid sequence of Anti-CEACAM V_{HH} CEA5 
EVQLVESGGGSVQAGGSLRLSCAASGDTYGSYWMGWFRQAPGQEREAVAAINRGGGYTVYADSV
KGRFTISRDNAKNTLYLQMNSLRPDDTADYYCAASGVLGGLHEDWFNYWGQGTLVTVSS
SEQ ID NO: 45: Amino-acid sequence of Anti-CD123 V_{HH} 57A07 
EVQLVESGGGLVQAGGSLRLSCAASGSIFSGNVMGWYRRQAPGKEREWVAAIASGGSIYYRDSVKG
RFTISRDNAKNTVYLQMNSLKPEDTAVYYCNSHPPTLPYWGLGTQVTVSS
SEQ ID NO: 46: Amino-acid sequence of Anti-CD123 V_{HH} 57B04 
EVQLVESGGGLVQPGGSLRLSCAASGINFRFNSMGWWRRAPGKEREWVAAITSGDITNYRDSVRG
RFTISRDNVKNTVYLQMNTLKLEDTAVYYCNTFPPIADYWGLGTQVTVSS
SEQ ID NO: 47: Amino-acid sequence of Anti-CD123 V_{HH} 51 D09 
EVQLVESGGGLVQPGGSLRLSCAASGSIFSGNTMGWYRQAPGKQRELVAAISSGGSTDYADSVKGR
FTISRDNSKNTVYLQMNSLRPEDTAVYYCNAAILLYRLYGYEEGDYWGLGTLVTVSS
SEQ ID NO: 48: Amino-acid sequence of Anti-CD123 V_{HH} 55C05 
EVQLVESGGGLVPAGDSLRLSCVASGRSLNTYTMGWFRQAPGKECEEVAAINWNGVYRDYADSAK
GRETASRDNAMNTVFLQMNSLKPEDTAVYFCATATQGWDRHTEPSDFGSWGLGTQVTVSS
SEQ ID NO: 49: Amino-acid sequence of Anti-CD123 V_{HH} 50F07 
EVQLVESGGGLVQPGGSLRLSCTGSGSTFSINAMGWYRQAPGKQRELVAAITSGGRTNYADSVKGR
FTISRDNSKNTVYLQMNSLRPEDTAVYYCNARISAGTAFWLWSDYEYWGLGTLVTVSS
SEQ ID NO: 50: Amino-acid sequence of Anti-CD123 V_{HH} 55F03 
EVQLVESGGGLVQAGGPLRLSCAASGRTFSSYVMGWFRQAPGKEREFVAAIYWSNGKTQYTDSVK
GRFTISGDNAKNTVYLQMNSLNPEDTAVYYCVADKDETGFRTLPIAYDYWGLGTQVTVSS
SEQ ID NO: 51: Amino-acid sequence of Anti-CD123 V_{HH} 55A01 
EVQLVESGGGSVQAGGSLRLSCTTSGRALNMYVMGWFRQAPGNEREFVAATSSSGGSTSYPDSVK
GRFTISRDNAKNTVYLQMNSLKPEDTAAYRCAASPYVSTPTMNILEEYRYWGLGTQVTVSS
SEQ ID NO: 52: Amino-acid sequence of Anti-c-MET V_{HH} 04E09 
EVQLVESGGGLVQPGGSLRLSCAASGFILDYYAIGWFRQAPGKEREGVLCIDASDDITYYADSVKGRF
TISRDNAKNTVYLQMNSLKPEDTGVYYCATPIGLSSSCLLEYDYDYWGQGTLVTVSS
SEQ ID NO: 53: Amino-acid sequence of Anti-c-MET V_{HH} 06B08 
EVQLVESGGGLVQAGGSLRLSCAASGRTISRYTMGWFRQAPGKEREFVAAISWSGDNTNYADSVKG
RFTISRPNTKNTMYLQMNSLKPEDTAVYYCAADYRSGSYYQASEWTRPSGYDYWGQGTLVTVSS
SEQ ID NO: 54: Amino-acid sequence of Anti-c-MET V_{HH} 06C12 
EVQLVESGGGLVQPGGSLRLSCAASGFSLDYFAIGWFRQAPGKEREEISCISNSDGSTYYANSVKGR
FTISIDNAKNTVYLQMTSLKPEDTAVYYCATPVGLGPFCKTTNDYDYSGQGTLVTVSS
SEQ ID NO: 55: Amino-acid sequence of Anti-c-MET V_{HH} 06F10 
EVQLVESGGGLVQPGGSLRLSCAASGFTLDYYAINWFRQAPGKEREGVSCISGGDGSTYYADSVKG
RFTISRDNAKNTVYLQMNSLKPEDTAVYYCATALGLSSSCHGDGYDYWGQGTLVTVSS
SEQ ID NO: 56: Amino-acid sequence of Anti-Her3 V_{HH} 21 F6 
EVQLVESGGGLVQAGGSLRLSCAASGRTYYLNAMGWFRQGPGKDREFVAAIDWSDGNKDYADSVK
GRFTISRDNAKNTVYLQMNSLKPEDTAVYYCAADTPPWGPMIYIESYDSWGQGTLVTVSS
SEQ ID NO: 57: Amino-acid sequence of Anti-Her3 V_{HH} 4C7 
EVQLVESGGGLVQAGGSLRLSCAASGFTFSSYPMSWVRQAPGKGPAWVSTVSPGGITTSYADSVKG
RFTISRDNAKNTLYLQMNSLKPEDTAVYYCLRDLNNRGQGTLVTVSS
SEQ ID NO: 58: Amino-acid sequence of Anti-Her3 V_{HH} 17B5 
EVQLVESGGGLVQPGGSLRLSCAASGSIGGLNAMAWYRQAPGKERELVAGIFGVGSTRYADSVKGR
FTISRDIAKNTVFLQMNSLNSEDTAVYYCRMSSVTRGSSDYWGQGTQVTVSS
SEQ ID NO: 59: Amino-acid sequence of Anti-Her3 V_{HH} 18G11 
EVQLVESGGGLVQPGGSLRLSCAASGTLFKINAMGWYRQAPGKRRELVALITSSDTTDYAESVEGRF
TISRDNTWNAVYLQMNSLKPEDTAVYYCHSDHYSMGVPEKRVIMYGQGTQVTVSS
SEQ ID NO: 60: Amino-acid sequence of Anti-Her3 V_{HH} 34C7 
EVQLVESGGGLVQPGGSLGLSCVASGSIFRINAMAWYRQAPGKQRELVAEITAGGSTNYADSVKGRF
TISVDNAWNTLYLQMNSLKVEDTAVYYCNLDHYTTWDRRSAYWGQGTQVTVSS
SEQ ID NO: 61: Amino-acid sequence of Anti-Her2 V_{HH} 47D5 
KVQLVESGGGLVQPGGSLRLSCAASGSIFGFNDMAWYRQAPGKQRELVALISRVGVTSSADSVKGR
FTISRVNAKDTVYLQMNSLKPEDTAVYYCYMDQRLDGSTLAYWGQGTQVTVSS
SEQ ID NO: 62: Amino-acid sequence of Anti-Her2 V_{HH} 2D3 
EVQLVESGGSLVQPGGSLRLSCAASGFTFDDYAMSWVRQVPGKGLEWVSSINWSGTHTDYADSVK
GRFTISRNNANNTLYLQMNSLKSEDTAVYYCAKNWRDAGTTWFEKSGSAGQGTQVTVSS
SEQ ID NO: 63: Amino-acid sequence of Anti-Her2 V_{HH} 5F7 
EVQLVESGGGLVQPGGSLRLSCAASGFTFSINTMGWYRQAPGKQRELVALISSIGDTYYADSVKGRF
TISRDNAKNTVYLQMNSLKPEDTAVYYCKRFRTAAQGTDYWGQGTQVTVSS
SEQ ID NO: 64: Amino-acid sequence of Anti-Her2 V_{HH} 13D11 
EVQLVESGGGLVHPGGSLRLSCVGSGFSLDDYGMTWVRRAPGKGLEWVSSINWSGTHTDYADSVK
GRFTISRDNAKNTLFLQMNSLNPEDTAVYYCGQGWKIVPTNPRGHGTQVTVSS
SEQ ID NO: 65: Amino-acid sequence of Anti-Her2 V_{HH} 2B4 
EVQLVESGGGLVQPGGSLRLSCVGSGFSLDDYAMTWVRQAPGKGLEWVSSINWSGTHTDYADSVK
GRFTISRDNAKNTLFLQMNSLSPEDTAVYYCNQGWKIRPTIPMGHGTQVTVSS
SEQ ID NO: 66: Amino-acid sequence of Anti-Her2 V_{HH} 2G2 
EVQLVESGGGLVQPGGSLRLSCVASGFSLDDYGMTWVRQAPGKGLEWVSSINWSGTHTDYTDPVK
GRFTISRDNAKNTLFLQMNNLTPEDTAVYYCNRGWKIVPTDLGGHGTQVTVSS
SEQ ID NO: 67: Amino-acid sequence of Anti-Her2 V_{HH} 13G11 
EVQLVESGGGLVQAGGSLRLSCAASGRTFISNYAMGWFRQAPGKEREFVATINWSGHSDYADSVK
GRFTISRDNAKNTVYLQMNNLKSEDTAVYYCAPGWGTAPLSTSVYWGQGTQVTVSS
SEQ ID NO: 68: Amino-acid sequence of Anti-Her2 V_{HH} 12E33 
EVQLVESGGGMVQAGGSLRLSCAASGLTLSNYGMGWFRQAPGKEREFVSSINWSGTHTYDADFVK
GRFIISRDNAKNTVYLQINSLKPEDTAVYYCAAGGWGTGRYNYWGQGTQVTVSS
SEQ ID NO: 69: Amino-acid sequence of Anti-Her2 V_{HH} 13F21 
EVQLVESGGGLVQSGGSLRLSCVASGTIVSINATSWYRQAPGNQRELVATIIGDGRTHYADSVKDRFT
ISRDAAANLVYLQMNSLKPSDTAIYSCNANGIESYGWGNRHFNYWTVGTQVTVSS
SEQ ID NO: 70: Amino-acid sequence of Anti-Her2 V_{HH} 11A101 
EVQLVESGGGLVQAGGSLRLSCAASGRTFNAMGWFRQAPGKEREFVAAISRSPGVTYYADSVKGRF
TTSRDNAKNTVYLQMNDLKPEDTAVYYCAADFYLATLAHEYDYWGQGTQVTVSS
SEQ ID NO: 71: Amino-acid sequence of Anti-Her2 V_{HH} 11A22 
EVQLVESGGGLVQAGGSLRLSCAASGRTFSSYAMAWFRQAPGTEREFIAGIRWSDGSTYYADSVKG
RFTISRDNAKNTVYLQMNSLKPEDTAVYYCAADFYVSTLAHEYDYWGQGTQVTVSS
SEQ ID NO: 72: Amino-acid sequence of Anti-Her2 V_{HH} 12D44

KVQLVESGGGLVQAGGSLRLSCAASGRTFSSYAMAWFRQAPGTEREFIAGIRWSDGSTYYADSVKG
RFTISRANAKNTVYLQMNGLKPEDTAVYYCAADFYVSTLAHEYDYWGQGTQVTVSS

## Claims

1. **Pharmaceutical** combination comprising:
- a first conjugate comprising at least one oligonucleotide and a single-domain antibody (sdAb) for binding to a first cell-surface molecule, which are covalently linked to each other, directly or via a linker;
- any one selected from:
o a saponin comprising an aglycone core structure selected from quillaic acid and gypsogenin and further comprising a first saccharide chain bound to the C₃ atom of the aglycone core structure and a second saccharide chain bound to the C₂₈ atom of the aglycone core structure;
and/or
o a derivative of the saponin, wherein the aldehyde group comprised by the aglycone core structure of the saponin has been transformed into a hydrazone bond through reaction with the N-ε-maleimidocaproic acid hydrazide (EMCH);
and/or
o a second conjugate comprising a binding molecule for binding to a second cell-surface molecule and the saponin and/or the derivative thereof, which binding molecule and the saponin and/or the derivative thereof are covalently linked to each other via a cleavable linker;
wherein the binding molecule is an immunoglobulin or at least one binding fragment or binding domain of said immunoglobulin;
wherein the second cell-surface molecule is the same as or different from the first cell-surface molecule; wherein, when the first and second cell-surface molecules are different, the first and second cell-surface molecules are present at the same target cell; wherein the cleavable linker is covalently bound to the aldehyde group in position C₂₃ of the aglycone core structure of the saponin and is a linker N-ε-maleimidocaproic acid hydrazide (EMCH) and covalently binds the saponin to a sulfhydryl group in the binding molecule;
wherein the aldehyde group comprised by the aglycone core structure of the saponin has been transformed into a hydrazone bond through reaction with the N-ε-maleimidocaproic acid hydrazide (EMCH);
and optionally further comprising
- a pharmaceutically acceptable excipient and/or pharmaceutically acceptable diluent.

2. Pharmaceutical combination of claim 1 in the form of a single pharmaceutical composition comprising the first conjugate and the saponin and/or the derivative thereof and/or the second conjugate and optionally further comprising a pharmaceutically acceptable excipient and/or pharmaceutically acceptable diluent, or
wherein the pharmaceutical combination is provided in the form of at least two, preferably two, pharmaceutical compositions comprising:
- a first pharmaceutical composition comprising the first conjugate and optionally comprising a pharmaceutically acceptable excipient and/or pharmaceutically acceptable diluent; and
- a second pharmaceutical composition comprising the saponin and/or the derivative thereof and/or the second conjugate, and optionally comprising a pharmaceutically acceptable excipient and/or pharmaceutically acceptable diluent.

3. Pharmaceutical combination of any one of the claims 1-3, wherein the sdAb is any one or more of: a V_{H} domain derived from a heavy chain of an antibody, preferably of immunoglobulin G origin, preferably of human origin; a V_{L} domain derived from a light chain of an antibody, preferably of immunoglobulin G origin, preferably of human origin; a V_{HH} domain such as derived from a heavy-chain only antibody (HCAb) such as from *Camelidae* origin or *Ig-NAR* origin such as a variable heavy chain new antigen receptor (V_{NAR}) domain, preferably the HCAb is from *Camelidae* origin; and preferably the sdAb is a V_{HH} domain derived from an HCAb from *Camelidae* origin (camelid V_{H}) such as derived from an HCAb from camel, lama, alpaca, dromedary, vicuna, guanaco and Bactrian camel.

4. Pharmaceutical combination of any one of the claims 1-3, wherein the sdAb is a single sdAb or are at least two, preferably two sdAbs, wherein the sdAb(s) can bind to the first cell-surface molecule such as HIVgp41, more preferably a receptor selected from: CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, prostate specific membrane antigen (PSMA), CanAg, integrin-alphaV, CA6, CD33, mesothelin, Cripto, CD3, CD30, CD239, CD70, CD123, CD352, DLL3, CD25, ephrinA4, MUC-1, Trop2, CEACAM5, CEACAM6, HER3, CD74, PTK7, Notch3, FGF2, C4.4A, FLT3, CD38, FGFR3, CD7, PD-L1, CTLA-4, CD52, PDGFRA, VEGFR1, VEGFR2, c-Met (HGFR), EGFR1, RANKL, ADAMTS5, CD16, CXCR7 (ACKR3), glucocorticoid-induced TNFR-related protein (GITR), most preferably selected from: HER2, c-Met, VEGFR2, CXCR7, CD71 and EGFR1,
preferably wherein the sdAb(s) is/are selected from: an anti-CD71 sdAb, an anti-HER2 sdAb, an anti-CD20 sdAb, an anti-CA125 sdAb, an anti-EpCAM (17-1A) sdAb, an anti-EGFR sdAb, an anti-CD30 sdAb, an anti-CD33 sdAb, an anti-vascular integrin alpha-v beta-3 sdAb, an anti-CD52 sdAb, an anti-CD22 sdAb, an anti-CEA sdAb, an anti-CD44v6 sdAb, an anti-FAP sdAb, an anti-CD19 sdAb, an anti-CanAg sdAb, an anti-CD56 sdAb, an anti-CD38 sdAb, an anti-CA6 sdAb, an anti-IGF-1R sdAb, an anti-integrin sdAb, an anti-syndecan-1 sdAb, an anti-CD79b, an anti-c-Met sdAb, an anti-EGFR1 sdAb, an anti-VEGFR2 sdAb, an anti-CXCR7 sdAb and an anti-HIVgp41, wherein the sdAb(s) is/are preferably V_{HH}(s), more preferably camelid V_{H},
more preferably wherein the first conjugate comprises an sdAb that can bind to HER2, CD71, HIVgp41 and/or EGFR, wherein the sdAb is preferably a V_{HH}, more preferably a camelid V_{H},
even more preferably wherein the first conjugate comprises an sdAb for binding to HER2 selected from: sdAb produced by clone 11A4, clone 18C3, clone 22G12, clone Q17 or clone Q17-C-tag; or an sdAb for binding to EGFR and produced by clone anti-EGFR Q86-C-tag; or an sdAb for binding to CD71 and produced by clone anti-CD71 Q52-C-tag; or an sdAb for binding to HIVgp41 and produced by clone anti-HIVgp41 Q8-C-tag; or an sdAb encoded by a cDNA of any one of the SEQ ID NO: 1, 3, 5, 7, 9, 11, 13, 15, 17, 19, 21, 23, 25, 27, 29 and 31; or any one of the sdAbs with an amino-acid sequence of SEQ ID NO: 2, 4, 6, 8, 10, 12, 14, 16, 18, 20, 22, 24, 26, 28, 30, 32, 36-72, wherein optionally the first conjugate further comprises a covalently bound sdAb for binding to albumin, such as any one or more of the sdAbs with an amino-acid sequence of SEQ ID NO: 33, 34 and 35, preferably the sdAb is a V_{HH}, more preferably a camelid V_{H}.

5. Pharmaceutical combination of any one of the claims 1-4, wherein the oligonucleotide is selected from any one or more of a(n): short interfering RNA (siRNA), short hairpin RNA (shRNA), anti-hairpin-shaped microRNA (miRNA), single-stranded RNA, aptamer RNA, double-stranded RNA (dsRNA), anti-microRNA (anti-miRNA, anti-miR), antisense oligonucleotide (ASO), mRNA, DNA, antisense DNA, locked nucleic acid (LNA), bridged nucleic acid (BNA), 2'-O,4'-aminoethylene bridged nucleic Acid (BNA^{NC}), BNA-based siRNA, and BNA-based antisense oligonucleotide (BNA-AON), and/or is selected from any one of an anti-miRNA, a BNA-AON or an siRNA, such as BNA-based siRNA, preferably selected from chemically modified siRNA, metabolically stable siRNA and chemically modified, metabolically stable siRNA, and/or
wherein the oligonucleotide is capable of silencing a gene, when present in a cell comprising such gene, wherein the gene is any one of genes: apolipoprotein B (apoB), transthyretin (TTR), proprotein convertase subtilisin/kexin type 9 (PCSK9), delta-aminolevulinate synthase 1 (ALAS1), antithrombin 3 (AT3), glycolate oxidase (GO), complement component C5 (CC5), X gene of hepatitis B virus (HBV), S gene of HBV, alpha-1 antitrypsin (AAT) and lactate dehydrogenase (LDH), and/or is capable of targeting an aberrant miRNA when present in a cell comprising such miRNA, and/or
wherein the oligonucleotide is capable of targeting an mRNA, when present in a cell comprising such mRNA, wherein the mRNA is involved in expression of any one of proteins: apoB, TTR, PCSK9, ALAS1, AT3, GO, CC5, expression product of X gene of HBV, expression product of S gene of HBV, AAT and LDH, or is capable of antagonizing or restoring an miRNA function such as inhibiting an oncogenic miRNA (onco-miR) or suppression of expression of an onco-miR, when present in a cell comprising such an miRNA.

6. Pharmaceutical combination of any one of the claims 1-5, wherein the saponin comprises an aglycone core structure being quillaic acid.

7. Pharmaceutical combination of any one of the claims 1-6, wherein
• the saponin comprises the first saccharide chain selected from:
GlcA-,
Glc-,
Gal-,
Rha-(1→2)-Ara-,
Gal-(1→2)-[Xyl-(1→3)]-GlcA-,
Glc-(1→2)-[Glc-(1→4)]-GlcA-,
Glc-(1→2)-Ara-(1→3)-[Gal-(1→2)]-GlcA-,
Xyl-(1→2)-Ara-(1→3)-[Gal-(1→2)]-GlcA-,
Glc-(1→3)-Gal-(1→2)-[Xyl-(1→3)]-Glc-(1→4)-Gal-,
Rha-(1→2)-Gal-(1→3)-[Glc-(1→2)]-GlcA-,
Ara-(1→4)-Rha-(1→2)-Glc-(1→2)-Rha-(1→2)-GlcA-,
Ara-(1→4)-Fuc-(1→2)-Glc-(1→2)-Rha-(1→2)-GlcA-,
Ara-(1→4)-Rha-(1→2)-Gal-(1→2)-Rha-(1→2)-GlcA-,
Ara-(1→4)-Fuc-(1→2)-Gal-(1→2)-Rha-(1→2)-GlcA-,
Ara-(1→4)-Rha-(1→2)-Glc-(1→2)-Fuc-(1→2)-GlcA-,
Ara-(1→4)-Fuc-(1→2)-Glc-(1→2)-Fuc-(1→2)-GlcA-,
Ara-(1→4)-Rha-(1→2)-Gal-(1→2)-Fuc-(1→2)-GlcA-,
Ara-(1→4)-Fuc-(1→2)-Gal-(1→2)-Fuc-(1→2)-GlcA-,
Xyl-(1→4)-Rha-(1→2)-Glc-(1→2)-Rha-(1→2)-GlcA-,
Xyl-(1→4)-Fuc-(1→2)-Glc-(1→2)-Rha-(1→2)-GlcA-,
Xyl-(1→4)-Rha-(1→2)-Gal-(1→2)-Rha-(1→2)-GlcA-,
Xyl-(1→4)-Fuc-(1→2)-Gal-(1→2)-Rha-(1→2)-GlcA-,
Xyl-(1→4)-Rha-(1→2)-Glc-(1→2)-Fuc-(1→2)-GlcA-,
Xyl-(1→4)-Fuc-(1→2)-Glc-(1→2)-Fuc-(1→2)-GlcA-,
Xyl-(1→4)-Rha-(1→2)-Gal-(1→2)-Fuc-(1→2)-GlcA-,
Xyl-(1→4)-Fuc-(1→2)-Gal-(1→2)-Fuc-(1→2)-GlcA-, and
and/or
• the saponin comprises the second saccharide chain selected from:
Glc-,
Gal-,
Rha-(1→2)-[Xyl-(1→4)]-Rha-,
Rha-(1→2)-[Ara-(1→3)-Xyl-(1→4)]-Rha-,
Ara-,
Xyl-,
Xyl-(1→4)-Rha-(1→2)-[R1-(→4)]-Fuc- wherein R1 is 4E-Methoxycinnamic acid,
Xyl-(1→4)-Rha-(1→2)-[R2-(→4)]-Fuc- wherein R2 is 4Z-Methoxycinnamic acid,
Xyl-(1→4)-[Gal-(1→3)]-Rha-(1→2)-4-OAc-Fuc-,
Xyl-(1→4)-[Glc-(1→3)]-Rha-(1→2)-3,4-di-OAc-Fuc-,
Xyl-(1→4)-[Glc-(1→3)]-Rha-(1→2)-[R3-(→4)]-3-OAc-Fuc- wherein R3 is 4E-Methoxycinnamic acid,
Glc-(1→3)-Xyl-(1→4)-[Glc-(1→3)]-Rha-(1→2)-4-OAc-Fuc-,
Glc-(1→3)-Xyl-(1→4)-Rha-(1→2)-4-OAc-Fuc-,
(Ara- or Xyl-)(1→3)-(Ara- or Xyl-)(1→4)-(Rha- or Fuc-)(1→2)-[4-OAc-(Rha- or Fuc-)(1→4)]-(Rha- or Fuc-),
Xyl-(1→3)-Xyl-(1→4)-Rha-(1→2)-[Qui-(1→4)]-Fuc-,
Api-(1→3)-Xyl-(1→4)-[Glc-(1→3)]-Rha-(1→2)-Fuc-,
Xyl-(1→4)-[Gal-(1→3)]-Rha-(1→2)-Fuc-,
Xyl-(1→4)-[Glc-(1→3)]-Rha-(1→2)-Fuc-,
Ara/Xyl-(1→4)-Rha/Fuc-(1→4)-[Glc/Gal-(1→2)]-Fuc-,
Api-(1→3)-Xyl-(1→4)-[Glc-(1→3)]-Rha-(1→2)-[R4-(→4)]-Fuc- wherein R4 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R5-(→4)]-Fuc- wherein R5 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[Rha-(1→3)]-4-OAc-Fuc-,
Api-(1→3)-Xyl-(1→4)-[Glc-(1→3)]-Rha-(1→2)-[Rha-(1→3)]-4-OAc-Fuc-,
6-OAc-Glc-(1→3)-Xyl-(1→4)-Rha-(1→2)-[3-OAc-Rha-(1→3)]-Fuc-,
Glc-(1→3)-Xyl-(1→4)-Rha-(1→2)-[3-OAc--Rha-(1→3)]-Fuc-,
Xyl-(1→3)-Xyl-(1→4)-Rha-(1→2)-[Qui-(1→4)]-Fuc-,
Glc-(1→3)-[Xyl-(1→4)]-Rha-(1→2)-[Qui-(1→4)]-Fuc-,
Glc-(1→3)-Xyl-(1→4)-Rha-(1→2)-[Xyl-(1→3)-4-OAc-Qui-(1→4)]-Fuc-,
Xyl-(1→3)-Xyl-(1→4)-Rha-(1→2)-[3,4-di-OAc-Qui-(1→4)]-Fuc-,
Glc-(1→3)-[Xyl-(1→4)]-Rha-(1→2)-Fuc-,
6-OAc-Glc-(1→3)-[Xyl-(1→4)]-Rha-(1→2)-Fuc-,
Glc-(1→3)-[Xyl-(1→3)-Xyl-(1→4)]-Rha-(1→2)-Fuc-,
Xyl-(1→3)-Xyl-(1→4)-Rha-(1→2)-[Xyl-(1→3)-4-OAc-Qui-(1→4)]-Fuc-,
Api/Xyl-(1→3)-Xyl-(1→4)-[Glc-(1→3)]-Rha-(1→2)-[Rha-(1→3)]-4OAc-Fuc-,
Api-(1→3)-Xyl-(1→4)-[Glc-(1→3)]-Rha-(1→2)-[Rha-(1→3)]-4OAc-Fuc-,
Api/Xyl-(1→3)-Xyl-(1→4)-[Glc-(1→3)]-Rha-(1→2)-[R6-(→4)]-Fuc- wherein R6 is 5-O-[5-O-Rha-(1→2)-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R7-(→4)]-Fuc- wherein R7 is 5-O-[5-O-Ara/Api-3,5-dihydroxy-6-methyl-octanoyl]-3,5-dihydroxy-6-methyl-octanoic acid),
Api/Xyl-(1→3)-Xyl-(1→4)-[Glc-(1→3)]-Rha-(1→2)-[R8-(→4)]-Fuc- wherein R8 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R9-(→4)]-Fuc- wherein R9 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R10-(→4)]-Fuc- wherein R10 is 5-O-[5-O-Ara/Api-3,5-dihydroxy-6-methyl-octanoyl]-3,5-dihydroxy-6-methyl-octanoic acid),
Api-(1→3)-Xyl-(1→4)-Rha-(1→2)-[R11-(→3)]-Fuc- wherein R11 is 5-O-[5-O-Ara/Api-3,5-dihydroxy-6-methyl-octanoyl]-3,5-dihydroxy-6-methyl-octanoic acid),
Xyl-(1→3)-Xyl-(1→4)-Rha-(1→2)-[R12-(→3)]-Fuc- wherein R12 is 5-O-[5-O-Ara/Api-3,5-dihydroxy-6-methyl-octanoyl]-3,5-dihydroxy-6-methyl-octanoic acid) and
Glc-(1→3)-[Glc-(1→6)]-Gal-.

8. Pharmaceutical combination of any one of the claims 1-7, wherein the maleimide group of the EMCH is derivatised by formation of a thioether bond with mercaptoethanol.

9. Pharmaceutical combination of any one of the claims 1-8, wherein the saponin is selected from: *Quillaja* bark saponin, dipsacoside B, saikosaponin A, saikosaponin D, macranthoidin A, esculentoside A, phytolaccagenin, aescinate, AS6.2, NP-005236, AMA-1, AMR, alpha-Hederin, NP-012672, NP-017777, NP-017778, NP-017774, NP-018110, NP-017772, NP-018109, NP-017888, NP-017889, NP-018108, SA1641, AE X55, NP-017674, NP-017810, AG1, NP-003881, NP-017676, NP-017677, NP-017706, NP-017705, NP-017773, NP-017775, SA1657, AG2, SO1861, GE1741, SO1542, SO1584, SO1658, SO1674, SO1832, SO1904, SO1862, QS-7, QS1861, QS-7 api, QS1862, QS-17, QS-18, QS-21 A-apio, QS-21 A-xylo, QS-21 B-apio, QS-21 B-xylo, beta-Aescin, Aescin la, Teaseed saponin I, Teaseedsaponin J, Assamsaponin F, Digitonin, Primula acid 1 and AS64R, or a combination thereof, preferably the saponin is any one or more of QS-21, SO1861, SA1641 and GE1741, preferably wherein the saponin is selected from: SO1861, SA1657, GE1741, SA1641, QS-21, QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, Quillajasaponin, Saponinum album, QS-18, Quil-A, Gyp1, gypsoside A, AG1, AG2, SO1542, SO1584, SO1658, SO1674, SO1832, SO1862, SO1904, , or a stereoisomer thereof.

10. Pharmaceutical combination of any one of the claims 1-9, wherein the immunoglobulin is any one or more of an antibody such as a monoclonal antibody, preferably a human antibody, an IgG, a molecule comprising or consisting of a sdAb, at least one V_{HH} domain or at least one V_{H} domain, a variable heavy chain new antigen receptor (V_{NAR}) domain, a Fab, an scFv, an Fv, a dAb, an F(ab)2, a Fcab fragment.

11. Pharmaceutical combination of any one of the claims 1-10, wherein the binding molecule binds to the second cell-surface molecule being a tumor-cell surface receptor such as a tumor-cell specific receptor, more preferably a cell-surface receptor selected from: CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, prostate specific membrane antigen (PSMA), CanAg, integrin-alphaV, CA6, CD33, mesothelin, Cripto, CD3, CD30, CD239, CD70, CD123, CD352, DLL3, CD25, ephrinA4, MUC-1, Trop2, CEACAM5, CEACAM6, HER3, CD74, PTK7, Notch3, FGF2, C4.4A, FLT3, CD38, FGFR3, CD7, PD-L1, CTLA-4, CD52, PDGFRA, VEGFR1, VEGFR2, c-Met (HGFR), EGFR1, RANKL, ADAMTS5, CD16, CXCR7 (ACKR3), glucocorticoid-induced TNFR-related protein (GITR), most preferably selected from: HER2, c-Met, VEGFR2, CXCR7, CD71, EGFR and EGFR1.

12. Pharmaceutical combination of any one of the claims 1-11, wherein the binding molecule is an immunoglobulin selected from an anti-CD71 antibody such as IgG type OKT-9, an anti-HER2 antibody such as trastuzumab (Herceptin), pertuzumab, an anti-CD20 antibody such as rituximab, ofatumumab, tositumomab, obinutuzumab ibritumomab, an anti-CA125 antibody such as oregovomab, an anti-EpCAM (17-1A) antibody such as edrecolomab, an anti-EGFR antibody such as cetuximab, matuzumab, panitumumab, nimotuzumab, an anti-CD30 antibody such as brentuximab, an anti-CD33 antibody such as gemtuzumab, huMy9-6, an anti-vascular integrin alpha-v beta-3 antibody such as etaracizumab, an anti-CD52 antibody such as alemtuzumab, an anti-CD22 antibody such as epratuzumab, pinatuzumab, binding fragment (Fv) of anti-CD22 antibody moxetumomab, humanized monoclonal antibody inotuzumab, an anti-CEA antibody such as labetuzumab, an anti-CD44v6 antibody such as bivatuzumab, an anti-FAP antibody such as sibrotuzumab, an anti-CD19 antibody such as huB4, an anti-CanAg antibody such as huC242, an anti-CD56 antibody such as huN901, an anti-CD38 antibody such as daratumumab, OKT-10 anti-CD38 monoclonal antibody, an anti-CA6 antibody such as DS6, an anti-IGF-1R antibody such as cixutumumab, 3B7, an anti-integrin antibody such as CNTO 95, an anti-syndecan-1 antibody such as B-B4, an anti-CD79b such as polatuzumab, an anti-HIVgp41 antibody, preferably any one of an anti-HIVgp41 antibody, an anti-CD71 antibody, an anti-HER2 antibody and an anti-EGFR antibody, more preferably any one of: trastuzumab, pertuzumab, cetuximab, matuzumab, an anti-CD71 antibody, OKT-9, most preferably trastuzumab, cetuximab, the anti-CD71 antibody OKT-9.

13. Pharmaceutical combination of any one of the claims 1-12, wherein the linker N-ε-maleimidocaproic acid hydrazide (EMCH) that is covalently bound to an aldehyde group in position C₂₃ of the aglycone core structure of the saponin is covalently bound to the sulfhydryl group of a cysteine in the binding molecule.

14. Pharmaceutical combination of any one of the claims 1-13, wherein the second conjugate comprises more than one covalently bound saponin.

15. *In vitro* or ex *vivo* method for transferring the first conjugate of any one of the claims 1-14 from outside a cell to inside said cell, preferably to the cytosol of said cell, comprising the steps of:
a) providing a cell which expresses the first cell-surface molecule according to any one of the claims 1-14 on its surface, and optionally expresses the second cell-surface molecule according to any one of the claims 1-14 on its surface, said cell preferably selected from a liver cell, an aberrant cell such as a virally infected cell, an auto-immune cell and a tumor cell;
b) providing the first conjugate of any one of the claims 1-14 for transferring into the cell provided in step a);
c) providing the saponin or the saponin derivative and/or the second conjugate of any one of the claims 1-14;
d) contacting the cell of step a) *in vitro* or ex *vivo* with the first conjugate of step b) and any one or more of the saponin, the saponin derivative and the conjugate of step c),
therewith establishing the transfer of the first conjugate from outside the cell into said cell, preferably into the cytosol of said cell.
